# EUROPEAN PATENT SPECIFICATION

(11) **EP 2 671 082 B1**
(45) Date of publication and mention of the grant of the patent: **15.01.2020**
(21) Application number: 12703940.2
(22) Date of filing: 02.02.2012
(51) Int. Cl.: G01N 33/574

(54) **METHODS AND COMPOSITONS RELATING TO INHIBITION OF IGF-1R**
VERFAHREN UND ZUSAMMENSETZUNGEN ZUR HEMMUNG VON IGF-1R
MÉTHODES ET COMPOSITIONS ASSOCIÉES À L'INHIBITION D'IGF-1R

(30) Priority: 02.02.2011 US 201161438918 P
(43) Date of publication of application: 11.12.2013
(73) Proprietor: Amgen Inc., California 91320 (US)
(72) Inventor: MCCAFFERY, Ian, Moorpark, CA 93021 (US); LU, Jian-Feng, Oak Park, CA 91377 (US)
(74) Representative: Dörries, Hans Ulrich
(86) International application number: PCT/US2012/023691
(87) International publication number: WO 2012/106556

(56) References cited:
- WO-A1-99/44062
- WO-A1-02/068960
- US-A1- 2009 093 488
- POLLAK MICHAEL N ET AL: "Insulin-like growth factors and neoplasia.", NATURE REVIEWS. CANCER JUL 2004 LNKD- PUBMED:15229476, vol. 4, no. 7, July 2004 (2004-07), pages 505-518, XP002674515, ISSN: 1474-175X

### SEQUENCE LISTING

<110> Amgen Inc. McCaffery, Ian Lu, Jian-Feng
<120> Methods and Compositions Relating to Inhibition of IGF-1R
<130> A-1610-WO-PCT
<140> To be assigned
   <141> 2012-02-02
<150> US 61/438,918
   <151> 2011-02-02
<160> 279
<170> PatentIn version 3.2
<210> 1
   <211> 336
   <212> DNA
   <213> Artificial Sequence
<220>
   <223> light chain variable region
<220>
   <221> CDS
   <222> (1)..(336)
<400> 1
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   agt gga tac aac tat ttg gat tgg tac ctg cag aag cca ggg cag tct      144
   Ser Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act ccg atc acc ttc ggc caa ggg aca cga ctg gag att aaa      336
   Leu Gln Thr Pro Ile Thr Phe Gly Gln Gly Thr Arg Leu Glu Ile Lys
               100                 105                 110
<210> 2
   <211> 112
   <212> PRT
   <213> Artificial Sequence
<220>
   <223> light chain variable region
<400> 2
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Ser Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Gln Gly Thr Arg Leu Glu Ile Lys
               100                 105                 110
<210> 3
   <211> 327
   <212> DNA
   <213> Artificial Sequence
<220>
   <223> light chain variable region
<220>
   <221> CDS
   <222> (1)..(327)
<400> 3
   atg act cag tct cca ctc tcc ctg ccc gtc acc cct gga gag ccg gcc       48
   Met Thr Gln Ser Pro Leu Ser Leu Pro Val Thr Pro Gly Glu Pro Ala
   1               5                   10                  15
   tcc atc tcc tgc agg tct agt cag agc ctc ctg cat agt aat gga tac       96
   Ser Ile Ser Cys Arg Ser Ser Gln Ser Leu Leu His Ser Asn Gly Tyr
               20                  25                  30
   aac tat ttg gat tgg tac ctg cag aag cca ggg cag tct cca cag ctc      144
   Asn Tyr Leu Asp Trp Tyr Leu Gln Lys Pro Gly Gln Ser Pro Gln Leu
           35                  40                  45
   ctg atc tat ttg ggt tct aat cgg gcc tcc ggg gtc cct gac agg ttc      192
   Leu Ile Tyr Leu Gly Ser Asn Arg Ala Ser Gly Val Pro Asp Arg Phe
       50                  55                  60
   agt ggc agt gga tca ggc aca gat ttt aca ctg aaa atc agc aga gtg      240
   Ser Gly Ser Gly Ser Gly Thr Asp Phe Thr Leu Lys Ile Ser Arg Val
   65                  70                  75                  80
   gag gct gag gat gtt ggg gtt tat tac tgc atg caa gct cta caa act      288
   Glu Ala Glu Asp Val Gly Val Tyr Tyr Cys Met Gln Ala Leu Gln Thr
                   85                  90                  95
   ccg atc acc ttc ggc caa ggg aca cga ctg gag att aaa                  327
   Pro Ile Thr Phe Gly Gln Gly Thr Arg Leu Glu Ile Lys
               100                 105
<210> 4
   <211> 109
   <212> PRT
   <213> Artificial Sequence
<220>
   <223> light chain variable region
<400> 4
   Met Thr Gln Ser Pro Leu Ser Leu Pro Val Thr Pro Gly Glu Pro Ala
   1               5                   10                  15
   Ser Ile Ser Cys Arg Ser Ser Gln Ser Leu Leu His Ser Asn Gly Tyr
               20                  25                  30
   Asn Tyr Leu Asp Trp Tyr Leu Gln Lys Pro Gly Gln Ser Pro Gln Leu
           35                  40                  45
   Leu Ile Tyr Leu Gly Ser Asn Arg Ala Ser Gly Val Pro Asp Arg Phe
       50                  55                  60
   Ser Gly Ser Gly Ser Gly Thr Asp Phe Thr Leu Lys Ile Ser Arg Val
   65                  70                  75                  80
   Glu Ala Glu Asp Val Gly Val Tyr Tyr Cys Met Gln Ala Leu Gln Thr
                   85                  90                  95
   Pro Ile Thr Phe Gly Gln Gly Thr Arg Leu Glu Ile Lys
               100                 105
<210> 5
   <211> 336
   <212> DNA
   <213> Artificial Sequence
<220>
   <223> light chain variable region
<220>
   <221> CDS
   <222> (1)..(336)
<400> 5
   gat gtt gtg atg act cag tct cca ctc tcc ctg ccc gtc acc cct gga      48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt      96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct     144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct     192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc     240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct     288
   Ser Arg Val Glu Ala Glu Asp Val Gly Val Tyr Tyr Cys Met Gln Ala
                   85                  90                  95
   cta caa act cca ctc act ttc ggc ggc ggg acc aag gtg gag atc aaa     336
   Leu Gln Thr Pro Leu Thr Phe Gly Gly Gly Thr Lys Val Glu Ile Lys
               100                 105                 110
<210> 6
   <211> 112
   <212> PRT
   <213> Artificial Sequence
<220>
   <223> light chain variable region
<400> 6
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 7
   <211> 336
   <212> DNA
   <213> Artificial Sequence
<220>
   <223> light chain variable region
<220>
   <221> CDS
   <222> (1)..(336)
<400> 7
   gaa att gtg atg acg cag tct cca ctc tcc ctg ccc gtc acc cct gga        48
   Glu Ile Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct cac act ttc ggc gga ggg acc aag gtg gag atc aaa       336
   Leu Gln Thr Pro His Thr Phe Gly Gly Gly Thr Lys Val Glu Ile Lys
               100                 105                 110
<210> 8
   <211> 112
   <212> PRT
   <213> Artificial Sequence
<220>
   <223> light chain variable region
<400> 8
   Glu Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro His Thr Phe Gly Gly Gly Thr Lys Val Glu Ile Lys
               100                 105                 110
<210> 9
   <211> 336
   <212> DNA
   <213> Artificial Sequence
<220>
   <223> light chain variable region
<220>
   <221> CDS
   <222> (1)..(336)
<400> 9
   gaa att gtg ctg act cag tct cca ctc tcc ctg ccc gtc acc cct gga       48
   Glu Ile Val Leu Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa acc cct ctc act ttc ggc cct ggg acc aaa gtg gat atc aaa      336
   Leu Gln Thr Pro Leu Thr Phe Gly Pro Gly Thr Lys Val Asp Ile Lys
               100                 105                 110
<210> 10
   <211> 112
   <212> PRT
   <213> Artificial Sequence
<220>
   <223> light chain variable region
<400> 10
   Glu Ile Val Leu Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Pro Gly Thr Lys Val Asp Ile Lys
               100                 105                 110
<210> 11
   <211> 336
   <212> DNA
   <213> Artificial Sequence
<220>
   <223> light chain variable region
<220>
   <221> CDS
   <222> (1)..(336)
<400> 11
   gat gtt gtg atg act cag tct cca ctc tcc ctg gcc gtc acc cct gga        48
   Asp Val Val Met Thr Gln Ser Pro Leu Ser Leu Ala Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act ccg ctc act ttc ggc gga ggg acc aag gtg gag atc aaa       336
   Leu Gln Thr Pro Leu Thr Phe Gly Gly Gly Thr Lys Val Glu Ile Lys
               100                 105                 110
<210> 12
   <211> 112
   <212> PRT
   <213> Artificial Sequence
<220>
   <223> light chain variable region
<400> 12
   Asp Val Val Met Thr Gln Ser Pro Leu Ser Leu Ala Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
             100                 105                 110
<210> 13
   <211> 336
   <212> DNA
   <213> Artificial Sequence
<220>
   <223> light chain variable region
<220>
   <221> CDS
   <222> (1)..(336)
<400> 13
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct ctc act ttc ggc gga ggg acc aag gtg gag atc aaa       336
   Leu Gln Thr Pro Leu Thr Phe Gly Gly Gly Thr Lys Val Glu Ile Lys
               100                 105                 110
<210> 14
   <211> 112
   <212> PRT
   <213> Artificial Sequence
<220>
   <223> light chain variable region
<400> 14
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 15
   <211> 336
   <212> DNA
   <213> Artificial Sequence
<220>
   <223> light chain variable region
<220>
   <221> CDS
   <222> (1)..(336)
<400> 15
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gaa gat gtt ggg gtt tat tac tgt atg caa gct       288
   Ser Arg Val Glu Ala Glu Asp Val Gly Val Tyr Tyr Cys Met Gln Ala
                   85                  90                  95
   cta caa acc ccc ctc act ttc ggc gga ggg acc aag gtg gag atc aaa       336
   Leu Gln Thr Pro Leu Thr Phe Gly Gly Gly Thr Lys Val Glu Ile Lys
               100                 105                 110
<210> 16
   <211> 112
   <212> PRT
   <213> Artificial Sequence
<220>
   <223> light chain variable region
<400> 16
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 17
   <211> 336
   <212> DNA
   <213> Artificial Sequence
<220>
   <223> light chain variable region
<220>
   <221> CDS
   <222> (1)..(336)
<400> 17
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act ccg ttc acc ttc ggc caa ggg aca cga ctg gag att aaa       336
   Leu Gln Thr Pro Phe Thr Phe Gly Gln Gly Thr Arg Leu Glu Ile Lys
               100                 105                 110
<210> 18
   <211> 112
   <212> PRT
   <213> Artificial Sequence
<220>
   <223> light chain variable region
<400> 18
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Phe Thr Phe Gly Gln Gly Thr Arg Leu Glu Ile Lys
               100                 105                 110
<210> 19
   <211> 336
   <212> DNA
   <213> Artificial Sequence
<220>
   <223> light chain variable region
<220>
   <221> CDS
   <222> (1)..(336)
<400> 19
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct ctg gcg ttc ggc caa ggg acc aag gtg gaa atc aaa       336
   Leu Gln Thr Pro Leu Ala Phe Gly Gln Gly Thr Lys Val Glu Ile Lys
               100                 105                 110
<210> 20
   <211> 112
   <212> PRT
   <213> Artificial Sequence
<220>
   <223> light chain variable region
<400> 20
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Ala Phe Gly Gln Gly Thr Lys Val Glu Ile Lys
               100                 105                 110
<210> 21
   <211> 336
   <212> DNA
   <213> Artificial Sequence
<220>
   <223> light chain variable region
<220>
   <221> CDS
   <222> (1)..(336)
<400> 21
   gaa att gtg ctg act cag tct cca ctc tcc ctg ccc gtc acc cct gga       48
   Glu Ile Val Leu Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg aat tgg tac ctg cag aag cca ggg cag tct      144
   Asn Gly Tyr Asn Tyr Leu Asn Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt gcc agt gga tca ggc aca gat ttt aca ctg aaa atc      240
   Asp Arg Phe Ser Ala Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act cct atc acc ttc ggc caa ggg aca cga ctg gag att aaa      336
   Leu Gln Thr Pro Ile Thr Phe Gly Gln Gly Thr Arg Leu Glu Ile Lys
               100                 105                 110
<210> 22
   <211> 112
   <212> PRT
   <213> Artificial Sequence
<220>
   <223> light chain variable region
<400> 22
   Glu Ile Val Leu Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asn Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Ala Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Gln Gly Thr Arg Leu Glu Ile Lys
               100                 105                 110
<210> 23
   <211> 333
   <212> DNA
   <213> Artificial Sequence
<220>
   <223> light chain variable region
<220>
   <221> CDS
   <222> (1)..(333)
<400> 23
   aat ttt atg ctg act cag ccc cac tct gtg tcg gag tct ccg ggg aag         48
   Asn Phe Met Leu Thr Gln Pro His Ser Val Ser Glu Ser Pro Gly Lys
   1               5                   10                  15
   acg gta acc atc tcc tgc acc cgc agc agt ggc agc att gcc agc aac         96
   Thr Val Thr Ile Ser Cys Thr Arg Ser Ser Gly Ser Ile Ala Ser Asn
               20                  25                  30
   tat gtg cag tgg tac cag cag cgc ccg ggc agt tcc ccc acc act gtg        144
   Tyr Val Gln Trp Tyr Gln Gln Arg Pro Gly Ser Ser Pro Thr Thr Val
           35                  40                  45
   atc tat gag gat aac caa aga ccc tct ggg gtc cct gat cgg ttc tct        192
   Ile Tyr Glu Asp Asn Gln Arg Pro Ser Gly Val Pro Asp Arg Phe Ser
       50                  55                  60
   ggc tcc atc gac agc tcc tcc aac tct gcc tcc ctc acc atc tct gga        240
   Gly Ser Ile Asp Ser Ser Ser Asn Ser Ala Ser Leu Thr Ile Ser Gly
   65                  70                  75                  80
   ctg aag act gag gac gag gct gac tac tac tgt cag tct tat gat agc        288
   Leu Lys Thr Glu Asp Glu Ala Asp Tyr Tyr Cys Gln Ser Tyr Asp Ser
                   85                  90                  95
   agc aat cag aga gtg ttc ggc gga ggg acc aag ctg acc gtc cta            333
   Ser Asn Gln Arg Val Phe Gly Gly Gly Thr Lys Leu Thr Val Leu
               100                 105                 110
<210> 24
   <211> 111
   <212> PRT
   <213> Artificial Sequence
<220>
   <223> light chain variable region
<400> 24
   Asn Phe Met Leu Thr Gln Pro His Ser Val Ser Glu Ser Pro Gly Lys
   1               5                   10                  15
   Thr Val Thr Ile Ser Cys Thr Arg Ser Ser Gly Ser Ile Ala Ser Asn
               20                  25                  30
   Tyr Val Gln Trp Tyr Gln Gln Arg Pro Gly Ser Ser Pro Thr Thr Val
           35                  40                  45
   Ile Tyr Glu Asp Asn Gln Arg Pro Ser Gly Val Pro Asp Arg Phe Ser
       50                  55                  60
   Gly Ser Ile Asp Ser Ser Ser Asn Ser Ala Ser Leu Thr Ile Ser Gly
   65                  70                  75                  80
   Leu Lys Thr Glu Asp Glu Ala Asp Tyr Tyr Cys Gln Ser Tyr Asp Ser
                   85                  90                  95
   Ser Asn Gln Arg Val Phe Gly Gly Gly Thr Lys Leu Thr Val Leu
               100                 105                 110
<210> 25
   <211> 336
   <212> DNA
   <213> Artificial Sequence
<220>
   <223> light chain variable region
<220>
   <221> CDS
   <222> (1)..(336)
<400> 25
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa acc ccg ctc act ttc ggc gga ggg acc aag gtg gag atc aaa       336
   Leu Gln Thr Pro Leu Thr Phe Gly Gly Gly Thr Lys Val Glu Ile Lys
               100                 105                 110
<210> 26
   <211> 112
   <212> PRT
   <213> Artificial Sequence
<220>
   <223> light chain variable region
<400> 26
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 27
   <211> 336
   <212> DNA
   <213> Artificial Sequence
<220>
   <223> light chain variable region
<220>
   <221> CDS
   <222> (1)..(336)
<400> 27
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct ctt act ttc ggc gga ggg acc aag gtg gag atc aaa       336
   Leu Gln Thr Pro Leu Thr Phe Gly Gly Gly Thr Lys Val Glu Ile Lys
               100                 105                 110
<210> 28
   <211> 112
   <212> PRT
   <213> Artificial Sequence
<220>
   <223> light chain variable region
<400> 28
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 29
   <211> 336
   <212> DNA
   <213> Artificial Sequence
<220>
   <223> light chain variable region
<220>
   <221> CDS
   <222> (1)..(336)
<400> 29
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg gat tgg tac ctg caa aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct tat cgg gcc tcc ggg gtc cct      192
   Pro Gln Leu Leu Ile Tyr Leu Gly Ser Tyr Arg Ala Ser Gly Val Pro
       50                  55                  60
   gac agg ttc agt gcc agt gga tca ggc aca gat ttt aca ctg aaa atc      240
   Asp Arg Phe Ser Ala Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act ccg atc acc ttc ggc caa ggg aca cga ctg gag att aaa      336
   Leu Gln Thr Pro Ile Thr Phe Gly Gln Gly Thr Arg Leu Glu Ile Lys
               100                 105                 110
<210> 30
   <211> 112
   <212> PRT
   <213> Artificial Sequence
<220>
   <223> light chain variable region
<400> 30
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Tyr Arg Ala Ser Gly Val Pro
       50                  55                  60
   Asp Arg Phe Ser Ala Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Gln Gly Thr Arg Leu Glu Ile Lys
               100                 105                 110
<210> 31
   <211> 336
   <212> DNA
   <213> Artificial Sequence
<220>
   <223> light chain variable region
<220>
   <221> CDS
   <222> (1)..(336)
<400> 31
   gat gtt gtg atg act cag tct cca ctc tcc ctg ccc gtc acc cct gga        48
   gag ccg gcc tcc atc tcc tgc agg tct agt cag agc ctc ctg cat agt        96
   aat gga tac aac tat ttg gat tgg tac ctg cag aag cca ggg cag tct       144
   cca cag ctc ctg atc tat ttg ggt tct aat cgg gcc tcc ggg gtc cct       192
   gac agg ttc agt ggc agt gga tca ggc aca gat ttt aca ctg aaa atc       240
   agc agg gtg gag gct gag gat gtt ggg gtt tat tac tgc atg caa ggt       288
   aca cac tgg cct ctg acg ttc ggc caa ggg acc aag gtg gag atc aaa       336
<210> 32
   <211> 112
   <212> PRT
   <213> Artificial Sequence
<220>
   <223> light chain variable region
<400> 32
<210> 33
   <211> 335
   <212> DNA
   <213> Artificial Sequence
<220>
   <223> light chain variable region
<220>
   <221> CDS
   <222> (1)..(333)
<400> 33
   gaa att gtg atg acg cag tct cca ctc tcc ctg ccc gtc acc cct gga        48
   Glu Ile Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct ctc act ttc ggc gga ggg acc aag gtg gag atc aa        335
   Leu Gln Thr Pro Leu Thr Phe Gly Gly Gly Thr Lys Val Glu Ile
               100                 105                 110
<210> 34
   <211> 111
   <212> PRT
   <213> Artificial Sequence
<220>
   <223> light chain variable region
<400> 34
   Glu Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile
               100                 105                 110
<210> 35
   <211> 321
   <212> DNA
   <213> Artificial Sequence
<220>
   <223> light chain variable region
<220>
   <221> CDS
   <222> (1)..(321)
<400> 35
   gac atc cag ttg acc cag tct cca tct tcc gtg tct gcg tct gtc gga       48
   Asp Ile Gln Leu Thr Gln Ser Pro Ser Ser Val Ser Ala Ser Val Gly
   1               5                   10                  15
   gac aga gtc acc atc act tgt cgg gcg agt cag ggt att agc agg tgg       96
   Asp Arg Val Thr Ile Thr Cys Arg Ala Ser Gln Gly Ile Ser Arg Trp
               20                  25                  30
   tta gcc tgg tat caa cag aaa cca ggg aaa gcc cct aga ctc ctg atc      144
   Leu Ala Trp Tyr Gln Gln Lys Pro Gly Lys Ala Pro Arg Leu Leu Ile
           35                  40                  45
   tat gct gcg tcc ggt tta caa agt ggg gtc cca tca agg ttc agc ggc      192
   Tyr Ala Ala Ser Gly Leu Gln Ser Gly Val Pro Ser Arg Phe Ser Gly
       50                  55                  60
   agt gga tct ggg aca gat ttc act ctc acc atc agc aac ctg cag cct      240
   Ser Gly Ser Gly Thr Asp Phe Thr Leu Thr Ile Ser Asn Leu Gln Pro
   65                  70                  75                  80
   gaa gat ttt gca act tac tat tgt caa cag gct agc agt ttt cca atc      288
   Glu Asp Phe Ala Thr Tyr Tyr Cys Gln Gln Ala Ser Ser Phe Pro Ile
                   85                  90                  95
   acc ttc ggc caa ggg aca cga ctg gag act aaa                          321
   Thr Phe Gly Gln Gly Thr Arg Leu Glu Thr Lys
               100                 105
<210> 36
   <211> 107
   <212> PRT
   <213> Artificial Sequence
<220>
   <223> light chain variable region
<400> 36
   Asp Ile Gln Leu Thr Gln Ser Pro Ser Ser Val Ser Ala Ser Val Gly
   1               5                   10                  15
   Asp Arg Val Thr Ile Thr Cys Arg Ala Ser Gln Gly Ile Ser Arg Trp
               20                  25                  30
   Leu Ala Trp Tyr Gln Gln Lys Pro Gly Lys Ala Pro Arg Leu Leu Ile
           35                  40                  45
   Tyr Ala Ala Ser Gly Leu Gln Ser Gly Val Pro Ser Arg Phe Ser Gly
       50                  55                  60
   Ser Gly Ser Gly Thr Asp Phe Thr Leu Thr Ile Ser Asn Leu Gln Pro
   65                  70                  75                  80
   Glu Asp Phe Ala Thr Tyr Tyr Cys Gln Gln Ala Ser Ser Phe Pro Ile
                   85                  90                  95
   Thr Phe Gly Gln Gly Thr Arg Leu Glu Thr Lys
               100                 105
<210> 37
   <211> 336
   <212> DNA
   <213> Artificial Sequence
<220>
   <223> light chain variable region
<220>
   <221> CDS
   <222> (1)..(336)
<400> 37
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt gga gtt tat tac tgc atg caa gct       288
   Ser Arg Val Glu Ala Glu Asp Val Gly Val Tyr Tyr Cys Met Gln Ala
                   85                  90                  95
   cta caa act ccg tac act ttt ggc cag ggg acc aag ctg gag atc aaa       336
   Leu Gln Thr Pro Tyr Thr Phe Gly Gln Gly Thr Lys Leu Glu Ile Lys
               100                 105                 110
<210> 38
   <211> 112
   <212> PRT
   <213> Artificial Sequence
<220>
   <223> light chain variable region
<400> 38
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Tyr Thr Phe Gly Gln Gly Thr Lys Leu Glu Ile Lys
               100                 105                 110
<210> 39
   <211> 336
   <212> DNA
   <213> Artificial Sequence
<220>
   <223> light chain variable region
<220>
   <221> CDS
   <222> (1)..(336)
<400> 39
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt      96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct     144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct     192
   Pro Gln Leu Leu Ile Tyr Leu Gly Ser Asn Arg Ala Ser Gly Val Pro
       50                  55                  60
   aac agg ttc agt ggc agt gga tca ggc aca gat ttt aca ctg aaa atc     240
   Asn Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct     288
   Ser Arg Val Glu Ala Glu Asp Val Gly Val Tyr Tyr Cys Met Gln Ala
                   85                  90                  95
   cta caa act cca ttc act ttc ggc cct ggg acc aaa gtg gat atc aaa     336
   Leu Gln Thr Pro Phe Thr Phe Gly Pro Gly Thr Lys Val Asp Ile Lys
               100                 105                 110
<210> 40
   <211> 112
   <212> PRT
   <213> Artificial Sequence
<220>
   <223> light chain variable region
<400> 40
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asn Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Phe Thr Phe Gly Pro Gly Thr Lys Val Asp Ile Lys
               100                 105                 110
<210> 41
   <211> 336
   <212> DNA
   <213> Artificial Sequence
<220>
   <223> light chain variable region
<220>
   <221> CDS
   <222> (1)..(336)
<400> 41
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   cat gga tac aac tat ttg gat tgg tac ctg cag aag cca ggg cag tct      144
   His Gly Tyr Asn Tyr Leu Asp Trp Tyr Leu Gln Lys Pro Gly Gln Ser
           35                  40                  45
   cca caa ctt ctg atc tat ttg ggt tct tat cgg gcc tcc ggg gtc cct      192
   Pro Gln Leu Leu Ile Tyr Leu Gly Ser Tyr Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa tct      288
   Ser Arg Val Glu Ala Glu Asp Val Gly Val Tyr Tyr Cys Met Gln Ser
                   85                  90                  95
   cta gaa gtt ccg ttc act ttt ggc cag ggg acc aag ctg gag atc aaa      336
   Leu Glu Val Pro Phe Thr Phe Gly Gln Gly Thr Lys Leu Glu Ile Lys
               100                 105                 110
<210> 42
   <211> 112
   <212> PRT
   <213> Artificial Sequence
<220>
   <223> light chain variable region
<400> 42
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   His Gly Tyr Asn Tyr Leu Asp Trp Tyr Leu Gln Lys Pro Gly Gln Ser
       35                  40                  45
   Pro Gln Leu Leu Ile Tyr Leu Gly Ser Tyr Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ser
                   85                  90                  95
   Leu Glu Val Pro Phe Thr Phe Gly Gln Gly Thr Lys Leu Glu Ile Lys
               100                 105                 110
<210> 43
   <211> 321
   <212> DNA
   <213> Artificial Sequence
<220>
   <223> light chain variable region
<220>
   <221> CDS
   <222> (1)..(321)
<400> 43
   tct tct gag ctg act cag gac cct gct gtg tct gtg gcc ttg gga cag       48
   Ser Ser Glu Leu Thr Gln Asp Pro Ala Val Ser Val Ala Leu Gly Gln
   1               5                   10                  15
   aca gtc agg atc aca tgc caa gga gac agc ctc aga att tat tat aca       96
   Thr Val Arg Ile Thr Cys Gln Gly Asp Ser Leu Arg Ile Tyr Tyr Thr
               20                  25                  30
   ggc tgg tac caa cag aag cca gga cag gcc cct gtg ctt gtc ctc ttt      144
   Gly Trp Tyr Gln Gln Lys Pro Gly Gln Ala Pro Val Leu Val Leu Phe
           35                  40                  45
   ggt aag aac aat cgg ccc tca ggg atc cca gac cga ttc tct ggc tcc      192
   Gly Lys Asn Asn Arg Pro Ser Gly Ile Pro Asp Arg Phe Ser Gly Ser
       50                  55                  60
   cac tca ggg aac aca gct tcc ttg acc atc act ggg gct caa gcg gaa      240
   His Ser Gly Asn Thr Ala Ser Leu Thr Ile Thr Gly Ala Gln Ala Glu
   65                  70                  75                  80
   gat gag gct gac tat tac tgt aac tcc cgg gac atc act ggt gtc cat      288
   Asp Glu Ala Asp Tyr Tyr Cys Asn Ser Arg Asp Ile Thr Gly Val His
                   85                  90                  95
   cga ttc ggc gga ggg acc aag ctg acc gtc cta                          321
   Arg Phe Gly Gly Gly Thr Lys Leu Thr Val Leu
               100                 105
<210> 44
   <211> 107
   <212> PRT
   <213> Artificial Sequence
<220>
   <223> light chain variable region
<400> 44
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ile Tyr Tyr Thr
               20                  25                  30
   Gly Trp Tyr Gln Gln Lys Pro Gly Gln Ala Pro Val Leu Val Leu Phe
           35                  40                  45
   Gly Lys Asn Asn Arg Pro Ser Gly Ile Pro Asp Arg Phe Ser Gly Ser
       50                  55                  60
   His Ser Gly Asn Thr Ala Ser Leu Thr Ile Thr Gly Ala Gln Ala Glu
   65                  70                  75                  80
   Asp Glu Ala Asp Tyr Tyr Cys Asn Ser Arg Asp Ile Thr Gly Val His
                   85                  90                  95
   Arg Phe Gly Gly Gly Thr Lys Leu Thr Val Leu
               100                 105
<210> 45
   <211> 336
   <212> DNA
   <213> Artificial Sequence
<220>
   <223> light chain variable region
<220>
   <221> CDS
   <222> (1)..(336)
<400> 45
   gaa att gtg ctg act cag tct cca ctc tcc ctg ccc gtc acc cct gga          48
   Glu Ile Val Leu Thr Gln Ser Pro Leu Ser Leu Pro Val Thr Pro Gly
   1               5                   10                  15
   gag ccg gcc tcc atc tcc tgc agg tct agt cag agc ctc ctg cat agt          96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct         144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct         192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc         240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct         288
   Ser Arg Val Glu Ala Glu Asp Val Gly Val Tyr Tyr Cys Met Gln Ala
                   85                  90                  95
   cta caa act cct ctc act ttc ggc gga ggg acc aag gtg gag atc aaa         336
   Leu Gln Thr Pro Leu Thr Phe Gly Gly Gly Thr Lys Val Glu Ile Lys
               100                 105                 110
<210> 46
   <211> 112
   <212> PRT
   <213> Artificial Sequence
<220>
   <223> light chain variable region
<400> 46
   Glu Ile Val Leu Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 47
   <211> 336
   <212> DNA
   <213> Artificial Sequence
<220>
   <223> light chain variable region
<220>
   <221> CDS
   <222> (1)..(336)
<400> 47
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct aac act ttc ggc gga ggg acc aag gtg gag atc aaa       336
   Leu Gln Thr Pro Asn Thr Phe Gly Gly Gly Thr Lys Val Glu Ile Lys
               100                 105                 110
<210> 48
   <211> 112
   <212> PRT
   <213> Artificial Sequence
<220>
   <223> light chain variable region
<400> 48
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Asn Thr Phe Gly Gly Gly Thr Lys Val Glu Ile Lys
               100                 105                 110
<210> 49
   <211> 336
   <212> DNA
   <213> Artificial Sequence
<220>
   <223> light chain variable region
<220>
   <221> CDS
   <222> (1)..(336)
<400> 49
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act cca atc act ttc ggc cct ggg acc aaa gtg gat atc aaa      336
   Leu Gln Thr Pro Ile Thr Phe Gly Pro Gly Thr Lys Val Asp Ile Lys
               100                 105                 110
<210> 50
   <211> 112
   <212> PRT
   <213> Artificial Sequence
<220>
   <223> light chain variable region
<400> 50
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Pro Gly Thr Lys Val Asp Ile Lys
               100                 105                 110
<210> 51
   <211> 336
   <212> DNA
   <213> Artificial Sequence
<220>
   <223> light chain variable region
<220>
   <221> CDS
   <222> (1)..(336)
<400> 51
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac acc tat ttg gat tgg tac ctg cag aag cca ggg cag tct       144
   Asn Gly Tyr Thr Tyr Leu Asp Trp Tyr Leu Gln Lys Pro Gly Gln Ser
           35                  40                  45
   cca caa ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agc ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag cct gag gat gtt ggg gtc tat tac tgc atg caa gct       288
   Ser Arg Val Glu Pro Glu Asp Val Gly Val Tyr Tyr Cys Met Gln Ala
                   85                  90                  95
   cta gaa atg ccc ctc act ttc ggc gga ggg acc aag gtg gag atc aaa       336
   Leu Glu Met Pro Leu Thr Phe Gly Gly Gly Thr Lys Val Glu Ile Lys
               100                 105                 110
<210> 52
   <211> 112
   <212> PRT
   <213> Artificial Sequence
<220>
   <223> light chain variable region
<400> 52
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Thr Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Pro Glu Asp Val Gly Val Tyr Tyr Cys Met Gln Ala
                   85                  90                  95
   Leu Glu Met Pro Leu Thr Phe Gly Gly Gly Thr Lys Val Glu Ile Lys
               100                 105                 110
<210> 53
   <211> 321
   <212> DNA
   <213> Artificial Sequence
<220>
   <223> light chain variable region
<220>
   <221> CDS
   <222> (1)..(321)
<400> 53
   gac atc cag ttg acc cag tct cca tcc ttc ctg tct gca tct gta gga       48
   Asp Ile Gln Leu Thr Gln Ser Pro Ser Phe Leu Ser Ala Ser Val Gly
   1               5                   10                  15
   gac aga gtc acc atc act tgc cgg gcc agt cag ggc att agc agt tat      96
   Asp Arg Val Thr Ile Thr Cys Arg Ala Ser Gln Gly Ile Ser Ser Tyr
               20                  25                  30
   tta gcc tgg tat cag caa aaa cca ggg aaa gcc cct aag ctc ctg atc     144
   Leu Ala Trp Tyr Gln Gln Lys Pro Gly Lys Ala Pro Lys Leu Leu Ile
           35                  40                  45
   tat gct gca tcc act ttg caa agt ggg gtc cca tca agg ttc agc ggc     192
   Tyr Ala Ala Ser Thr Leu Gln Ser Gly Val Pro Ser Arg Phe Ser Gly
       50                  55                  60
   agt gga tct ggg aca gaa ttc act ctc aca atc agc agc ctg cag cct     240
   Ser Gly Ser Gly Thr Glu Phe Thr Leu Thr Ile Ser Ser Leu Gln Pro
   65                  70                  75                  80
   gaa gat ttt gca act tat tac tgt caa cag ctt aat agt tac ccc ctc     288
   Glu Asp Phe Ala Thr Tyr Tyr Cys Gln Gln Leu Asn Ser Tyr Pro Leu
                   85                  90                  95
   act ttc ggc gga ggg acc aag gtg gag atc aaa                         321
   Thr Phe Gly Gly Gly Thr Lys Val Glu Ile Lys
               100                 105
<210> 54
   <211> 107
   <212> PRT
   <213> Artificial Sequence
<220>
   <223> light chain variable region
<400> 54
   Asp Ile Gln Leu Thr Gln Ser Pro Ser Phe Leu Ser Ala Ser Val Gly
   1               5                   10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Leu
                   85                  90                  95
   Thr Phe Gly Gly Gly Thr Lys Val Glu Ile Lys
                       100                 105
<210> 55
   <211> 315
   <212> DNA
   <213> Artificial Sequence
<220>
   <223> light chain variable region
<220>
   <221> CDS
   <222> (1)..(315)
<400> 55
   tcc tat gtg ctg act cag cca ccc tca gtg tcc gtg tcc cca gga cag        48
   Ser Tyr Val Leu Thr Gln Pro Pro Ser Val Ser Val Ser Pro Gly Gln
   1               5                   10                  15
   aca gcc agc atc acc tgc tct gga gat aaa ttg ggg gat aaa tat gtt        96
   Thr Ala Ser Ile Thr Cys Ser Gly Asp Lys Leu Gly Asp Lys Tyr Val
               20                  25                  30
   ggc tgg tat cag caa aag gca ggc caa gcc cct gtt ttg gtc atc tat       144
   Gly Trp Tyr Gln Gln Lys Ala Gly Gln Ala Pro Val Leu Val Ile Tyr
           35                  40                  45
   caa gac aac aag cga ccc tca ggg atc cct gag cga ttc tct ggc tcc       192
   Gln Asp Asn Lys Arg Pro Ser Gly Ile Pro Glu Arg Phe Ser Gly Ser
       50                  55                  60
   aac tct ggg aac aca gcc agt ctg acc atc agc ggg acc cag gct atg       240
   Asn Ser Gly Asn Thr Ala Ser Leu Thr Ile Ser Gly Thr Gln Ala Met
   65                  70                  75                  80
   gat gag gct gac tat tac tgt cag gcg tgg gac agc ggc acg gtg ttc       288
   Asp Glu Ala Asp Tyr Tyr Cys Gln Ala Trp Asp Ser Gly Thr Val Phe
                   85                  90                  95
   ggc gga ggg acc aag ctg acc gtc cta                                   315
   Gly Gly Gly Thr Lys Leu Thr Val Leu
               100                 105
<210> 56
   <211> 105
   <212> PRT
   <213> Artificial Sequence
<220>
   <223> light chain variable region
<400> 56
   Ser Tyr Val Leu Thr Gln Pro Pro Ser Val Ser Val Ser Pro Gly Gln
   1               5                   10                  15
   Thr Ala Ser Ile Thr Cys Ser Gly Asp Lys Leu Gly Asp Lys Tyr Val
               20                  25                  30
   Gly Trp Tyr Gln Gln Lys Ala Gly Gln Ala Pro Val Leu Val Ile Tyr
           35                  40                  45
   Gln Asp Asn Lys Arg Pro Ser Gly Ile Pro Glu Arg Phe Ser Gly Ser
       50                  55                  60
   Asn Ser Gly Asn Thr Ala Ser Leu Thr Ile Ser Gly Thr Gln Ala Met
   65                  70                  75                  80
   Asp Glu Ala Asp Tyr Tyr Cys Gln Ala Trp Asp Ser Gly Thr Val Phe
                   85                  90                  95
   Gly Gly Gly Thr Lys Leu Thr Val Leu
               100                 105
<210> 57
   <211> 336
   <212> DNA
   <213> Artificial Sequence
<220>
   <223> light chain variable region
<220>
   <221> CDS
   <222> (1)..(336)
<400> 57
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa acc ccc ctc act ttc ggc gga ggg acc aag gtg gag atc aaa      336
   Leu Gln Thr Pro Leu Thr Phe Gly Gly Gly Thr Lys Val Glu Ile Lys
               100                 105                 110
<210> 58
   <211> 112
   <212> PRT
   <213> Artificial Sequence
<220>
   <223> light chain variable region
<400> 58
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 59
   <211> 336
   <212> DNA
   <213> Artificial Sequence
<220>
   <223> light chain variable region
<220>
   <221> CDS
   <222> (1)..(336)
<400> 59
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg gaa gct       288
   Ser Arg Val Glu Ala Glu Asp Val Gly Val Tyr Tyr Cys Met Glu Ala
                   85                  90                  95
   cta caa act cca ttc act ttc ggc cct ggg acc aag gtg gaa atc aaa       336
   Leu Gln Thr Pro Phe Thr Phe Gly Pro Gly Thr Lys Val Glu Ile Lys
               100                 105                 110
<210> 60
   <211> 112
   <212> PRT
   <213> Artificial Sequence
<220>
   <223> light chain variable region
<400> 60
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Glu Ala
                   85                  90                  95
   Leu Gln Thr Pro Phe Thr Phe Gly Pro Gly Thr Lys Val Glu Ile Lys
               100                 105                 110
<210> 61
   <211> 321
   <212> DNA
   <213> Artificial Sequence
<220>
   <223> light chain variable region
<220>
   <221> CDS
   <222> (1)..(321)
<400> 61
   gac atc cag ttg acc cag tct cca tcc tcc ctg tct gcg tct gtg gga        48
   Asp Ile Gln Leu Thr Gln Ser Pro Ser Ser Leu Ser Ala Ser Val Gly
   1               5                   10                  15
   gac aga gtc acc atc act tgc cgg tca agt caa ggc att ggt tac ttc        96
   Asp Arg Val Thr Ile Thr Cys Arg Ser Ser Gln Gly Ile Gly Tyr Phe
               20                  25                  30
   tta aat tgg tat cag cag gaa cca ggg aaa gcc cca aag atc ctg atc       144
   Leu Asn Trp Tyr Gln Gln Glu Pro Gly Lys Ala Pro Lys Ile Leu Ile
           35                  40                  45
   tct gct gca tcc act ttg caa agt ggg gtc cca tca agg ttc agt ggc       192
   Ser Ala Ala Ser Thr Leu Gln Ser Gly Val Pro Ser Arg Phe Ser Gly
       50                  55                  60
   agt gga tct ggg aca gat ttc aca ctc tcc atc aac aat ctg caa ccc       240
   Ser Gly Ser Gly Thr Asp Phe Thr Leu Ser Ile Asn Asn Leu Gln Pro
   65                  70                  75                  80
   gca gat ttt gcg aca tac tac tgt caa cag agt cac agt ccc ccg tac       288
   Ala Asp Phe Ala Thr Tyr Tyr Cys Gln Gln Ser His Ser Pro Pro Tyr
                   85                  90                  95
   act ttc ggc cag ggg acc aag gtg gag atc aaa                           321
   Thr Phe Gly Gln Gly Thr Lys Val Glu Ile Lys
               100                 105
<210> 62
   <211> 107
   <212> PRT
   <213> Artificial Sequence
<220>
   <223> light chain variable region
<400> 62
   Asp Ile Gln Leu Thr Gln Ser Pro Ser Ser Leu Ser Ala Ser Val Gly
   1               5                   10                  15
   Asp Arg Val Thr Ile Thr Cys Arg Ser Ser Gln Gly Ile Gly Tyr Phe
               20                  25                  30
   Leu Asn Trp Tyr Gln Gln Glu Pro Gly Lys Ala Pro Lys Ile Leu Ile
           35                  40                  45
   Ser Ala Ala Ser Thr Leu Gln Ser Gly Val Pro Ser Arg Phe Ser Gly
       50                  55                  60
   Ser Gly Ser Gly Thr Asp Phe Thr Leu Ser Ile Asn Asn Leu Gln Pro
   65                  70                  75                  80
   Ala Asp Phe Ala Thr Tyr Tyr Cys Gln Gln Ser His Ser Pro Pro Tyr
                   85                  90                  95
   Thr Phe Gly Gln Gly Thr Lys Val Glu Ile Lys
               100                 105
<210> 63
   <211> 336
   <212> DNA
   <213> Artificial Sequence
<220>
   <223> light chain variable region
<220>
   <221> CDS
   <222> (1)..(336)
<400> 63
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act ccg ctc act ttc ggc gga ggg acc aag gtg gag atc aaa      336
   Leu Gln Thr Pro Leu Thr Phe Gly Gly Gly Thr Lys Val Glu Ile Lys
               100                 105                 110
<210> 64
   <211> 112
   <212> PRT
   <213> Artificial Sequence
<220>
   <223> light chain variable region
<400> 64
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 65
   <211> 336
   <212> DNA
   <213> Artificial Sequence
<220>
   <223> light chain variable region
<220>
   <221> CDS
   <222> (1)..(336)
<400> 65
   gaa att gtg ctg act cag tct cca ctc tcc ctg ccc gtc acc cct gga        48
   Glu Ile Val Leu Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atg tat ttg gtt tct aat cgg gcc tcc ggg gtc cct       192
   Pro Gln Leu Leu Met Tyr Leu Val Ser Asn Arg Ala Ser Gly Val Pro
       50                  55                  60
   gag agg ttc agt ggc agt gga tca ggc aca gat ttt aca ctg aaa atc       240
   Glu Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa act       288
   Ser Arg Val Glu Ala Glu Asp Val Gly Val Tyr Tyr Cys Met Gln Thr
                   85                  90                  95
   cta caa act cct ctc agt ttt ggc cag ggg acc aag ctg gag atc aaa       336
   Leu Gln Thr Pro Leu Ser Phe Gly Gln Gly Thr Lys Leu Glu Ile Lys
               100                 105                 110
<210> 66
   <211> 112
   <212> PRT
   <213> Artificial Sequence
<220>
   <223> light chain variable region
<400> 66
   Glu Ile Val Leu Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Met Tyr Leu Val Ser Asn Arg Ala Ser Gly Val Pro
       50                  55                  60
   Glu Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Thr
                   85                  90                  95
   Leu Gln Thr Pro Leu Ser Phe Gly Gln Gly Thr Lys Leu Glu Ile Lys
               100                 105                 110
<210> 67
   <211> 336
   <212> DNA
   <213> Artificial Sequence
<220>
   <223> light chain variable region
<220>
   <221> CDS
   <222> (1)..(336)
<400> 67
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt          96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct         144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct         192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc         240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct         288
   Ser Arg Val Glu Ala Glu Asp Val Gly Val Tyr Tyr Cys Met Gln Ala
                   85                  90                  95
   cta caa act ccg ctc act ttc ggc gga ggg acc aag gtg gag atc aaa         336
   Leu Gln Thr Pro Leu Thr Phe Gly Gly Gly Thr Lys Val Glu Ile Lys
               100                 105                 110
<210> 68
   <211> 112
   <212> PRT
   <213> Artificial Sequence
<220>
   <223> light chain variable region
<400> 68
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 69
   <211> 330
   <212> DNA
   <213> Artificial Sequence
<220>
   <223> light chain variable region
<220>
   <221> CDS
   <222> (1)..(330)
<400> 69
   aat ttt atg ctg act cag ccc cac tct gtg tcg gcg tct ccg ggg aag        48
   Asn Phe Met Leu Thr Gln Pro His Ser Val Ser Ala Ser Pro Gly Lys
   1               5                   10                  15
   acg gtt acc atc tcc tgc acc cgc agc agt ggc gac att gac aac aac        96
   Thr Val Thr Ile Ser Cys Thr Arg Ser Ser Gly Asp Ile Asp Asn Asn
               20                  25                  30
   tat gtg cag tgg tac cag cag cgc ccg ggc aat tcc ccc acc aat gtg       144
   Tyr Val Gln Trp Tyr Gln Gln Arg Pro Gly Asn Ser Pro Thr Asn Val
           35                  40                  45
   att tat gag gat aac cga aga ccc tct ggg gtc ccg gat cgc ttc tct       192
   Ile Tyr Glu Asp Asn Arg Arg Pro Ser Gly Val Pro Asp Arg Phe Ser
       50                  55                  60
   ggc tcc atc gac agc tcc tcc aac tct gcc tcc ctc acc atc tct gga       240
   Gly Ser Ile Asp Ser Ser Ser Asn Ser Ala Ser Leu Thr Ile Ser Gly
   65                  70                  75                  80
   ctg cag cct gag gac gag gct gac tac tat tgt cag tct tat caa agc       288
   Leu Gln Pro Glu Asp Glu Ala Asp Tyr Tyr Cys Gln Ser Tyr Gln Ser
                   85                  90                  95
   gac aat tgg gtg ttc ggc gga ggg acc aag gtg acc gtc cta               330
   Asp Asn Trp Val Phe Gly Gly Gly Thr Lys Val Thr Val Leu
               100                 105                 110
<210> 70
   <211> 110
   <212> PRT
   <213> Artificial Sequence
<220>
   <223> light chain variable region
<400> 70
   Asn Phe Met Leu Thr Gln Pro His Ser Val Ser Ala Ser Pro Gly Lys
   1               5                   10                  15
   Thr Val Thr Ile Ser Cys Thr Arg Ser Ser Gly Asp Ile Asp Asn Asn
               20                  25                  30
   Tyr Val Gln Trp Tyr Gln Gln Arg Pro Gly Asn Ser Pro Thr Asn Val
           35                  40                  45
   Ile Tyr Glu Asp Asn Arg Arg Pro Ser Gly Val Pro Asp Arg Phe Ser
       50                  55                  60
   Gly Ser Ile Asp Ser Ser Ser Asn Ser Ala Ser Leu Thr Ile Ser Gly
   65                  70                  75                  80
   Leu Gln Pro Glu Asp Glu Ala Asp Tyr Tyr Cys Gln Ser Tyr Gln Ser
                   85                  90                  95
   Asp Asn Trp Val Phe Gly Gly Gly Thr Lys Val Thr Val Leu
               100                 105                 110
<210> 71
   <211> 330
   <212> DNA
   <213> Artificial Sequence
<220>
   <223> light chain variable region
<220>
   <221> CDS
   <222> (1)..(330)
<400> 71
   aat ttt atg ctg act cag ccc cac tct gtg tcg gag tct ccg ggg aag       48
   Asn Phe Met Leu Thr Gln Pro His Ser Val Ser Glu Ser Pro Gly Lys
   1               5                   10                  15
   acg gta acc atc tcc tgc acc cgc agc agt ggc agc att gcc agc aac       96
   Thr Val Thr Ile Ser Cys Thr Arg Ser Ser Gly Ser Ile Ala Ser Asn
               20                  25                  30
   tat gtg cag tgg tac cag cag cgc ccg ggc agt tcc ccc acc act gtg      144
   Tyr Val Gln Trp Tyr Gln Gln Arg Pro Gly Ser Ser Pro Thr Thr Val
           35                  40                  45
   atc tat gag gat aac caa aga ccc tct ggg gtc cct gat cga ttc tct      192
   Ile Tyr Glu Asp Asn Gln Arg Pro Ser Gly Val Pro Asp Arg Phe Ser
       50                  55                  60
   ggc tcc atc gac agc tcc tcc aac tct gcc tcc ctc acc atc tct gga      240
   Gly Ser Ile Asp Ser Ser Ser Asn Ser Ala Ser Leu Thr Ile Ser Gly
   65                  70                  75                  80
   ctg aag act gag gac gag gct gac tac tac tgt cag tct tat gat agc      288
   Leu Lys Thr Glu Asp Glu Ala Asp Tyr Tyr Cys Gln Ser Tyr Asp Ser
                   85                  90                  95
   agc aat gtg gtg ttc ggc gga ggg acc aag ctg acc gtc cta              330
   Ser Asn Val Val Phe Gly Gly Gly Thr Lys Leu Thr Val Leu
               100                 105                 110
<210> 72
   <211> 110
   <212> PRT
   <213> Artificial Sequence
<220>
   <223> light chain variable region
<400> 72
   Asn Phe Met Leu Thr Gln Pro His Ser Val Ser Glu Ser Pro Gly Lys
   1               5                   10                  15
   Thr Val Thr Ile Ser Cys Thr Arg Ser Ser Gly Ser Ile Ala Ser Asn
               20                  25                  30
   Tyr Val Gln Trp Tyr Gln Gln Arg Pro Gly Ser Ser Pro Thr Thr Val
           35                  40                  45
   Ile Tyr Glu Asp Asn Gln Arg Pro Ser Gly Val Pro Asp Arg Phe Ser
       50                  55                  60
   Gly Ser Ile Asp Ser Ser Ser Asn Ser Ala Ser Leu Thr Ile Ser Gly
   65                  70                  75                  80
   Leu Lys Thr Glu Asp Glu Ala Asp Tyr Tyr Cys Gln Ser Tyr Asp Ser
                   85                  90                  95
   Ser Asn Val Val Phe Gly Gly Gly Thr Lys Leu Thr Val Leu
               100                 105                 110
<210> 73
   <211> 336
   <212> DNA
   <213> Artificial Sequence
<220>
   <223> light chain variable region
<220>
   <221> CDS
   <222> (1)..(336)
<400> 73
   gat gtt gtg atg act cag tct cca ctc tcc ctg ccc gtc acc cct ggg        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aac cgg gac tct ggg gtc cca       192
   Pro Gln Leu Leu Ile Tyr Leu Gly Ser Asn Arg Asp Ser Gly Val Pro
       50                  55                  60
   gac aga ttc agc ggc agt ggg tca ggc act gat ttc aca ctg aaa atc       240
   Asp Arg Phe Ser Gly Ser Gly Ser Gly Thr Asp Phe Thr Leu Lys Ile
   65                  70                  75                  80
   agc agg gtg gag gct gag gat gtt ggg gtt tat tac tgc atg caa ggt       288
   Ser Arg Val Glu Ala Glu Asp Val Gly Val Tyr Tyr Cys Met Gln Gly
                   85                  90                  95
   aca cac tgg ccg tac act ttt ggc cag ggg acc agg ctg gag atc aaa       336
   Thr His Trp Pro Tyr Thr Phe Gly Gln Gly Thr Arg Leu Glu Ile Lys
               100                 105                 110
<210> 74
   <211> 112
   <212> PRT
   <213> Artificial Sequence
<220>
   <223> light chain variable region
<400> 74
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Arg Leu Glu Ile Lys
               100                 105                 110
<210> 75
   <211> 336
   <212> DNA
   <213> Artificial Sequence
<220>
   <223> light chain variable region
<220>
   <221> CDS
   <222> (1)..(336)
<400> 75
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag tcg gcc tcc atc tcc tgc agg tct agt cag agc ctc ctg cat agt       96
   Glu Ser Ala Ser Ile Ser Cys Arg Ser Ser Gln Ser Leu Leu His Ser
               20                  25                  30
   aat gga tac aac ttt ttg gat tgg tac ctg cag aag cca ggg cag tct      144
   Asn Gly Tyr Asn Phe Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act cct ctc act ttc ggc gga ggg acc aag gtg gag atc aaa      336
   Leu Gln Thr Pro Leu Thr Phe Gly Gly Gly Thr Lys Val Glu Ile Lys
               100                 105                 110
<210> 76
   <211> 112
   <212> PRT
   <213> Artificial Sequence
<220>
   <223> light chain variable region
<400> 76
   Asp Val Val Met Thr Gln Ser Pro Leu Ser Leu Pro Val Thr Pro Gly
   1               5                   10                  15
   Glu Ser Ala Ser Ile Ser Cys Arg Ser Ser Gln Ser Leu Leu His Ser
               20                  25                  30
   Asn Gly Tyr Asn Phe Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 77
   <211> 336
   <212> DNA
   <213> Artificial Sequence
<220>
   <223> light chain variable region
<220>
   <221> CDS
   <222> (1)..(336)
<400> 77
   gat gtt gtg atg act cag tct cca ctc tcc ctg ccc gtc acc cct gga      48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt      96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa acc ccc ctc act ttc ggc gga ggg acc aag gtg gag atc aaa      336
   Leu Gln Thr Pro Leu Thr Phe Gly Gly Gly Thr Lys Val Glu Ile Lys
               100                 105                 110
<210> 78
   <211> 112
   <212> PRT
   <213> Artificial Sequence
<220>
   <223> light chain variable region
<400> 78
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 79
   <211> 321
   <212> DNA
   <213> Artificial Sequence
<220>
   <223> light chain variable region
<220>
   <221> CDS
   <222> (1)..(321)
<400> 79
   gaa acg aca ctc acg cag tct cca gcc acc ctg tct ttg tct cca ggg        48
   Glu Thr Thr Leu Thr Gln Ser Pro Ala Thr Leu Ser Leu Ser Pro Gly
   1               5                   10                  15
   caa aga gcc acc ctc tcc tgc agg gcc agt cag agt gtc tac aac tac        96
   Gln Arg Ala Thr Leu Ser Cys Arg Ala Ser Gln Ser Val Tyr Asn Tyr
               20                  25                  30
   tta gcc tgg tac caa cag aag cct ggc cag gct ccc agg ctc ctc atc       144
   Leu Ala Trp Tyr Gln Gln Lys Pro Gly Gln Ala Pro Arg Leu Leu Ile
           35                  40                  45
   tat gat gca tcc aga agg gca act ggc atc cca gcc agg ttc agt ggc       192
   Tyr Asp Ala Ser Arg Arg Ala Thr Gly Ile Pro Ala Arg Phe Ser Gly
       50                  55                  60
   agt ggg tct ggg aca gac ttc act ctc acc atc agc agc cta gag cct       240
   Ser Gly Ser Gly Thr Asp Phe Thr Leu Thr Ile Ser Ser Leu Glu Pro
   65                  70                  75                  80
   gaa gat ttt gca gtt tat tac tgt cag cag cgt aac aac tgg ccg ctc       288
   Glu Asp Phe Ala Val Tyr Tyr Cys Gln Gln Arg Asn Asn Trp Pro Leu
                   85                  90                  95
   act ttc ggt gga ggg acc aag gtg gag atc aaa                            321
   Thr Phe Gly Gly Gly Thr Lys Val Glu Ile Lys
               100                 105
<210> 80
   <211> 107
   <212> PRT
   <213> Artificial Sequence
<220>
   <223> light chain variable region
<400> 80
   Glu Thr Thr Leu Thr Gln Ser Pro Ala Thr Leu Ser Leu Ser Pro Gly
   1               5                   10                  15
   Gln Arg Ala Thr Leu Ser Cys Arg Ala Ser Gln Ser Val Tyr Asn Tyr
               20                  25                  30
   Leu Ala Trp Tyr Gln Gln Lys Pro Gly Gln Ala Pro Arg Leu Leu Ile
           35                  40                  45
   Tyr Asp Ala Ser Arg Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Asn Asn Trp Pro Leu
                   85                  90                  95
   Thr Phe Gly Gly Gly Thr Lys Val Glu Ile Lys
               100                 105
<210> 81
   <211> 321
   <212> DNA
   <213> Artificial Sequence
<220>
   <223> light chain variable region
<220>
   <221> CDS
   <222> (1)..(321)
<400> 81
   gac atc cag ttg acc cag tct cca tcc tcc ctg tct gct tct gtt gga       48
   Asp Ile Gln Leu Thr Gln Ser Pro Ser Ser Leu Ser Ala Ser Val Gly
   1               5                   10                  15
   gac agc gtc acc atc tct tgc cgg gca agt cag agt cct ggc atc ttt      96
   Asp Ser Val Thr Ile Ser Cys Arg Ala Ser Gln Ser Pro Gly Ile Phe
               20                  25                  30
   tta aat tgg tat cag cag ata cca ggg aaa gcc cct aaa ctc ctg atc     144
   Leu Asn Trp Tyr Gln Gln Ile Pro Gly Lys Ala Pro Lys Leu Leu Ile
           35                  40                  45
   tac gct aca tcc act ctg gaa agt ggg gtc ccc ccc agg ttc acc ggc     192
   Tyr Ala Thr Ser Thr Leu Glu Ser Gly Val Pro Pro Arg Phe Thr Gly
       50                  55                  60
   agt gga tct ggg aca gat ttc act ctc acc atc agc agt ctg caa cct     240
   Ser Gly Ser Gly Thr Asp Phe Thr Leu Thr Ile Ser Ser Leu Gln Pro
   65                  70                  75                  80
   gag gac ttt gca act tac tac tgt caa cag agt aac agt gtt ccg ctc     288
   Glu Asp Phe Ala Thr Tyr Tyr Cys Gln Gln Ser Asn Ser Val Pro Leu
                   85                  90                  95
   act ttc ggc ggc ggg acc aag gtg gag atc aaa                         321
   Thr Phe Gly Gly Gly Thr Lys Val Glu Ile Lys
               100                 105
<210> 82
   <211> 107
   <212> PRT
   <213> Artificial Sequence
<220>
   <223> light chain variable region
<400> 82
   Asp Ile Gln Leu Thr Gln Ser Pro Ser Ser Leu Ser Ala Ser Val Gly
   1               5                   10                  15
   Asp Ser Val Thr Ile Ser Cys Arg Ala Ser Gln Ser Pro Gly Ile Phe
               20                  25                  30
   Leu Asn Trp Tyr Gln Gln Ile Pro Gly Lys Ala Pro Lys Leu Leu Ile
           35                  40                  45
   Tyr Ala Thr Ser Thr Leu Glu Ser Gly Val Pro Pro Arg Phe Thr Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Asn Ser Val Pro Leu
                   85                  90                  95
   Thr Phe Gly Gly Gly Thr Lys Val Glu Ile Lys
                       100                 105
<210> 83
   <211> 336
   <212> DNA
   <213> Artificial Sequence
<220>
   <223> light chain variable region
<220>
   <221> CDS
   <222> (1)..(336)
<400> 83
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca cta aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct cta acc ttc ggc caa ggg aca cga ctg gag att aaa       336
   Leu Gln Thr Pro Leu Thr Phe Gly Gln Gly Thr Arg Leu Glu Ile Lys
               100                 105                 110
<210> 84
   <211> 112
   <212> PRT
   <213> Artificial Sequence
<220>
   <223> light chain variable region
<400> 84
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
               35                  40                  45
   Pro Gln Leu Leu Ile Tyr Leu Gly Ser Asn Arg Ala Ser Gly Val Pro
           50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gln Gly Thr Arg Leu Glu Ile Lys
               100                 105                 110
<210> 85
   <211> 321
   <212> DNA
   <213> Artificial Sequence
<220>
   <223> light chain variable region
<220>
   <221> CDS
   <222> (1)..(321)
<400> 85
   gaa att gtg atg acg cag tct cca gcc acc ctg tct gtg tct cca ggg      48
   Glu Ile Val Met Thr Gln Ser Pro Ala Thr Leu Ser Val Ser Pro Gly
   1               5                   10                  15
   gaa aga gcc acc ttc tcc tgt agg gcc agt cag agt gtt ggc agc aac      96
   Glu Arg Ala Thr Phe Ser Cys Arg Ala Ser Gln Ser Val Gly Ser Asn
               20                  25                  30
   tta gcc tgg tac cag cag aaa cct ggc cag gct ccc agg ctc ctc atc      144
   Leu Ala Trp Tyr Gln Gln Lys Pro Gly Gln Ala Pro Arg Leu Leu Ile
           35                  40                  45
   tat gat gca tcc aac agg gcc act ggc atc cca gcc agg ttc agt ggc      192
   Tyr Asp Ala Ser Asn Arg Ala Thr Gly Ile Pro Ala Arg Phe Ser Gly
       50                  55                  60
   agt ggg tct ggg aca gac ttc act ctc acc atc agc aga ctg gag cct      240
   Ser Gly Ser Gly Thr Asp Phe Thr Leu Thr Ile Ser Arg Leu Glu Pro
   65                  70                  75                  80
   gaa gat ttt gca gtg tat tac tgt cag cag cgt agc aac tgg ccc ctc      288
   Glu Asp Phe Ala Val Tyr Tyr Cys Gln Gln Arg Ser Asn Trp Pro Leu
                   85                  90                  95
   act ttc ggc gga ggg acc aag gtg gag atc aaa                          321
   Thr Phe Gly Gly Gly Thr Lys Val Glu Ile Lys
               100                 105
<210> 86
   <211> 107
   <212> PRT
   <213> Artificial Sequence
<220>
   <223> light chain variable region
<400> 86
   Glu Ile Val Met Thr Gln Ser Pro Ala Thr Leu Ser Val Ser Pro Gly
   1               5                   10                  15
   Glu Arg Ala Thr Phe Ser Cys Arg Ala Ser Gln Ser Val Gly Ser Asn
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Arg Leu Glu Pro
   65                  70                  75                  80
   Glu Asp Phe Ala Val Tyr Tyr Cys Gln Gln Arg Ser Asn Trp Pro Leu
                   85                  90                  95
   Thr Phe Gly Gly Gly Thr Lys Val Glu Ile Lys
               100                 105
<210> 87
   <211> 336
   <212> DNA
   <213> Artificial Sequence
<220>
   <223> light chain variable region
<220>
   <221> CDS
   <222> (1)..(336)
<400> 87
   gat gtt gtg atg act cag tct cca ctc tcc ctg ccc gtc acc cct gga      48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt      96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act ccg ctc act ttc ggc gga ggg acc aag gtg gag atc aaa      336
   Leu Gln Thr Pro Leu Thr Phe Gly Gly Gly Thr Lys Val Glu Ile Lys
               100                 105                 110
<210> 88
   <211> 112
   <212> PRT
   <213> Artificial Sequence
<220>
   <223> light chain variable region
<400> 88
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 89
   <211> 336
   <212> DNA
   <213> Artificial Sequence
<220>
   <223> light chain variable region
<220>
   <221> CDS
   <222> (1)..(336)
<400> 89
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tac ttg ggt tct act cgg gcc tcc ggc gtc cct       192
   Pro Gln Leu Leu Ile Tyr Leu Gly Ser Thr Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct       288
   Ser Arg Val Glu Ala Glu Asp Val Gly Val Tyr Tyr Cys Met Gln Ala
                   85                  90                  95
   cta caa act cct tac act ttc ggc gga ggg acc aag gtg gag atc aaa       336
   Leu Gln Thr Pro Tyr Thr Phe Gly Gly Gly Thr Lys Val Glu Ile Lys
               100                 105                 110
<210> 90
   <211> 112
   <212> PRT
   <213> Artificial Sequence
<220>
   <223> light chain variable region
<400> 90
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Thr Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Tyr Thr Phe Gly Gly Gly Thr Lys Val Glu Ile Lys
               100                 105                 110
<210> 91
   <211> 336
   <212> DNA
   <213> Artificial Sequence
<220>
   <223> light chain variable region
<220>
   <221> CDS
   <222> (1)..(336)
<400> 91
   gat gtt gtg atg act cag tct cca ctc tcc ctg ccc gtc acc cct gga      48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt      96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct     144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct     192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc     240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct     288
   Ser Arg Val Glu Ala Glu Asp Val Gly Val Tyr Tyr Cys Met Gln Ala
                   85                  90                  95
   cta caa act ccc ctc act ttc ggc gga ggg acc aag gtg gag atc aaa     336
   Leu Gln Thr Pro Leu Thr Phe Gly Gly Gly Thr Lys Val Glu Ile Lys
               100                 105                 110
<210> 92
   <211> 112
   <212> PRT
   <213> Artificial Sequence
<220>
   <223> light chain variable region
<400> 92
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 93
   <211> 336
   <212> DNA
   <213> Artificial Sequence
<220>
   <223> light chain variable region
<220>
   <221> CDS
   <222> (1)..(336)
<400> 93
   gat gtt gtg atg act cag tct cca ctc tcc ctg ccc gtc acc cct gga        48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat act        96
   Glu Pro Ala Ser Ile Ser Cys Arg Ser Ser Gln Ser Leu Leu His Thr
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cgg ctc ctg atc tat ttg ggt ttt aat cgg gcc tcc ggg gtc cct       192
   Pro Arg Leu Leu Ile Tyr Leu Gly Phe Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgt atg caa ggt       288
   Ser Arg Val Glu Ala Glu Asp Val Gly Val Tyr Tyr Cys Met Gln Gly
                   85                  90                  95
   cta caa act ccc ctc act ttc ggc gga ggg acc aag gtg gag atc aaa       336
   Leu Gln Thr Pro Leu Thr Phe Gly Gly Gly Thr Lys Val Glu Ile Lys
               100                 105                 110
<210> 94
   <211> 112
   <212> PRT
   <213> Artificial Sequence
<220>
   <223> light chain variable region
<400> 94
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Thr
               20                  25                  30
   Asn Gly Tyr Asn Tyr Leu Asp Trp Tyr Leu Gln Lys Pro Gly Gln Ser
           35                  40                  45
   Pro Arg Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Leu Gln Thr Pro Leu Thr Phe Gly Gly Gly Thr Lys Val Glu Ile Lys
               100                 105                 110
<210> 95
   <211> 336
   <212> DNA
   <213> Artificial Sequence
<220>
   <223> light chain variable region
<220>
   <221> CDS
   <222> (1)..(336)
<400> 95
   gat gtt gtg atg act cag tct cca ctc tcc ctg ccc gtc acc cct gga       48
   Asp Val Val Met Thr Gln Ser Pro Leu Ser Leu Pro Val Thr Pro Gly
   1               5                   10                  15
   gag ccg gcc tcc atc tcc tgc agg tct agt cag agc ctc ctg cat agt        96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct       144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct       192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc       240
   Asp Arg Phe Ser Gly Ser Gly Ser Gly Thr Asp Phe Thr Leu Lys Ile
   65                  70                  75                  80
   agc agg gtg gag gct gag gat gtt ggg gtt tat tat tgc atg caa gct       288
   Ser Arg Val Glu Ala Glu Asp Val Gly Val Tyr Tyr Cys Met Gln Ala
                   85                  90                  95
   aca cac tgg ccg tac act ttt ggc cag ggg acc aag ctg gag atc aaa       336
   Thr His Trp Pro Tyr Thr Phe Gly Gln Gly Thr Lys Leu Glu Ile Lys
               100                 105                 110
<210> 96
   <211> 112
   <212> PRT
   <213> Artificial Sequence
<220>
   <223> light chain variable region
<400> 96
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Thr His Trp Pro Tyr Thr Phe Gly Gln Gly Thr Lys Leu Glu Ile Lys
               100                 105                 110
<210> 97
   <211> 330
   <212> DNA
   <213> Artificial Sequence
<220>
   <223> light chain variable region
<220>
   <221> CDS
   <222> (1)..(330)
<400> 97
   aat ttt atg ctg act cag ccc cac tct gtg tcg gag tct ccg ggg aag        48
   Asn Phe Met Leu Thr Gln Pro His Ser Val Ser Glu Ser Pro Gly Lys
   1               5                   10                  15
   acg gta agc atc tcc tgc acc cgc aac agt ggc agc att gcc agc aac        96
   Thr Val Ser Ile Ser Cys Thr Arg Asn Ser Gly Ser Ile Ala Ser Asn
               20                  25                  30
   ttt gtg cag tgg tac cag cag cgc ccg ggc agt gcc ccc acc att gta       144
   Phe Val Gln Trp Tyr Gln Gln Arg Pro Gly Ser Ala Pro Thr Ile Val
           35                  40                  45
   atc tat gag gat aac caa aga ccc tct gcg gtc cct act cgg ttc tct       192
   Ile Tyr Glu Asp Asn Gln Arg Pro Ser Ala Val Pro Thr Arg Phe Ser
       50                  55                  60
   ggc tcc atc gac agg tcc tcc aac tct gcc tcc ctc acc atc tct gga       240
   Gly Ser Ile Asp Arg Ser Ser Asn Ser Ala Ser Leu Thr Ile Ser Gly
   65                  70                  75                  80
   ctg acg act gag gac gag gct gac tac tac tgt cag tct tat gat agc       288
   Leu Thr Thr Glu Asp Glu Ala Asp Tyr Tyr Cys Gln Ser Tyr Asp Ser
                   85                  90                  95
   gcc aat gtc att ttc ggc ggg ggg acc aag ctg acc gtc cta               330
   Ala Asn Val Ile Phe Gly Gly Gly Thr Lys Leu Thr Val Leu
               100                 105                 110
<210> 98
   <211> 110
   <212> PRT
   <213> Artificial Sequence
<220>
   <223> light chain variable region
<400> 98
   Asn Phe Met Leu Thr Gln Pro His Ser Val Ser Glu Ser Pro Gly Lys
   1               5                   10                  15
   Thr Val Ser Ile Ser Cys Thr Arg Asn Ser Gly Ser Ile Ala Ser Asn
               20                  25                  30
   Phe Val Gln Trp Tyr Gln Gln Arg Pro Gly Ser Ala Pro Thr Ile Val
           35                  40                  45
   Ile Tyr Glu Asp Asn Gln Arg Pro Ser Ala Val Pro Thr Arg Phe Ser
       50                  55                  60
   Gly Ser Ile Asp Arg Ser Ser Asn Ser Ala Ser Leu Thr Ile Ser Gly
   65                  70                  75                  80
   Leu Thr Thr Glu Asp Glu Ala Asp Tyr Tyr Cys Gln Ser Tyr Asp Ser
                   85                  90                  95
   Ala Asn Val Ile Phe Gly Gly Gly Thr Lys Leu Thr Val Leu
               100                 105                 110
<210> 99
   <211> 324
   <212> DNA
   <213> Artificial Sequence
<220>
   <223> light chain variable region
<220>
   <221> CDS
   <222> (1)..(324)
<400> 99
   gaa acg aca ctc acg cag tct cca ggc acc ctg tct ttg tct cca ggg      48
   Glu Thr Thr Leu Thr Gln Ser Pro Gly Thr Leu Ser Leu Ser Pro Gly
   1               5                   10                  15
   gag aga gcc acc ctc tcc tgc agg gcc agt cag act atc agc agc agc      96
   Glu Arg Ala Thr Leu Ser Cys Arg Ala Ser Gln Thr Ile Ser Ser Ser
               20                  25                  30
   cac tta gcc tgg tac cag cag aaa cct ggc cag tct ccc agg ctc ctc     144
   His Leu Ala Trp Tyr Gln Gln Lys Pro Gly Gln Ser Pro Arg Leu Leu
           35                  40                  45
   atc tat ggt gcg ggc tac agg gcc acc ggc att cca gac agg ttc agt     192
   Ile Tyr Gly Ala Gly Tyr Arg Ala Thr Gly Ile Pro Asp Arg Phe Ser
       50                  55                  60
   ggc agt ggg tct ggc aca gac ttc act ctc acc atc agc aga ctg gag     240
   Gly Ser Gly Ser Gly Thr Asp Phe Thr Leu Thr Ile Ser Arg Leu Glu
   65                  70                  75                  80
   cct gaa gat ttt gca gtg tat tac tgt cag cac tat ggt agt tca ctc     288
   Pro Glu Asp Phe Ala Val Tyr Tyr Cys Gln His Tyr Gly Ser Ser Leu
                   85                  90                  95
   cgg acg ttc ggc caa ggg acc aag gtg gaa atc aaa                     324
   Arg Thr Phe Gly Gln Gly Thr Lys Val Glu Ile Lys
               100                 105
<210> 100
   <211> 108
   <212> PRT
   <213> Artificial Sequence
<220>
   <223> light chain variable region
<400> 100
   Glu Thr Thr Leu Thr Gln Ser Pro Gly Thr Leu Ser Leu Ser Pro Gly
   1               5                   10                  15
   Glu Arg Ala Thr Leu Ser Cys Arg Ala Ser Gln Thr Ile Ser Ser Ser
               20                  25                  30
   His Leu Ala Trp Tyr Gln Gln Lys Pro Gly Gln Ser Pro Arg Leu Leu
           35                  40                  45
   Ile Tyr Gly Ala Gly Tyr Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln His Tyr Gly Ser Ser Leu
                   85                  90                  95
   Arg Thr Phe Gly Gln Gly Thr Lys Val Glu Ile Lys
               100                 105
<210> 101
   <211> 330
   <212> DNA
   <213> Artificial Sequence
<220>
   <223> light chain variable region
<220>
   <221> CDS
   <222> (1)..(330)
<400> 101
   aat ttt atg ctg act cag ccc cac tct gtg tcg gag tct ccg ggg aag         48
   Asn Phe Met Leu Thr Gln Pro His Ser Val Ser Glu Ser Pro Gly Lys
   1               5                   10                  15
   acg gta acc atc tcc tgc acc ggc agc ggt ggc aac att gcc agc aat         96
   Thr Val Thr Ile Ser Cys Thr Gly Ser Gly Gly Asn Ile Ala Ser Asn
               20                  25                  30
   tat gtg cag tgg tac cag cag cgc ccg ggc agg gcc ccc acc act gtg        144
   Tyr Val Gln Trp Tyr Gln Gln Arg Pro Gly Arg Ala Pro Thr Thr Val
           35                  40                  45
   atc tat gag gat aat cga aga ccc tct ggg gtc cct gat cgg ttc tct        192
   Ile Tyr Glu Asp Asn Arg Arg Pro Ser Gly Val Pro Asp Arg Phe Ser
       50                  55                  60
   ggc tcc atc gac agc tcc tcc aac tct gcc tcc ctc acc atc tct gga        240
   Gly Ser Ile Asp Ser Ser Ser Asn Ser Ala Ser Leu Thr Ile Ser Gly
   65                  70                  75                  80
   ctg aag act gaa gac gag gct gac tac tac tgt cag tct tat gat ccc        288
   Leu Lys Thr Glu Asp Glu Ala Asp Tyr Tyr Cys Gln Ser Tyr Asp Pro
                   85                  90                  95
   tac aat cga gtg ttc ggc gga ggg acc aag ctg acc gtc cta                330
   Tyr Asn Arg Val Phe Gly Gly Gly Thr Lys Leu Thr Val Leu
               100                 105                 110
<210> 102
   <211> 110
   <212> PRT
   <213> Artificial Sequence
<220>
   <223> light chain variable region
<400> 102
   Asn Phe Met Leu Thr Gln Pro His Ser Val Ser Glu Ser Pro Gly Lys
   1               5                   10                  15
   Thr Val Thr Ile Ser Cys Thr Gly Ser Gly Gly Asn Ile Ala Ser Asn
               20                  25                  30
   Tyr Val Gln Trp Tyr Gln Gln Arg Pro Gly Arg Ala Pro Thr Thr Val
           35                  40                  45
   Ile Tyr Glu Asp Asn Arg Arg Pro Ser Gly Val Pro Asp Arg Phe Ser
       50                  55                  60
   Gly Ser Ile Asp Ser Ser Ser Asn Ser Ala Ser Leu Thr Ile Ser Gly
   65                  70                  75                  80
   Leu Lys Thr Glu Asp Glu Ala Asp Tyr Tyr Cys Gln Ser Tyr Asp Pro
                   85                  90                  95
   Tyr Asn Arg Val Phe Gly Gly Gly Thr Lys Leu Thr Val Leu
               100                 105                 110
<210> 103
   <211> 336
   <212> DNA
   <213> Artificial Sequence
<220>
   <223> light chain variable region
<220>
   <221> CDS
   <222> (1)..(336)
<400> 103
   gaa att gtg atg acg cag tct cca ctc tcc ctg ccc gtc acc cct gga       48
   Glu Ile Val Met Thr Gln Ser Pro Leu Ser Leu Pro Val Thr Pro Gly
   1               5                   10                  15
   gag ccg gcc tcc atc tcc tgc agg tct agt cag agc ctc ctg cat act       96
   Glu Pro Ala Ser Ile Ser Cys Arg Ser Ser Gln Ser Leu Leu His Thr
               20                  25                  30
   aat gga tac gac tat ttg gat tgg tac ctg cag aag cca ggg cag tct      144
   Asn Gly Tyr Asp Tyr Leu Asp Trp Tyr Leu Gln Lys Pro Gly Gln Ser
           35                  40                  45
   cca cag ctt ctg atc tat ttg ggt tct act cgg gcc tcc ggg gtc cct      192
   Pro Gln Leu Leu Ile Tyr Leu Gly Ser Thr Arg Ala Ser Gly Val Pro
       50                  55                  60
   gac agg ttc agt ggc agt gga tcg ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   ttt caa act ccg ctc act ttc ggc gga ggg acc aag atg gag atc aaa      336
   Phe Gln Thr Pro Leu Thr Phe Gly Gly Gly Thr Lys Met Glu Ile Lys
               100                 105                 110
<210> 104
   <211> 112
   <212> PRT
   <213> Artificial Sequence
<220>
   <223> light chain variable region
<400> 104
   Glu Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Thr
               20                  25                  30
   Asn Gly Tyr Asp Tyr Leu Asp Trp Tyr Leu Gln Lys Pro Gly Gln Ser
           35                  40                  45
   Pro Gln Leu Leu Ile Tyr Leu Gly Ser Thr Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Phe Gln Thr Pro Leu Thr Phe Gly Gly Gly Thr Lys Met Glu Ile Lys
               100                 105                 110
<210> 105
   <211> 351
   <212> DNA
   <213> Artificial Sequence
<220>
   <223> heavy chain variable region
<220>
   <221> CDS
   <222> (1)..(351)
<400> 105
   gag gtg cag ctg gtg gag acc ggc cca gga ctg gtg aag cct tcg ggg       48
   Glu Val Gln Leu Val Glu Thr Gly Pro Gly Leu Val Lys Pro Ser Gly
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga ttt aat tac tat gat agt agt gtc tgg ggc cag gga acc ctg      336
   Ala Arg Phe Asn Tyr Tyr Asp Ser Ser Val Trp Gly Gln Gly Thr Leu
               100                 105                 110
   gtc acc gtc tca agc                                                  351
   Val Thr Val Ser Ser
           115
<210> 106
   <211> 117
   <212> PRT
   <213> Artificial Sequence
<220>
   <223> heavy chain variable region
<400> 106
   Glu Val Gln Leu Val Glu Thr Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Phe Asn Tyr Tyr Asp Ser Ser Val Trp Gly Gln Gly Thr Leu
               100                 105                 110
   Val Thr Val Ser Ser
           115
<210> 107
   <211> 348
   <212> **DNA**
   <213> Artificial Sequence
<220>
   <223> heavy chain variable region
<220>
   <221> CDS
   <222> (1)..(348)
<400> 107
   gag gtg cag ctg gtg gag acc ggc cca gga ctg gtg aag cct tcg ggg        48
   Glu Val Gln Leu Val Glu Thr Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga ggg gtt gag cag att gac tac tgg ggc cag gga acc ctg gtc       336
   Ala Arg Gly Val Glu Gln Ile Asp Tyr Trp Gly Gln Gly Thr Leu Val
               100                 105                 110
   acc gtc tca agc                                                       348
   Thr Val Ser Ser
           115
<210> 108
   <211> 116
   <212> PRT
   <213> Artificial Sequence
<220>
   <223> heavy chain variable region
<400> 108
   Glu Val Gln Leu Val Glu Thr Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Gly Val Glu Gln Ile Asp Tyr Trp Gly Gln Gly Thr Leu Val
               100                 105                 110
   Thr Val Ser Ser
           115
<210> 109
   <211> 354
   <212> DNA
   <213> Artificial Sequence
<220>
   <223> heavy chain variable region
<220>
   <221> CDS
   <222> (1)..(354)
<400> 109
   cag gtg cag ctg cag gag tcg ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg act gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aaa aat tta gca gca ggg gcg gtt gcc tac tgg ggc cag ggc acc       336
   Ala Lys Asn Leu Ala Ala Gly Ala Val Ala Tyr Trp Gly Gln Gly Thr
               100                 105                 110
   ctg gtc acc gtc tca agc                                               354
   Leu Val Thr Val Ser Ser
           115
<210> 110
   <211> 118
   <212> PRT
   <213> Artificial Sequence
<220>
   <223> heavy chain variable region
<400> 110
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
                   20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Lys Asn Leu Ala Ala Gly Ala Val Ala Tyr Trp Gly Gln Gly Thr
               100                 105                 110
   Leu Val Thr Val Ser Ser
           115
<210> 111
   <211> 351
   <212> DNA
   <213> Artificial Sequence
<220>
   <223> heavy chain variable region
<220>
   <221> CDS
   <222> (1)..(351)
<400> 111
   cag gtg cag cta cag cag tgg ggc gca gga ctg ttg aag cct tcg gag      48
   Gln Val Gln Leu Gln Gln Trp Gly Ala Gly Leu Leu Lys Pro Ser Glu
   1               5                   10                  15
   acc ctg tcc ctc acc tgc gct gtc tct ggt ggg tcc ttc agt ggt tac      96
   Thr Leu Ser Leu Thr Cys Ala Val Ser Gly Gly Ser Phe Ser Gly Tyr
               20                  25                  30
   tac tgg agc tgg atc cgt cag ccc cca ggg aag ggg ctg gag tgg att     144
   Tyr Trp Ser Trp Ile Arg Gln Pro Pro Gly Lys Gly Leu Glu Trp Ile
           35                  40                  45
   ggg gaa atc aat cat agt gga agt acc aac tac aac cgg tcc ctc aag     192
   Gly Glu Ile Asn His Ser Gly Ser Thr Asn Tyr Asn Arg Ser Leu Lys
       50                  55                  60
   agt cga gtc acc ata tca gta gac acg tcc aag aac cag ttc tcc ctg     240
   Ser Arg Val Thr Ile Ser Val Asp Thr Ser Lys Asn Gln Phe Ser Leu
   65                  70                  75                  80
   aag ctg agc tct gtg acc gcc gcg gac acg gct gtg tat tac tgt gcg     288
   Lys Leu Ser Ser Val Thr Ala Ala Asp Thr Ala Val Tyr Tyr Cys Ala
                   85                  90                  95
   aga ctt tca tat ggt tcg ggc gtt gac tac tgg ggc cag ggc acc ctg     336
   Arg Leu Ser Tyr Gly Ser Gly Val Asp Tyr Trp Gly Gln Gly Thr Leu
               100                 105                 110
   gtc acc gtc tca agc                                                 351
   Val Thr Val Ser Ser
           115
<210> 112
   <211> 117
   <212> PRT
   <213> Artificial Sequence
<220>
   <223> heavy chain variable region
<400> 112
   Gln Val Gln Leu Gln Gln Trp Gly Ala Gly Leu Leu Lys Pro Ser Glu
   1               5                   10                  15
   Thr Leu Ser Leu Thr Cys Ala Val Ser Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Glu Ile Asn His Ser Gly Ser Thr Asn Tyr Asn Arg Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Leu Ser Tyr Gly Ser Gly Val Asp Tyr Trp Gly Gln Gly Thr Leu
               100                 105                 110
   Val Thr Val Ser Ser
           115
<210> 113
   <211> 360
   <212> DNA
   <213> Artificial Sequence
<220>
   <223> heavy chain variable region
<220>
   <221> CDS
   <222> (1)..(360)
<400> 113
   cag ctg cag ctg cag gag tcg ggc cca gga ctg gtg aag cct tca cag        48
   Gln Leu Gln Leu Gln Glu Ser Gly Pro Gly Leu Val Lys Pro Ser Gln
   1               5                   10                  15
   acc ctg tcc ctc acc tgc act gtc tct ggt ggc tcc atc agc agt agt        96
   Thr Leu Ser Leu Thr Cys Thr Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg agg tat agc agc agc cgc aat gat gct ttt gat atc tgg ggc caa       336
   Ala Arg Tyr Ser Ser Ser Arg Asn Asp Ala Phe Asp Ile Trp Gly Gln
               100                 105                 110
   ggg aca atg gtc acc gtc tca agc                                       360
   Gly Thr Met Val Thr Val Ser Ser
           115                 120
<210> 114
   <211> 120
   <212> PRT
   <213> Artificial Sequence
<220>
   <223> heavy chain variable region
<400> 114
   Gln Leu Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Tyr Ser Ser Ser Arg Asn Asp Ala Phe Asp Ile Trp Gly Gln
               100                 105                 110
   Gly Thr Met Val Thr Val Ser Ser
           115                 120
<210> 115
   <211> 354
   <212> DNA
   <213> Artificial Sequence
<220>
   <223> heavy chain variable region
<220>
   <221> CDS
   <222> (1)..(354)
<400> 115
   cag gtg cag ctg cag gag tcg ggc cca gga ctg gtg aag cct tcg ggg       48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga gat ggg cag ctg gat gct ttt gat atc tgg ggc caa ggg aca      336
   Ala Arg Asp Gly Gln Leu Asp Ala Phe Asp Ile Trp Gly Gln Gly Thr
               100                 105                 110
   atg gtc acc gtc tca agc                                              354
   Met Val Thr Val Ser Ser
           115
<210> 116
   <211> 118
   <212> PRT
   <213> Artificial Sequence
<220>
   <223> heavy chain variable region
<400> 116
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Asp Gly Gln Leu Asp Ala Phe Asp Ile Trp Gly Gln Gly Thr
               100                 105                 110
   Met Val Thr Val Ser Ser
           115
<210> 117
   <211> 354
   <212> DNA
   <213> Artificial Sequence
<220>
   <223> heavy chain variable region
<220>
   <221> CDS
   <222> (1)..(354)
<400> 117
   cag gtg cag ctg cag gag tcg ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga ttt tgg gac tac tac ggt atg gac gtc tgg ggc caa ggg acc       336
   Ala Arg Phe Trp Asp Tyr Tyr Gly Met Asp Val Trp Gly Gln Gly Thr
               100                 105                 110
   acg gtc acc gtc tca agc                                               354
   Thr Val Thr Val Ser Ser
           115
<210> 118
   <211> 118
   <212> PRT
   <213> Artificial Sequence
<220>
   <223> heavy chain variable region
<400> 118
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Phe Trp Asp Tyr Tyr Gly Met Asp Val Trp Gly Gln Gly Thr
               100                 105                 110
   Thr Val Thr Val Ser Ser
           115
<210> 119
   <211> 351
   <212> DNA
   <213> Artificial Sequence
<220>
   <223> heavy chain variable region
<220>
   <221> CDS
   <222> (1)..(351)
<400> 119
   cag gtg cag cta cag cag tgg ggc cca gga ctg gtg aag cct tcg ggg       48
   Gln Val Gln Leu Gln Gln Trp Gly Pro Gly Leu Val Lys Pro Ser Gly
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   gag agt cga gtc acc ata tca gta gac aag tcc aag aac cag ttc tcc      240
   Glu Ser Arg Val Thr Ile Ser Val Asp Lys Ser Lys Asn Gln Phe Ser
   65                  70                  75                  80
   ctg aag ctg agc tct gtg acc gcc gca gac acg gcc gtg tat tac tgt      288
   Leu Lys Leu Ser Ser Val Thr Ala Ala Asp Thr Ala Val Tyr Tyr Cys
                   85                  90                  95
   gcg aga gat cgg tac tac ggt atg gac gtc tgg ggc caa ggg acc acg      336
   Ala Arg Asp Arg Tyr Tyr Gly Met Asp Val Trp Gly Gln Gly Thr Thr
               100                 105                 110
   gtc acc gtc tca agc                                                  351
   Val Thr Val Ser Ser
           115
<210> 120
   <211> 117
   <212> PRT
   <213> Artificial Sequence
<220>
   <223> heavy chain variable region
<400> 120
   Gln Val Gln Leu Gln Gln Trp Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Glu Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Asp Arg Tyr Tyr Gly Met Asp Val Trp Gly Gln Gly Thr Thr
               100                 105                 110
   Val Thr Val Ser Ser
           115
<210> 121
   <211> 354
   <212> DNA
   <213> Artificial Sequence
<220>
   <223> heavy chain variable region
<220>
   <221> CDS
   <222> (1)..(354)
<400> 121
   gag gtg cag ctg gtc gag tct ggc cca gga ctg gtg aag cct tcg ggg      48
   Glu Val Gln Leu Val Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg     144
   Asn Trp Trp Ser Trp Val Arg Gln Pro Pro Gly Lys Gly Leu Glu Trp
           35                  40                  45
   att ggg tac atc tat tat agt ggg agc acc tac tac aac ccg tcc ctc     192
   Ile Gly Tyr Ile Tyr Tyr Ser Gly Ser Thr Tyr Tyr Asn Pro Ser Leu
       50                  55                  60
   aag agt cga gtc acc atg tca gta gac acg tcc aag aac cag ttc tcc     240
   Lys Ser Arg Val Thr Met Ser Val Asp Thr Ser Lys Asn Gln Phe Ser
   65                  70                  75                  80
   ctg aag ctg agc tct gtg acc gcc gca gac acg gcc gtg tat tac tgt     288
   Leu Lys Leu Ser Ser Val Thr Ala Ala Asp Thr Ala Val Tyr Tyr Cys
                   85                  90                  95
   gcg aga tgg agc tac ttg gat gct ttt gat atc tgg ggc caa ggg aca     336
   Ala Arg Trp Ser Tyr Leu Asp Ala Phe Asp Ile Trp Gly Gln Gly Thr
               100                 105                 110
   atg gtc acc gtc tca agc                                             354
   Met Val Thr Val Ser Ser
           115
<210> 122
   <211> 118
   <212> PRT
   <213> Artificial Sequence
<220>
   <223> heavy chain variable region
<400> 122
   Glu Val Gln Leu Val Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Tyr Ile Tyr Tyr Ser Gly Ser Thr Tyr Tyr Asn Pro Ser Leu
       50                  55                  60
   Lys Ser Arg Val Thr Met Ser Val Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Trp Ser Tyr Leu Asp Ala Phe Asp Ile Trp Gly Gln Gly Thr
               100                 105                 110
   Met Val Thr Val Ser Ser
           115
<210> 123
   <211> 354
   <212> DNA
   <213> Artificial Sequence
<220>
   <223> heavy chain variable region
<220>
   <221> CDS
   <222> (1)..(354)
<400> 123
   gag gtg cag ctg gtg gag tct ggc cca gga ctg gtg aag cct tcg ggg        48
   Glu Val Gln Leu Val Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga gat tac gat att ttc ggt atg gac gtc tgg ggc caa ggg acc       336
   Ala Arg Asp Tyr Asp Ile Phe Gly Met Asp Val Trp Gly Gln Gly Thr
               100                 105                 110
   acg gtc acc gtc tca agc                                                354
   Thr Val Thr Val Ser Ser
           115
<210> 124
   <211> 118
   <212> PRT
   <213> Artificial Sequence
<220>
   <223> heavy chain variable region
<400> 124
   Glu Val Gln Leu Val Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
   50 55 60
   Lys Ser Arg Val Thr Ile Ser Val Asp Lys Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg Asp Tyr Asp Ile Phe Gly Met Asp Val Trp Gly Gln Gly Thr
   100 105 110
   Thr Val Thr Val Ser Ser
   115
<210> 125
   <211> 354
   <212> DNA
   <213> Artificial Sequence
<220>
   <223> heavy chain variable region
<220>
   <221> CDS
   <222> (1)..(354)
<400> 125
   cag ctg cag ctg cag gag tcg ggc cca gga ctg gtg aag cct tcg ggg      48
   Gln Leu Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg     144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc     192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag tcc tcc     240
   Lys Ser Arg Val Thr Ile Ser Val Asp Lys Ser Lys Asn Gln Ser Ser
   65                  70                  75                  80
   ctg aag ctg agc tct gtg acc gcc gcg gac acg gcc gtg tat tac tgt     288
   Leu Lys Leu Ser Ser Val Thr Ala Ala Asp Thr Ala Val Tyr Tyr Cys
                   85                  90                  95
   gcg aga gcc aac aga gat gat gct ttt gat atc tgg ggc caa ggg aca     336
   Ala Arg Ala Asn Arg Asp Asp Ala Phe Asp Ile Trp Gly Gln Gly Thr
               100                 105                 110
   atg gtc acc gtc tca agc                                             354
                                 Met Val Thr Val Ser Ser
                                         115
<210> 126
   <211> 118
   <212> PRT
   <213> Artificial Sequence
<220>
   <223> heavy chain variable region
<400> 126
   Gln Leu Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Ser Ser
   65                  70                  75                  80
   Leu Lys Leu Ser Ser Val Thr Ala Ala Asp Thr Ala Val Tyr Tyr Cys
                   85                  90                  95
   Ala Arg Ala Asn Arg Asp Asp Ala Phe Asp Ile Trp Gly Gln Gly Thr
               100                 105                 110
   Met Val Thr Val Ser Ser
           115
<210> 127
   <211> 357
   <212> DNA
   <213> Artificial Sequence
<220>
   <223> heavy chain variable region
<220>
   <221> CDS
   <222> (1)..(357)
<400> 127
   gag gtg cag ctg gtg gag tct ggg gga ggc ttg gta cag ccg ggg ggg       48
   Glu Val Gln Leu Val Glu Ser Gly Gly Gly Leu Val Gln Pro Gly Gly
   1               5                   10                  15
   tcc ctg aga ctc tcc tgt gca gcc tct gga ttc acc ttt agc agc tat        96
   Ser Leu Arg Leu Ser Cys Ala Ala Ser Gly Phe Thr Phe Ser Ser Tyr
               20                  25                  30
   gcc atg agc tgg gtc cgc cag gct cca ggg aag ggg ctg gag tgg gtc       144
   Ala Met Ser Trp Val Arg Gln Ala Pro Gly Lys Gly Leu Glu Trp Val
           35                  40                  45
   tca gct att agt ggt agt ggt ggt agc aca tac tac gca gac tcc gtg       192
   Ser Ala Ile Ser Gly Ser Gly Gly Ser Thr Tyr Tyr Ala Asp Ser Val
       50                  55                  60
   aag ggc cgg ttc acc atc tcc aga gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agt ctg agc gcc gac gac acg gcc gta tat ttc tgt       288
   Leu Gln Met Asn Ser Leu Ser Ala Asp Asp Thr Ala Val Tyr Phe Cys
                   85                  90                  95
   gcg tcg ggt ggc tgg tac ggg gac tac ttt gac tac tgg ggc cag gga       336
   Ala Ser Gly Gly Trp Tyr Gly Asp Tyr Phe Asp Tyr Trp Gly Gln Gly
               100                 105                 110
   acc ctg gtc acc gtc tca agc                                           357
   Thr Leu Val Thr Val Ser Ser
           115
<210> 128
   <211> 119
   <212> PRT
   <213> Artificial Sequence
<220>
   <223> heavy chain variable region
<400> 128
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Ser Ala Asp Asp Thr Ala Val Tyr Phe Cys
                   85                  90                  95
   Ala Ser Gly Gly Trp Tyr Gly Asp Tyr Phe Asp Tyr Trp Gly Gln Gly
               100                 105                 110
   Thr Leu Val Thr Val Ser Ser
           115
<210> 129
   <211> 363
   <212> DNA
   <213> Artificial Sequence
<220>
   <223> heavy chain variable region
<220>
   <221> CDS
   <222> (1)..(363)
<400> 129
   cag gtg cag ctg cag gag tcc ggc cca gga ctg gtg aag cct tcg gag       48
   Gln Val Gln Leu Gln Glu Ser Gly Pro Gly Leu Val Lys Pro Ser Glu
   1               5                   10                  15
   acc ctg tcc ctc acc tgc act gtc tct ggt ggc tcc atc agc agt agt       96
   Thr Leu Ser Leu Thr Cys Thr Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga gaa ggg aac cga acg gtg act agt gct ttt gat atc tgg ggc      336
   Ala Arg Glu Gly Asn Arg Thr Val Thr Ser Ala Phe Asp Ile Trp Gly
               100                 105                 110
   caa ggg aca atg gtc acc gtc tca agc                                  363
   Gln Gly Thr Met Val Thr Val Ser Ser
           115                 120
<210> 130
   <211> 121
   <212> PRT
   <213> Artificial Sequence
<220>
   <223> heavy chain variable region
<400> 130
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Glu Gly Asn Arg Thr Val Thr Ser Ala Phe Asp Ile Trp Gly
               100                 105                 110
   Gln Gly Thr Met Val Thr Val Ser Ser
           115                 120
<210> 131
   <211> 357
   <212> DNA
   <213> Artificial Sequence
<220>
   <223> heavy chain variable region
<220>
   <221> CDS
   <222> (1)..(357)
<400> 131
   cag gtg cag ctg cag gag tcc ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg        144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc        192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc        240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gtg tac tac tgt        288
   Leu Lys Leu Ser Ser Val Thr Ala Ala Asp Thr Ala Val Tyr Tyr Cys
                   85                  90                  95
   gcg aga ggg ctg ggg gat agt agt ggt tat atc ctt tgg ggc caa ggg        336
   Ala Arg Gly Leu Gly Asp Ser Ser Gly Tyr Ile Leu Trp Gly Gln Gly
               100                 105                 110
   aca atg gtc acc gtc tca agc                                            357
   Thr Met Val Thr Val Ser Ser
           115
<210> 132
   <211> 119
   <212> PRT
   <213> Artificial Sequence
<220>
   <223> heavy chain variable region
<400> 132
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Gly Leu Gly Asp Ser Ser Gly Tyr Ile Leu Trp Gly Gln Gly
               100                 105                 110
   Thr Met Val Thr Val Ser Ser
           115
<210> 133
   <211> 357
   <212> DNA
   <213> Artificial Sequence
<220>
   <223> heavy chain variable region
<220>
   <221> CDS
   <222> (1)..(357)
<400> 133
   cag gtg cag ctg cag gag tcc ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gtg tac tac tgt       288
   Leu Lys Leu Ser Ser Val Thr Ala Ala Asp Thr Ala Val Tyr Tyr Cys
                   85                  90                  95
   gcg aga ggg ctg ggg gat agt agt ggt tat atc ctt tgg ggc caa ggg       336
   Ala Arg Gly Leu Gly Asp Ser Ser Gly Tyr Ile Leu Trp Gly Gln Gly
               100                 105                 110
   aca atg gtc acc gtc tca agc                                           357
   Thr Met Val Thr Val Ser Ser
           115
<210> 134
   <211> 119
   <212> PRT
   <213> Artificial Sequence
<220>
   <223> heavy chain variable region
<400> 134
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Gly Leu Gly Asp Ser Ser Gly Tyr Ile Leu Trp Gly Gln Gly
               100                 105                 110
   Thr Met Val Thr Val Ser Ser
           115
<210> 135
   <211> 357
   <212> DNA
   <213> Artificial Sequence
<220>
   <223> heavy chain variable region
<220>
   <221> CDS
   <222> (1)..(357)
<400> 135
   cag gtg cag ctg cag gag tcg ggc cca gga ctg gtg aag cct tcg ggg        48
   acc ctg tcc ctc acc tgc gct gtc tct ggt ggc tcc atc agc agt agt        96
   aac tgg tgg agt tgg gtc cgc cag ccc cca ggg aag ggg ctg gag tgg       144
   att ggg gaa atc tat cat agt ggg agc acc aac tac aac ccg tcc ctc       192
   aag agt cga gtc acc ata tca gta gac aag tcc aag aac cag ttc tcc       240
   ctg aag ctg agc tct gtg acc gcc gcg gac acg gcc gtg tat tac tgt       288
   gcg aga tgg acc ggg cgt act gat gct ttt gat atc tgg ggc caa ggg       336
   aca atg gtc acc gtc tca agc                                           357
<210> 136
   <211> 119
   <212> PRT
   <213> Artificial Sequence
<220>
   <223> heavy chain variable region
<400> 136
<210> 137
   <211> 354
   <212> DNA
   <213> Artificial Sequence
<220>
   <223> heavy chain variable region
<220>
   <221> CDS
   <222> (1)..(354)
<400> 137
   cag gtg cag ctg cag gag tcc ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga caa ggg gcg tta gat gct ttt gat atc tgg ggc caa ggg acc       336
   Ala Arg Gln Gly Ala Leu Asp Ala Phe Asp Ile Trp Gly Gln Gly Thr
               100                 105                 110
   acg gtc acc gtc tca agc                                               354
   Thr Val Thr Val Ser Ser
           115
<210> 138
   <211> 118
   <212> PRT
   <213> Artificial Sequence
<220>
   <223> heavy chain variable region
<400> 138
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Gln Gly Ala Leu Asp Ala Phe Asp Ile Trp Gly Gln Gly Thr
               100                 105                 110
   Thr Val Thr Val Ser Ser
           115
<210> 139
   <211> 366
   <212> DNA
   <213> Artificial Sequence
<220>
   <223> heavy chain variable region
<220>
   <221> CDS
   <222> (1)..(366)
<400> 139
   cag gtg cag ctg gtg gag tcc ggg gga ggc gtg gtc cga cct ggg ggg       48
   Gln Val Gln Leu Val Glu Ser Gly Gly Gly Val Val Arg Pro Gly Gly
   1               5                   10                  15
   tcc ctg aga ctc tcc tgt gca gcg tct gga ttc acc ttt agc agc tat       96
   Ser Leu Arg Leu Ser Cys Ala Ala Ser Gly Phe Thr Phe Ser Ser Tyr
               20                  25                  30
   gcc atg agc tgg gtc cgc cag gct cca ggg aag ggg ctg gag tgg gtc      144
   Ala Met Ser Trp Val Arg Gln Ala Pro Gly Lys Gly Leu Glu Trp Val
           35                  40                  45
   tca act att agt ggt agt ggt ggt agc aca tac tac gca gac tcc gtg      192
   Ser Thr Ile Ser Gly Ser Gly Gly Ser Thr Tyr Tyr Ala Asp Ser Val
       50                  55                  60
   aag ggc cgg ttc acc atc tcc aga gac aat tcc aag aac acg ctg tat      240
   Lys Gly Arg Phe Thr Ile Ser Arg Asp Asn Ser Lys Asn Thr Leu Tyr
   65                  70                  75                  80
   ctg cag atg aac agc ctg aga gcc gag gac acg gcc gta tat tac tgt      288
   Leu Gln Met Asn Ser Leu Arg Ala Glu Asp Thr Ala Val Tyr Tyr Cys
                   85                  90                  95
   gcg aaa gag cgt ggc agt ggc tgg tcc tta gac aat atg gac gtc tgg      336
   Ala Lys Glu Arg Gly Ser Gly Trp Ser Leu Asp Asn Met Asp Val Trp
               100                 105                 110
   ggc caa ggg acc acg gtc acc gtc tca agc                              366
   Gly Gln Gly Thr Thr Val Thr Val Ser Ser
           115                 120
<210> 140
   <211> 122
   <212> PRT
   <213> Artificial Sequence
<220>
   <223> heavy chain variable region
<400> 140
   Gln Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Thr Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Arg Gly Ser Gly Trp Ser Leu Asp Asn Met Asp Val Trp
               100                 105                 110
   Gly Gln Gly Thr Thr Val Thr Val Ser Ser
           115                 120
<210> 141
   <211> 357
   <212> DNA
   <213> Artificial Sequence
<220>
   <223> heavy chain variable region
<220>
   <221> CDS
   <222> (1)..(357)
<400> 141
   cag gtg cag ctg gtg gag tct ggc cca gga ctg gtg aag cct tcg ggg      48
   Gln Val Gln Leu Val Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg     144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc     192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc     240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gtg tat tac tgt     288
   Leu Lys Leu Ser Ser Val Thr Ala Ala Asp Thr Ala Val Tyr Tyr Cys
                   85                  90                  95
   gcg aga gat agc agt ggg ttc tac ggt atg gac gtc tgg ggc caa ggg     336
   Ala Arg Asp Ser Ser Gly Phe Tyr Gly Met Asp Val Trp Gly Gln Gly
               100                 105                 110
   acc acg gtc acc gtc tca agc                                         357
   Thr Thr Val Thr Val Ser Ser
           115
<210> 142
   <211> 119
   <212> PRT
   <213> Artificial Sequence
<220>
   <223> heavy chain variable region
<400> 142
   Gln Val Gln Leu Val Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Asp Ser Ser Gly Phe Tyr Gly Met Asp Val Trp Gly Gln Gly
               100                 105                 110
   Thr Thr Val Thr Val Ser Ser
           115
<210> 143
   <211> 360
   <212> DNA
   <213> Artificial Sequence
<220>
   <223> heavy chain variable region
<220>
   <221> CDS
   <222> (1)..(360)
<400> 143
   cag gtg cag ctg cag gag tcg ggc cca gga ctg gtg aag cct tcg ggg       48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg act gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga agc agc agc tgg tac tgg aat gct ttt gat atc tgg ggc caa      336
   Ala Arg Ser Ser Ser Trp Tyr Trp Asn Ala Phe Asp Ile Trp Gly Gln
               100                 105                 110
   ggg aca atg gtc acc gtc tca agc                                      360
   Gly Thr Met Val Thr Val Ser Ser
           115                 120
<210> 144
   <211> 120
   <212> PRT
   <213> Artificial Sequence
<220>
   <223> heavy chain variable region
<400> 144
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Ser Ser Ser Trp Tyr Trp Asn Ala Phe Asp Ile Trp Gly Gln
               100                 105                 110
   Gly Thr Met Val Thr Val Ser Ser
           115                 120
<210> 145
   <211> 351
   <212> DNA
   <213> Artificial Sequence
<220>
   <223> heavy chain variable region
<220>
   <221> CDS
   <222> (1)..(351)
<400> 145
   cag gtg cag cta cag cag tgg ggc cca gca ctg gtg aag cct tcg ggg         48
   Gln Val Gln Leu Gln Gln Trp Gly Pro Ala Leu Val Lys Pro Ser Gly
   1               5                   10                  15
   acc ctg tcc ctc acc tgc tct gtc tct ggt gtc tcc atc acc agt aat         96
   Thr Leu Ser Leu Thr Cys Ser Val Ser Gly Val Ser Ile Thr Ser Asn
               20                  25                  30
   atc tgg tgg agt tgg gtc cgc cag tcc cca ggg aag ggg ctg gag tgg        144
   Ile Trp Trp Ser Trp Val Arg Gln Ser Pro Gly Lys Gly Leu Glu Trp
           35                  40                  45
   att ggg gaa gtc tat cat agt ggg agc acc aac tac aac ccg tcc ctc        192
   Ile Gly Glu Val Tyr His Ser Gly Ser Thr Asn Tyr Asn Pro Ser Leu
       50                  55                  60
   aag agt cga gtc acc ata tca gta gac aag tcc aag aac cag ttc tcc        240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gct gtg tat tac tgt        288
   Leu Lys Leu Ser Ser Val Thr Ala Ala Asp Thr Ala Val Tyr Tyr Cys
                   85                  90                  95
   gcg ggg tac cgt agc ttc ggg gag tcc tac tgg ggc cag gga acc ctg        336
   Ala Gly Tyr Arg Ser Phe Gly Glu Ser Tyr Trp Gly Gln Gly Thr Leu
               100                 105                 110
   gtc acc gtc tca agc                                                    351
   Val Thr Val Ser Ser
           115
<210> 146
   <211> 117
   <212> PRT
   <213> Artificial Sequence
<220>
   <223> heavy chain variable region
<400> 146
   Gln Val Gln Leu Gln Gln Trp Gly Pro Ala Leu Val Lys Pro Ser Gly
   1               5                   10                  15
   Thr Leu Ser Leu Thr Cys Ser Val Ser Gly Val Ser Ile Thr Ser Asn
               20                  25                  30
   Ile Trp Trp Ser Trp Val Arg Gln Ser Pro Gly Lys Gly Leu Glu Trp
           35                  40                  45
   Ile Gly Glu Val Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Gly Tyr Arg Ser Phe Gly Glu Ser Tyr Trp Gly Gln Gly Thr Leu
               100                 105                 110
   Val Thr Val Ser Ser
           115
<210> 147
   <211> 366
   <212> DNA
   <213> Artificial Sequence
<220>
   <223> heavy chain variable region
<220>
   <221> CDS
   <222> (1)..(366)
<400> 147
   cag gtg cag cta cag cag tgg ggc gca ggg ctg ttg aag cct tcg gag        48
   Gln Val Gln Leu Gln Gln Trp Gly Ala Gly Leu Leu Lys Pro Ser Glu
   1               5                   10                  15
   acc ctg tct ctc acc tgc gtt gtc tat ggt ggg tcc ttc agc gat ttc        96
   Thr Leu Ser Leu Thr Cys Val Val Tyr Gly Gly Ser Phe Ser Asp Phe
               20                  25                  30
   tac tgg agc tgg atc cgc cag ccc cca ggg aag ggg cca gag tgg att       144
   Tyr Trp Ser Trp Ile Arg Gln Pro Pro Gly Lys Gly Pro Glu Trp Ile
           35                  40                  45
   ggg gaa gtc aat cct aga gga agc acc aac tac aac ccg tcc ctc aag       192
   Gly Glu Val Asn Pro Arg Gly Ser Thr Asn Tyr Asn Pro Ser Leu Lys
       50                  55                  60
   agt cga gcc acc ata tca cta gac acg tcc aag aac cag ttc tcc ctg       240
   Ser Arg Ala Thr Ile Ser Leu Asp Thr Ser Lys Asn Gln Phe Ser Leu
   65                  70                  75                  80
   aag ctg agt tct gtg acc gcc gcg gac acg gct gtg tat ttc tgt gcg       288
   Lys Leu Ser Ser Val Thr Ala Ala Asp Thr Ala Val Tyr Phe Cys Ala
                   85                  90                  95
   aga ggt cct cgg ccc ggg aga gat ggc tac aat tac ttt gac aac tgg       336
   Arg Gly Pro Arg Pro Gly Arg Asp Gly Tyr Asn Tyr Phe Asp Asn Trp
               100                 105                 110
   ggc cag ggc acc ctg gtc acc gtc tca agc                               366
   Gly Gln Gly Thr Leu Val Thr Val Ser Ser
           115                 120
<210> 148
   <211> 122
   <212> **PRT**
   <213> Artificial Sequence
<220>
   <223> heavy chain variable region
<400> 148
   Gln Val Gln Leu Gln Gln Trp Gly Ala Gly Leu Leu Lys Pro Ser Glu
   1               5                   10                  15
   Thr Leu Ser Leu Thr Cys Val Val Tyr Gly Gly Ser Phe Ser Asp Phe
               20                  25                  30
   Tyr Trp Ser Trp Ile Arg Gln Pro Pro Gly Lys Gly Pro Glu Trp Ile
           35                  40                  45
   Gly Glu Val Asn Pro Arg Gly Ser Thr Asn Tyr Asn Pro Ser Leu Lys
       50                  55                  60
   Ser Arg Ala Thr Ile Ser Leu Asp Thr Ser Lys Asn Gln Phe Ser Leu
   65                  70                  75                  80
   Lys Leu Ser Ser Val Thr Ala Ala Asp Thr Ala Val Tyr Phe Cys Ala
                   85                  90                  95
   Arg Gly Pro Arg Pro Gly Arg Asp Gly Tyr Asn Tyr Phe Asp Asn Trp
               100                 105                 110
   Gly Gln Gly Thr Leu Val Thr Val Ser Ser
           115                 120
<210> 149
   <211> 357
   <212> DNA
   <213> Artificial Sequence
<220>
   <223> heavy chain variable region
<220>
   <221> CDS
   <222> (1)..(357)
<400> 149
   cag gtg cag ctg cag gag tcg ggc cca gga ctg gtg aag cct tcg gag       48
   Gln Val Gln Leu Gln Glu Ser Gly Pro Gly Leu Val Lys Pro Ser Glu
   1               5                   10                  15
   acc ctg tcc ctc acc tgc act gtc tct ggt ggc tcc atc agc agt agt       96
   Thr Leu Ser Leu Thr Cys Thr Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga ggt ata gca gca gct ggt caa ggt gac tac tgg ggc cag gga      336
   Ala Arg Gly Ile Ala Ala Ala Gly Gln Gly Asp Tyr Trp Gly Gln Gly
               100                 105                 110
   acc ctg gtc acc gtc tca agc                                          357
   Thr Leu Val Thr Val Ser Ser
           115
<210> 150
   <211> 119
   <212> PRT
   <213> Artificial Sequence
<220>
   <223> heavy chain variable region
<400> 150
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Gly Ile Ala Ala Ala Gly Gln Gly Asp Tyr Trp Gly Gln Gly
               100                 105                 110
   Thr Leu Val Thr Val Ser Ser
           115
<210> 151
   <211> 363
   <212> DNA
   <213> Artificial Sequence
<220>
   <223> heavy chain variable region
<220>
   <221> CDS
   <222> (1)..(363)
<400> 151
   cag gtg cag ctg cag gag tcg ggc cca gga ctg gtg aag cct tcg gag      48
   Gln Val Gln Leu Gln Glu Ser Gly Pro Gly Leu Val Lys Pro Ser Glu
   1               5                   10                  15
   acc ctg tcc ctc acc tgc act gtc tct ggt ggc tcc atc agc agt agt      96
   Thr Leu Ser Leu Thr Cys Thr Val Ser Gly Gly Ser Ile Ser Ser Ser
               20                  25                  30
   agt tac tac tgg ggc tgg atc cgc cag ccc cca ggg aag ggg ctg gag      144
   Ser Tyr Tyr Trp Gly Trp Ile Arg Gln Pro Pro Gly Lys Gly Leu Glu
           35                  40                  45
   tgg att ggg agt atc tat tat agt ggg agc acc tac tac aac ccg tcc      192
   Trp Ile Gly Ser Ile Tyr Tyr Ser Gly Ser Thr Tyr Tyr Asn Pro Ser
       50                  55                  60
   ctc aag agt cga gtc acc ata tcc gta gac acg tcc aag aac cag ttc      240
   Leu Lys Ser Arg Val Thr Ile Ser Val Asp Thr Ser Lys Asn Gln Phe
   65                  70                  75                  80
   tcc ctg aag ctg agc tct gtg acc gcc gcg gac acg gcc gtg tat tac      288
   Ser Leu Lys Leu Ser Ser Val Thr Ala Ala Asp Thr Ala Val Tyr Tyr
                   85                  90                  95
   tgt gcg aga gat ggg gga tac tac tac tac ggt atg gac gtc tgg ggc      336
   Cys Ala Arg Asp Gly Gly Tyr Tyr Tyr Tyr Gly Met Asp Val Trp Gly
               100                 105                 110
   caa ggg acc acg gtc acc gtc tca agc                                  363
   Gln Gly Thr Thr Val Thr Val Ser Ser
           115                 120
<210> 152
   <211> 121
   <212> PRT
   <213> Artificial Sequence
<220>
   <223> heavy chain variable region
<400> 152
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Gly Trp Ile Arg Gln Pro Pro Gly Lys Gly Leu Glu
           35                  40                  45
   Trp Ile Gly Ser Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Gly Gly Tyr Tyr Tyr Tyr Gly Met Asp Val Trp Gly
               100                 105                 110
   Gln Gly Thr Thr Val Thr Val Ser Ser
           115                 120
<210> 153
   <211> 351
   <212> DNA
   <213> Artificial Sequence
<220>
   <223> heavy chain variable region
<220>
   <221> CDS
   <222> (1)..(351)
<400> 153
   cag gtg cag ctg cag gag tcg ggc cca gga ctg gtg aag cct tcg ggg      48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg     144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc     192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc     240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt     288
   Leu Lys Leu Ser Ser Val Thr Ala Ala Asp Thr Ala Val Tyr Tyr Cys
                   85                  90                  95
   gcg agt agt ggt tat gat gct ttt gat atc tgg ggc caa ggg acc acg     336
   Ala Ser Ser Gly Tyr Asp Ala Phe Asp Ile Trp Gly Gln Gly Thr Thr
               100                 105                 110
   gtc acc gtc tca agc                                                 351
   Val Thr Val Ser Ser
           115
<210> 154
   <211> 117
   <212> PRT
   <213> Artificial Sequence
<220>
   <223> heavy chain variable region
<400> 154
   Gln Val Gln Leu Gln Glu Ser Gly Pro Gly Leu Val Lys Pro Ser Gly
   1 5 10 15
   Thr Leu Ser Leu Thr Cys Ala Val Ser Gly Gly Ser Ile Ser Ser Ser
   20 25 30
   Asn Trp Trp Ser Trp Val Arg Gln Pro Pro Gly Lys Gly Leu Glu Trp
   35 40 45
   Ile Gly Glu Ile Tyr His Ser Gly Ser Thr Asn Tyr Asn Pro Ser Leu
   50 55 60
   Lys Ser Arg Val Thr Ile Ser Val Asp Lys Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Ser Ser Gly Tyr Asp Ala Phe Asp Ile Trp Gly Gln Gly Thr Thr
   100 105 110
   Val Thr Val Ser Ser
   115
<210> 155
   <211> 357
   <212> DNA
   <213> Artificial Sequence
<220>
   <223> heavy chain variable region
<220>
   <221> CDS
   <222> (1)..(357)
<400> 155
   cag gtg cag ctg cag gag tcg ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aat tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gca cga tac agc tat gga acg gta gga att gac tac tgg ggc cag gga       336
   Ala Arg Tyr Ser Tyr Gly Thr Val Gly Ile Asp Tyr Trp Gly Gln Gly
               100                 105                 110
   acc ctg gtc acc gtc tca agc                                           357
   Thr Leu Val Thr Val Ser Ser
           115
<210> 156
   <211> 119
   <212> PRT
   <213> Artificial Sequence
<220>
   <223> heavy chain variable region
<400> 156
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Tyr Ser Tyr Gly Thr Val Gly Ile Asp Tyr Trp Gly Gln Gly
               100                 105                 110
   Thr Leu Val Thr Val Ser Ser
           115
<210> 157
   <211> 351
   <212> DNA
   <213> Artificial Sequence
<220>
   <223> heavy chain variable region
<220>
   <221> CDS
   <222> (1)..(351)
   <223> heavy chain variable region
<400> 157
   gag gtg cag ctg gtg cag tct ggg gga ggc gtg gtc cag cct ggg acg       48
   Glu Val Gln Leu Val Gln Ser Gly Gly Gly Val Val Gln Pro Gly Thr
   1               5                   10                  15
   tcc ctg aga ctc tcc tgt gca gcc tct gga ttc agc ttc aga agt cat       96
   Ser Leu Arg Leu Ser Cys Ala Ala Ser Gly Phe Ser Phe Arg Ser His
               20                  25                  30
   ggc atg cac tgg gtc cgc cag gct cca ggc aag ggg ctg gag tgg gtg      144
   Gly Met His Trp Val Arg Gln Ala Pro Gly Lys Gly Leu Glu Trp Val
           35                  40                  45
   gca gtt ata tca tat gat gga agt aat aaa tac tat gca gac tcc gtg      192
   Ala Val Ile Ser Tyr Asp Gly Ser Asn Lys Tyr Tyr Ala Asp Ser Val
       50                  55                  60
   aag ggc cga ttc acc atc tcc aga gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg aga gct gag gac acg gct gtg tat tac tgt      288
   Leu Gln Met Asn Ser Leu Arg Ala Glu Asp Thr Ala Val Tyr Tyr Cys
                   85                  90                  95
   gcg act ata ggg ccg ggg gga ttt gac tac tgg ggc cag ggc acc ctg      336
   Ala Thr Ile Gly Pro Gly Gly Phe Asp Tyr Trp Gly Gln Gly Thr Leu
               100                 105                 110
   gtc acc gtc tca agc                                                  351
   Val Thr Val Ser Ser
           115
<210> 158
   <211> 117
   <212> PRT
   <213> Artificial Sequence
<220>
   <223> heavy chain variable region
<400> 158
   Glu Val Gln Leu Val Gln Ser Gly Gly Gly Val Val Gln Pro Gly Thr
   1               5                   10                  15
   Ser Leu Arg Leu Ser Cys Ala Ala Ser Gly Phe Ser Phe Arg Ser His
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Thr Ile Gly Pro Gly Gly Phe Asp Tyr Trp Gly Gln Gly Thr Leu
               100                 105                 110
   Val Thr Val Ser Ser
           115
<210> 159
   <211> 357
   <212> DNA
   <213> Artificial Sequence
<220>
   <223> heavy chain variable region
<220>
   <221> CDS
   <222> (1)..(357)
<400> 159
   cag gtg cag ctg cag gag tcc ggc cca gga ctg gtg aag cct tcg gag      48
   Gln Val Gln Leu Gln Glu Ser Gly Pro Gly Leu Val Lys Pro Ser Glu
   1               5                   10                  15
   acc ctg tcc ctc acc tgc act gtc tct ggt ggc tcc att aga aat tac      96
   Thr Leu Ser Leu Thr Cys Thr Val Ser Gly Gly Ser Ile Arg Asn Tyr
               20                  25                  30
   tac tgg agt tgg atc cgg cag ccc cca ggg aag gga ctg gag tgg att     144
   Tyr Trp Ser Trp Ile Arg Gln Pro Pro Gly Lys Gly Leu Glu Trp Ile
           35                  40                  45
   ggg tat att tct gac agt ggg aat acc aac tac aat ccc tcc ctc aag     192
   Gly Tyr Ile Ser Asp Ser Gly Asn Thr Asn Tyr Asn Pro Ser Leu Lys
       50                  55                  60
   agt cga gtc acc ata tca gta gac acg tcc aag aac cag ttc tcc cta     240
   Ser Arg Val Thr Ile Ser Val Asp Thr Ser Lys Asn Gln Phe Ser Leu
   65                  70                  75                  80
   aag ctg acc tct gtg acc gcc aca gac acg gct gcg tat ttc tgt gcg     288
   Lys Leu Thr Ser Val Thr Ala Thr Asp Thr Ala Ala Tyr Phe Cys Ala
                   85                  90                  95
   aga cat cga agc agc tgg gca tgg tac ttc gat ctc tgg ggc cgt ggc     336
   Arg His Arg Ser Ser Trp Ala Trp Tyr Phe Asp Leu Trp Gly Arg Gly
               100                 105                 110
   acc ctg gtc acc gtc tca agc                                        357
   Thr Leu Val Thr Val Ser Ser
           115
<210> 160
   <211> 119
   <212> PRT
   <213> Artificial Sequence
<220>
   <223> heavy chain variable region
<400> 160
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Arg Asn Tyr
               20                  25                  30
   Tyr Trp Ser Trp Ile Arg Gln Pro Pro Gly Lys Gly Leu Glu Trp Ile
           35                  40                  45
   Gly Tyr Ile Ser Asp Ser Gly Asn Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Thr Ser Val Thr Ala Thr Asp Thr Ala Ala Tyr Phe Cys Ala
                   85                  90                  95
   Arg His Arg Ser Ser Trp Ala Trp Tyr Phe Asp Leu Trp Gly Arg Gly
               100                 105                 110
   Thr Leu Val Thr Val Ser Ser
           115
<210> 161
   <211> 354
   <212> DNA
   <213> Artificial Sequence
<220>
   <223> heavy chain variable region
<220>
   <221> CDS
   <222> (1)..(354)
<400> 161
   cag gtg cag ctg cag gag tcg ggc cca gga ctg gtg aag cct tcg gag        48
   Gln Val Gln Leu Gln Glu Ser Gly Pro Gly Leu Val Lys Pro Ser Glu
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga gtg ggc agt ggc tgg tac gtt gac tac tgg ggc cag gga acc       336
   Ala Arg Val Gly Ser Gly Trp Tyr Val Asp Tyr Trp Gly Gln Gly Thr
               100                 105                 110
   ctg gtc acc gtc tca agc                                               354
   Leu Val Thr Val Ser Ser
           115
<210> 162
   <211> 118
   <212> PRT
   <213> Artificial Sequence
<220>
   <223> heavy chain variable region
<400> 162
   Gln Val Gln Leu Gln Glu Ser Gly Pro Gly Leu Val Lys Pro Ser Glu
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Val Gly Ser Gly Trp Tyr Val Asp Tyr Trp Gly Gln Gly Thr
               100                 105                 110
   Leu Val Thr Val Ser Ser
           115
<210> 163
   <211> 360
   <212> DNA
   <213> Artificial Sequence
<220>
   <223> heavy chain variable region
<220>
   <221> CDS
   <222> (1)..(360)
<400> 163
   cag gtg cag ctg cag gag tcc ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga gtt tct ggc tac tac tac tac ggt atg gac gtc tgg ggc caa       336
   Ala Arg Val Ser Gly Tyr Tyr Tyr Tyr Gly Met Asp Val Trp Gly Gln
               100                 105                 110
   ggg acc acg gtc acc gtc tca agc                                       360
   Gly Thr Thr Val Thr Val Ser Ser
           115                 120
<210> 164
   <211> 120
   <212> **PRT**
   <213> **Artificial** Sequence
<220>
   <223> heavy chain variable region
<400> 164
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Val Ser Gly Tyr Tyr Tyr Tyr Gly Met Asp Val Trp Gly Gln
               100                 105                 110
   Gly Thr Thr Val Thr Val Ser Ser
           115                 120
<210> 165
   <211> 369
   <212> DNA
   <213> Artificial Sequence
<220>
   <223> heavy chain variable region
<220>
   <221> CDS
   <222> (1)..(369)
<400> 165
   gag gtc cag ctg gta cag tct ggg gga ggc gtg gtc cag cct ggg agg      48
   Glu Val Gln Leu Val Gln Ser Gly Gly Gly Val Val Gln Pro Gly Arg
   1               5                   10                  15
   tcc ctg aga ctc tcc tgt gca gcc tct gga ttc acc ttc agt agc tat      96
   Ser Leu Arg Leu Ser Cys Ala Ala Ser Gly Phe Thr Phe Ser Ser Tyr
               20                  25                  30
   ggc atg cac tgg gtc cgc cag gct cca ggc aag ggg ctg gag tgg gtg     144
   Gly Met His Trp Val Arg Gln Ala Pro Gly Lys Gly Leu Glu Trp Val
           35                  40                  45
   gca gtt ata tca tat gat gga agt aat aaa tac tat gca gac tcc gtg       192
   Ala Val Ile Ser Tyr Asp Gly Ser Asn Lys Tyr Tyr Ala Asp Ser Val
       50                  55                  60
   aag ggc cga ttc acc atc tcc aga gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg aga gct gag gac acg gct gtg tat tac tgt       288
   Leu Gln Met Asn Ser Leu Arg Ala Glu Asp Thr Ala Val Tyr Tyr Cys
                   85                  90                  95
   gcg aaa gcg tat agc agt ggc tgg tac gac tac tac ggt atg gac gtc       336
   Ala Lys Ala Tyr Ser Ser Gly Trp Tyr Asp Tyr Tyr Gly Met Asp Val
               100                 105                 110
   tgg ggc caa ggg acc acg gtc acc gtc tca agc                           369
   Trp Gly Gln Gly Thr Thr Val Thr Val Ser Ser
           115                 120
<210> 166
   <211> 123
   <212> PRT
   <213> Artificial Sequence
<220>
   <223> heavy chain variable region
<400> 166
   Glu Val Gln Leu Val Gln Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ala Tyr Ser Ser Gly Trp Tyr Asp Tyr Tyr Gly Met Asp Val
               100                 105                 110
   Trp Gly Gln Gly Thr Thr Val Thr Val Ser Ser
           115                 120
<210> 167
   <211> 351
   <212> DNA
   <213> Artificial Sequence
<220>
   <223> heavy chain variable region
<220>
   <221> CDS
   <222> (1)..(351)
<400> 167
   cag gtg cag ctg cag gag tcg ggc cca gga ctg gtg aag cct tcg ggg      48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga gcc agc gtt gat gct ttt gat atc tgg ggc caa ggg aca atg      336
   Ala Arg Ala Ser Val Asp Ala Phe Asp Ile Trp Gly Gln Gly Thr Met
               100                 105                 110
   gtc acc gtc tca agc                                                  351
   Val Thr Val Ser Ser
           115
<210> 168
   <211> 117
   <212> PRT
   <213> Artificial Sequence
<220>
   <223> heavy chain variable region
<400> 168
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Ala Ser Val Asp Ala Phe Asp Ile Trp Gly Gln Gly Thr Met
               100                 105                 110
   Val Thr Val Ser Ser
           115
<210> 169
   <211> 357
   <212> DNA
   <213> Artificial Sequence
<220>
   <223> heavy chain variable region
<220>
   <221> CDS
   <222> (1)..(357)
<400> 169
   cag gtg cag ctg cag gag tcc ggc cca gga ctg gtg aag cct tcg ggg        48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gtg tac tac tgt       288
   Leu Lys Leu Ser Ser Val Thr Ala Ala Asp Thr Ala Val Tyr Tyr Cys
                   85                  90                  95
   gcg aga ggg ctg ggg gat agt agt ggt tat atc ctt tgg ggc caa ggg       336
   Ala Arg Gly Leu Gly Asp Ser Ser Gly Tyr Ile Leu Trp Gly Gln Gly
               100                 105                 110
   aca atg gtc acc gtc tca agc                                           357
   Thr Met Val Thr Val Ser Ser
           115
<210> 170
   <211> 119
   <212> PRT
   <213> Artificial Sequence
<220>
   <223> heavy chain variable region
<400> 170
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Gly Leu Gly Asp Ser Ser Gly Tyr Ile Leu Trp Gly Gln Gly
               100                 105                 110
   Thr Met Val Thr Val Ser Ser
           115
<210> 171
   <211> 348
   <212> DNA
   <213> Artificial Sequence
<220>
   <223> heavy chain variable region
<220>
   <221> CDS
   <222> (1)..(348)
<400> 171
   cag gta cag ctg cag cag tca ggc cca gga ctg gtg aag cct tcg ggg        48
   Gln Val Gln Leu Gln Gln Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg       144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg act ccc gag gac acg gct gtg tat tac tgt       288
   Leu Lys Leu Ser Ser Val Thr Pro Glu Asp Thr Ala Val Tyr Tyr Cys
                   85                  90                  95
   gca aga gat cac ggc ccc ttt gac tac tgg ggc cgg gga acc ctg gtc       336
   Ala Arg Asp His Gly Pro Phe Asp Tyr Trp Gly Arg Gly Thr Leu Val
               100                 105                 110
   acc gtc tca agc                                                       348
   Thr Val Ser Ser
           115
<210> 172
   <211> 116
   <212> PRT
   <213> Artificial Sequence
<220>
   <223> heavy chain variable region
<400> 172
   Gln Val Gln Leu Gln Gln Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Pro Glu Asp Thr Ala Val Tyr Tyr Cys
                   85                  90                  95
   Ala Arg Asp His Gly Pro Phe Asp Tyr Trp Gly Arg Gly Thr Leu Val
               100                 105                 110
   Thr Val Ser Ser
           115
<210> 173
   <211> 360
   <212> DNA
   <213> Artificial Sequence
<220>
   <223> heavy chain variable region
<220>
   <221> CDS
   <222> (1)..(360)
<400> 173
   cag gtg cag ctg gtg caa tct ggg gga ggc gtg gtc cag cct ggg agg      48
   Gln Val Gln Leu Val Gln Ser Gly Gly Gly Val Val Gln Pro Gly Arg
   1               5                   10                  15
   tcc ctg aga ctc tcc tgt gca gcc tct gga ttc gcc ttc agt agc tat      96
   Ser Leu Arg Leu Ser Cys Ala Ala Ser Gly Phe Ala Phe Ser Ser Tyr
               20                  25                  30
   ggc atg cac tgg gtc cgc cag gct cca ggg aag ggg ctg gag tgg gtt     144
   Gly Met His Trp Val Arg Gln Ala Pro Gly Lys Gly Leu Glu Trp Val
           35                  40                  45
   tca tac att agt agt agt agt agt acc ata tac tac gca gac tct gtg     192
   Ser Tyr Ile Ser Ser Ser Ser Ser Thr Ile Tyr Tyr Ala Asp Ser Val
       50                  55                  60
   aag ggc cga ttc acc atc tcc aga gac aat tcc aag aac acg ctg tat     240
   Lys Gly Arg Phe Thr Ile Ser Arg Asp Asn Ser Lys Asn Thr Leu Tyr
   65                  70                  75                  80
   ctg caa atg aac agc ctg aga gcc gag gac acg gct gtg tat tac tgt     288
   Leu Gln Met Asn Ser Leu Arg Ala Glu Asp Thr Ala Val Tyr Tyr Cys
                   85                  90                  95
   gcg aga gat cga ttt ggg tcg ggg cac ttg ccc gac tac tgg ggc cag     336
   Ala Arg Asp Arg Phe Gly Ser Gly His Leu Pro Asp Tyr Trp Gly Gln
               100                 105                 110
   gga acc ctg gtc acc gtc tca agc                                     360
   Gly Thr Leu Val Thr Val Ser Ser
           115                 120
<210> 174
   <211> 120
   <212> PRT
   <213> Artificial Sequence
<220>
   <223> heavy chain variable region
<400> 174
   Gln Val Gln Leu Val Gln Ser Gly Gly Gly Val Val Gln Pro Gly Arg
   1               5                   10                  15
   Ser Leu Arg Leu Ser Cys Ala Ala Ser Gly Phe Ala Phe Ser Ser Tyr
               20                  25                  30
   Gly Met His Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Phe Gly Ser Gly His Leu Pro Asp Tyr Trp Gly Gln
               100                 105                 110
   Gly Thr Leu Val Thr Val Ser Ser
           115                 120
<210> 175
   <211> 357
   <212> DNA
   <213> Artificial Sequence
<220>
   <223> heavy chain variable region
<220>
   <221> CDS
   <222> (1)..(357)
<400> 175
   cag gtg cag cta cag cag tgg ggc gca gga ctg ttg aag cct tcg gag      48
   Gln Val Gln Leu Gln Gln Trp Gly Ala Gly Leu Leu Lys Pro Ser Glu
   1               5                   10                  15
   acc ctg tcc ctc acc tgc gct gtc tat ggt ggg tcc ttc agt ggt tac      96
   Thr Leu Ser Leu Thr Cys Ala Val Tyr Gly Gly Ser Phe Ser Gly Tyr
               20                  25                  30
   tac tgg agc tgg atc cgc cag ccc cca ggg aag ggg ctg gag tgg att      144
   Tyr Trp Ser Trp Ile Arg Gln Pro Pro Gly Lys Gly Leu Glu Trp Ile
           35                  40                  45
   ggg gaa atc aat cat agt gga agc acc aac tac aac ccg tcc ctc aag      192
   Gly Glu Ile Asn His Ser Gly Ser Thr Asn Tyr Asn Pro Ser Leu Lys
       50                  55                  60
   agt cga gtc acc ata tca gta gac acg tcc aag aac cag ttc tcc ctg      240
   Ser Arg Val Thr Ile Ser Val Asp Thr Ser Lys Asn Gln Phe Ser Leu
   65                  70                  75                  80
   aag ctg agc tct gtg acc gcc gcg gac acg gct gtg tat tac tgt gcg      288
   Lys Leu Ser Ser Val Thr Ala Ala Asp Thr Ala Val Tyr Tyr Cys Ala
                   85                  90                  95
   aga gtt ggg tat agc agt ggc cgt gac gtt gac tac tgg ggc cag ggc      336
   Arg Val Gly Tyr Ser Ser Gly Arg Asp Val Asp Tyr Trp Gly Gln Gly
               100                 105                 110
   acc ctg gtc acc gtc tca agc                                          357
   Thr Leu Val Thr Val Ser Ser
           115
<210> 176
   <211> 119
   <212> PRT
   <213> Artificial Sequence
<220>
   <223> heavy chain variable region
<400> 176
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Glu Ile Asn His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Val Gly Tyr Ser Ser Gly Arg Asp Val Asp Tyr Trp Gly Gln Gly
               100                 105                 110
   Thr Leu Val Thr Val Ser Ser
           115
<210> 177
   <211> 360
   <212> DNA
   <213> Artificial Sequence
<220>
   <223> heavy chain variable region
<220>
   <221> CDS
   <222> (1)..(360)
<400> 177
   gag gtc cag ctg gtg gag tct ggc cca gga ctg gtg aag cct tcg ggg        48
   Glu Val Gln Leu Val Glu Ser Gly Pro Gly Leu Val Lys Pro Ser Gly
   1               5                   10                  15
   acc ctg tcc ctc acc tgc gct gtc tct ggt ggc tcc atc agc agt agt        96
   Thr Leu Ser Leu Thr Cys Ala Val Ser Gly Gly Ser Ile Ser Ser Ser
               20                  25                  30
   aac tgg tgg agt tgg atc cgg cag ccc cca ggg aag ggg ctg gag tgg       144
   Asn Trp Trp Ser Trp Ile Arg Gln Pro Pro Gly Lys Gly Leu Glu Trp
           35                  40                  45
   att ggg gaa atc tat cat agt ggg agc acc aac tac aac ccg tcc ctc       192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc       240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt       288
   Leu Lys Leu Ser Ser Val Thr Ala Ala Asp Thr Ala Val Tyr Tyr Cys
                   85                  90                  95
   gcg aga gat agc agc agc tgg tac tac ggt atg gac gtc tgg ggc caa       336
   Ala Arg Asp Ser Ser Ser Trp Tyr Tyr Gly Met Asp Val Trp Gly Gln
               100                 105                 110
   ggg acc acg gtc acc gtc tca agc                                       360
   Gly Thr Thr Val Thr Val Ser Ser
           115                 120
<210> 178
   <211> 120
   <212> PRT
   <213> Artificial Sequence
<220>
   <223> heavy chain variable region
<400> 178
   Glu Val Gln Leu Val Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Ile Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Asp Ser Ser Ser Trp Tyr Tyr Gly Met Asp Val Trp Gly Gln
               100                 105                 110
   Gly Thr Thr Val Thr Val Ser Ser
           115                 120
<210> 179
   <211> 348
   <212> DNA
   <213> Artificial Sequence
<220>
   <223> heavy chain variable region
<220>
   <221> CDS
   <222> (1)..(348)
<400> 179
   gag gtc cag ctg gtg gag tcc ggc cca gga ctg gtg aag cct tcg gag      48
   Glu Val Gln Leu Val Glu Ser Gly Pro Gly Leu Val Lys Pro Ser Glu
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg     144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc     192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc     240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gta tat tat tgt      288
   Leu Lys Leu Ser Ser Val Thr Ala Ala Asp Thr Ala Val Tyr Tyr Cys
                   85                  90                  95
   gcg aga tcg acg tgg tcc ctt gac tac tgg ggc cag ggc acc ctg gtc      336
   Ala Arg Ser Thr Trp Ser Leu Asp Tyr Trp Gly Gln Gly Thr Leu Val
               100                 105                 110
   acc gtc tca agc                                                      348
   Thr Val Ser Ser
           115
<210> 180
   <211> 116
   <212> PRT
   <213> Artificial Sequence
<220>
   <223> heavy chain variable region
<400> 180
   Glu Val Gln Leu Val Glu Ser Gly Pro Gly Leu Val Lys Pro Ser Glu
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Ser Thr Trp Ser Leu Asp Tyr Trp Gly Gln Gly Thr Leu Val
               100                 105                 110
   Thr Val Ser Ser
           115
<210> 181
   <211> 354
   <212> DNA
   <213> Artificial Sequence
<220>
   <223> heavy chain variable region
<220>
   <221> CDS
   <222> (1)..(354)
<400> 181
   gag gtc cag ctg gtg gag tct ggc cca gga ctg gtg aag cct tcg ggg       48
   Glu Val Gln Leu Val Glu Ser Gly Pro Gly Leu Val Lys Pro Ser Gly
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gta tat tac tgt      288
   Leu Lys Leu Ser Ser Val Thr Ala Ala Asp Thr Ala Val Tyr Tyr Cys
                   85                  90                  95
   gcg aga ctc tcg ttt gcc gat cct ttt gat atc tgg ggc caa ggg aca      336
   Ala Arg Leu Ser Phe Ala Asp Pro Phe Asp Ile Trp Gly Gln Gly Thr
               100                 105                 110
   atg gtc acc gtc tca agc                                              354
   Met Val Thr Val Ser Ser
           115
<210> 182
   <211> 118
   <212> PRT
   <213> Artificial Sequence
<220>
   <223> heavy chain variable region
<400> 182
   Glu Val Gln Leu Val Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Leu Ser Phe Ala Asp Pro Phe Asp Ile Trp Gly Gln Gly Thr
               100                 105                 110
   Met Val Thr Val Ser Ser
           115
<210> 183
   <211> 366
   <212> DNA
   <213> Artificial Sequence
<220>
   <223> heavy chain variable region
<220>
   <221> CDS
   <222> (1)..(366)
<400> 183
   cag gtc cag ctg gtg cag tct ggg gct gag gtg aag aag cct ggg tcc        48
   Gln Val Gln Leu Val Gln Ser Gly Ala Glu Val Lys Lys Pro Gly Ser
   1               5                   10                  15
   tcg gtg aag gtc tcc tgc aag gct tct gga ggc acc ttc agc agc tat        96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg       144
   Ala Ile Ser Trp Val Arg Gln Ala Pro Gly Gln Gly Leu Glu Trp Met
           35                  40                  45
   gga agg atc atc ccc atc ctt ggt ata gca aac tac gca cag aag ttc       192
   Gly Arg Ile Ile Pro Ile Leu Gly Ile Ala Asn Tyr Ala Gln Lys Phe
       50                  55                  60
   cag ggc aga gtc acg att acc gcg gac aaa tcc acg agc aca gcc tac       240
   Gln Gly Arg Val Thr Ile Thr Ala Asp Lys Ser Thr Ser Thr Ala Tyr
   65                  70                  75                  80
   atg gag ctg agc agc ctg aga tct gag gac acg gcc gtg tat tac tgt       288
   Met Glu Leu Ser Ser Leu Arg Ser Glu Asp Thr Ala Val Tyr Tyr Cys
                   85                  90                  95
   gca tat ggt tcg ggg agt tat tac gac tac tac tac atg gac gtc tgg       336
   Ala Tyr Gly Ser Gly Ser Tyr Tyr Asp Tyr Tyr Tyr Met Asp Val Trp
               100                 105                 110
   ggc aaa ggg acc acg gtc acc gtc tca agc                               366
   Gly Lys Gly Thr Thr Val Thr Val Ser Ser
                             115                 120
<210> 184
   <211> 122
   <212> PRT
   <213> Artificial Sequence
<220>
   <223> heavy chain variable region
<400> 184
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Arg Ile Ile Pro Ile Leu Gly Ile Ala Asn Tyr Ala Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Gly Ser Gly Ser Tyr Tyr Asp Tyr Tyr Tyr Met Asp Val Trp
               100                 105                 110
   Gly Lys Gly Thr Thr Val Thr Val Ser Ser
           115                 120
<210> 185
   <211> 357
   <212> DNA
   <213> Artificial Sequence
<220>
   <223> heavy chain variable region
<220>
   <221> CDS
   <222> (1)..(357)
<400> 185
   gag gtc cag ctg gtg cag tct ggg gga ggc ttg gtc cag cct ggg ggg      48
   Glu Val Gln Leu Val Gln Ser Gly Gly Gly Leu Val Gln Pro Gly Gly
   1               5                   10                  15
   tcc ctg aga ctc tcc tgt tca gcc tcc gga ttc acc ttc agt agc tat       96
   Ser Leu Arg Leu Ser Cys Ser Ala Ser Gly Phe Thr Phe Ser Ser Tyr
               20                  25                  30
   gct atg cac tgg gtc cgc cag gct cca ggg aag gga ctg gaa tat gtt      144
   Ala Met His Trp Val Arg Gln Ala Pro Gly Lys Gly Leu Glu Tyr Val
           35                  40                  45
   tca act att agt agt aat ggg gat agc aca tac tac gca gac tcc gtg      192
   Ser Thr Ile Ser Ser Asn Gly Asp Ser Thr Tyr Tyr Ala Asp Ser Val
       50                  55                  60
   aag ggc aga ttc acc atc tcc aga gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg aga gct gag gac acg gct gtg tat tac tgt      288
   Leu Gln Met Asn Ser Leu Arg Ala Glu Asp Thr Ala Val Tyr Tyr Cys
                   85                  90                  95
   gcg aaa gaa gaa gta tgg cta cag gct ttt gat atc tgg ggc caa ggg      336
   Ala Lys Glu Glu Val Trp Leu Gln Ala Phe Asp Ile Trp Gly Gln Gly
               100                 105                 110
   aca atg gtc acc gtc tca agc                                          357
   Thr Met Val Thr Val Ser Ser
           115
<210> 186
   <211> 119
   <212> PRT
   <213> Artificial Sequence
<220>
   <223> heavy chain variable region
<400> 186
   Glu Val Gln Leu Val Gln Ser Gly Gly Gly Leu Val Gln Pro Gly Gly
   1               5                   10                  15
   Ser Leu Arg Leu Ser Cys Ser Ala Ser Gly Phe Thr Phe Ser Ser Tyr
               20                  25                  30
   Ala Met His Trp Val Arg Gln Ala Pro Gly Lys Gly Leu Glu Tyr Val
           35                  40                  45
   Ser Thr Ile Ser Ser Asn Gly Asp Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Glu Val Trp Leu Gln Ala Phe Asp Ile Trp Gly Gln Gly
               100                 105                 110
   Thr Met Val Thr Val Ser Ser
           115
<210> 187
   <211> 345
   <212> DNA
   <213> Artificial Sequence
<220>
   <223> heavy chain variable region
<220>
   <221> CDS
   <222> (1)..(345)
<400> 187
   cag ctg cag ctg cag gag tcg ggc cca gga ctg gtg aag cct tcg gag      48
   Gln Leu Gln Leu Gln Glu Ser Gly Pro Gly Leu Val Lys Pro Ser Glu
   1               5                   10                  15
   acc ctg tcc ctc acc tgc act gtc tct ggt ggc tcc atc agt agt aac      96
   Thr Leu Ser Leu Thr Cys Thr Val Ser Gly Gly Ser Ile Ser Ser Asn
               20                  25                  30
   tgg tgg agt tgg gtc cgc cag ccc cca ggg aag ggg ctg gag tgg att      144
   Trp Trp Ser Trp Val Arg Gln Pro Pro Gly Lys Gly Leu Glu Trp Ile
           35                  40                  45
   ggg gaa atc tat cat agt ggg agc acc aac tac aac ccc tcc ctc aag      192
   Gly Glu Ile Tyr His Ser Gly Ser Thr Asn Tyr Asn Pro Ser Leu Lys
       50                  55                  60
   agt cga gtc acc atc tca gta gac acg tcc aag aac cag ttc tcc ctg      240
   Ser Arg Val Thr Ile Ser Val Asp Thr Ser Lys Asn Gln Phe Ser Leu
   65                  70                  75                  80
   aag ctg agc tct gtg acc gct gcg gac acg gcc gtg tat tac tgt gcg      288
   Lys Leu Ser Ser Val Thr Ala Ala Asp Thr Ala Val Tyr Tyr Cys Ala
                   85                  90                  95
   aga gat aag gga tac atg gac gtc tgg ggc aaa ggg acc acg gtc acc      336
   Arg Asp Lys Gly Tyr Met Asp Val Trp Gly Lys Gly Thr Thr Val Thr
               100                 105                 110
   gtc tca agc                                                          345
   Val Ser Ser
           115
<210> 188
   <211> 115
   <212> PRT
   <213> Artificial Sequence
<220>
   <223> heavy chain variable region
<400> 188
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Asn
               20                  25                  30
   Trp Trp Ser Trp Val Arg Gln Pro Pro Gly Lys Gly Leu Glu Trp Ile
           35                  40                  45
   Gly Glu Ile Tyr His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Asp Lys Gly Tyr Met Asp Val Trp Gly Lys Gly Thr Thr Val Thr
               100                 105                 110
   Val Ser Ser
           115
<210> 189
   <211> 363
   <212> DNA
   <213> Artificial Sequence
<220>
   <223> heavy chain variable region
<220>
   <221> CDS
   <222> (1)..(363)
<400> 189
   cag gta cag ctg cag cag tca ggg gct gag gtg aag aag cct ggg tcc        48
   Gln Val Gln Leu Gln Gln Ser Gly Ala Glu Val Lys Lys Pro Gly Ser
   1               5                   10                  15
   tcg gtg aag gtc tcc tgc aag gct tct gga ggc acc ttc agc agc tat        96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg       144
   Ala Ile Ser Trp Val Arg Gln Ala Pro Gly Gln Gly Leu Glu Trp Met
           35                  40                  45
   gga agg atc atc cct atc ctt ggt ata gca aac tac gca cag aag ttc       192
   Gly Arg Ile Ile Pro Ile Leu Gly Ile Ala Asn Tyr Ala Gln Lys Phe
       50                  55                  60
   cag ggc aga gtc acg att acc gcg gac aaa tcc acg agc aca gcc tac       240
   Gln Gly Arg Val Thr Ile Thr Ala Asp Lys Ser Thr Ser Thr Ala Tyr
   65                  70                  75                  80
   atg gag ctg agc agc ctg aga tct gag gac acg gcc gtg tat tac tgt       288
   Met Glu Leu Ser Ser Leu Arg Ser Glu Asp Thr Ala Val Tyr Tyr Cys
                   85                  90                  95
   gcg aga gat cat agg ttc gac tac gcc tgg tac ttc gat ctc tgg ggc       336
   Ala Arg Asp His Arg Phe Asp Tyr Ala Trp Tyr Phe Asp Leu Trp Gly
               100                 105                 110
   cgt ggc acc ctg gtc acc gtc tca agc                                   363
   Arg Gly Thr Leu Val Thr Val Ser Ser
           115                 120
<210> 190
   <211> 121
   <212> PRT
   <213> Artificial Sequence
<220>
   <223> heavy chain variable region
<400> 190
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Arg Ile Ile Pro Ile Leu Gly Ile Ala Asn Tyr Ala Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp His Arg Phe Asp Tyr Ala Trp Tyr Phe Asp Leu Trp Gly
               100                 105                 110
   Arg Gly Thr Leu Val Thr Val Ser Ser
           115                 120
<210> 191
   <211> 351
   <212> DNA
   <213> Artificial Sequence
<220>
   <223> heavy chain variable region
<220>
   <221> CDS
   <222> (1)..(351)
<400> 191
   cag gtg cag ctg cag gag tcg ggc cca gga ctg ctg aag cct tcg ggg       48
   Gln Val Gln Leu Gln Glu Ser Gly Pro Gly Leu Leu Lys Pro Ser Gly
   1               5                   10                  15
   acc ctg tcc ctc acc tgc gct gtc tct ggt ggc tcc atc agc agt agc       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg gag ggg ctg gag tgg      144
   Asn Trp Trp Ser Trp Val Arg Gln Pro Pro Gly Glu Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtc tat tac tgt      288
   Leu Lys Leu Ser Ser Val Thr Ala Ala Asp Thr Ala Val Tyr Tyr Cys
                   85                  90                  95
   gcg aga gat cta acg ggg agt ctt gac tac tgg ggc cag gga acc ctg      336
   Ala Arg Asp Leu Thr Gly Ser Leu Asp Tyr Trp Gly Gln Gly Thr Leu
               100                 105                 110
   gtc acc gtc tca agc                                                  351
   Val Thr Val Ser Ser
           115
<210> 192
   <211> 117
   <212> PRT
   <213> Artificial Sequence
<220>
   <223> heavy chain variable region
<400> 192
   Gln Val Gln Leu Gln Glu Ser Gly Pro Gly Leu Leu Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Glu Gly Leu Glu Trp
   35 40 45
   Ile Gly Glu Ile Tyr His Ser Gly Ser Thr Asn Tyr Asn Pro Ser Leu
   50 55 60
   Lys Ser Arg Val Thr Ile Ser Val Asp Lys Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg Asp Leu Thr Gly Ser Leu Asp Tyr Trp Gly Gln Gly Thr Leu
   100 105 110
   Val Thr Val Ser Ser
   115
<210> 193
   <211> 351
   <212> DNA
   <213> Artificial Sequence
<220>
   <223> heavy chain variable region
<220>
   <221> CDS
   <222> (1)..(351)
<400> 193
   cag gtg cag ctg cag gag tcc ggc cca gga ctg gtg aag cct tcg ggg      48
   Gln Val Gln Leu Gln Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga ata cgc tat gat gct ttt gat atc tgg ggc caa ggg aca atg      336
   Ala Arg Ile Arg Tyr Asp Ala Phe Asp Ile Trp Gly Gln Gly Thr Met
               100                 105                 110
   gtc acc gtc tca agc                                                  351
   Val Thr Val Ser Ser
           115
<210> 194
   <211> 117
   <212> PRT
   <213> Artificial Sequence
<220>
   <223> heavy chain variable region
<400> 194
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Ile Arg Tyr Asp Ala Phe Asp Ile Trp Gly Gln Gly Thr Met
               100                 105                 110
   Val Thr Val Ser Ser
           115
<210> 195
   <211> 354
   <212> DNA
   <213> Artificial Sequence
<220>
   <223> heavy chain variable region
<220>
   <221> CDS
   <222> (1)..(354)
<400> 195
   cag gtg cag ctg cag gag tcg ggc cca gga ctg gtg aag cct tcg gag       48
   Gln Val Gln Leu Gln Glu Ser Gly Pro Gly Leu Val Lys Pro Ser Glu
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gct gcg gac acg gcc gtg tat tac tgt      288
   Leu Lys Leu Ser Ser Val Thr Ala Ala Asp Thr Ala Val Tyr Tyr Cys
                   85                  90                  95
   gcc gtg acg gca gcc cat gat gct ttt gat atc tgg ggc caa ggg aca      336
   Ala Val Thr Ala Ala His Asp Ala Phe Asp Ile Trp Gly Gln Gly Thr
               100                 105                 110
   atg gtc acc gtc tca agc                                              354
   Met Val Thr Val Ser Ser
           115
<210> 196
   <211> 118
   <212> PRT
   <213> Artificial Sequence
<220>
   <223> heavy chain variable region
<400> 196
   Gln Val Gln Leu Gln Glu Ser Gly Pro Gly Leu Val Lys Pro Ser Glu
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Val Thr Ala Ala His Asp Ala Phe Asp Ile Trp Gly Gln Gly Thr
               100                 105                 110
   Met Val Thr Val Ser Ser
           115
<210> 197
   <211> 357
   <212> DNA
   <213> Artificial Sequence
<220>
   <223> heavy chain variable region
<220>
   <221> CDS
   <222> (1)..(357)
<400> 197
   cag gtg cag cta cag cag tgg ggc cca gga ctg gtg aag cct tcg ggg       48
   Gln Val Gln Leu Gln Gln Trp Gly Pro Gly Leu Val Lys Pro Ser Gly
   1               5                   10                  15
   acc ctg tcc ctc acc tgc gct gtc tct ggt ggc tcc atc agc agt agt       96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg      144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc      192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc      240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt      288
   Leu Lys Leu Ser Ser Val Thr Ala Ala Asp Thr Ala Val Tyr Tyr Cys
                   85                  90                  95
   gcg aga gac agc agt ggc caa ggg tac ttt gac tac tgg ggc cag ggc      336
   Ala Arg Asp Ser Ser Gly Gln Gly Tyr Phe Asp Tyr Trp Gly Gln Gly
               100                 105                 110
   acc ctg gtc acc gtc tca agc                                          357
   Thr Leu Val Thr Val Ser Ser
           115
<210> 198
   <211> 119
   <212> PRT
   <213> Artificial Sequence
<220>
   <223> heavy chain variable region
<400> 198
   Gln Val Gln Leu Gln Gln Trp Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Asp Ser Ser Gly Gln Gly Tyr Phe Asp Tyr Trp Gly Gln Gly
               100                 105                 110
   Thr Leu Val Thr Val Ser Ser
           115
<210> 199
   <211> 354
   <212> **DNA**
   <213> Artificial Sequence
<220>
   <223> heavy chain variable region
<220>
   <221> CDS
   <222> (1)..(354)
<400> 199
   gag gtg cag ctg gtg cag tct ggg gct gag gtg aag aag cct ggg gcc      48
   Glu Val Gln Leu Val Gln Ser Gly Ala Glu Val Lys Lys Pro Gly Ala
   1               5                   10                  15
   tca gtg aag gtc tcc tgc aag gct tct gga tac acc ttc act agc tat      96
   Ser Val Lys Val Ser Cys Lys Ala Ser Gly Tyr Thr Phe Thr Ser Tyr
               20                  25                  30
   gct atg cat tgg gtg cgc cag gcc ccc gga caa agg ctt gag tgg atg     144
   Ala Met His Trp Val Arg Gln Ala Pro Gly Gln Arg Leu Glu Trp Met
           35                  40                  45
   gga tgg atc aac gct ggc aat ggt aac aca aaa tat tca cag aag ttc     192
   Gly Trp Ile Asn Ala Gly Asn Gly Asn Thr Lys Tyr Ser Gln Lys Phe
       50                  55                  60
   cag ggc aga gtc acc atg acc agg gac acg tcc acg agc aca gtc tac     240
   Gln Gly Arg Val Thr Met Thr Arg Asp Thr Ser Thr Ser Thr Val Tyr
   65                  70                  75                  80
   atg gag ctg agc agc ctg aga tct gag gac acg gcc gtg tat tac tgt     288
   Met Glu Leu Ser Ser Leu Arg Ser Glu Asp Thr Ala Val Tyr Tyr Cys
                   85                  90                  95
   gct aga cac tcg tac tac tac ggt atg gac gtc tgg ggc caa ggc acc     336
   Ala Arg His Ser Tyr Tyr Tyr Gly Met Asp Val Trp Gly Gln Gly Thr
               100                 105                 110
   ctg gtc acc gtc tca agc                                             354
   Leu Val Thr Val Ser Ser
           115
<210> 200
   <211> 118
   <212> PRT
   <213> Artificial Sequence
<220>
   <223> heavy chain variable region
<400> 200
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Ala Met His Trp Val Arg Gln Ala Pro Gly Gln Arg Leu Glu Trp Met
           35                  40                  45
   Gly Trp Ile Asn Ala Gly Asn Gly Asn Thr Lys Tyr Ser Gln Lys Phe
       50                  55                  60
   Gln Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg His Ser Tyr Tyr Tyr Gly Met Asp Val Trp Gly Gln Gly Thr
               100                 105                 110
   Leu Val Thr Val Ser Ser
           115
<210> 201
   <211> 360
   <212> DNA
   <213> Artificial Sequence
<220>
   <223> heavy chain variable region
<220>
   <221> CDS
   <222> (1)..(360)
<400> 201
   cag gtg cag cta cag cag tgg ggc gca gga ctg ttg aag cct tcg gag       48
   Gln Val Gln Leu Gln Gln Trp Gly Ala Gly Leu Leu Lys Pro Ser Glu
   1               5                   10                  15
   acc ctg tcc ctc acc tgc gct gtc tat ggt ggg tcc ttc agt ggt tac       96
   Thr Leu Ser Leu Thr Cys Ala Val Tyr Gly Gly Ser Phe Ser Gly Tyr
               20                  25                  30
   tac tgg agc tgg atc cgc cag ccc cca ggg aag ggg ctg gag tgg att      144
   Tyr Trp Ser Trp Ile Arg Gln Pro Pro Gly Lys Gly Leu Glu Trp Ile
           35                  40                  45
   ggg gaa atc aat cat agt gga agc acc aac tac aac ccg tcc ctc aag      192
   Gly Glu Ile Asn His Ser Gly Ser Thr Asn Tyr Asn Pro Ser Leu Lys
       50                  55                  60
   agt cga gtc acc ata tcg gta gac acg tcc aag aac cag ttc tcc ctg      240
   Ser Arg Val Thr Ile Ser Val Asp Thr Ser Lys Asn Gln Phe Ser Leu
   65                  70                  75                  80
   aag ctg agc tct gtg acc gcc gcg gac acg gct gtg tat tac tgt gcg      288
   Lys Leu Ser Ser Val Thr Ala Ala Asp Thr Ala Val Tyr Tyr Cys Ala
                   85                  90                  95
   aga gtc ggg tat agc cac ggc gaa gaa gtc ctg gac gtc tgg ggc aaa      336
   Arg Val Gly Tyr Ser His Gly Glu Glu Val Leu Asp Val Trp Gly Lys
               100                 105                 110
   ggg acc acg gtc acc gtc tca agc                                      360
   Gly Thr Thr Val Thr Val Ser Ser
           115                 120
<210> 202
   <211> 120
   <212> PRT
   <213> Artificial Sequence
<220>
   <223> heavy chain variable region
<400> 202
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
                   20                  25                  30
   Tyr Trp Ser Trp Ile Arg Gln Pro Pro Gly Lys Gly Leu Glu Trp Ile
           35                  40                  45
   Gly Glu Ile Asn His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Val Gly Tyr Ser His Gly Glu Glu Val Leu Asp Val Trp Gly Lys
               100                 105                 110
   Gly Thr Thr Val Thr Val Ser Ser
           115                 120
<210> 203
   <211> 354
   <212> DNA
   <213> Artificial Sequence
<220>
   <223> heavy chain variable region
<220>
   <221> CDS
   <222> (1)..(354)
<400> 203
   cag gtg cag ctg cag gag tcg ggc cca gga ctg gtg aag cct tcg gag        48
   Gln Val Gln Leu Gln Glu Ser Gly Pro Gly Leu Val Lys Pro Ser Glu
   1               5                   10                  15
   acc ctg tcc ctc acc tgc act gtc tct ggt ggc tcc atc ggc aat tat        96
   Thr Leu Ser Leu Thr Cys Thr Val Ser Gly Gly Ser Ile Gly Asn Tyr
               20                  25                  30
   gac tgg agt tgg atc cgg cag ccc cca ggg aag gga ctg gag tgg att       144
   Asp Trp Ser Trp Ile Arg Gln Pro Pro Gly Lys Gly Leu Glu Trp Ile
           35                  40                  45
   ggg act atc tac tct agt ggg agt acg tac tac agt ccg tcc ctc aag       192
   Gly Thr Ile Tyr Ser Ser Gly Ser Thr Tyr Tyr Ser Pro Ser Leu Lys
       50                  55                  60
   agt cga ctc acc ata tca gta gac aag tcc aag aac cgg ttc tcc ctg       240
   Ser Arg Leu Thr Ile Ser Val Asp Lys Ser Lys Asn Arg Phe Ser Leu
   65                  70                  75                  80
   aag ctg agc tct gtg acc gcc gcg gac acg gcc gtg tat tac tgt gcg       288
   Lys Leu Ser Ser Val Thr Ala Ala Asp Thr Ala Val Tyr Tyr Cys Ala
                   85                  90                  95
   aga gca cga ggg tat agc agc ccc ttc gac ccc tgg ggc cag ggc acc       336
   Arg Ala Arg Gly Tyr Ser Ser Pro Phe Asp Pro Trp Gly Gln Gly Thr
               100                 105                 110
   ctg gtc acc gtc tca agc                                               354
   Leu Val Thr Val Ser Ser
           115
<210> 204
   <211> 118
   <212> PRT
   <213> Artificial Sequence
<220>
   <223> heavy chain variable region
<400> 204
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Gly Asn Tyr
               20                  25                  30
   Asp Trp Ser Trp Ile Arg Gln Pro Pro Gly Lys Gly Leu Glu Trp Ile
           35                  40                  45
   Gly Thr Ile Tyr Ser Ser Gly Ser Thr Tyr Tyr Ser Pro Ser Leu Lys
       50                  55                  60
   Ser Arg Leu Thr Ile Ser Val Asp Lys Ser Lys Asn Arg Phe Ser Leu
   65                  70                  75                  80
   Lys Leu Ser Ser Val Thr Ala Ala Asp Thr Ala Val Tyr Tyr Cys Ala
                   85                  90                  95
   Arg Ala Arg Gly Tyr Ser Ser Pro Phe Asp Pro Trp Gly Gln Gly Thr
               100                 105                 110
   Leu Val Thr Val Ser Ser
           115
<210> 205
   <211> 357
   <212> DNA
   <213> Artificial Sequence
<220>
   <223> heavy chain variable region
<220>
   <221> CDS
   <222> (1)..(357)
<400> 205
   cag gtc cag ctg gta cag tct ggg gct gag gtg aag aag cct ggg tcc       48
   Gln Val Gln Leu Val Gln Ser Gly Ala Glu Val Lys Lys Pro Gly Ser
   1               5                   10                  15
   tcg gtg aag gtc tcc tgc aag gct tct gga ggc acc ttc agc agc tat       96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga ata atc aac cct agt ggt ggt agc aca agc tac gca cag aag ttc      192
   Gly Ile Ile Asn Pro Ser Gly Gly Ser Thr Ser Tyr Ala Gln Lys Phe
       50                  55                  60
   cag ggc aga gtc acc att acc agg gac aca tcc gcg agc aca gcc tac      240
   Gln Gly Arg Val Thr Ile Thr Arg Asp Thr Ser Ala Ser Thr Ala Tyr
   65                  70                  75                  80
   atg gag ctg agc agc ctg aga tct gaa gac acg gct gtg tat tac tgt      288
   Met Glu Leu Ser Ser Leu Arg Ser Glu Asp Thr Ala Val Tyr Tyr Cys
                   85                  90                  95
   gcg aga gat cgg tgg agg tac gat gct ttt gat atc tgg ggc caa ggg      336
   Ala Arg Asp Arg Trp Arg Tyr Asp Ala Phe Asp Ile Trp Gly Gln Gly
               100                 105                 110
   aca atg gtc acc gtc tca agc                                          357
   Thr Met Val Thr Val Ser Ser
           115
<210> 206
   <211> 119
   <212> PRT
   <213> Artificial Sequence
<220>
   <223> heavy chain variable region
<400> 206
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Ile Ile Asn Pro Ser Gly Gly Ser Thr Ser Tyr Ala Gln Lys Phe
       50                  55                  60
   Gln Gly Arg Val Thr Ile Thr Arg Asp Thr Ser Ala Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Arg Trp Arg Tyr Asp Ala Phe Asp Ile Trp Gly Gln Gly
               100                 105                 110
   Thr Met Val Thr Val Ser Ser
           115
<210> 207
   <211> 348
   <212> DNA
   <213> Artificial Sequence
<220>
   <223> heavy chain variable region
<220>
   <221> CDS
   <222> (1)..(348)
<400> 207
   gag gtg cag ctg gtg gag tct ggc cca gga ctg gtg aag cct tcg ggg      48
   Glu Val Gln Leu Val Glu Ser Gly Pro Gly Leu Val Lys Pro Ser Gly
   1               5                   10                  15
   acc ctg tcc ctc acc tgc gct gtc tct ggt ggc tcc atc agc agt agt      96
   Thr Leu Ser Leu Thr Cys Ala Val Ser Gly Gly Ser Ile Ser Ser Ser
               20                  25                  30
   aac tgg tgg agt tgg gtc cgc cag ccc cca ggg aag ggg ctg gag tgg     144
   Asn Trp Trp Ser Trp Val Arg Gln Pro Pro Gly Lys Gly Leu Glu Trp
           35                  40                  45
   att ggg gaa atc tat cat agt ggg agc acc aac tac aac ccg tcc ctc     192
   Ile Gly Glu Ile Tyr His Ser Gly Ser Thr Asn Tyr Asn Pro Ser Leu
       50                  55                  60
   aag agt cga gtc acc ata tca gta gac aag tcc aag aac cag ttc tcc     240
   Lys Ser Arg Val Thr Ile Ser Val Asp Lys Ser Lys Asn Gln Phe Ser
   65                  70                  75                  80
   ctg aag ctg agc tct gtg acc gcc gcg gac acg gcc gtg tat tac tgt     288
   Leu Lys Leu Ser Ser Val Thr Ala Ala Asp Thr Ala Val Tyr Tyr Cys
                   85                  90                  95
   gcg aga gaa aaa tcg ggt atg gac gtc tgg ggc caa ggg acc acg gtc     336
   Ala Arg Glu Lys Ser Gly Met Asp Val Trp Gly Gln Gly Thr Thr Val
               100                 105                 110
   acc gtc tca agc                                                     348
   Thr Val Ser Ser
           115
<210> 208
   <211> 116
   <212> PRT
   <213> Artificial Sequence
<220>
   <223> heavy chain variable region
<400> 208
   Glu Val Gln Leu Val Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr Asn Tyr Asn Pro Ser Leu
       50                  55                  60
   Lys Ser Arg Val Thr Ile Ser Val Asp Lys Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Glu Lys Ser Gly Met Asp Val Trp Gly Gln Gly Thr Thr Val
               100                 105                 110
   Thr Val Ser Ser
           115
<210> 209
   <211> 321
   <212> DNA
   <213> Artificial Sequence
<220>
   <223> light chain constant region
<220>
   <221> CDS
   <222> (1)..(321)
<400> 209
   cga act gtg gct gca cca tct gtc ttc atc ttc ccg cca tct gat gag        48
   cag ttg aaa tct gga act gcc tct gtt gtg tgc ctg ctg aat aac ttc        96
   tat ccc aga gag gcc aaa gta cag tgg aag gtg gat aac gcc ctc caa       144
   tcg ggt aac tcc cag gag agt gtc aca gag cag gac agc aag gac agc       192
   acc tac agc ctc agc agc acc ctg acg ctg agc aaa gca gac tac gag       240
   aaa cac aaa gtc tac gcc tgc gaa gtc acc cat cag ggc ctg agc tcg       288
   ccc gtc aca aag agc ttc aac agg gga gag tgt                           321
<210> 210
   <211> 107
   <212> PRT
   <213> Artificial Sequence
<220>
   <223> light chain constant region
<400> 210
<210> 211
   <211> 990
   <212> DNA
   <213> Artificial Sequence
<220>
   <223> heavy chain constant region
<220>
   <221> CDS
   <222> (1)..(990)
<400> 211
   gcc tcc acc aag ggc cca tcg gtc ttc ccc ctg gca ccc tcc tcc aag       48
   agc acc tct ggg ggc aca gcg gcc ctg ggc tgc ctg gtc aag gac tac       96
   ttc ccc gaa ccg gtg acg gtg tcg tgg aac tca ggc gcc ctg acc agc      144
   ggc gtg cac acc ttc ccg gct gtc cta cag tcc tca gga ctc tac tcc      192
   ctc agc agc gtg gtg acc gtg ccc tcc agc agc ttg ggc acc cag acc      240
   tac atc tgc aac gtg aat cac aag ccc agc aac acc aag gtg gac aag      288
   aaa gtt gag ccc aaa tct tgt gac aaa act cac aca tgc cca ccg tgc      336
   cca gca cct gaa ctc ctg ggg gga ccg tca gtc ttc ctc ttc ccc cca      384
   aaa ccc aag gac acc ctc atg atc tcc cgg acc cct gag gtc aca tgc      432
   gtg gtg gtg gac gtg agc cac gaa gac cct gag gtc aag ttc aac tgg      480
   tac gtg gac ggc gtg gag gtg cat aat gcc aag aca aag ccg cgg gag      528
   gag cag tac aac agc acg tac cgt gtg gtc agc gtc ctc acc gtc ctg      576
   cac cag gac tgg ctg aat ggc aag gag tac aag tgc aag gtc tcc aac      624
   aaa gcc ctc cca gcc ccc atc gag aaa acc atc tcc aaa gcc aaa ggg       672
   cag ccc cga gaa cca cag gtg tac acc ctg ccc cca tcc cgg gat gag       720
   ctg acc aag aac cag gtc agc ctg acc tgc ctg gtc aaa ggc ttc tat       768
   ccc agc gac atc gcc gtg gag tgg gag agc aat ggg cag ccg gag aac       816
   aac tac aag acc acg cct ccc gtg ctg gac tcc gac ggc tcc ttc ttc       864
   ctc tat agc aag ctc acc gtg gac aag agc agg tgg cag cag ggg aac       912
   gtc ttc tca tgc tcc gtg atg cat gag gct ctg cac aac cac tac acg       960
   cag aag agc ctc tcc ctg tct ccg ggt aaa                               990
<210> 212
   <211> 330
   <212> PRT
   <213> Artificial Sequence
<220>
   <223> heavy chain constant region
<400> 212
<210> 213
   <211> 9
   <212> PRT
   <213> Artificial Sequence
<220>
   <223> Light chain CDR3
<220>
   <221> MISC_FEATURE
   <222> (8)..(8)
   <223> Xaa can be any naturally occuring amino acid
<400> 213
   Met Gln Ala Leu Gln Thr Pro Xaa Thr
   1               5
<210> 214
   <211> 9
   <212> PRT
   <213> Artificial Sequence
<220>
   <223> Light chain CDR3
<220>
   <221> MISC_FEATURE
   <222> (3)..(3)
   <223> Xaa is arginine or serine
<220>
   <221> MISC_FEATURE
   <222> (4)..(4)
   <223> Xaa is asparagine or serine
<220>
   <221> MISC_FEATURE
   <222> (5)..(5)
   <223> Xaa is serine or asparagine
<220>
   <221> MISC_FEATURE
   <222> (6)..(6)
   <223> Xaa is glycine, alanine, valine, leucine, isoleucine, proline, phenylalinine, methionine, tryptophan or cysteine
<400> 214
   Gln Gln Xaa Xaa Xaa Xaa Pro Leu Thr
   1               5
<210> 215
   <211> 10
   <212> PRT
   <213> Artificial Sequence
<220>
   <223> Light chain CDR3
<220>
   <221> MISC_FEATURE
   <222> (8)..(9)
   <223> Xaa is arginine, valine, or isoleucine or no amino acid
<400> 215
   Gln Ser Tyr Asp Ser Ser Asn Xaa Xaa Val
   1               5                   10
<210> 216
   <211> 8
   <212> PRT
   <213> Artificial Sequence
<220>
   <223> Heavy chain CDR3
<400> 216
   Ser Arg Leu Asp Ala Phe Asp Ile
   1               5
<210> 217
   <211> 10
   <212> PRT
   <213> Artificial Sequence
<220>
   <223> Heavy chain CDR3
<220>
   <221> MISC_FEATURE
   <222> (2)..(2)
   <223> Xaa can be any naturally occurring amino acid
<400> 217
   Ser Xaa Tyr Asp Tyr Tyr Gly Met Asp Val
   1               5                   10
<210> 218
   <211> 11
   <212> PRT
   <213> Artificial Sequence
<220>
   <223> Heavy chain CDR3
<220>
   <221> MISC_FEATURE
   <222> (3)..(3)
   <223> Xaa can be any naturally occurring amino acid
<220>
   <221> MISC_FEATURE
   <222> (5)..(5)
   <223> Xaa can be any naturally occurring amino acid
<400> 218
   His Arg Xaa Asp Xaa Ala Trp Tyr Phe Asp Leu
   1 5 10
<210> 219
   <211> 4
   <212> PRT
   <213> Artificial Sequence
<220>
   <223> Heavy chain CDR3
<220>
   <221> MISC_FEATURE
   <222> (1)..(4)
<400> 219
   Asp Ser Ser Gly
   1
<210> 220
   <211> 16
   <212> PRT
   <213> Artificial Sequence
<220>
   <223> Light chain CDR1
<220>
   <221> MISC_FEATURE
   <222> (1)..(16)
<400> 220
   Arg Ser Ser Gln Ser Leu Leu His Ser Asn Gly Tyr Asn Tyr Leu Asp
   1 5 10 15
<210> 221
   <211> 11
   <212> PRT
   <213> Artificial Sequence
<220>
   <223> Light chain CDR1
<220>
   <221> MISC_FEATURE
   <222> (5)..(5)
   <223> Xaa is glycine or serine
<220>
   <221> MISC_FEATURE
   <222> (6)..(6)
   <223> Xaa is isoleucine or valine
<220>
   <221> MISC_FEATURE
   <222> (7)..(7)
   <223> Xaa is glycine or serine
<220>
   <221> MISC_FEATURE
   <222> (8)..(8)
   <223> Xaa is any amino acid
<220>
   <221> MISC_FEATURE
   <222> (9)..(9)
   <223> Xaa is tyrosine or phenyalanine
<220>
   <221> MISC_FEATURE
   <222> (11)..(11)
   <223> Xaa is alanine or asparagine
<400> 221
   Arg Ala Ser Gln Xaa Xaa Xaa Xaa Xaa Leu Xaa
   1               5                   10
<210> 222
   <211> 11
   <212> PRT
   <213> Artificial Sequence
<220>
   <223> Light chain CDR1
<220>
   <221> MISC_FEATURE
   <222> (6)..(6)
   <223> Xaa is leucine or serine
<220>
   <221> MISC_FEATURE
   <222> (7)..(11)
   <223> Xaa is independently any amino acid
<400> 222
   Arg Ser Ser Gln Ser Xaa Xaa Xaa Xaa Xaa Xaa
   1               5                   10
<210> 223
   <211> 7
   <212> PRT
   <213> Artificial Sequence
<220>
   <223> Light chain CDR2
<400> 223
   Leu Gly Ser Asn Arg Ala Ser
   1 5
<210> 224
   <211> 7
   <212> PRT
   <213> Artificial Sequence
<220>
   <223> Light chain CDR2
<400> 224
   Ala Ala Ser Thr Leu Gln Ser
   1 5
<210> 225
   <211> 7
   <212> PRT
   <213> Artificial Sequence
<220>
   <223> Light chain CDR2
<220>
   <221> MISC_FEATURE
   <222> (4)..(4)
   <223> Xaa can be any naturally occurring amino acid
<400> 225
   Glu Asp Asn Xaa Arg Pro Ser
   1 5
<210> 226
   <211> 6
   <212> PRT
   <213> Artificial Sequence
<220>
   <223> Heavy chain CDR1
<400> 226
   Ser Ser Asn Trp Trp Ser
   1 5
<210> 227
   <211> 5
   <212> PRT
   <213> Artificial Sequence
<220>
   <223> Heavy chain CDR1
<220>
   <221> MISC_FEATURE
   <222> (1)..(1)
   <223> Xaa can be any naturally occurring amino acid
<400> 227
   Xaa Tyr Tyr Trp Ser
   1               5
<210> 228
   <211> 5
   <212> PRT
   <213> Artificial Sequence
<220>
   <223> Heavy chain CDR1
<220>
   <221> MISC_FEATURE
   <222> (5)..(5)
   <223> Xaa is serine or histidine
<400> 228
   Ser Tyr Ala Met Xaa
   1               5
<210> 229
   <211> 16
   <212> PRT
   <213> Artificial Sequence
<220>
   <223> Heavy chain CDR2
<220>
   <221> MISC_FEATURE
   <222> (1)..(1)
   <223> Xaa = glutamic acid or isoleucine
<220>
   <221> MISC_FEATURE
   <222> (2)..(2)
   <223> Xaa = isoleucine or valine
<220>
   <221> MISC_FEATURE
   <222> (3)..(3)
   <223> Xaa = tyrosine or asparagine
<220>
   <221> MISC_FEATURE
   <222> (4)..(4)
   <223> Xaa = histidine or tyrosine
<220>
   <221> MISC_FEATURE
   <222> (9)..(9)
   <223> Xaa = asparagine or tyrosine
<400> 229
   Xaa Xaa Xaa Xaa Ser Gly Ser Thr Xaa Tyr Asn Pro Ser Leu Lys Ser
   1               5                   10                  15
<210> 230
   <211> 17
   <212> PRT
   <213> Artificial Sequence
<220>
   <223> Heavy chain CDR2
<220>
   <221> MISC_FEATURE
   <222> (1)..(1)
   <223> Xaa = any amino acid
<220>
   <221> MISC_FEATURE
   <222> (4)..(4)
   <223> Xaa = glycine or serine
<220>
   <221> MISC_FEATURE
   <222> (7)..(7)
   <223> Xaa = glycine or serine
<400> 230
   Xaa Ile Ser Xaa Ser Gly Xaa Ser Thr Tyr Tyr Ala Asp Ser Val Lys
   1               5                   10                  15
   Gly
<210> 231
   <211> 1162
   <212> PRT
   <213> Artificial Sequence
<220>
   <223> huIGF-1R:Fc
<220>
   <221> MISC_FEATURE
   <222> (1)..(1162)
<400> 231
   Met Lys Ser Gly Ser Gly Gly Gly Ser Pro Thr Ser Leu Trp Gly Leu
   1               5                   10                  15
   Leu Phe Leu Ser Ala Ala Leu Ser Leu Trp Pro Thr Ser Gly Glu Ile
               20                  25                  30
   Cys Gly Pro Gly Ile Asp Ile Arg Asn Asp Tyr Gln Gln Leu Lys Arg
           35                  40                  45
   Leu Glu Asn Cys Thr Val Ile Glu Gly Tyr Leu His Ile Leu Leu Ile
       50                  55                  60
   Ser Lys Ala Glu Asp Tyr Arg Ser Tyr Arg Phe Pro Lys Leu Thr Val
   65                  70                  75                  80
   Ile Thr Glu Tyr Leu Leu Leu Phe Arg Val Ala Gly Leu Glu Ser Leu
                   85                  90                  95
   Gly Asp Leu Phe Pro Asn Leu Thr Val Ile Arg Gly Trp Lys Leu Phe
               100                 105                 110
   Tyr Asn Tyr Ala Leu Val Ile Phe Glu Met Thr Asn Leu Lys Asp Ile
           115                 120                 125
   Gly Leu Tyr Asn Leu Arg Asn Ile Thr Arg Gly Ala Ile Arg Ile Glu
       130                 135                 140
   Lys Asn Ala Asp Leu Cys Tyr Leu Ser Thr Val Asp Trp Ser Leu Ile
   145                 150                 155                 160
   Leu Asp Ala Val Ser Asn Asn Tyr Ile Val Gly Asn Lys Pro Pro Lys
                   165                 170                 175
   Glu Cys Gly Asp Leu Cys Pro Gly Thr Met Glu Glu Lys Pro Met Cys
               180                 185                 190
   Glu Lys Thr Thr Ile Asn Asn Glu Tyr Asn Tyr Arg Cys Trp Thr Thr
           195                 200                 205
   Asn Arg Cys Gln Lys Met Cys Pro Ser Thr Cys Gly Lys Arg Ala Cys
       210                 215                 220
   Thr Glu Asn Asn Glu Cys Cys His Pro Glu Cys Leu Gly Ser Cys Ser
   225                 230                 235                 240
   Ala Pro Asp Asn Asp Thr Ala Cys Val Ala Cys Arg His Tyr Tyr Tyr
                   245                 250                 255
   Ala Gly Val Cys Val Pro Ala Cys Pro Pro Asn Thr Tyr Arg Phe Glu
               260                 265                 270
   Gly Trp Arg Cys Val Asp Arg Asp Phe Cys Ala Asn Ile Leu Ser Ala
   275 280 285
   Glu Ser Ser Asp Ser Glu Gly Phe Val Ile His Asp Gly Glu Cys Met
   290 295 300
   Gln Glu Cys Pro Ser Gly Phe Ile Arg Asn Gly Ser Gln Ser Met Tyr
   305 310 315 320
   Cys Ile Pro Cys Glu Gly Pro Cys Pro Lys Val Cys Glu Glu Glu Lys
   325 330 335
   Lys Thr Lys Thr Ile Asp Ser Val Thr Ser Ala Gln Met Leu Gln Gly
   340 345 350
   Cys Thr Ile Phe Lys Gly Asn Leu Leu Ile Asn Ile Arg Arg Gly Asn
   355 360 365
   Asn Ile Ala Ser Glu Leu Glu Asn Phe Met Gly Leu Ile Glu Val Val
   370 375 380
   Thr Gly Tyr Val Lys Ile Arg His Ser His Ala Leu Val Ser Leu Ser
   385 390 395 400
   Phe Leu Lys Asn Leu Arg Leu Ile Leu Gly Glu Glu Gln Leu Glu Gly
   405 410 415
   Asn Tyr Ser Phe Tyr Val Leu Asp Asn Gln Asn Leu Gln Gln Leu Trp
   420 425 430
   Asp Trp Asp His Arg Asn Leu Thr Ile Lys Ala Gly Lys Met Tyr Phe
   435 440 445
   Ala Phe Asn Pro Lys Leu Cys Val Ser Glu Ile Tyr Arg Met Glu Glu
   450 455 460
   Val Thr Gly Thr Lys Gly Arg Gln Ser Lys Gly Asp Ile Asn Thr Arg
   465 470 475 480
   Asn Asn Gly Glu Arg Ala Ser Cys Glu Ser Asp Val Leu His Phe Thr
   485 490 495
   Ser Thr Thr Thr Ser Lys Asn Arg Ile Ile Ile Thr Trp His Arg Tyr
   500 505 510
   Arg Pro Pro Asp Tyr Arg Asp Leu Ile Ser Phe Thr Val Tyr Tyr Lys
   515 520 525
   Glu Ala Pro Phe Lys Asn Val Thr Glu Tyr Asp Gly Gln Asp Ala Cys
       530                 535                 540
   Gly Ser Asn Ser Trp Asn Met Val Asp Val Asp Leu Pro Pro Asn Lys
   545                 550                 555                 560
   Asp Val Glu Pro Gly Ile Leu Leu His Gly Leu Lys Pro Trp Thr Gln
                   565                 570                 575
   Tyr Ala Val Tyr Val Lys Ala Val Thr Leu Thr Met Val Glu Asn Asp
               580                 585                 590
   His Ile Arg Gly Ala Lys Ser Glu Ile Leu Tyr Ile Arg Thr Asn Ala
           595                 600                 605
   Ser Val Pro Ser Ile Pro Leu Asp Val Leu Ser Ala Ser Asn Ser Ser
       610                 615                 620
   Ser Gln Leu Ile Val Lys Trp Asn Pro Pro Ser Leu Pro Asn Gly Asn
   625                 630                 635                 640
   Leu Ser Tyr Tyr Ile Val Arg Trp Gln Arg Gln Pro Gln Asp Gly Tyr
                   645                 650                 655
   Leu Tyr Arg His Asn Tyr Cys Ser Lys Asp Lys Ile Pro Ile Arg Lys
               660                 665                 670
   Tyr Ala Asp Gly Thr Ile Asp Ile Glu Glu Val Thr Glu Asn Pro Lys
           675                 680                 685
   Thr Glu Val Cys Gly Gly Glu Lys Gly Pro Cys Cys Ala Cys Pro Lys
       690                 695                 700
   Thr Glu Ala Glu Lys Gln Ala Glu Lys Glu Glu Ala Glu Tyr Arg Lys
   705                 710                 715                 720
   Val Phe Glu Asn Phe Leu His Asn Ser Ile Phe Val Pro Arg Pro Glu
                   725                 730                 735
   Arg Lys Arg Arg Asp Val Met Gln Val Ala Asn Thr Thr Met Ser Ser
               740                 745                 750
   Arg Ser Arg Asn Thr Thr Ala Ala Asp Thr Tyr Asn Ile Thr Asp Pro
           755                 760                 765
   Glu Glu Leu Glu Thr Glu Tyr Pro Phe Phe Glu Ser Arg Val Asp Asn
       770                 775                 780
   Lys Glu Arg Thr Val Ile Ser Asn Leu Arg Pro Phe Thr Leu Tyr Arg
   785 790 795 800
   Ile Asp Ile His Ser Cys Asn His Glu Ala Glu Lys Leu Gly Cys Ser
   805 810 815
   Ala Ser Asn Phe Val Phe Ala Arg Thr Met Pro Ala Glu Gly Ala Asp
   820 825 830
   Asp Ile Pro Gly Pro Val Thr Trp Glu Pro Arg Pro Glu Asn Ser Ile
   835 840 845
   Phe Leu Lys Trp Pro Glu Pro Glu Asn Pro Asn Gly Leu Ile Leu Met
   850 855 860
   Tyr Glu Ile Lys Tyr Gly Ser Gln Val Glu Asp Gln Arg Glu Cys Val
   865 870 875 880
   Ser Arg Gln Glu Tyr Arg Lys Tyr Gly Gly Ala Lys Leu Asn Arg Leu
   885 890 895
   Asn Pro Gly Asn Tyr Thr Ala Arg Ile Gln Ala Thr Ser Leu Ser Gly
   900 905 910
   Asn Gly Ser Trp Thr Asp Pro Val Phe Phe Tyr Val Gln Ala Lys Thr
   915 920 925
   Gly Tyr Glu Asn Phe Ile His Leu Asp Glu Val Asp Gly Cys Lys Pro
   930 935 940
   Cys Ile Cys Thr Val Pro Glu Val Ser Ser Val Phe Ile Phe Pro Pro
   945 950 955 960
   Lys Pro Lys Asp Val Leu Thr Ile Thr Leu Thr Pro Lys Val Thr Cys
   965 970 975
   Val Val Val Asp Ile Ser Lys Asp Asp Pro Glu Val Gln Phe Ser Trp
   980 985 990
   Phe Val Asp Asp Val Glu Val His Thr Ala Gln Thr Gln Pro Arg Glu
   995 1000 1005
   Glu Gln Phe Asn Ser Thr Phe Arg Ser Val Ser Glu Leu Pro Ile
   1010 1015 1020
   Met His Gln Asp Trp Leu Asn Gly Lys Glu Phe Lys Cys Arg Val
   1025 1030 1035
   Asn Ser Ala Ala Phe Pro Ala Pro Ile Glu Lys Thr Ile Ser Lys
   1040 1045 1050
   Thr Lys Gly Arg Pro Lys Ala Pro Gln Val Tyr Thr Ile Pro Pro
   1055 1060 1065
   Pro Lys Glu Gln Met Ala Lys Asp Lys Val Ser Leu Thr Cys Met
   1070 1075 1080
   Ile Thr Asp Phe Phe Pro Glu Asp Ile Thr Val Glu Trp Gln Trp
   1085 1090 1095
   Asn Gly Gln Pro Ala Glu Asn Tyr Lys Asn Thr Gln Pro Ile Met
   1100 1105 1110
   Asp Thr Asp Gly Ser Tyr Phe Val Tyr Ser Lys Leu Asn Val Gln
   1115 1120 1125
   Lys Ser Asn Trp Glu Ala Gly Asn Thr Phe Thr Cys Ser Val Leu
   1130 1135 1140
   His Glu Gly Leu His Asn His His Thr Glu Lys Ser Leu Ser His
   1145 1150 1155
   Ser Pro Gly Lys
   1160
<210> 232
   <211> 1180
   <212> PRT
   <213> Artificial Sequence
<220>
   <223> hu INSR:fC
<220>
   <221> MISC_FEATURE
   <222> (1)..(1180)
<400> 232
   Met Gly Thr Gly Gly Arg Arg Gly Ala Ala Ala Ala Pro Leu Leu Val
   1 5 10 15
   Ala Val Ala Ala Leu Leu Leu Gly Ala Ala Gly His Leu Tyr Pro Gly
   20 25 30
   Glu Val Cys Pro Gly Met Asp Ile Arg Asn Asn Leu Thr Arg Leu His
   35 40 45
   Glu Leu Glu Asn Cys Ser Val Ile Glu Gly His Leu Gln Ile Leu Leu
       50                  55                  60
   Met Phe Lys Thr Arg Pro Glu Asp Phe Arg Asp Leu Ser Phe Pro Lys
   65                  70                  75                  80
   Leu Ile Met Ile Thr Asp Tyr Leu Leu Leu Phe Arg Val Tyr Gly Leu
                   85                  90                  95
   Glu Ser Leu Lys Asp Leu Phe Pro Asn Leu Thr Val Ile Arg Gly Ser
               100                 105                 110
   Arg Leu Phe Phe Asn Tyr Ala Leu Val Ile Phe Glu Met Val His Leu
           115                 120                 125
   Lys Glu Leu Gly Leu Tyr Asn Leu Met Asn Ile Thr Arg Gly Ser Val
       130                 135                 140
   Arg Ile Glu Lys Asn Asn Glu Leu Cys Tyr Leu Ala Thr Ile Asp Trp
   145                 150                 155                 160
   Ser Arg Ile Leu Asp Ser Val Glu Asp Asn His Ile Val Leu Asn Lys
                   165                 170                 175
   Asp Asp Asn Glu Glu Cys Gly Asp Ile Cys Pro Gly Thr Ala Lys Gly
               180                 185                 190
   Lys Thr Asn Cys Pro Ala Thr Val Ile Asn Gly Gln Phe Val Glu Arg
           195                 200                 205
   Cys Trp Thr His Ser His Cys Gln Lys Val Cys Pro Thr Ile Cys Lys
       210                 215                 220
   Ser His Gly Cys Thr Ala Glu Gly Leu Cys Cys His Ser Glu Cys Leu
   225                 230                 235                 240
   Gly Asn Cys Ser Gln Pro Asp Asp Pro Thr Lys Cys Val Ala Cys Arg
                   245                 250                 255
   Asn Phe Tyr Leu Asp Gly Arg Cys Val Glu Thr Cys Pro Pro Pro Tyr
               260                 265                 270
   Tyr His Phe Gln Asp Trp Arg Cys Val Asn Phe Ser Phe Cys Gln Asp
           275                 280                 285
   Leu His His Lys Cys Lys Asn Ser Arg Arg Gln Gly Cys His Gln Tyr
   290 295 300
   Val Ile His Asn Asn Lys Cys Ile Pro Glu Cys Pro Ser Gly Tyr Thr
   305 310 315 320
   Met Asn Ser Ser Asn Leu Leu Cys Thr Pro Cys Leu Gly Pro Cys Pro
   325 330 335
   Lys Val Cys His Leu Leu Glu Gly Glu Lys Thr Ile Asp Ser Val Thr
   340 345 350
   Ser Ala Gln Glu Leu Arg Gly Cys Thr Val Ile Asn Gly Ser Leu Ile
   355 360 365
   Ile Asn Ile Arg Gly Gly Asn Asn Leu Ala Ala Glu Leu Glu Ala Asn
   370 375 380
   Leu Gly Leu Ile Glu Glu Ile Ser Gly Tyr Leu Lys Ile Arg Arg Ser
   385 390 395 400
   Tyr Ala Leu Val Ser Leu Ser Phe Phe Arg Lys Leu Arg Leu Ile Arg
   405 410 415
   Gly Glu Thr Leu Glu Ile Gly Asn Tyr Ser Phe Tyr Ala Leu Asp Asn
   420 425 430
   Gln Asn Leu Arg Gln Leu Trp Asp Trp Ser Lys His Asn Leu Thr Thr
   435 440 445
   Thr Gln Gly Lys Leu Phe Phe His Tyr Asn Pro Lys Leu Cys Leu Ser
   450 455 460
   Glu Ile His Lys Met Glu Glu Val Ser Gly Thr Lys Gly Arg Gln Glu
   465 470 475 480
   Arg Asn Asp Ile Ala Leu Lys Thr Asn Gly Asp Lys Ala Ser Cys Glu
   485 490 495
   Asn Glu Leu Leu Lys Phe Ser Tyr Ile Arg Thr Ser Phe Asp Lys Ile
   500 505 510
   Leu Leu Arg Trp Glu Pro Tyr Trp Pro Pro Asp Phe Arg Asp Leu Leu
   515 520 525
   Gly Phe Met Leu Phe Tyr Lys Glu Ala Pro Tyr Gln Asn Val Thr Glu
   530 535 540
   Phe Asp Gly Gln Asp Ala Cys Gly Ser Asn Ser Trp Thr Val Val Asp
   545 550 555 560
   Ile Asp Pro Pro Leu Arg Ser Asn Asp Pro Lys Ser Gln Asn His Pro
   565 570 575
   Gly Trp Leu Met Arg Gly Leu Lys Pro Trp Thr Gln Tyr Ala Ile Phe
   580 585 590
   Val Lys Thr Leu Val Thr Phe Ser Asp Glu Arg Arg Thr Tyr Gly Ala
   595 600 605
   Lys Ser Asp Ile Ile Tyr Val Gln Thr Asp Ala Thr Asn Pro Ser Val
   610 615 620
   Pro Leu Asp Pro Ile Ser Val Ser Asn Ser Ser Ser Gln Ile Ile Leu
   625 630 635 640
   Lys Trp Lys Pro Pro Ser Asp Pro Asn Gly Asn Ile Thr His Tyr Leu
   645 650 655
   Val Phe Trp Glu Arg Gln Ala Glu Asp Ser Glu Leu Phe Glu Leu Asp
   660 665 670
   Tyr Cys Leu Lys Gly Leu Lys Leu Pro Ser Arg Thr Trp Ser Pro Pro
   675 680 685
   Phe Glu Ser Glu Asp Ser Gln Lys His Asn Gln Ser Glu Tyr Glu Asp
   690 695 700
   Ser Ala Gly Glu Cys Cys Ser Cys Pro Lys Thr Asp Ser Gln Ile Leu
   705 710 715 720
   Lys Glu Leu Glu Glu Ser Ser Phe Arg Lys Thr Phe Glu Asp Tyr Leu
   725 730 735
   His Asn Val Val Phe Val Pro Arg Lys Thr Ser Ser Gly Thr Gly Ala
   740 745 750
   Glu Asp Pro Arg Pro Ser Arg Lys Arg Arg Ser Leu Gly Asp Val Gly
   755 760 765
   Asn Val Thr Val Ala Val Pro Thr Val Ala Ala Phe Pro Asn Thr Ser
   770 775 780
   Ser Thr Ser Val Pro Thr Ser Pro Glu Glu His Arg Pro Phe Glu Lys
   785 790 795 800
   Val Val Asn Lys Glu Ser Leu Val Ile Ser Gly Leu Arg His Phe Thr
                   805                 810                 815
   Gly Tyr Arg Ile Glu Leu Gln Ala Cys Asn Gln Asp Thr Pro Glu Glu
               820                 825                 830
   Arg Cys Ser Val Ala Ala Tyr Val Ser Ala Arg Thr Met Pro Glu Ala
           835                 840                 845
   Lys Ala Asp Asp Ile Val Gly Pro Val Thr His Glu Ile Phe Glu Asn
       850                 855                 860
   Asn Val Val His Leu Met Trp Gln Glu Pro Lys Glu Pro Asn Gly Leu
   865                 870                 875                 880
   Ile Val Leu Tyr Glu Val Ser Tyr Arg Arg Tyr Gly Asp Glu Glu Leu
                   885                 890                 895
   His Leu Cys Val Ser Arg Lys His Phe Ala Leu Glu Arg Gly Cys Arg
               900                 905                 910
   Leu Arg Gly Leu Ser Pro Gly Asn Tyr Ser Val Arg Ile Arg Ala Thr
           915                 920                 925
   Ser Leu Ala Gly Asn Gly Ser Trp Thr Glu Pro Thr Tyr Phe Tyr Val
       930                 935                 940
   Thr Asp Tyr Leu Asp Val Pro Ser Asn Ile Ala Lys Val Asp Gly Cys
   945                 950                 955                 960
   Lys Pro Cys Ile Cys Thr Val Pro Glu Val Ser Ser Val Phe Ile Phe
                   965                 970                 975
   Pro Pro Lys Pro Lys Asp Val Leu Thr Ile Thr Leu Thr Pro Lys Val
               980                 985                 990
   Thr Cys Val Val Val Asp Ile Ser  Lys Asp Asp Pro Glu  Val Gln Phe
           995                 1000                1005
   Ser Trp  Phe Val Asp Asp Val  Glu Val His Thr Ala  Gln Thr Gln
       1010                1015                1020
   Pro Arg  Glu Glu Gln Phe Asn  Ser Thr Phe Arg Ser  Val Ser Glu
       1025                1030                1035
   Leu Pro  Ile Met His Gln Asp  Trp Leu Asn Gly Lys  Glu Phe Lys
       1040                1045                1050
   Cys Arg Val Asn Ser Ala Ala Phe Pro Ala Pro Ile Glu Lys Thr
   1055 1060 1065
   Ile Ser Lys Thr Lys Gly Arg Pro Lys Ala Pro Gln Val Tyr Thr
   1070 1075 1080
   Ile Pro Pro Pro Lys Glu Gln Met Ala Lys Asp Lys Val Ser Leu
   1085 1090 1095
   Thr Cys Met Ile Thr Asp Phe Phe Pro Glu Asp Ile Thr Val Glu
   1100 1105 1110
   Trp Gln Trp Asn Gly Gln Pro Ala Glu Asn Tyr Lys Asn Thr Gln
   1115 1120 1125
   Pro Ile Met Asp Thr Asp Gly Ser Tyr Phe Val Tyr Ser Lys Leu
   1130 1135 1140
   Asn Val Gln Lys Ser Asn Trp Glu Ala Gly Asn Thr Phe Thr Cys
   1145 1150 1155
   Ser Val Leu His Glu Gly Leu His Asn His His Thr Glu Lys Ser
   1160 1165 1170
   Leu Ser His Ser Pro Gly Lys
   1175 1180
<210> 233
   <211> 1062
   <212> PRT
   <213> Artificial Sequence
<220>
   <223> hu IGF-1R:avidin
<400> 233
   Met Lys Ser Gly Ser Gly Gly Gly Ser Pro Thr Ser Leu Trp Gly Leu
   1 5 10 15
   Leu Phe Leu Ser Ala Ala Leu Ser Leu Trp Pro Thr Ser Gly Glu Ile
   20 25 30
   Cys Gly Pro Gly Ile Asp Ile Arg Asn Asp Tyr Gln Gln Leu Lys Arg
   35 40 45
   Leu Glu Asn Cys Thr Val Ile Glu Gly Tyr Leu His Ile Leu Leu Ile
   50 55 60
   Ser Lys Ala Glu Asp Tyr Arg Ser Tyr Arg Phe Pro Lys Leu Thr Val
   65                  70                  75                  80
   Ile Thr Glu Tyr Leu Leu Leu Phe Arg Val Ala Gly Leu Glu Ser Leu
                   85                  90                  95
   Gly Asp Leu Phe Pro Asn Leu Thr Val Ile Arg Gly Trp Lys Leu Phe
               100                 105                 110
   Tyr Asn Tyr Ala Leu Val Ile Phe Glu Met Thr Asn Leu Lys Asp Ile
           115                 120                 125
   Gly Leu Tyr Asn Leu Arg Asn Ile Thr Arg Gly Ala Ile Arg Ile Glu
       130                 135                 140
   Lys Asn Ala Asp Leu Cys Tyr Leu Ser Thr Val Asp Trp Ser Leu Ile
   145                 150                 155                 160
   Leu Asp Ala Val Ser Asn Asn Tyr Ile Val Gly Asn Lys Pro Pro Lys
                   165                 170                 175
   Glu Cys Gly Asp Leu Cys Pro Gly Thr Met Glu Glu Lys Pro Met Cys
               180                 185                 190
   Glu Lys Thr Thr Ile Asn Asn Glu Tyr Asn Tyr Arg Cys Trp Thr Thr
           195                 200                 205
   Asn Arg Cys Gln Lys Met Cys Pro Ser Thr Cys Gly Lys Arg Ala Cys
       210                 215                 220
   Thr Glu Asn Asn Glu Cys Cys His Pro Glu Cys Leu Gly Ser Cys Ser
   225                 230                 235                 240
   Ala Pro Asp Asn Asp Thr Ala Cys Val Ala Cys Arg His Tyr Tyr Tyr
                   245                 250                 255
   Ala Gly Val Cys Val Pro Ala Cys Pro Pro Asn Thr Tyr Arg Phe Glu
               260                 265                 270
   Gly Trp Arg Cys Val Asp Arg Asp Phe Cys Ala Asn Ile Leu Ser Ala
           275                 280                 285
   Glu Ser Ser Asp Ser Glu Gly Phe Val Ile His Asp Gly Glu Cys Met
       290                 295                 300
   Gln Glu Cys Pro Ser Gly Phe Ile Arg Asn Gly Ser Gln Ser Met Tyr
   305                 310                 315                 320
   Cys Ile Pro Cys Glu Gly Pro Cys Pro Lys Val Cys Glu Glu Glu Lys
   325 330 335
   Lys Thr Lys Thr Ile Asp Ser Val Thr Ser Ala Gln Met Leu Gln Gly
   340 345 350
   Cys Thr Ile Phe Lys Gly Asn Leu Leu Ile Asn Ile Arg Arg Gly Asn
   355 360 365
   Asn Ile Ala Ser Glu Leu Glu Asn Phe Met Gly Leu Ile Glu Val Val
   370 375 380
   Thr Gly Tyr Val Lys Ile Arg His Ser His Ala Leu Val Ser Leu Ser
   385 390 395 400
   Phe Leu Lys Asn Leu Arg Leu Ile Leu Gly Glu Glu Gln Leu Glu Gly
   405 410 415
   Asn Tyr Ser Phe Tyr Val Leu Asp Asn Gln Asn Leu Gln Gln Leu Trp
   420 425 430
   Asp Trp Asp His Arg Asn Leu Thr Ile Lys Ala Gly Lys Met Tyr Phe
   435 440 445
   Ala Phe Asn Pro Lys Leu Cys Val Ser Glu Ile Tyr Arg Met Glu Glu
   450 455 460
   Val Thr Gly Thr Lys Gly Arg Gln Ser Lys Gly Asp Ile Asn Thr Arg
   465 470 475 480
   Asn Asn Gly Glu Arg Ala Ser Cys Glu Ser Asp Val Leu His Phe Thr
   485 490 495
   Ser Thr Thr Thr Ser Lys Asn Arg Ile Ile Ile Thr Trp His Arg Tyr
   500 505 510
   Arg Pro Pro Asp Tyr Arg Asp Leu Ile Ser Phe Thr Val Tyr Tyr Lys
   515 520 525
   Glu Ala Pro Phe Lys Asn Val Thr Glu Tyr Asp Gly Gln Asp Ala Cys
   530 535 540
   Gly Ser Asn Ser Trp Asn Met Val Asp Val Asp Leu Pro Pro Asn Lys
   545 550 555 560
   Asp Val Glu Pro Gly Ile Leu Leu His Gly Leu Lys Pro Trp Thr Gln
   565 570 575
   Tyr Ala Val Tyr Val Lys Ala Val Thr Leu Thr Met Val Glu Asn Asp
   580 585 590
   His Ile Arg Gly Ala Lys Ser Glu Ile Leu Tyr Ile Arg Thr Asn Ala
   595 600 605
   Ser Val Pro Ser Ile Pro Leu Asp Val Leu Ser Ala Ser Asn Ser Ser
   610 615 620
   Ser Gln Leu Ile Val Lys Trp Asn Pro Pro Ser Leu Pro Asn Gly Asn
   625 630 635 640
   Leu Ser Tyr Tyr Ile Val Arg Trp Gln Arg Gln Pro Gln Asp Gly Tyr
   645 650 655
   Leu Tyr Arg His Asn Tyr Cys Ser Lys Asp Lys Ile Pro Ile Arg Lys
   660 665 670
   Tyr Ala Asp Gly Thr Ile Asp Ile Glu Glu Val Thr Glu Asn Pro Lys
   675 680 685
   Thr Glu Val Cys Gly Gly Glu Lys Gly Pro Cys Cys Ala Cys Pro Lys
   690 695 700
   Thr Glu Ala Glu Lys Gln Ala Glu Lys Glu Glu Ala Glu Tyr Arg Lys
   705 710 715 720
   Val Phe Glu Asn Phe Leu His Asn Ser Ile Phe Val Pro Arg Pro Glu
   725 730 735
   Arg Lys Arg Arg Asp Val Met Gln Val Ala Asn Thr Thr Met Ser Ser
   740 745 750
   Arg Ser Arg Asn Thr Thr Ala Ala Asp Thr Tyr Asn Ile Thr Asp Pro
   755 760 765
   Glu Glu Leu Glu Thr Glu Tyr Pro Phe Phe Glu Ser Arg Val Asp Asn
   770 775 780
   Lys Glu Arg Thr Val Ile Ser Asn Leu Arg Pro Phe Thr Leu Tyr Arg
   785 790 795 800
   Ile Asp Ile His Ser Cys Asn His Glu Ala Glu Lys Leu Gly Cys Ser
   805 810 815
   Ala Ser Asn Phe Val Phe Ala Arg Thr Met Pro Ala Glu Gly Ala Asp
               820                 825                 830
   Asp Ile Pro Gly Pro Val Thr Trp Glu Pro Arg Pro Glu Asn Ser Ile
           835                 840                 845
   Phe Leu Lys Trp Pro Glu Pro Glu Asn Pro Asn Gly Leu Ile Leu Met
       850                 855                 860
   Tyr Glu Ile Lys Tyr Gly Ser Gln Val Glu Asp Gln Arg Glu Cys Val
   865                 870                 875                 880
   Ser Arg Gln Glu Tyr Arg Lys Tyr Gly Gly Ala Lys Leu Asn Arg Leu
                   885                 890                 895
   Asn Pro Gly Asn Tyr Thr Ala Arg Ile Gln Ala Thr Ser Leu Ser Gly
               900                 905                 910
   Asn Gly Ser Trp Thr Asp Pro Val Phe Phe Tyr Val Gln Ala Lys Thr
           915                 920                 925
   Gly Tyr Glu Ala Ala Ala Ala Arg Lys Cys Ser Leu Thr Gly Lys Trp
       930                 935                 940
   Thr Asn Asp Leu Gly Ser Asn Met Thr Ile Gly Ala Val Asn Ser Lys
   945                 950                 955                 960
   Gly Glu Phe Thr Gly Thr Tyr Thr Thr Ala Val Thr Ala Thr Ser Asn
                   965                 970                 975
   Glu Ile Lys Glu Ser Pro Leu His Gly Thr Gln Asn Thr Ile Asn Lys
               980                 985                 990
   Arg Thr Gln Pro Thr Phe Gly Phe  Thr Val Asn Trp Lys  Phe Ser Glu
           995                 1000                1005
   Ser Thr  Thr Val Phe Thr Gly  Gln Cys Phe Ile Asp  Arg Asn Gly
       1010                1015                1020
   Lys Glu  Val Leu Lys Thr Met  Trp Leu Leu Arg Ser  Ser Val Asn
       1025                1030                1035
   Asp Ile  Gly Asp Asp Trp Lys  Ala Thr Arg Val Gly  Ile Asn Ile
       1040                1045                1050
   Phe Thr  Arg Leu Arg Thr Gln  Lys Glu
       1055                1060
<210> 234
   <211> 107
   <212> PRT
   <213> Artificial Sequence
<220>
   <223> human kappa light chain constant region
<400> 234
<210> 235
   <211> 330
   <212> PRT
   <213> Artificial Sequence
<220>
   <223> heavy chain constant region
<400> 235
<210> 236
   <211> 16
   <212> PRT
   <213> Artificial Sequence
<220>
   <223> light chain CDR1
<220>
   <221> MISC_FEATURE
   <222> (9)..(9)
   <223> Xaa is serine or threonine residue
<220>
   <221> MISC_FEATURE
   <222> (10)..(10)
   <223> Xaa is asparagine, serine or histidine residue
<220>
   <221> MISC_FEATURE
   <222> (14)..(14)
   <223> Xaa is tyrosine or phenylalanine residue
<220>
   <221> MISC_FEATURE
   <222> (16)..(16)
   <223> Xaa is aspartate or asparagine residue
<400> 236
   Arg Ser Ser Gln Ser Leu Leu His Xaa Xaa Gly Tyr Asn Xaa Leu Xaa
   1               5                   10                  15
<210> 237
   <211> 13
   <212> PRT
   <213> Artificial Sequence
<220>
   <223> light chain CDR1
<220>
   <221> MISC_FEATURE
   <222> (6)..(6)
   <223> Xaa is serine or aspartate residue
<220>
   <221> MISC_FEATURE
   <222> (8)..(8)
   <223> Xaa is alanine or aspartate residue
<220>
   <221> MISC_FEATURE
   <222> (9)..(9)
   <223> Xaa is serine or asparagine residue
<400> 237
   Thr Arg Ser Ser Gly Xaa Ile Xaa Xaa Asn Tyr Val Gln
   1               5                   10
<210> 238
   <211> 11
   <212> PRT
   <213> Artificial Sequence
<220>
   <223> light chain CDR1
<220>
   <221> MISC_FEATURE
   <222> (5)..(5)
   <223> Xaa is glycine or serine residue
<220>
   <221> MISC_FEATURE
   <222> (6)..(6)
   <223> Xaa is isoleucine, valine or proline residue
<220>
   <221> MISC_FEATURE
   <222> (7)..(7)
   <223> Xaa is serine, glycine or tyrosine residue
<220>
   <221> MISC_FEATURE
   <222> (8)..(8)
   <223> Xaa is any amino acid
<220>
   <221> MISC_FEATURE
   <222> (9)..(9)
   <223> Xaa is phenylalanine, tyrosine, asparagine or tryptophan residue
<220>
   <221> MISC_FEATURE
   <222> (11)..(11)
   <223> Xaa is alanine or asparagine residue
<400> 238
   Arg Ala Ser Gln Xaa Xaa Xaa Xaa Xaa Leu Xaa
   1               5                   10
<210> 239
   <211> 7
   <212> PRT
   <213> Artificial Sequence
<220>
   <223> light chain CDR2
<220>
   <221> MISC_FEATURE
   <222> (2)..(2)
   <223> Xaa is glycine or valine residue
<220>
   <221> MISC_FEATURE
   <222> (3)..(3)
   <223> Xaa is serine or phenylalanine residue
<220>
   <221> MISC_FEATURE
   <222> (4)..(4)
   <223> Xaa is asparagine, tyrosine or threonine residue
<220>
   <221> MISC_FEATURE
   <222> (6)..(6)
   <223> Xaa is alanine or aspartate residue
<400> 239
   Leu Xaa Xaa Xaa Arg Xaa Ser
   1               5
<210> 240
   <211> 7
   <212> PRT
   <213> Artificial Sequence
<220>
   <223> light chain CDR2
<220>
   <221> MISC_FEATURE
   <222> (2)..(2)
   <223> Xaa is alanine or threonine residue
<220>
   <221> MISC_FEATURE
   <222> (4)..(4)
   <223> Xaa is threonine or glycine residue
<220>
   <221> MISC_FEATURE
   <222> (6)..(6)
   <223> Xaa is glutamine or glutamate residue
<400> 240
   Ala Xaa Ser Xaa Leu Xaa Ser
   1               5
<210> 241
   <211> 7
   <212> PRT
   <213> Artificial Sequence
<220>
   <223> light chain CDR2
<220>
   <221> MISC_FEATURE
   <222> (1)..(1)
   <223> Xaa is glutamate, glutamine or glycine residue
<220>
   <221> MISC_FEATURE
   <222> (2)..(2)
   <223> Xaa is aspartate or lysine residue
<220>
   <221> MISC_FEATURE
   <222> (4)..(4)
   <223> Xaa is any amino acid residue
<400> 241
   Xaa Xaa Asn Xaa Arg Pro Ser
   1 5
<210> 242
   <211> 9
   <212> PRT
   <213> Artificial Sequence
<220>
   <223> light chain CDR3
<220>
   <221> MISC_FEATURE
   <222> (2)..(2)
   <223> Xaa is glutamine or glutamate residue
<220>
   <221> MISC_FEATURE
   <222> (3)..(3)
   <223> Xaa is alanine, glycine, serine or threonine residue
<220>
   <221> MISC_FEATURE
   <222> (4)..(4)
   <223> Xaa is leucine or threonine residue
<220>
   <221> MISC_FEATURE
   <222> (5)..(5)
   <223> Xaa is glutamine, glutamate or histidine residue
<220>
   <221> MISC_FEATURE
   <222> (6)..(6)
   <223> Xaa is threonine, tryptophan, methionine or valine residue
<220>
   <221> MISC_FEATURE
   <222> (8)..(8)
   <223> Xaa is nonpolar side chain
<220>
   <221> MISC_FEATURE
   <222> (9)..(9)
   <223> Xaa is threonine, serine or alanine residue
<400> 242
   Met Xaa Xaa Xaa Xaa Xaa Pro Xaa Xaa
   1               5
<210> 243
   <211> 9
   <212> PRT
   <213> Artificial Sequence
<220>
   <223> light chain CDR3
<220>
   <221> MISC_FEATURE
   <222> (3)..(3)
   <223> Xaa is arginine, serine, leucine or alanine residue
<220>
   <221> MISC_FEATURE
   <222> (4)..(4)
   <223> Xaa is asparagine, serine or histidine residue
<220>
   <221> MISC_FEATURE
   <222> (5)..(5)
   <223> Xaa is serine or asparagine residue
<220>
   <221> MISC_FEATURE
   <222> (6)..(6)
   <223> Xaa is nonpolar side chain
<220>
   <221> MISC_FEATURE
   <222> (8)..(8)
   <223> Xaa is leucine, isoleucine, tyrosine or tryptophan residue
<400> 243
   Gln Gln Xaa Xaa Xaa Xaa Pro Xaa Thr
   1               5
<210> 244
   <211> 10
   <212> PRT
   <213> Artificial Sequence
<220>
   <223> light chain CDR3
<220>
   <221> MISC_FEATURE
   <222> (4)..(4)
   <223> Xaa is aspartate or glutamine residue
<220>
   <221> MISC_FEATURE
   <222> (6)..(6)
   <223> Xaa is serine or aspartate residue
<220>
   <221> MISC_FEATURE
   <222> (8)..(8)
   <223> Xaa is glutamine, valine or tryptophan residue
<220>
   <221> MISC_FEATURE
   <222> (9)..(9)
   <223> Xaa is arginine residue or no residue
<400> 244
   Gln Ser Tyr Xaa Ser Xaa Asn Xaa Xaa Val
   1               5                   10
<210> 245
   <211> 6
   <212> PRT
   <213> Artificial Sequence
<220>
   <223> heavy chain CDR1
<220>
   <221> MISC_FEATURE
   <222> (1)..(1)
   <223> Xaa is serine residue or no residue
<220>
   <221> MISC_FEATURE
   <222> (2)..(2)
   <223> Xaa is serine or asparagine residue
<220>
   <221> MISC_FEATURE
   <222> (3)..(3)
   <223> Xaa is asparagine and isoleucine residue
<400> 245
   Xaa Xaa Xaa Trp Trp Ser
   1               5
<210> 246
   <211> 5
   <212> PRT
   <213> Artificial Sequence
<220>
   <223> heavy chain CDR1
<220>
   <221> MISC_FEATURE
   <222> (1)..(1)
   <223> Xaa is glycine, asparagine or aspartate residue
<220>
   <221> MISC_FEATURE
   <222> (2)..(2)
   <223> Xaa is tyrosine or phenylalanine residue
<400> 246
   Xaa Xaa Tyr Trp Ser
   1               5
<210> 247
   <211> 5
   <212> PRT
   <213> Artificial Sequence
<220>
   <223> heavy chain CDR1
<220>
   <221> MISC_FEATURE
   <222> (3)..(3)
   <223> Xaa is alanine or glycine residue
<220>
   <221> MISC_FEATURE
   <222> (4)..(4)
   <223> Xaa is methionine or isoleucine residue
<220>
   <221> MISC_FEATURE
   <222> (5)..(5)
   <223> Xaa is serine or histidine residue
<400> 247
   Ser Tyr Xaa Xaa Xaa
   1               5
<210> 248
   <211> 16
   <212> PRT
   <213> Artificial Sequence
<220>
   <223> heavy chain CDR2
<220>
   <221> MISC_FEATURE
   <222> (1)..(1)
   <223> Xaa is glutamate, tyrosine or serine residue
<220>
   <221> MISC_FEATURE
   <222> (2)..(2)
   <223> Xaa is isoleucine or valine residue
<220>
   <221> MISC_FEATURE
   <222> (3)..(3)
   <223> Xaa is tyrosine, asparagine or serine residue
<220>
   <221> MISC_FEATURE
   <222> (4)..(4)
   <223> Xaa is histidine, tyrosine, aspartate,or proline residue
<220>
   <221> MISC_FEATURE
   <222> (5)..(5)
   <223> Xaa is serine or arginine residue
<220>
   <221> MISC_FEATURE
   <222> (7)..(7)
   <223> Xaa is serine or arginine residue
<220>
   <221> MISC_FEATURE
   <222> (9)..(9)
   <223> Xaa is asparagine or tyrosine residue
<220>
   <221> MISC_FEATURE
   <222> (15)..(15)
   <223> Xaa is lysine or glutamate residue
<400> 248
   Xaa Xaa Xaa Xaa Xaa Gly Xaa Thr Xaa Tyr Asn Pro Ser Leu Xaa Ser
   1               5                   10                  15
<210> 249
   <211> 17
   <212> PRT
   <213> Artificial Sequence
<220>
   <223> heavy chain CDR2
<220>
   <221> MISC_FEATURE
   <222> (1)..(1)
   <223> Xaa is threonine, alanine, valine or tyrosine residue
<220>
   <221> MISC_FEATURE
   <222> (4)..(4)
   <223> Xaa is glycine, serine or tyrosine residue
<220>
   <221> MISC_FEATURE
   <222> (5)..(5)
   <223> Xaa is serine, asparagine or aspartate residue
<220>
   <221> MISC_FEATURE
   <222> (6)..(6)
   <223> Xaa is glycine or serine residue
<220>
   <221> MISC_FEATURE
   <222> (7)..(7)
   <223> Xaa is glycine, serine or aspartate residue
<220>
   <221> MISC_FEATURE
   <222> (8)..(8)
   <223> Xaa is serine, threonine or asparagine residue
<220>
   <221> MISC_FEATURE
   <222> (9)..(9)
   <223> Xaa is threonine, lysine or isoleucine residue
<400> 249
   Xaa Ile Ser Xaa Xaa Xaa Xaa Xaa Xaa Tyr Tyr Ala Asp Ser Val Lys
   1               5                   10                  15
   Gly
<210> 250
   <211> 12
   <212> PRT
   <213> Artificial Sequence
<220>
   <223> heavy chain CDR3
<220>
   <221> MISC_FEATURE
   <222> (1)..(1)
   <223> Xaa is glutamate or no residue
<220>
   <221> MISC_FEATURE
   <222> (2)..(2)
   <223> Xaa is tyrosine, glycine or serine or no residue
<220>
   <221> MISC_FEATURE
   <222> (3)..(3)
   <223> Xaa is serine, asparagine, tryptophan or glutamate or no residue
<220>
   <221> MISC_FEATURE
   <222> (4)..(4)
   <223> Xaa is serine, aspartate, tryptophan, alanine, arginine, threonine, glutamine, leucine or glutamate or no residue
<220>
   <221> MISC_FEATURE
   <222> (5)..(5)
   <223> Xaa is serine, glycine, asparagine, threonine, tryptophan, alanine, valine or isoleucine residue
<220>
   <221> MISC_FEATURE
   <222> (6)..(6)
   <223> Xaa is arginine, glutamine, tyrosine, valine, alanine, glycine, serine, phenylalanine or tryptophan residue
<220>
   <221> MISC_FEATURE
   <222> (7)..(7)
   <223> Xaa is leucine, asparagine, aspartate, threonine, tryptophan, tyrosine, valine, alanine, or histidine residue
<220>
   <221> MISC_FEATURE
   <222> (8)..(8)
   <223> Xaa is aspartate, serine, asparagine or glutamine residue
<220>
   <221> MISC_FEATURE
   <222> (9)..(9)
   <223> Xaa is alanine or proline residue
<400> 250
   Xaa Xaa Xaa Xaa Xaa Xaa Xaa Xaa Xaa Phe Asp Ile
   1               5                   10
<210> 251
   <211> 14
   <212> PRT
   <213> Artificial Sequence
<220>
   <223> heavy chain CDR3
<220>
   <221> MISC_FEATURE
   <222> (1)..(1)
   <223> Xaa is alanine or no residue
<220>
   <221> MISC_FEATURE
   <222> (2)..(2)
   <223> Xaa is glutamate, tryosine or glycine or no residue
<220>
   <221> MISC_FEATURE
   <222> (3)..(3)
   <223> Xaa is serine, asparagine, tryptophan, glutamate or no residue
<220>
   <221> MISC_FEATURE
   <222> (4)..(4)
   <223> Xaa is aspartate, glycine, serine, or valine residue, or no residue
<220>
   <221> MISC_FEATURE
   <222> (5)..(5)
   <223> Xaa is serine, glycine, or aspartate residue, or no residue
<220>
   <221> MISC_FEATURE
   <222> (6)..(6)
   <223> Xaa is glycine, phenylalanine, aspartate, serine, tryptophan, or tyrosine residue, or no residue
<220>
   <221> MISC_FEATURE
   <222> (7)..(7)
   <223> Xaa is a tyrosine, tryptophan, serine, or aspartate residue, or no residue
<220>
   <221> MISC_FEATURE
   <222> (8)..(8)
   <223> Xaa is aspartate, arginine, serine, glycine, tyrosine, or tryptophan residue
<220>
   <221> MISC_FEATURE
   <222> (9)..(9)
   <223> Xaa is tyrosine, isoleucine, leucine, phenylalanine, or lysine residue
<220>
   <221> MISC_FEATURE
   <222> (10)..(10)
   <223> Xaa is tyrosine, phenylalanine, aspartate, or glycine residue
<220>
   <221> MISC_FEATURE
   <222> (11)..(11)
   <223> Xaa is glycine, tyrosine, or asparagine residue
<400> 251
   Xaa Xaa Xaa Xaa Xaa Xaa Xaa Xaa Xaa Xaa Xaa Met Asp Val
   1               5                   10
<210> 252
   <211> 11
   <212> PRT
   <213> Artificial Sequence
<220>
   <223> heavy chain CDR3
<220>
   <221> MISC_FEATURE
   <222> (1)..(1)
   <223> Xaa is aspartate or valine residue, or no residue
<220>
   <221> MISC_FEATURE
   <222> (2)..(2)
   <223> Xaa is glycine, tyrosine, arginine, or aspartate residue, or no residue,
<220>
   <221> MISC_FEATURE
   <222> (3)..(3)
   <223> Xaa is asparagine, leucine, glycine, isoleucine, serine, valine, phenylalanine, or tyrosine residue, or no residue
<220>
   <221> MISC_FEATURE
   <222> (4)..(4)
   <223> Xaa is leucine, serine, tryptophan, alanine, tyrosine, isoleucine, glycine, or aspartate residue, or no residue
<220>
   <221> MISC_FEATURE
   <222> (5)..(5)
   <223> Xaa is glycine, alanine, tyrosine, serine, aspartate, or leucine residue
<220>
   <221> MISC_FEATURE
   <222> (6)..(6)
   <223> Xaa is valine, alanine, glycine, threonine, proline, histidine, or glutamine residue
<220>
   <221> MISC_FEATURE
   <222> (7)..(7)
   <223> Xaa is glutamate, glycine, serine, aspartate, glycine, valine, tryptophan, histidine, or arginine residue
<220>
   <221> MISC_FEATURE
   <222> (8)..(8)
   <223> Xaa is glutamine, alanine, glycine, tyrosine, proline, leucine, aspartate, or serine residue
<220>
   <221> MISC_FEATURE
   <222> (9)..(9)
   <223> Xaa is nonpolar side chain residue
<220>
   <221> MISC_FEATURE
   <222> (10)..(10)
   <223> Xaa is aspartate or alanine residue
<400> 252
   Xaa Xaa Xaa Xaa Xaa Xaa Xaa Xaa Xaa Xaa Tyr
   1               5                   10
<210> 253
   <211> 14
   <212> PRT
   <213> Artificial Sequence
<220>
   <223> Heavy chain CDR3
<220>
   <221> MISC_FEATURE
   <222> (1)..(1)
   <223> Xaa is glycine residue, or no residue
<220>
   <221> MISC_FEATURE
   <222> (2)..(2)
   <223> Xaa is proline residue, or no residue
<220>
   <221> MISC_FEATURE
   <222> (3)..(3)
   <223> Xaa is arginine or aspartate residue, or no residue
<220>
   <221> MISC_FEATURE
   <222> (4)..(4)
   <223> Xaa is histidine or proline residue
<220>
   <221> MISC_FEATURE
   <222> (5)..(5)
   <223> Xaa is arginine or glycine residue
<220>
   <221> MISC_FEATURE
   <222> (6)..(6)
   <223> Xaa is arginine, serine, or phenylalanine residue
<220>
   <221> MISC_FEATURE
   <222> (7)..(7)
   <223> Xaa is aspartate or serine residue
<220>
   <221> MISC_FEATURE
   <222> (8)..(8)
   <223> Xaa is glycine, tryptophan, or tyrosine residue
<220>
   <221> MISC_FEATURE
   <222> (9)..(9)
   <223> Xaa is tyrosine or alanine residue
<220>
   <221> MISC_FEATURE
   <222> (10)..(10)
   <223> Xaa is asparagine or tryptophan residue
<220>
   <221> MISC_FEATURE
   <222> (14)..(14)
   <223> Xaa is asparagine or leucine residue
<400> 253
   Xaa Xaa Xaa Xaa Xaa Xaa Xaa Xaa Xaa Xaa Tyr Phe Asp Xaa
   1               5                       10
<210> 254
   <211> 15
   <212> PRT
   <213> Artificial Sequence
<220>
   <223> heavy chain CDR3
<220>
   <221> MISC_FEATURE
   <222> (1)..(1)
   <223> Xaa is phenylalanine residue, or no residue
<220>
   <221> MISC_FEATURE
   <222> (2)..(2)
   <223> Xaa is asparagine or glycine residue, or no residue
<220>
   <221> MISC_FEATURE
   <222> (3)..(3)
   <223> Xaa is tyrosine or a leucine residue, or no residue
<220>
   <221> MISC_FEATURE
   <222> (4)..(4)
   <223> Xaa is a tyrosine or glycine residue, or no residue
<220>
   <221> MISC_FEATURE
   <222> (8)..(8)
   <223> Xaa is a glycine, serine, or valine residue
<220>
   <221> MISC_FEATURE
   <222> (9)..(9)
   <223> Xaa is tyrosine, phenylalanine, tryptophan, or glutamine residue, or no residue
<220>
   <221> MISC_FEATURE
   <222> (10)..(10)
   <223> Xaa is tyrosine, glycine, or isoleucine residue, or no residue
<220>
   <221> MISC_FEATURE
   <222> (11)..(11)
   <223> Xaa is tyrosine, leucine, or glycine residue, or no residue
<220>
   <221> MISC_FEATURE
   <222> (12)..(12)
   <223> Xaa is methionine, glycine, or phenylalanine residue, or no residue
<220>
   <221> MISC_FEATURE
   <222> (13)..(13)
   <223> Xaa is aspartate or methionine residue, or no residue
<220>
   <221> MISC_FEATURE
   <222> (14)..(14)
   <223> Xaa is a valine, aspartate, or tyrosine residue, or no residue
<220>
   <221> MISC_FEATURE
   <222> (15)..(15)
   <223> Xaa is a valine residue, or no residue
<400> 254
   Xaa Xaa Xaa Xaa Asp Ser Ser Xaa Xaa Xaa Xaa Xaa Xaa Xaa Xaa
   1               5                   10                  15
<210> 255
   <211> 8
   <212> DNA
   <213> Artificial Sequence
<220>
   <223> polypeptide
<400> 255
   dykddddk 8
<210> 256
   <211> 37
   <212> DNA
   <213> Artificial Sequence
<220>
   <223> nucleic acid
<400> 256
   agcaagcttc caccatgaag tctggctccg gaggagg 37
<210> 257
   <211> 36
   <212> DNA
   <213> Artificial Sequence
<220>
   <223> nucleic acid
<400> 257
   atttgtcgac ttcgtccaga tggatgaagt tttcat 36
<210> 258
   <211> 3486
   <212> DNA
   <213> Artificial Sequence
<220>
   <223> nucleic acid
<400> 258
   atgaagtctg gctccggagg agggtccccg acctcgctgt gggggctcct gtttctctcc     60
   gccgcgctct cgctctggcc gacgagtgga gaaatctgcg ggccaggcat cgacatccgc    120
   aacgactatc agcagctgaa gcgcctggag aactgcacgg tgatcgaggg ctacctccac    180
   atcctgctca tctccaaggc cgaggactac cgcagctacc gcttccccaa gctcacggtc    240
   attaccgagt acttgctgct gttccgagtg gctggcctcg agagcctcgg agacctcttc    300
   cccaacctca cggtcatccg cggctggaaa ctcttctaca actacgccct ggtcatcttc    360
   gagatgacca atctcaagga tattgggctt tacaacctga ggaacattac tcggggggcc 420
   atcaggattg agaaaaatgc tgacctctgt tacctctcca ctgtggactg gtccctgatc 480
   ctggatgcgg tgtccaataa ctacattgtg gggaataagc ccccaaagga atgtggggac 540
   ctgtgtccag ggaccatgga ggagaagccg atgtgtgaga agaccaccat caacaatgag 600
   tacaactacc gctgctggac cacaaaccgc tgccagaaaa tgtgcccaag cacgtgtggg 660
   aagcgggcgt gcaccgagaa caatgagtgc tgccaccccg agtgcctggg cagctgcagc 720
   gcgcctgaca acgacacggc ctgtgtagct tgccgccact actactatgc cggtgtctgt 780
   gtgcctgcct gcccgcccaa cacctacagg tttgagggct ggcgctgtgt ggaccgtgac 840
   ttctgcgcca acatcctcag cgccgagagc agcgactccg aggggtttgt gatccacgac 900
   ggcgagtgca tgcaggagtg cccctcgggc ttcatccgca acggcagcca gagcatgtac 960
   tgcatccctt gtgaaggtcc ttgcccgaag gtctgtgagg aagaaaagaa aacaaagacc 1020
   attgattctg ttacttctgc tcagatgctc caaggatgca ccatcttcaa gggcaatttg 1080
   ctcattaaca tccgacgggg gaataacatt gcttcagagc tggagaactt catggggctc 1140
   atcgaggtgg tgacgggcta cgtgaagatc cgccattctc atgccttggt ctccttgtcc 1200
   ttcctaaaaa accttcgcct catcctagga gaggagcagc tagaagggaa ttactccttc 1260
   tacgtcctcg acaaccagaa cttgcagcaa ctgtgggact gggaccaccg caacctgacc 1320
   atcaaagcag ggaaaatgta ctttgctttc aatcccaaat tatgtgtttc cgaaatttac 1380
   cgcatggagg aagtgacggg gactaaaggg cgccaaagca aaggggacat aaacaccagg 1440
   aacaacgggg agagagcctc ctgtgaaagt gacgtcctgc atttcacctc caccaccacg 1500
   tcgaagaatc gcatcatcat aacctggcac cggtaccggc cccctgacta cagggatctc 1560
   atcagcttca ccgtttacta caaggaagca ccctttaaga atgtcacaga gtatgatggg 1620
   caggatgcct gcggctccaa cagctggaac atggtggacg tggacctccc gcccaacaag 1680
   gacgtggagc ccggcatctt actacatggg ctgaagccct ggactcagta cgccgtttac 1740
   gtcaaggctg tgaccctcac catggtggag aacgaccata tccgtggggc caagagtgag 1800
   atcttgtaca ttcgcaccaa tgcttcagtt ccttccattc ccttggacgt tctttcagca 1860
   tcgaactcct cttctcagtt aatcgtgaag tggaaccctc cctctctgcc caacggcaac 1920
   ctgagttact acattgtgcg ctggcagcgg cagcctcagg acggctacct ttaccggcac 1980
   aattactgct ccaaagacaa aatccccatc aggaagtatg ccgacggcac catcgacatt 2040
   gaggaggtca cagagaaccc caagactgag gtgtgtggtg gggagaaagg gccttgctgc 2100
   gcctgcccca aaactgaagc cgagaagcag gccgagaagg aggaggctga ataccgcaaa 2160
   gtctttgaga atttcctgca caactccatc ttcgtgccca gacctgaaag gaagcggaga 2220
   gatgtcatgc aagtggccaa caccaccatg tccagccgaa gcaggaacac cacggccgca 2280
   gacacctaca acatcactga cccggaagag ctggagacag agtacccttt ctttgagagc 2340
   agagtggata acaaggagag aactgtcatt tctaaccttc ggcctttcac attgtaccgc 2400
   atcgatatcc acagctgcaa ccacgaggct gagaagctgg gctgcagcgc ctccaacttc 2460
   gtctttgcaa ggactatgcc cgcagaagga gcagatgaca ttcctgggcc agtgacctgg 2520
   gagccaaggc ctgaaaactc catcttttta aagtggccgg aacctgagaa tcccaatgga 2580
   ttgattctaa tgtatgaaat aaaatacgga tcacaagttg aggatcagcg agaatgtgtg 2640
   tccagacagg aatacaggaa gtatggaggg gccaagctaa accggctaaa cccggggaac 2700
   tacacagccc ggattcaggc cacatctctc tctgggaatg ggtcgtggac agatcctgtg 2760
   ttcttctatg tccaggccaa aacaggatat gaaaacttca tccatctgga cgaagtcgac 2820
   ggttgtaagc cttgcatatg tacagtccca gaagtatcat ctgtcttcat cttcccccca 2880
   aagcccaagg atgtgctcac cattactctg actcctaagg tcacgtgtgt tgtggtagac 2940
   atcagcaagg atgatcccga ggtccagttc agctggtttg tagatgatgt ggaggtgcac 3000
   acagctcaga cgcaaccccg ggaggagcag ttcaacagca ctttccgctc agtcagtgaa 3060
   cttcccatca tgcaccagga ctggctcaat ggcaaggagt tcaaatgcag ggtaaacagt 3120
   gcagctttcc ctgcccccat cgagaaaacc atctccaaaa ccaaaggcag accgaaggct 3180
   ccacaggtgt acaccattcc acctcccaag gagcagatgg ccaaggataa agtcagtctg 3240
   acctgcatga taacagactt cttccctgaa gacattactg tggagtggca gtggaatggg 3300
   cagccagcgg agaactacaa gaacactcag cccatcatgg acacagatgg ctcttacttc 3360
   gtctacagca agctcaatgt gcagaagagc aactgggagg caggaaatac tttcacctgc 3420
   tctgtgttac atgagggcct gcacaaccac catactgaga agagcctctc ccactctcct 3480
   ggtaaa 3486
<210> 259
   <211> 32
   <212> DNA
   <213> Artificial Sequence
<220>
   <223> nucleic acid
<400> 259
   agcaagcttc caccatgggc accgggggcc gg 32
<210> 260
   <211> 37
   <212> DNA
   <213> Artificial Sequence
<220>
   <223> nucleic acid
<400> 260
   atttgtcgac ttttgcaata tttgacggga cgtctaa 37
<210> 261
   <211> 3540
   <212> DNA
   <213> Artificial Sequence
<220>
   <223> nucleic acid
<400> 261
   atgggcaccg ggggccggcg gggggcggcg gccgcgccgc tgctggtggc ggtggccgcg 60
   ctgctactgg gcgccgcggg ccacctgtac cccggagagg tgtgtcccgg catggatatc 120
   cggaacaacc tcactaggtt gcatgagctg gagaattgct ctgtcatcga aggacacttg 180
   cagatactct tgatgttcaa aacgaggccc gaagatttcc gagacctcag tttccccaaa 240
   ctcatcatga tcactgatta cttgctgctc ttccgggtct atgggctcga gagcctgaag 300
   gacctgttcc ccaacctcac ggtcatccgg ggatcacgac tgttctttaa ctacgcgctg 360
   gtcatcttcg agatggttca cctcaaggaa ctcggcctct acaacctgat gaacatcacc 420
   cggggttctg tccgcatcga gaagaacaat gagctctgtt acttggccac tatcgactgg 480
   tcccgtatcc tggattccgt ggaggataat cacatcgtgt tgaacaaaga tgacaacgag 540
   gagtgtggag acatctgtcc gggtaccgcg aagggcaaga ccaactgccc cgccaccgtc 600
   atcaacgggc agtttgtcga acgatgttgg actcatagtc actgccagaa agtttgcccg 660
   accatctgta agtcacacgg ctgcaccgcc gaaggcctct gttgccacag cgagtgcctg 720
   ggcaactgtt ctcagcccga cgaccccacc aagtgcgtgg cctgccgcaa cttctacctg 780
   gacggcaggt gtgtggagac ctgcccgccc ccgtactacc acttccagga ctggcgctgt 840
   gtgaacttca gcttctgcca ggacctgcac cacaaatgca agaactcgcg gaggcagggc 900
   tgccaccagt acgtcattca caacaacaag tgcatccctg agtgtccctc cgggtacacg 960
   atgaattcca gcaacttgct gtgcacccca tgcctgggtc cctgtcccaa ggtgtgccac 1020
   ctcctagaag gcgagaagac catcgactcg gtgacgtctg cccaggagct ccgaggatgc 1080
   accgtcatca acgggagtct gatcatcaac attcgaggag gcaacaatct ggcagctgag 1140
   ctagaagcca acctcggcct cattgaagaa atttcagggt atctaaaaat ccgccgatcc 1200
   tacgctctgg tgtcactttc cttcttccgg aagttacgtc tgattcgagg agagaccttg 1260
   gaaattggga actactcctt ctatgccttg gacaaccaga acctaaggca gctctgggac 1320
   tggagcaaac acaacctcac caccactcag gggaaactct tcttccacta taaccccaaa 1380
   ctctgcttgt cagaaatcca caagatggaa gaagtttcag gaaccaaggg gcgccaggag 1440
   agaaacgaca ttgccctgaa gaccaatggg gacaaggcat cctgtgaaaa tgagttactt 1500
   aaattttctt acattcggac atcttttgac aagatcttgc tgagatggga gccgtactgg 1560
   ccccccgact tccgagacct cttggggttc atgctgttct acaaagaggc cccttatcag 1620
   aatgtgacgg agttcgatgg gcaggatgcg tgtggttcca acagttggac ggtggtagac 1680
   attgacccac ccctgaggtc caacgacccc aaatcacaga accacccagg gtggctgatg 1740
   cggggtctca agccctggac ccagtatgcc atctttgtga agaccctggt caccttttcg 1800
   gatgaacgcc ggacctatgg ggccaagagt gacatcattt atgtccagac agatgccacc 1860
   aacccctctg tgcccctgga tccaatctca gtgtctaact catcatccca gattattctg 1920
   aagtggaaac caccctccga ccccaatggc aacatcaccc actacctggt tttctgggag 1980
   aggcaggcgg aagacagtga gctgttcgag ctggattatt gcctcaaagg gctgaagctg 2040
   ccctcgagga cctggtctcc accattcgag tctgaagatt ctcagaagca caaccagagt 2100
   gagtatgagg attcggccgg cgaatgctgc tcctgtccaa agacagactc tcagatcctg 2160
   aaggagctgg aggagtcctc gtttaggaag acgtttgagg attacctgca caacgtggtt 2220
   ttcgtcccca gaaaaacctc ttcaggcact ggtgccgagg accctaggcc atctcggaaa 2280
   cgcaggtccc ttggcgatgt tgggaatgtg acggtggccg tgcccacggt ggcagctttc 2340
   cccaacactt cctcgaccag cgtgcccacg agtccggagg agcacaggcc ttttgagaag 2400
   gtggtgaaca aggagtcgct ggtcatctcc ggcttgcgac acttcacggg ctatcgcatc 2460
   gagctgcagg cttgcaacca ggacacccct gaggaacggt gcagtgtggc agcctacgtc 2520
   agtgcgagga ccatgcctga agccaaggct gatgacattg ttggccctgt gacgcatgaa 2580
   atctttgaga acaacgtcgt ccacttgatg tggcaggagc cgaaggagcc caatggtctg 2640
   atcgtgctgt atgaagtgag ttatcggcga tatggtgatg aggagctgca tctctgcgtc 2700
   tcccgcaagc acttcgctct ggaacggggc tgcaggctgc gtgggctgtc accggggaac 2760
   tacagcgtgc gaatccgggc cacctccctt gcgggcaacg gctcttggac ggaacccacc 2820
   tatttctacg tgacagacta tttagacgtc ccgtcaaata ttgcaaaagt cgacggttgt 2880
   aagccttgca tatgtacagt cccagaagta tcatctgtct tcatcttccc cccaaagccc 2940
   aaggatgtgc tcaccattac tctgactcct aaggtcacgt gtgttgtggt agacatcagc 3000
   aaggatgatc ccgaggtcca gttcagctgg tttgtagatg atgtggaggt gcacacagct 3060
   cagacgcaac cccgggagga gcagttcaac agcactttcc gctcagtcag tgaacttccc 3120
   atcatgcacc aggactggct caatggcaag gagttcaaat gcagggtaaa cagtgcagct 3180
   ttccctgccc ccatcgagaa aaccatctcc aaaaccaaag gcagaccgaa ggctccacag 3240
   gtgtacacca ttccacctcc caaggagcag atggccaagg ataaagtcag tctgacctgc 3300
   atgataacag acttcttccc tgaagacatt actgtggagt ggcagtggaa tgggcagcca 3360
   gcggagaact acaagaacac tcagcccatc atggacacag atggctctta cttcgtctac   3420
   agcaagctca atgtgcagaa gagcaactgg gaggcaggaa atactttcac ctgctctgtg   3480
   ttacatgagg gcctgcacaa ccaccatact gagaagagcc tctcccactc tcctggtaaa   3540
<210> 262
   <211> 1409
   <212> DNA
   <213> Artificial Sequence
<220>
   <223> nucleic acid
<400> 262
   tctagaccac catggacatc aggctcagct tagttttcct tgtccttttc ataaaaggtg 60
   tccagtgtga ggtagaactg gtggagtctg ggggcggctt agtacaacct ggaaggtcca 120
   tgacactctc ctgtgcagcc tcgggattca ctttcagaac ctatggcatg gcctgggtcc 180
   gccaggcccc aacgaagggt ctggagtggg tctcatcaat tactgctagt ggtggtacca 240
   cctactatcg agactccgtg aagggccgct tcactatttt tagggataat gcaaaaagta 300
   ccctatacct gcagatggac agtccgaggt ctgaggacac ggccacttat ttctgtacat 360
   caatttcgga atactggggc cacggagtca tggtcaccgt ctctagtgcc tccaccaagg 420
   gcccatcggt cttccccctg gcaccctcct ccaagagcac ctctgggggc acagcggccc 480
   tgggctgcct ggtcaaggac tacttccccg aaccggtgac ggtgtcgtgg aactcaggcg 540
   ccctgaccag cggcgtgcac accttcccgg ctgtcctaca gtcctcagga ctctactccc 600
   tcagcagcgt ggtgaccgtg ccctccagca gcttgggcac ccagacctac atctgcaacg 660
   tgaatcacaa gcccagcaac accaaggtgg acaagaaagt tgagcccaaa tcttgtgaca 720
   aaactcacac atgcccaccg tgcccagcac ctgaactcct ggggggaccg tcagtcttcc 780
   tcttcccccc aaaacccaag gacaccctca tgatctcccg gacccctgag gtcacatgcg 840
   tggtggtgga cgtgagccac gaagaccctg aggtcaagtt caactggtac gtggacggcg 900
   tggaggtgca taatgccaag acaaagccgc gggaggagca gtacaacagc acgtaccgtg 960
   tggtcagcgt cctcaccgtc ctgcaccagg actggctgaa tggcaaggag tacaagtgca 1020
   aggtctccaa caaagccctc ccagccccca tcgagaaaac catctccaaa gccaaagggc 1080
   agccccgaga accacaggtg tacaccctgc ccccatcccg ggatgagctg accaagaacc 1140
   aggtcagcct gacctgcctg gtcaaaggct tctatcccag cgacatcgcc gtggagtggg 1200
   agagcaatgg gcagccggag aacaactaca agaccacgcc tcccgtgctg gactccgacg 1260
   gctccttctt cctctatagc aagctcaccg tggacaagag caggtggcag caggggaacg 1320
   tcttctcatg ctccgtgatg catgaggctc tgcacaacca ctacacgcag aagagcctct 1380
   ccctgtctcc gggtaaatga taagtcgac 1409
<210> 263
   <211> 22
   <212> PRT
   <213> Artificial Sequence
<220>
   <223> protein
<400> 263
   Met Asp Met Arg Val Pro Ala Gln Leu Leu Gly Leu Leu Leu Leu Trp
   1 5 10 15
   Leu Arg Gly Ala Arg Cys
   20
<210> 264
   <211> 22
   <212> PRT
   <213> Artificial Sequence
<220>
   <223> protein
<400> 264
   Met Asp Met Arg Val Pro Ala Gln Leu Leu Gly Leu Leu Leu Leu Trp
   1 5 10 15
   Leu Arg Gly Ala Arg Cys
   20
<210> 265
   <211> 28
   <212> DNA
   <213> Artificial Sequence
<220>
   <223> nucleic acid
<400> 265
   gcaagcttgg gagaaatctg cgggccag 28
<210> 266
   <211> 32
   <212> DNA
   <213> Artificial Sequence
<220>
   <223> nucleic acid
<400> 266
   attgcggccg cttcatatcc tgttttggcc tg 32
<210> 267
   <211> 30
   <212> DNA
   <213> Artificial Sequence
<220>
   <223> nucleic acid
<400> 267
   attgcggccg ccccacattc ctttgggggc 30
<210> 268
   <211> 28
   <212> DNA
   <213> Artificial Sequence
<220>
   <223> nucleic acid
<400> 268
   agcaagcttg gacctgtgtc cagggacc 28
<210> 269
   <211> 30
   <212> DNA
   <213> Artificial Sequence
<220>
   <223> nucleic acid
<400> 269
   attgcggccg cgcaaggacc ttcacaaggg 30
<210> 270
   <211> 29
   <212> DNA
   <213> Artificial Sequence
<220>
   <223> nucleic acid
<400> 270
   agcaagcttg ccgaaggtct gtgaggaag 29
<210> 271
   <211> 30
   <212> DNA
   <213> Artificial Sequence
<220>
   <223> nucleic acid
<400> 271
   attgcggccg cactttcaca ggaggctctc 30
<210> 272
   <211> 30
   <212> DNA
   <213> Artificial Sequence
<220>
   <223> nucleic acid
<400> 272
   agcaagcttg gacgtcctgc atttcacctc 30
<210> 273
   <211> 32
   <212> DNA
   <213> Artificial Sequence
<220>
   <223> nucleic acid
<400> 273
   attgcggccg cggtgcgaat gtacaagatc tc 32
<210> 274
   <211> 32
   <212> DNA
   <213> Artificial Sequence
<220>
   <223> nucleic acid
<400> 274
   agcaagcttg aatgcttcag ttccttccat tc 32
<210> 275
   <211> 33
   <212> DNA
   <213> Artificial Sequence
<220>
   <223> nucleic acid
<400> 275
   attgcggccg cagtccttgc aaagacgaag ttg 33
<210> 276
   <211> 29
   <212> DNA
   <213> Artificial Sequence
<220>
   <223> nucleic acid
<400> 276
   agcaagcttg atgcccgcag aaggagcag 29
<210> 277
   <211> 32
   <212> DNA
   <213> Artificial Sequence
<220>
   <223> nucleic acid
<400> 277
   attgcggccg ctttaatggc cactctggtt tc 32
<210> 278
   <211> 29
   <212> DNA
   <213> Artificial Sequence
<220>
   <223> nucleic acid
<400> 278
   agcaagcttg ggagaaatct gcgggccag 29
<210> 279
   <211> 29
   <212> DNA
   <213> Artificial Sequence
<220>
   <223> nucleic acid
<400> 279
   agcaagcttg ggagaaatct gcgggccag 29

## Description

### CROSS-REFERENCE TO RELATED APPLICATIONS

This application claims the benefit of U. S. Provisional Application No. 61/438,918, filed February 2, 2011.

### REFERENCE TO THE SEQUENCE LISTING

The present application is being filed along with a Sequence Listing in electronic format via EFS-Web. The Sequence Listing is provided as a text file entitled A1610WOPCTst25.txt, created January 26, 2012, which is 388,961 bytes in size.

### FIELD OF THE INVENTION

This application describes methods and compositions relating to the treatment of tumor diseases such as pancreatic cancer and other cancers and proliferative diseases. This application provides methods for determining whether a subject with pancreatic cancer is likely to respond to treatment with ganitumab.

### BACKGROUND OF THE INVENTION

Pancreatic cancer is one of the most aggressive and deadly forms of cancer. With the current standard of care median overall survival is about six months from diagnosis. More and better treatment options are urgently needed, as are methods of determining which patients are more likely to benefit from such treatments.

US2009/0093488 describes diagnostic and prognostic methods for predicting the effectiveness of treatment with an IGF-1R kinase inhibitor using epithelial and/or mesenchymal biomarkers, which may include assessing the level of IGF-1 and/or IGF-2 in the tumour of the blood or serum of the patient.

Pollak MN, et al., 2004. Nature Reviews Cancer. 4:505-518 describes that high levels of circulating IGF1 are associated with increased risk of common cancers and that higher levels of IGF-1 are associated with higher rates of cell division.

WO02/068960 discloses methods for prognosis after therapy for prostate cancer, which measures the levels of IGFBP-2 and IGFBP3.

### BRIEF DESCRIPTION OF THE DRAWINGS

Figure 1 provides nucleotide sequences encoding light chain variable domains L1 through L52 and heavy chain variable domains H1 through H52.
Figure 2 provides amino acid sequences of light chain variable domains L1 through L52. CDR and FR regions are indicated.
Figure 3 provides amino acid sequences of heavy chain variable domains H1 through H52. CDR and FR regions are indicated.
Figure 4 provides amino acid sequences of the light chain CDR1 regions of light chain variable domains L1 through L52. Consensus sequences for groups of related CDR sequences are also provided.
Figure 5 provides amino acid sequences of the light chain CDR2 regions of light chain variable domains L1 through L52. Consensus sequences for groups of related CDR sequences are also provided.
Figure 6 provides amino acid sequences of the light chain CDR3 regions of light chain variable domains L1 through L52. Consensus sequences for groups of related CDR sequences are also provided.
Figure 7 provides amino acid sequences of the heavy chain CDR1 regions of heavy chain variable domains H1 through H52. Consensus sequences for groups of related CDR sequences are also provided.
Figure 8 provides amino acid sequences of the heavy chain CDR2 regions of heavy chain variable domains H1 through H52. Consensus sequences for groups of related CDR sequences are also provided.
Figure 9 provides amino acid sequences of the heavy chain CDR3 regions of heavy chain variable domains H1 through H52. Consensus sequences for groups of related CDR sequences are also provided.
Figure 10 provides the amino acid sequence of a human IGF-1R extracellular domain fused to a human IgG1 Fc region (underlined) with an intervening caspace-3 cleavage site (bold).
Figure 11 provides the amino acid sequence of a human insulin receptor extracellular domain fused to a human IgG1 Fc region (underlined).
Figure 12 provides the protein sequence of a human IGF-1R extracellular domain (including signal peptide) fused at the C-terminus with chicken avidin. The initiating met in the IGF-1R ECD is designated position 1 in this figure.
Figure 13 provides the polypeptide sequence of a human kappa light chain antibody constant region and a human IgG1 heavy chain antibody constant region.
Figure 14 provides a graph illustrating that four phage-displayed antibodies bind significantly better to an IGF-1R-Fc molecule than they bind to an insulin-receptor-Fc or a murine Fc.
Figure 15 provides graphs illustrating the ability of certain antibodies to compete for binding to IGF-1R with IGF-1 and IGF-2.
Figure 16 provides graphs illustrating the ability of certain antibodies to inhibit the growth of 32D hu IGF-1R+IRS-1 cells.
Figure 17 provides graphs illustrating the ability of certain antibodies to inhibit the growth of Balb/C 3T3 hu IGF-1R cells.
Figure 18 provides the amino acid sequences of ganitumab (AMG 479).
Figure 19 provides the amino acid sequences of conatumumab (AMG 655).
Figure 20 provides the study schema for a placebo-controlled, randomized phase 2 study of conatumumab (AMG 655) or ganitumab (AMG 479) or placebo plus gemcitabine in patients with metastatic pancreatic cancer.
Figure 21 provides overall survival data for the study of Figure 20.
Figure 22 provides progression free survival data for the study of Figure 20.
Figure 23 provides a graph illustrating the effect of ganitumab exposure on overall survival.
Figure 24 provides a graph illustrating the effect of ganitumab exposure on progression free survival.
Figure 25 provides a graph illustrating the observed ganitumab AUCₛₛ distributions at 20 mg/kg in patients with non-pancreatic tumors and 12 mg/kg in patients with pancreatic or non-pancreatic tumors and a predicted AUCₛₛ distribution at 20 mg/kg for patients with pancreatic tumors.
Figure 26 provides projected progression free survival profiles for placebo and 12 and 20 mg/kg ganitumab.
Figure 27 provides an analysis of the relationship between overall survival and baseline levels of the IGF biomarkers by Cox proportional hazard model. Data are tabulated and HR shown graphically as a Forest plot with error bars representing 95% confidence intervals.
Figure 28A and B provide a comparison of the overall survival of subgroups having higher-than-median and lower-than-median baseline levels of the IGF biomarkers, treated with either ganitumab or placebo, by Cox proportional hazard model. Data are tabulated and HR shown graphically as a Forest plot with error bars representing 95% confidence intervals.
Figure 29 provides a Kaplan-Maier plot illustrating the difference in overall survival between high and low total IGF-1 patient groups. The arrow indicates the line corresponding to the ganitumab-treated high total IGF-1 subgroup.
Figure 30 provides a Kaplan-Maier plot illustrating the difference in overall survival between high and low free IGF-1 patient groups. The arrow indicates the line corresponding to the ganitumab-treated high free IGF-1 subgroup.
Figure 31 provides a Kaplan-Maier plot illustrating the difference in overall survival between high and low total IGF-2 patient groups. The arrow indicates the line corresponding to the ganitumab-treated high IGF-2 subgroup.
Figure 32 provides a Kaplan-Maier plot illustrating the difference in overall survival between high and low total IGFBP-2 patient groups. The arrow indicates the line corresponding to the ganitumab-treated low IGFBP-2 subgroup.
Figure 33 provides a Kaplan-Maier plot illustrating the difference in overall survival between high and low IGFBP-3 patient groups. The arrow indicates the line corresponding to the ganitumab-treated high IGFBP-3 subgroup.
Figure 34 provides a graph illustrating the best tumor response achieved for each of twelve human subjects treated with an inhibitor of IGF-1 receptor signaling.
Figure 35 provides a scatter plot for pairs of biomarkers.

### SUMMARY OF THE INVENTION

Herein described is a method of determining whether a cancerous condition in a patient is likely to respond to a treatment that reduces signaling mediated by IGF-1 or IGF-2, comprising determining the concentration of a circulating biomarker in said patient's serum, wherein the amount of said circulating biomarker is predictive of a response to said treatment. The said treatment may comprise administering an inhibitor of signaling through an IGF-1R signaling pathway. Alternatively, said inhibitor inhibits signaling upstream of IGF-1R or inhibits IGF-1 or IGF-2. The inhibitor may inhibit the transcription or translation of IGF-1 or IGF-2 or the processing or secretion of IGF-1 or IGF-2. The inhibitor may bind to IGF-1 or IGF-2 or inhibit the binding of IGF-1 or IGF-2 to IGF-1R. The inhibitor may be an anti-IGF-1 or anti-IGF-2 antibody, antigen binding fragment of said antibody, isolated IGF-1 or IGF-2 binding protein, recombinant human IGF-1 or IGF-2 binding protein, IGFBP1, IGFBP2, IGFBP3, IGFBP4, IGFBP5, IGFBP6, or IGFBP7. The inhibitor may be an inhibitor of IGF-1R or a small molecule inhibitor of IGF-1R. Said small molecule inhibitor of IGF-1R may be a kinase inhibitor. Said small molecule inhibitor may be OSI-906, linsitinib, BMS-754807, INSM-18, XL228, AXL1717, BMS-536924, NVP-ADW742, GSK621659A, GSK1838705A, A-928605, AZD4253, TAE226, or AG1024. Said inhibitor may be an anti-IGF-1R antibody, or an antigen binding fragment of said antibody. Said antibody may inhibit binding of IGF-1 or IGF-2 to IGF-1R or inhibit binding of IGF-1 and IGF-2 to IGF-1R or downregulate IGF-1R. Said antibody may be fully human, humanized, or chimeric. The said anti-IGF-1R antibody may be ganitumab, AMG 479, figitumumab, CP-751,871, cixutumumab, IMC-A12, dalotuzumab, MK0646, RG1507, robatumumab, SCH 717454, AVE-1642a, MEDI-573, BIIB022, rhuMab IGFR, L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, or L52H52. Said inhibitor may affect an IGF-1 or IGF-2 binding protein, IGFBP1, IGFBP2, IGFBP3, IGFBP4, IGFBP5, IGFBP6, or IGFBP7 or inhibit downstream signaling of IGF-1R. Said inhibitor may inhibit the Ras/Raf signaling pathway or the PI3K signaling pathway or inhibit Shc, Grb2, SOS, Ras, Raf, MEK, ERK, Elk-1, IRS1, PI3K, or AKT/PKB. Said inhibitor may be an mTOR inhibitor which may be everolimus. The cancerous condition may be characterized by IGF-1R signaling activity and may be of a type that responds to treatment using an inhibitor of IGF-1R signaling. Said patient may have a tumor which may be a solid tumor. Said tumor may be a primary tumor. Said tumor may be a metastatic tumor. Said tumor may be a pancreatic cancer tumor, a sarcoma tumor, a Ewing's sarcoma tumor, an ovarian tumor, a breast cancer tumor, a small cell lung cancer tumor, a non-small cell lung cancer tumor, a colorectal cancer tumor with an activating KRAS mutation, a colorectal cancer tumor with a wild-type KRAS allele, a prostate cancer tumor, a hepatic cancer tumor, a head and neck cancer tumor, a carcinoid tumor, a gastric cancer tumor, a multiple myeloma tumor, a neuroendocrine cancer tumor, an adrenal cell carcinoma tumor, or a hepatocellular carcinoma tumor. Said pancreatic cancer tumor may be a metastatic pancreatic cancer tumor. Said pancreatic cancer tumor may be locally advanced pancreatic cancer tumor. Said cancerous condition may be Acute Lymphoblastic Leukemia, Adrenocortical Carcinoma, an AIDS-Related Cancer, AIDS-Related Lymphoma, Anal Cancer, Childhood Cerebellar Astrocytoma, Childhood Cerebral Astrocytoma, Basal Cell Carcinoma, Extrahepatic Bile Duct Cancer, Bladder Cancer, Osteosarcoma/Malignant Fibrous Histiocytoma Bone Cancer, a Brain Tumor (e.g., Brain Stem Glioma, Cerebellar Astrocytoma, Cerebral Astrocytoma/Malignant Glioma, Ependymoma, Medulloblastoma, a Supratentorial Primitive Neuroectodermal Tumor, Visual Pathway or Hypothalamic Glioma), Breast Cancer, a Bronchial Adenoma/Carcinoid, Burkitt's Lymphoma, Carcinoid Tumor, Gastrointestinal Carcinoid Tumor, Carcinoma of Unknown Primary, Primary Central Nervous System, Cerebellar Astrocytoma, Cerebral Astrocytoma/Malignant Glioma, Cervical Cancer, a Childhood Cancer, Chronic Lymphocytic Leukemia, Chronic Myelogenous Leukemia, a Chronic Myeloproliferative Disorder, Colon Cancer, Colorectal Cancer, Cutaneous T-Cell Lymphoma, Endometrial Cancer, Ependymoma, Esophageal Cancer, a Ewing's Family Tumor, Extracranial Germ Cell Tumor, Extragonadal Germ Cell Tumor, Extrahepatic Bile Duct Cancer, Intraocular Melanoma Eye Cancer, Retinoblastoma Eye Cancer, Gallbladder Cancer, Gastric (Stomach) Cancer, Gastrointestinal Carcinoid Tumor, a Germ Cell Tumor (e.g., Extracranial, Extragonadal, or Ovarian), Gestational Trophoblastic Tumor, Glioma (e.g., Adult, Childhood Brain Stem, Childhood Cerebral Astrocytoma, Childhood Visual Pathway or Hypothalamic), Hairy Cell Leukemia, Head and Neck Cancer, Hepatocellular (Liver) Cancer, Hodgkin's Lymphoma, Hypopharyngeal Cancer, Hypothalamic or Visual Pathway Glioma, Intraocular Melanoma, Islet Cell Carcinoma (Endocrine Pancreas), Kaposi's Sarcoma, Kidney (Renal Cell) Cancer, Laryngeal Cancer, Leukemia (e.g., Acute Lymphoblastic, Acute Myeloid, Chronic Lymphocytic, Chronic Myelogenous, or Hairy Cell), Lip or Oral Cavity Cancer, Liver Cancer, Non-Small Cell Lung Cancer, Small Cell Lung Cancer, Lymphoma (e.g., AIDS-Related, Burkitt's, Cutaneous T-Cell, Hodgkin's, Non-Hodgkin's, or Primary Central Nervous System), Waldenström's Macroglobulinemia, Malignant Fibrous Histiocytoma of Bone/Osteosarcoma, Medulloblastoma, Melanoma, Intraocular (Eye) Melanoma, Merkel Cell Carcinoma, Mesothelioma, Metastatic Squamous Neck Cancer with Occult Primary, Multiple Endocrine Neoplasia Syndrome, Multiple Myeloma/Plasma Cell Neoplasm, Mycosis Fungoides, a Myelodysplastic Syndrome, a Myelodysptastic/Myeloproliferative Disease, Myelogenous Leukemia, Chronic Myeloid Leukemia, Multiple Myeloma, a Chronic Myeloproliferative Disorder, Nasal Cavity or Paranasal Sinus Cancer, Nasopharyngeal Cancer, Neuroblastoma, Oral Cancer, Oropharyngeal Cancer, Osteosarcoma/Malignant Fibrous Histiocytoma of Bone, Ovarian Cancer, Ovarian Epithelial Cancer, Ovarian Germ Cell Tumor, Ovarian Low Malignant Potential Tumor, Pancreatic Cancer, Islet Cell Pancreatic Cancer, Paranasal Sinus or Nasal Cavity Cancer, Parathyroid Cancer, Penile Cancer, Pheochromocytoma, Pineoblastoma, Pituitary Tumor, Plasma Cell Neoplasm/Multiple Myeloma, Pleuropulmonary Blastoma, Primary Central Nervous System Lymphoma, Prostate Cancer, Rectal Cancer, Renal Cell (Kidney) Cancer, Renal Pelvis or Ureter Transitional Cell Cancer, Retinoblastoma, Rhabdomyosarcoma, Salivary Gland Cancer, Soft Tissue Sarcoma, Uterine Sarcoma, Sezary Syndrome, non-Melanoma Skin Cancer, Merkel Cell Skin Carcinoma, Small Intestine Cancer, Soft Tissue Sarcoma, Squamous Cell Carcinoma, Cutaneous T-Cell Lymphoma, Testicular Cancer, Thymoma, Thymic Carcinoma, Thyroid Cancer, Gestational Trophoblastic Tumor, Carcinoma of Unknown Primary Site, Cancer of Unknown Primary Site, Urethral Cancer, Endometrial Uterine Cancer, Uterine Sarcoma, Vaginal Cancer, Visual Pathway or Hypothalamic Glioma, Vulvar Cancer, Waldenström's Macroglobulinemia, or Wilms' Tumor. Said circulating biomarker may be total IGF-1, free IGF-1, total IGF-2, free IGF-2, IGFBP-1, IGFBP-2, IGFBP-3, IGFBP-4, IGFBP-5, IGFBP-6, or IGFBP-7. Herein described is a method, wherein, said circulating biomarker is total IGF-1, and wherein a higher total IGF-1 concentration indicates that said patient is more likely to respond to said treatment than a lower total IGF-1 concentration. Said patient's total IGF-1 concentration is higher if it is greater than about 118 ng/mL and lower if it is less than about 118 ng/mL. Said circulating biomarker may be total IGF-2, wherein a higher total IGF-2 concentration indicates that said patient is more likely to respond to said treatment than a lower total IGF-2 concentration. Said patient's total IGF-2 concentration is higher if it is greater than about 1734 ng/mL and lower if it is less than about 1734 ng/mL. Herein described is a method, wherein said circulating biomarker is IGFBP-1, and wherein a lower IGFBP-1 concentration indicates that said patient is more likely to respond to said treatment than a higher IGFBP-1 concentration. Said patient's total IGFBP-1 concentration is higher if it is greater than about 30 ng/mL and lower if it is less than about 30 ng/mL. Herein described is a method, wherein said circulating biomarker is IGFBP-2, and wherein a lower IGFBP-2 concentration indicates that said patient is more likely to respond to said treatment than a higher total IGFBP-2 concentration. Said patient's total IGFBP-2 concentration is higher if it is greater than about 170 ng/mL and lower if it is less than about 170 ng/mL. Herein described is a method, wherein circulating biomarker is IGFBP-3, and wherein a higher IGFBP-3 concentration indicates that said patient is more likely to respond to said treatment than a lower total IGFBP-3 concentration. Said patient's total IGFBP-3 concentration is higher if it is greater than about 1.9 µg/mL and lower if it is less than about 1.9 µg/mL. Herein described is a method, wherein said circulating biomarker is IGFBP-4, and wherein a higher IGFBP-4 concentration indicates that said patient is more likely to respond to said treatment than a lower total IGFBP-4 concentration. Said patient's total IGFBP-4 concentration is higher if it is greater than about 40 ng/mL and lower if it is less than about 40 ng/mL. Methods herein described may further comprise treating said patient with said treatment. Said method may further comprises treating said patient with said treatment if said patient's circulating biomarker concentration indicates that said patient is more likely to respond to said treatment. In methods herein described, said treatment may comprise administering an inhibitor of signaling through an IGF-1R signaling pathway or an inhibitor which inhibits signaling upstream of IGF-1R. Alternatively, said inhibitor inhibits IGF-1 or IGF-2, the transcription or translation of IGF-1 or IGF-2 the processing or secretion of IGF-1 or IGF-2, binds to IGF-1 or IGF-2, or inhibits the binding of IGF-1 or IGF-2 to IGF-1R. Said inhibitor may be an anti-IGF-1 or anti-IGF-2 antibody, antigen binding fragment of said antibody, isolated IGF-1 or IGF-2 binding protein, recombinant human IGF-1 or IGF-2 binding protein, IGFBP1, IGFBP2, IGFBP3, IGFBP4, IGFBP5, IGFBP6, or IGFBP7 or an inhibitor of IGF-1R. Said inhibitor may be a small molecule inhibitor of IGF-1R which may be a kinase inhibitor. Said small molecule inhibitor may be OSI-906, linsitinib, BMS-754807, INSM-18, XL228, AXL1717, BMS-536924, NVP-ADW742, GSK621659A, GSK1838705A, A-928605, AZD4253, TAE226, or AG1024. Said inhibitor may be an anti-IGF-1R antibody, or an antigen binding fragment of said antibody or an antibody which inhibits binding of IGF-1 or IGF-2 to IGF-1R or an antibody which inhibits binding of IGF-1 and IGF-2 to IGF-1R. Said antibody may downregulate IGF-1R. Said antibody may be fully human, humanized, or chimeric. Said anti-IGF-1R antibody may be ganitumab, AMG 479, figitumumab, CP-751,871, cixutumumab, IMC-A12, dalotuzumab, MK0646, RG1507, robatumumab, SCH 717454, AVE-1642a, MEDI-573, BIIB022, rhuMab IGFR, L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, or L52H52. Said inhibitor may affect an IGF-1 or IGF-2 binding protein or affect IGFBP1, IGFBP2, IGFBP3, IGFBP4, IGFBP5, IGFBP6, or IGFBP7. Said inhibitor may inhibit downstream signaling of IGF-1R or inhibit the Ras/Raf signaling pathway or the PI3K signaling pathway. The inhibitor may inhibit Shc, Grb2, SOS, Ras, Raf, MEK, ERK, Elk-1, IRS1, PI3K, or AKT/PKB. Said inhibitor may be an mTOR inhibitor which may be everolimus. Said response may be an increase in survival, an increase in progression free survival, or an objective response. Said objective response may be stable disease, a partial response or a complete response. Said complete response may be a durable complete response.

Herein described is a method of determining whether a cancer condition in a patient is likely to respond to a treatment with an inhibitor of signaling through an IGF-1R signaling pathway, comprising determining the exposure of said patient to said inhibitor, wherein said cancer condition is likely to respond to said treatment if said exposure of said patient is about equal to or greater than the median exposure of patients with said cancer condition treated with said inhibitor. Said inhibitor may inhibit signaling upstream of IGF-1R, inhibit IGF-1 or IGF-2, inhibit the transcription or translation of IGF-1 or IGF-2, or inhibit the processing or secretion of IGF-1 or IGF-2. Said inhibitor may bind to IGF-1 or IGF-2 or inhibit the binding of IGF-1 or IGF-2 to IGF-1R. Said inhibitor may be an anti-IGF-1 or anti-IGF-2 antibody, antigen binding fragment of said antibody, isolated IGF-1 or IGF-2 binding protein, recombinant human IGF-1 or IGF-2 binding protein, IGFBP1, IGFBP2, IGFBP3, IGFBP4, IGFBP5, IGFBP6, or IGFBP7. Said inhibitor may be an inhibitor of IGF-1R. Said inhibitor may be a small molecule inhibitor of IGF-1R which may be a kinase inhibitor. Said small molecule inhibitor may be OSI-906, linsitinib, BMS-754807, INSM-18, XL228, AXL1717, BMS-536924, NVP-ADW742, GSK621659A GSK1838705A, A-928605, AZD4253, TAE226, or AG1024. Said inhibitor may be an anti-IGF-1R antibody, or an antigen binding fragment of said antibody. Said antibody or fragment may inhibit binding of IGF-1 or IGF-2 to IGF-1R, inhibit binding of IGF-1 and IGF-2 to IGF-1R, or downregulate 1GF-1R. Said antibody or fragment may be fully human, humanized, or chimeric. Said anti-IGF-1R antibody or fragment may be ganitumab, AMG 479, figitumumab, CP-751,871, cixutumumab, IMC-A12, dalotuzumab, MK0646, RG1507, robatumumab, SCH 717454, AVE-1642a, MEDI-573, BIIB022, rhuMab IGFR, L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, or L52H52. Said inhibitor may affect an IGF-1 or IGF-2 binding protein or affect IGFBP1, IGFBP2, IGFBP3, IGFBP4, IGFBP5, IGFBP6, or IGFBP7. Said inhibitor may inhibit downstream signaling of IGF-1R, the Ras/Raf signaling pathway, the PI3K signaling pathway, Shc, Grb2, SOS, Ras, Raf, MEK, ERK, Elk-1, IRS1, PI3K, or AKT/PKB. Said inhibitor may be an mTOR inhibitor which may be everolimus. Said cancerous condition may be characterized by IGF-1R signaling activity and repond to treatment using an inhibitor of IGF-1R signaling. Said patient may have a tumor may be that may be a solid tumor. Said tumor may be a primary tumor or a metastatic tumor. Said tumor may be a pancreatic cancer tumor, a sarcoma tumor, a Ewing's sarcoma tumor, an ovarian tumor, a breast cancer tumor, a small cell lung cancer tumor, a non-small cell lung cancer tumor, a colorectal cancer tumor with an activating KRAS mutation, a colorectal cancer tumor with a wild-type KRAS allele, a prostate cancer tumor, a hepatic cancer tumor, a head and neck cancer tumor, a carcinoid tumor, a gastric cancer tumor, a multiple myeloma tumor, a neuroendocrine cancer tumor, an adrenal cell carcinoma tumor, or a hepatocellular carcinoma tumor. Said pancreatic cancer tumor may be a metastatic pancreatic cancer tumor. Said pancreatic cancer tumor may be a locally advanced pancreatic cancer tumor. Said method may further comprise administering to said patient a dose of an anti-IGF-1R antibody such as 12 mg/kg dose or 20 mg/kg dose. Said antibody may be ganitumab. Said cancerous condition may be pancreatic cancer. Said pancreatic cancer may be metastatic or locally advanced. The method may further comprised administering to said patient a second dose of said anti-IGF-1R antibody. e.g., two weeks after the first dose. Said patient may receive at least one additional dose of said anti-IGF-1R antibody e.g., once every two weeks. The method may further comprise determining the concentration of a second circulating biomarker, determining the concentration of a third circulating biomarker or determining the concentration of a fourth circulating biomarker. Said biomarkers may be IGFBP-2 and total IGF-1 or IGFBP-2 and IGF-2 or IGFBP-2 and IGFBP-3 or total IGF-1 and IGF-2/ or total IGF-1 and IGFBP-3 or IGF-2 and IGFBPF-3. Said circulating biomarker may be total IGF-1, free IGF-1, total IGF-2, free IGF-2, IGFBP-1, IGFBP-3, IGFBP-4, IGFBP-5, IGFBP-6, or IGFBP-7, and said concentration of said circulating biomarker may be greater than the median value from a reference range derived from an analysis of at least 100 patients having said cancerous condition. Said circulating biomarker may be IGFBP-2 and said concentration of said circulating biomarker may be less than the median value from a reference range derived from an analysis of at least 100 patients having said cancerous condition. Said cancer condition is likely to respond to said treatment if said patient's AUCₛₛ is greater than or equal to the median value. Said median value may be 19.2 mg•h/mL and may be the concentration of said circulating biomarker before treatment that is determined.

The present invention provides a method for assessing the prognosis for a patient comprising determining the amount of a circulating biomarker, wherein said patient has pancreatic cancer and is being treated with ganitumab, wherein a higher concentration of said circulating biomarker indicates that said patient has a better prognosis of a ganitumab-dependent increase in overall survival, and wherein said circulating biomarker is total IGF-2 and wherein said patient's total IGF-2 concentration is higher if it is greater than 1734 ng/mL. Herein described is a cancerous condition that may be characterized by IGF-1R signaling activity or said cancerous condition may be of a type that responds to treatment using an inhibitor of IGF-1R signaling. Said patient may have a tumor. Said tumor may be a solid tumor or said tumor may be primary tumor or said tumor may be a metastatic tumor. According to the present invention said tumor is a pancreatic cancer tumor. Herein described is a sarcoma tumor, a Ewing's sarcoma tumor, an ovarian tumor, a breast cancer tumor a small cell lung cancer tumor, a non-small cell lung cancer tumor, a colorectal cancer tumor with an activating KRAS mutation, a colorectal cancer tumor with a wild-type KRAS allele, a prostate cancer tumor, a hepatic cancer tumor, a head and neck cancer tumor, a carcinoid tumor, a gastric cancer tumor, a multiple myeloma tumor, a neuroendocrine cancer tumor, an adrenal cell carcinoma tumor, or a hepatocellular carcinoma tumor. In another embodiment, said pancreatic cancer tumor is a metastatic pancreatic cancer tumor. In another embodiment, said pancreatic cancer tumor is a locally advanced pancreatic cancer tumor. Said cancerous condition may be Acute Lymphoblastic Leukemia, Adrenocortical Carcinoma, an AIDS-Related Cancer, AIDS-Related Lymphoma, Anal Cancer, Childhood Cerebellar Astrocytoma, Childhood Cerebral Astrocytoma, Basal Cell Carcinoma, Extrahepatic Bile Duct Cancer, Bladder Cancer, Osteosarcoma/Malignant Fibrous Histiocytoma Bone Cancer, a Brain Tumor (e.g., Brain Stem Glioma, Cerebellar Astrocytoma, Cerebral Astrocytoma/Malignant Glioma, Ependymoma, Medulloblastoma, a Supratentorial Primitive Neuroectodermal Tumor, Visual Pathway or Hypothalamic Glioma), Breast Cancer, a Bronchial Adenoma/Carcinoid, Burkitt's Lymphoma, Carcinoid Tumor, Gastrointestinal Carcinoid Tumor, Carcinoma of Unknown Primary, Primary Central Nervous System, Cerebellar Astrocytoma, Cerebral Astrocytoma/Malignant Glioma, Cervical Cancer, a Childhood Cancer, Chronic Lymphocytic Leukemia, Chronic Myelogenous Leukemia, a Chronic Myeloproliferative Disorder, Colon Cancer, Colorectal Cancer, Cutaneous T-Cell Lymphoma, Endometrial Cancer, Ependymoma, Esophageal Cancer, a Ewing's Family Tumor, Extracranial Germ Cell Tumor, Extragonadal Germ Cell Tumor, Extrahepatic Bile Duct Cancer, Intraocular Melanoma Eye Cancer, Retinoblastoma Eye Cancer, Gallbladder Cancer, Gastric (Stomach) Cancer, Gastrointestinal Carcinoid Tumor, a Germ Cell Tumor (e.g., Extracranial, Extragonadal, or Ovarian), Gestational Trophoblastic Tumor, Glioma (e.g., Adult, Childhood Brain Stem, Childhood Cerebral Astrocytoma, Childhood Visual Pathway or Hypothalamic), Hairy Cell Leukemia, Head and Neck Cancer, Hepatocellular (Liver) Cancer, Hodgkin's Lymphoma, Hypopharyngeal Cancer, Hypothalamic or Visual Pathway Glioma, Intraocular Melanoma, Islet Cell Carcinoma (Endocrine Pancreas), Kaposi's Sarcoma, Kidney (Renal Cell) Cancer, Laryngeal Cancer, Leukemia (e.g., Acute Lymphoblastic, Acute Myeloid, Chronic Lymphocytic, Chronic Myelogenous, or Hairy Cell), Lip or Oral Cavity Cancer, Liver Cancer, Non-Small Cell Lung Cancer, Small Cell Lung Cancer, Lymphoma (e.g., AIDS-Related, Burkitt's, Cutaneous T-Cell, Hodgkin's, Non-Hodgkin's, or Primary Central Nervous System), Waldenström's Macroglobulinemia, Malignant Fibrous Histiocytoma of Bone/Osteosarcoma, Medulloblastoma, Melanoma, Intraocular (Eye) Melanoma, Merkel Cell Carcinoma, Mesothelioma, Metastatic Squamous Neck Cancer with Occult Primary, Multiple Endocrine Neoplasia Syndrome, Multiple Myeloma/Plasma Cell Neoplasm, Mycosis Fungoides, a Myelodysplastic Syndrome, a Myelodysplastic/Myeloproliferative Disease, Myelogenous Leukemia, Chronic Myeloid Leukemia, Multiple Myeloma, a Chronic Myeloproliferative Disorder, Nasal Cavity or Paranasal Sinus Cancer, Nasopharyngeal Cancer, Neuroblastoma, Oral Cancer, Oropharyngeal Cancer, Osteosarcoma/Malignant Fibrous Histiocytoma of Bone, Ovarian Cancer, Ovarian Epithelial Cancer, Ovarian Germ Cell Tumor, Ovarian Low Malignant Potential Tumor, Islet Cell Pancreatic Cancer, Paranasal Sinus or Nasal Cavity Cancer, Parathyroid Cancer, Penile Cancer, Pheochromocytoma, Pineoblastoma, Pituitary Tumor, Plasma Cell Neoplasm/Multiple Myeloma, Pleuropulmonary Blastoma, Primary Central Nervous System Lymphoma, Prostate Cancer, Rectal Cancer, Renal Cell (Kidney) Cancer, Renal Pelvis or Ureter Transitional Cell Cancer, Retinoblastoma, Rhabdomyosarcoma, Salivary Gland Cancer, Soft Tissue Sarcoma, Uterine Sarcoma, Sezary Syndrome, non-Melanoma Skin Cancer, Merkel Cell Skin Carcinoma, Small Intestine Cancer, Soft Tissue Sarcoma, Squamous Cell Carcinoma, Cutaneous T-Cell Lymphoma, Testicular Cancer, Thymoma, Thymic Carcinoma, Thyroid Cancer, Gestational Trophoblastic Tumor, Carcinoma of Unknown Primary Site, Cancer of Unknown Primary Site, Urethral Cancer, Endometrial Uterine Cancer, Uterine Sarcoma, Vaginal Cancer, Visual Pathway or Hypothalamic Glioma, Vulvar Cancer, Waldenström's Macroglobulinemia, or Wilms' Tumor. Said circulating biomarker is total IGF-2, wherein a higher IGF-2 concentration indicates that said patient has a better prognosis said patient's total IGF-2 concentration being higher if it is greater than about 1734 ng/mL. In another embodiment, said method further comprises determining the concentration of a second circulating biomarker. In another embodiment, said method further comprises determining the concentration of a third circulating biomarker. In another embodiment, said method further comprises determining the concentration of a fourth circulating biomarker. The second, third or fourth biomarkers are selected from IGFBP-2, total IGF-1, IGFBP-3, the ratio of IGF-2/IGFBP-2 and the ratio of IGFBP-2/IGFBP-3. Preferably, the said second biomarker is the ratio of IGF-2/IGFBP-2 or the ratio of IGFBP-2/IGFBP-3.

Herein described is a kit for practicing any of the foregoing methods.

### DETAILED DESCRIPTION OF THE INVENTION

Herein described are compositions, kits, and methods relating to molecules that bind to the Insulin-Like Growth Factor Receptor ("IGF-1R"), including molecules that agonize or antagonize IGF-1R, such as anti-IGF-1R antibodies, antibody fragments, and antibody derivatives, *e.g*., antagonistic anti-IGF-1R antibodies, antibody fragments, or antibody derivatives. Also herein described are nucleic acids, and derivatives and fragments thereof, comprising a sequence of nucleotides that encodes all or a portion of a polypeptide that binds to IGF-1R, *e.g.,* a nucleic acid encoding all or part of an anti-IGF-1R antibody, antibody fragment, or antibody derivative, plasmids and vectors comprising such nucleic acids, and cells or cell lines comprising such nucleic acids and/or vectors and plasmids. The methods include, for example, methods of making, identifying, or isolating molecules that bind to IGF-1R, such as anti-IGF-1R antibodies, methods of determining whether a molecule binds to IGF-1R, methods of determining whether a molecule agonizes or antagonizes IGF-1R, methods of making compositions, such as pharmaceutical compositions, comprising a molecule that binds to IGF-1R, and methods for administering a molecule that binds IGF-1R to a subject, for example, methods for treating a condition mediated by IGF-1R, and for agonizing or antagonizing a biological activity of IGF-1R, IGF-1, and/or IGF-2 *in vivo* or *in vitro.*

Polynucleotide and polypeptide sequences are indicated using standard one- or three-letter abbreviations. Unless otherwise indicated, polypeptide sequences have their amino termini at the left and their carboxy termini at the right and single-stranded nucleic acid sequences, and the top strand of double-stranded nucleic acid sequences, have their 5' termini at the left and their 3' termini at the right. A particular polypeptide or polynucleotide sequence also can be described by explaining how it differs from a reference sequence.

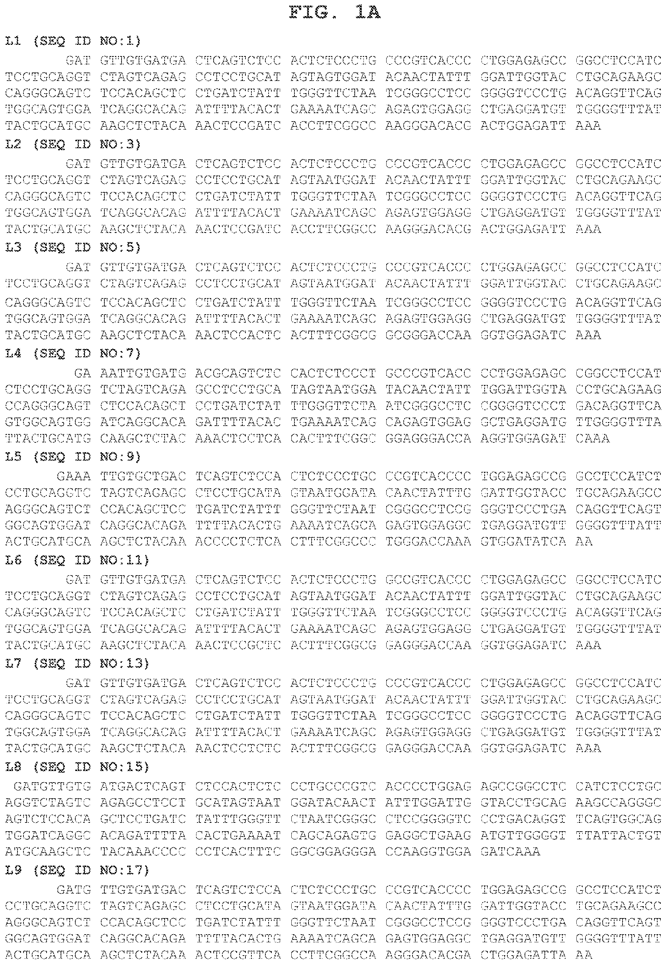

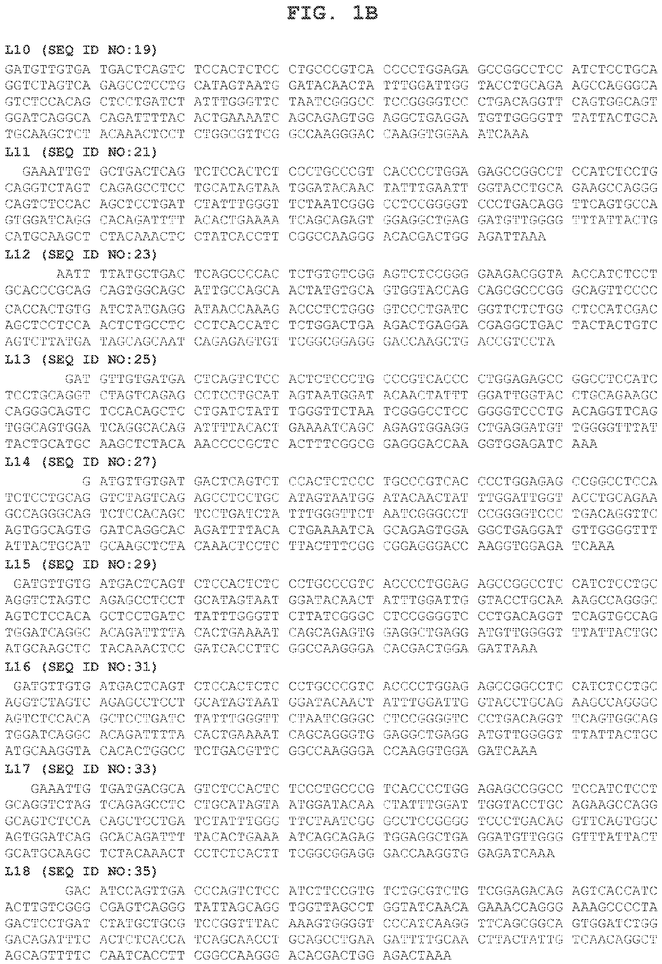

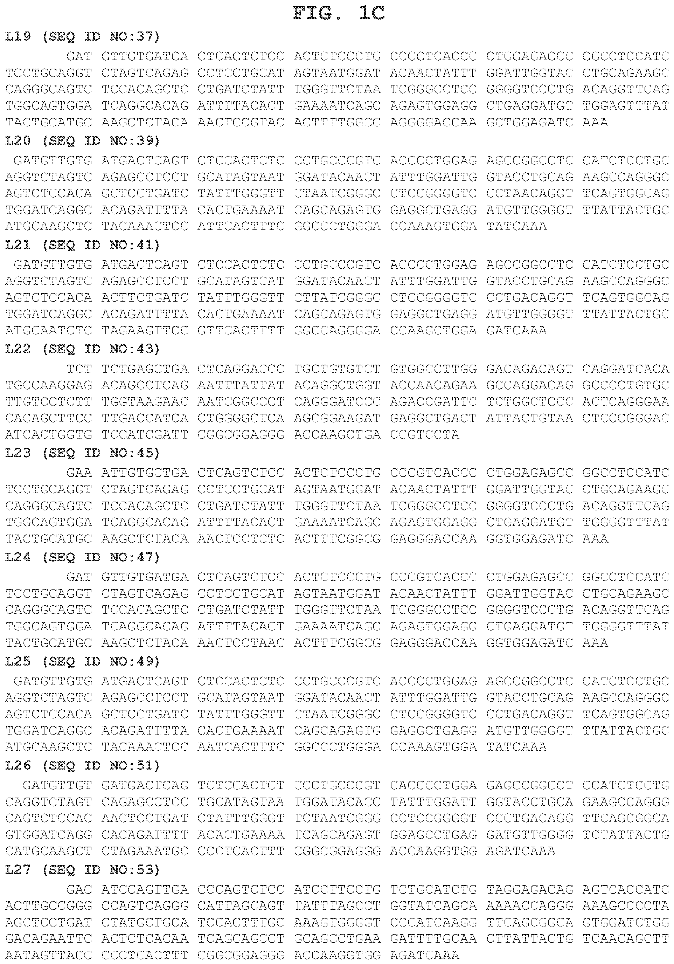

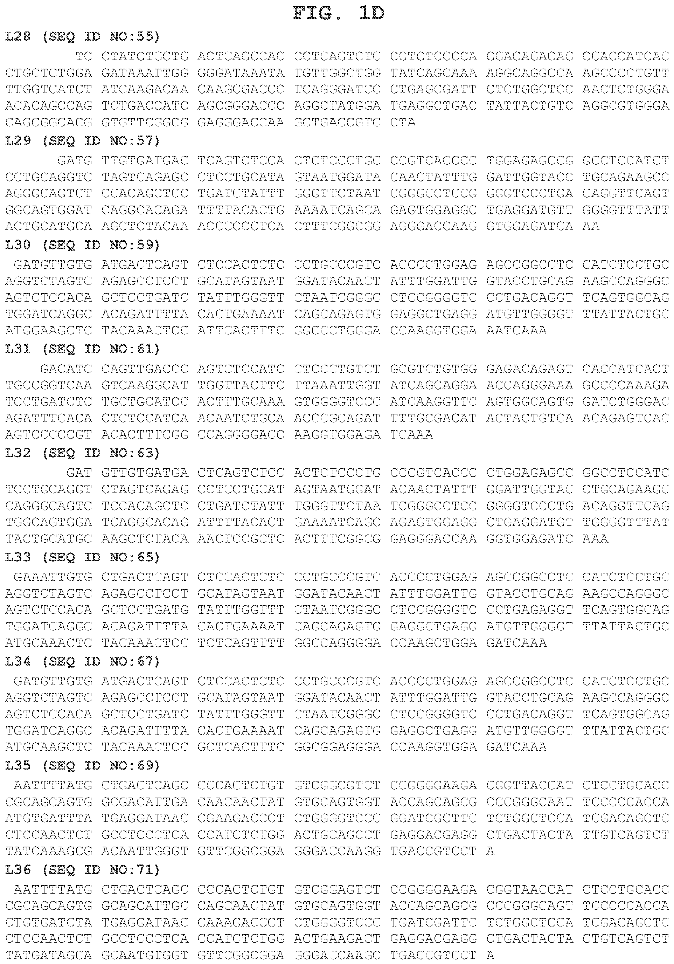

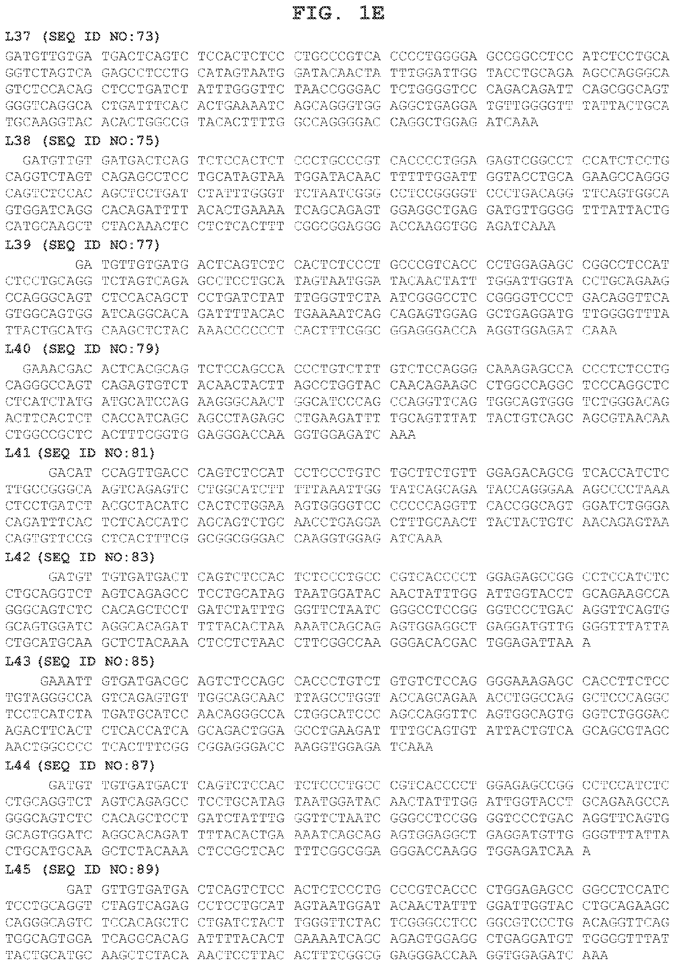

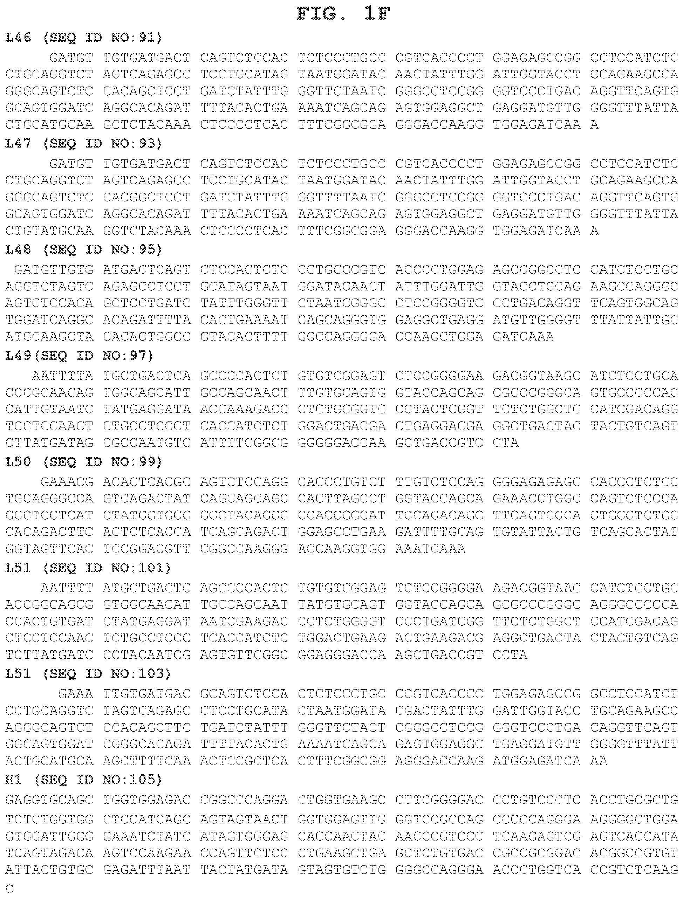

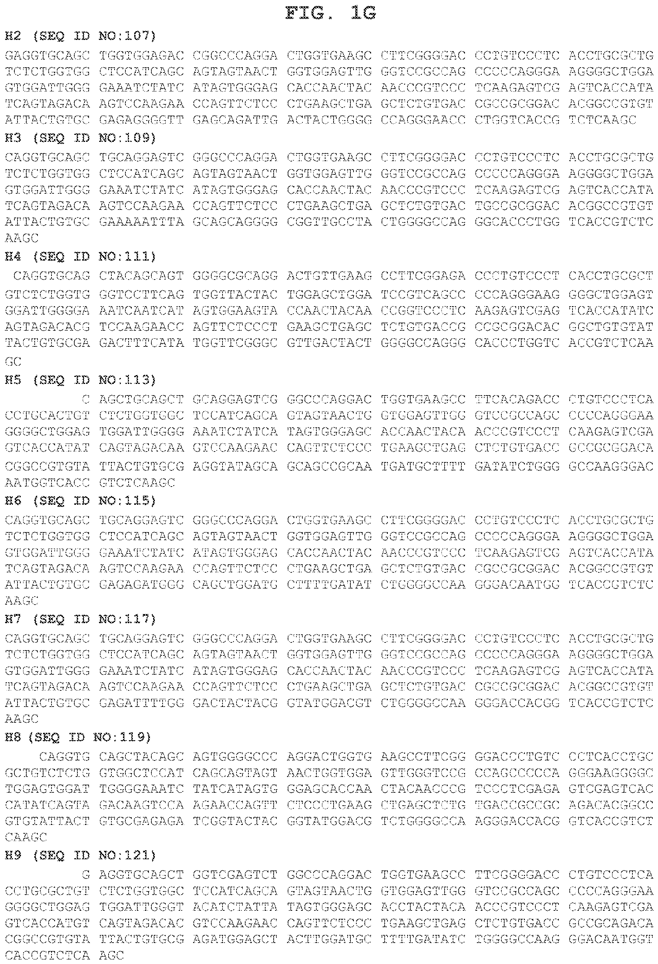

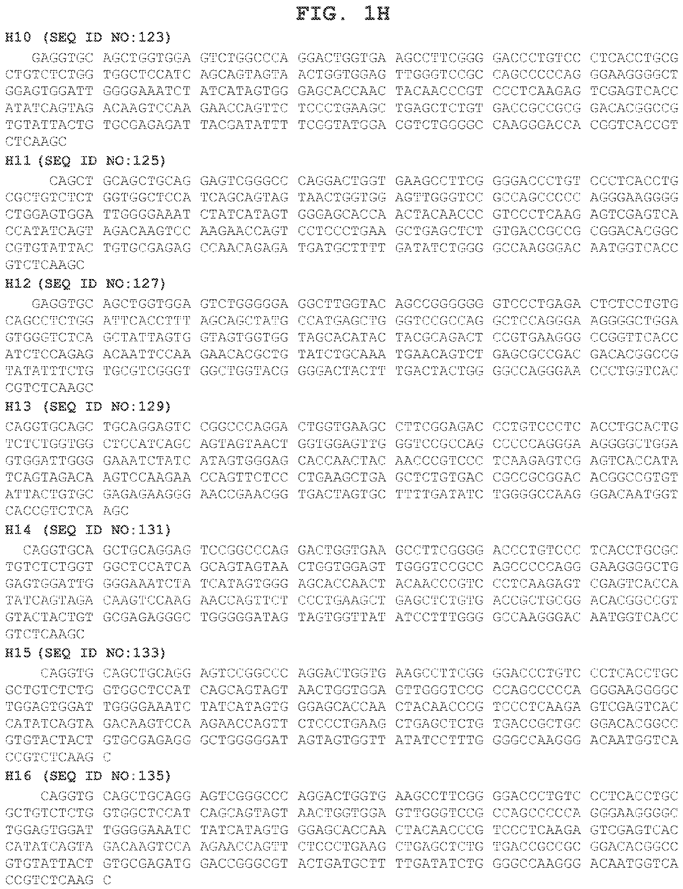

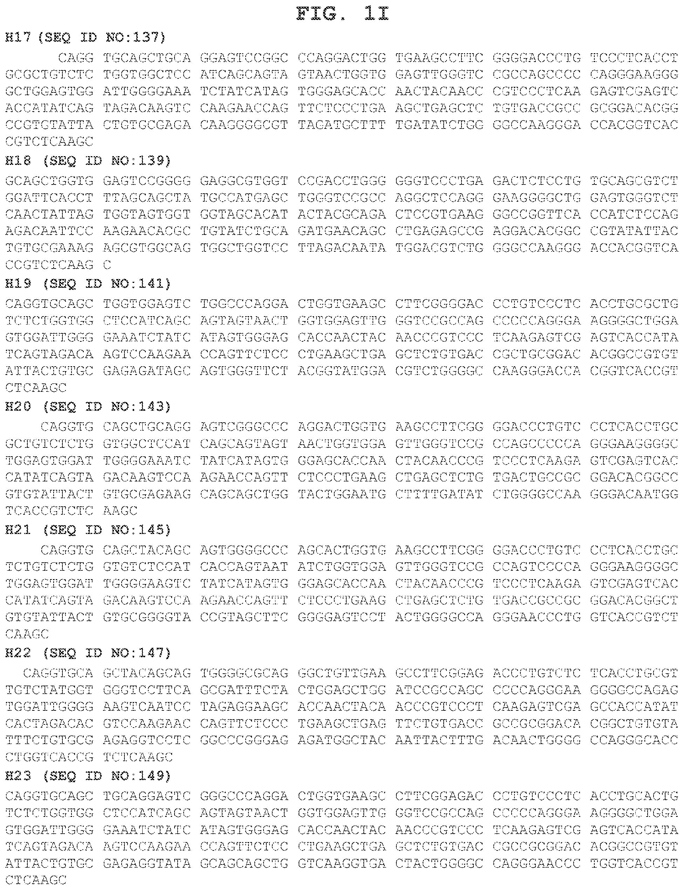

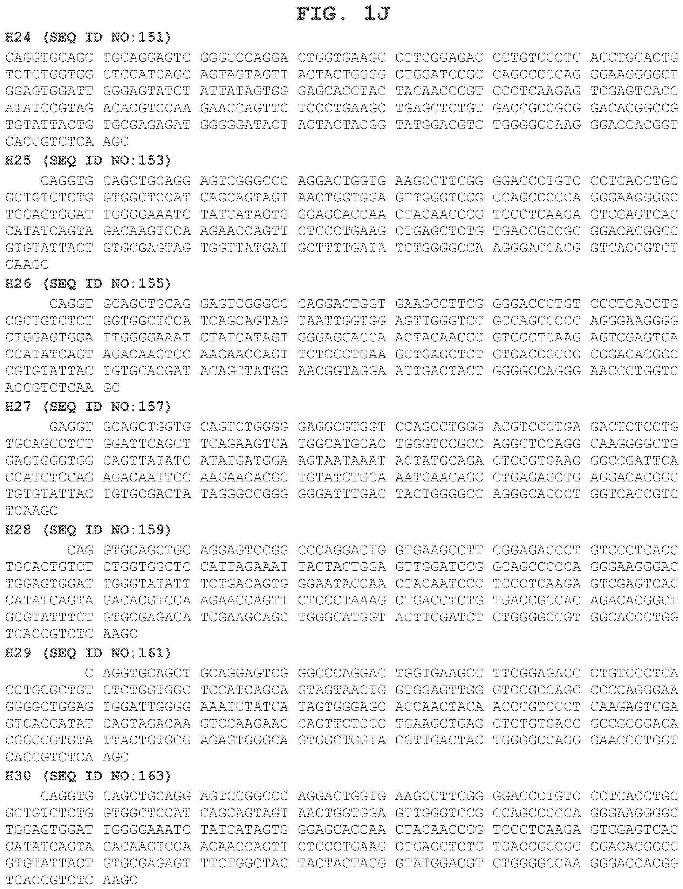

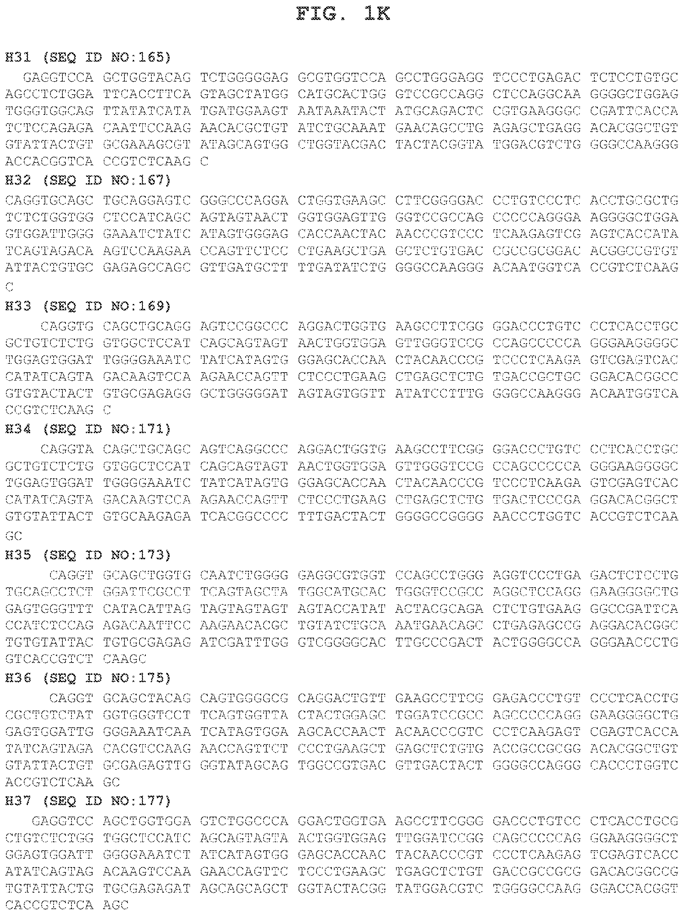

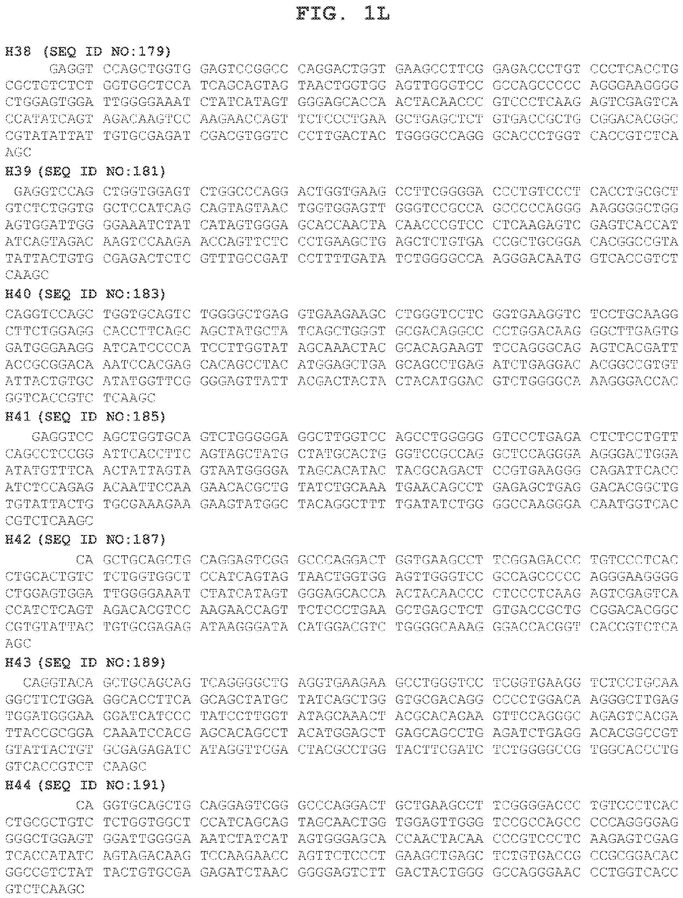

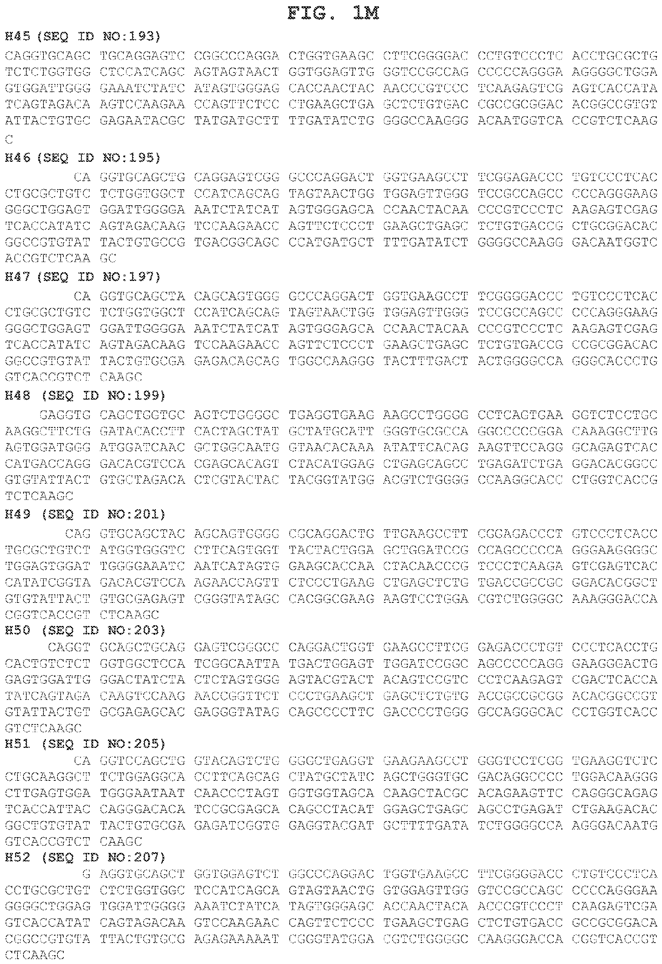

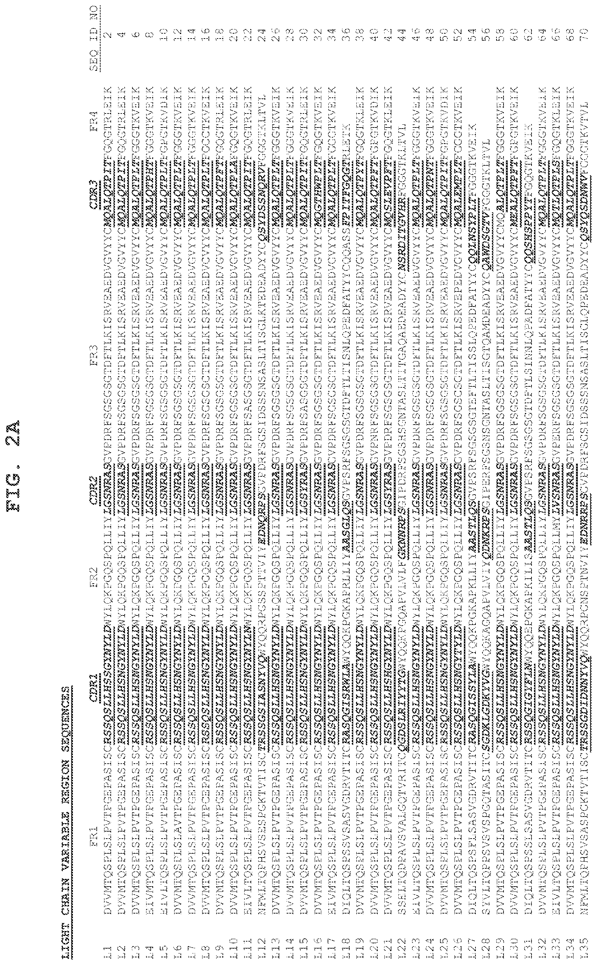

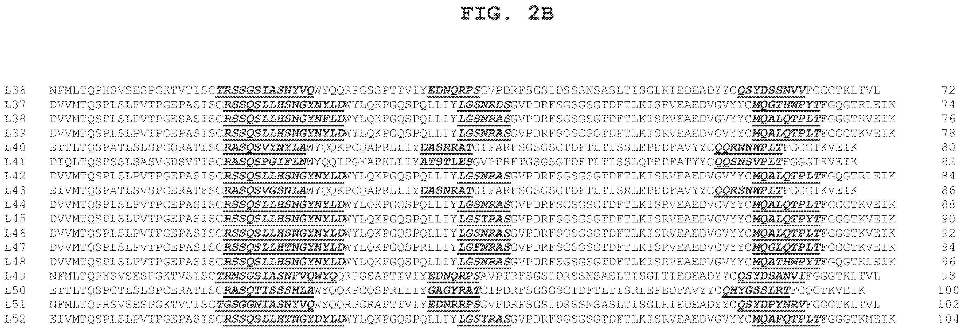

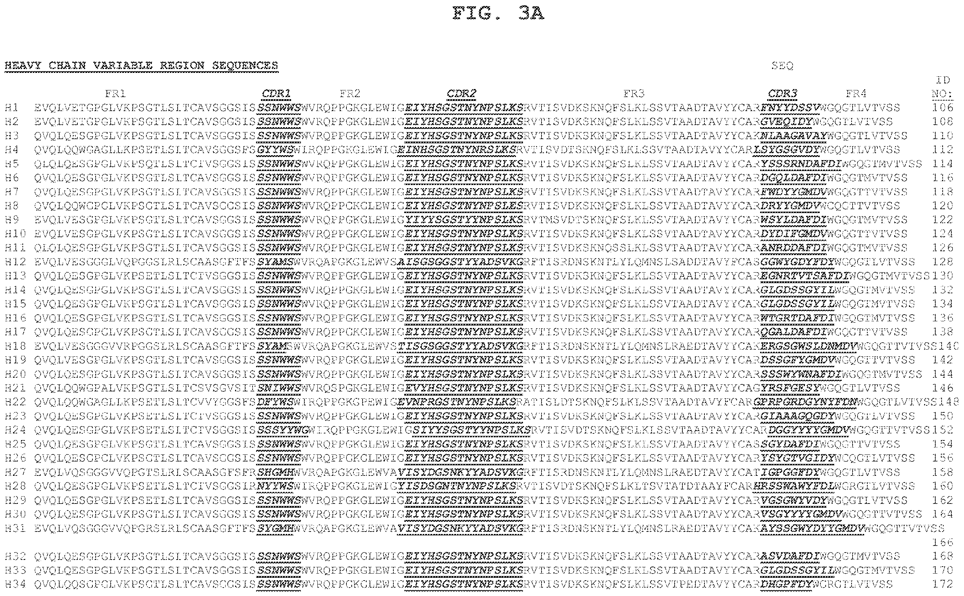

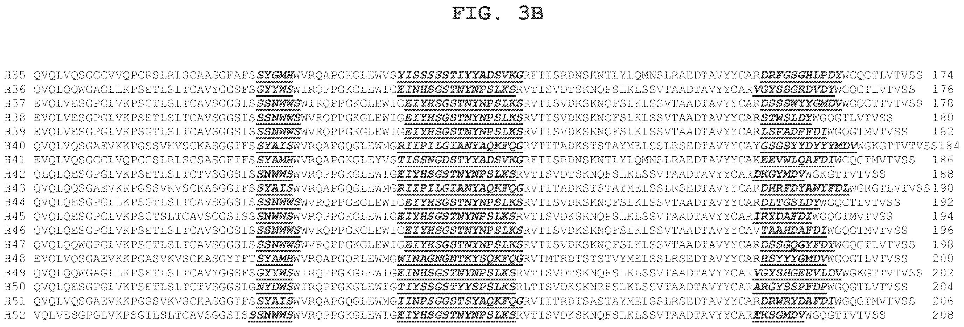

Polynucleotide and polypeptide sequences of particular light and heavy chain variable domains are shown in Figures 1, 2 and 3,, where they are labeled, for example, L1 ("light chain variable domain 1"), H1 ("heavy chain variable domain 1"), *etc.* Antibodies comprising a light chain and heavy chain from Figures 2 and 3 are indicated by combining the name of the light chain and the name of the heavy chain variable domains. For example, "L4H7," indicates an antibody comprising the light chain variable domain of L4 and the heavy chain variable domain of H7.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. See, *e.g.,* Sambrook et al. Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

The following terms, unless otherwise indicated, shall be understood to have the following meanings:
The term "isolated molecule" (where the molecule is, for example, a polypeptide, a polynucleotide, or an antibody) is a molecule that by virtue of its origin or source of derivation (1) is not associated with naturally associated components that accompany it in its native state, (2) is substantially free of other molecules from the same species (3) is expressed by a cell from a different species, or (4) does not occur in nature. Thus, a molecule that is chemically synthesized, or synthesized in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

The terms "IGF-1R inhibitor" and "IGF-1R antagonist" are used interchangeably. Each is a molecule that detectably inhibits at least one function of IGF-1R. Conversely, an "IGF-1R agonist" is a molecule that detectably increases at least one function of IGF-1R. The inhibition caused by an IGF-1R inhibitor need not be complete so long as it is detectable using an assay. Any assay of a function of IGF-1R can be used, examples of which are provided herein. Examples of functions of IGF-1R that can be inhibited by an IGF-1R inhibitor, or increased by an IGF-1R agonist, include binding to IGF-1, IGF-12, and/or another IGF-1R-activating molecule, kinase activity, downstream signaling, and so on. Examples of types of IGF-1R inhibitors and IGF-1R agonists include, but are not limited to, IGF-1R binding polypeptides such as antigen binding proteins (e.g., IGF-1R inhibiting antiben binding proteins), antibodies, antibody fragments, and antibody derivatives.

The terms "peptide," "polypeptide" and "protein" each refers to a molecule comprising two or more amino acid residues joined to each other by peptide bonds. These terms encompass, *e.g*., native and artificial proteins, protein fragments and polypeptide analogs (such as muteins, variants, and fusion proteins) of a protein sequence as well as post-translationally, or otherwise covalently or non-covalently, modified proteins. A peptide, polypeptide, or protein may be monomeric or polymeric.

The term "polypeptide fragment" as used herein refers to a polypeptide that has an amino-terminal and/or carboxy-terminal deletion as compared to a corresponding full-length protein. Fragments can be, for example, at least 5, 6, 7, 8, 9, 10, 11, 12, 13, 14, 15, 20, 50, 70, 80, 90, 100, 150 or 200 amino acids in length. Fragments can also be, for example, at most 1,000, 750, 500, 250, 200, 175, 150, 125, 100, 90, 80, 70, 60, 50, 40, 30, 20, 15, 14, 13, 12, 11, or 10 amino acids in length. A fragment can further comprise, at either or both of its ends, one or more additional amino acids, for example, a sequence of amino acids from a different naturally-occurring protein (*e.g*., an Fc or leucine zipper domain) or an artificial amino acid sequence (e*.g*., an artificial linker sequence).

Polypeptides herein described include polypeptides that have been modified in any way and for any reason, for example, to: (1) reduce susceptibility to proteolysis, (2) reduce susceptibility to oxidation, (3) alter binding affinity for forming protein complexes, (4) alter binding affinities, and (4) confer or modify other physicochemical or functional properties. Analogs include muteins of a polypeptide. For example, single or multiple amino acid substitutions (*e.g*., conservative amino acid substitutions) may be made in the naturally occurring sequence (*e.g*., in the portion of the polypeptide outside the domain(s) forming intermolecular contacts. A "conservative amino acid substitution" is one that does not substantially change the structural characteristics of the parent sequence (*e.g.,* a replacement amino acid should not tend to break a helix that occurs in the parent sequence, or disrupt other types of secondary structure that characterize the parent sequence or are necessary for its functionality). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et at. Nature 354:105 (1991).

Herein described are non-peptide analogs of IGF-1R binding polypeptides. Non-peptide analogs are commonly used in the pharmaceutical industry as drugs with properties analogous to those of the template peptide. These types of non-peptide compound are termed "peptide mimetics" or "peptidomimetics". Fauchere, J. Adv. Drug Res. 15:29 (1986); Veber and Freidinger TINS p.392 (1985); and Evans et al. J. Med. Chem. 30:1229 (1987). Peptide mimetics that are structurally similar to therapeutically useful peptides may be used to produce an equivalent therapeutic or prophylactic effect. Generally, peptidomimetics are structurally similar to a paradigm polypeptide (*i.e.,* a polypeptide that has a desired biochemical property or pharmacological activity), such as a human antibody, but have one or more peptide linkages optionally replaced by a linkage selected from the group consisting of: --CH₂NH--, --CH₂S--, --CH₂--CH₂-, --CH=CH-(*cis* and *trans*), --COCH₂--, --CH(OH)CH₂--, and --CH₂SO--, by methods well known in the art. Systematic substitution of one or more amino acids of a consensus sequence with a D-amino acid of the same type (*e.g.,* D-lysine in place of L-lysine) may also be used to generate more stable peptides. In addition, constrained peptides comprising a consensus sequence or a substantially identical consensus sequence variation may be generated by methods known in the art (Rizo and Gierasch Ann. Rev. Biochem. 61:387 (1992)), for example, by adding internal cysteine residues capable of forming intramolecular disulfide bridges which cyclize the peptide.

A "variant" of a polypeptide (*e.g.,* an antibody) comprises an amino acid sequence wherein one or more amino acid residues are inserted into, deleted from and/or substituted into the amino acid sequence relative to another polypeptide sequence. Variants include fusion proteins.

A "derivative" of a polypeptide is a polypeptide (*e.g*., an antibody) that has been chemically modified, *e.g*., via conjugation to another chemical moiety such as, for example, polyethylene glycol, albumin (*e.g.,* human serum albumin), phosphorylation, and glycosylation. Unless otherwise indicated, the term "antibody" includes, in addition to antibodies comprising two full-length heavy chains and two full-length light chains, derivatives, variants, fragments, and muteins thereof, examples of which are described below.

An "antigen binding protein" is a protein comprising a portion that binds to an antigen and, optionally, a scaffold or framework portion that allows the antigen binding portion to adopt a conformation that promotes binding of the antigen binding protein to the antigen. Examples of antigen binding proteins include antibodies, antibody fragments (e.g., an antigen binding portion of an antibody), antibody derivatives, and antibody analogs. The antigen binding protein can comprise, for example, an alternative protein scaffold or artificial scaffold with grafted CDRs or CDR derivatives. Such scaffolds include, but are not limited to, antibody-derived scaffolds comprising mutations introduced to, for example, stabilize the three-dimensional structure of the antigen binding protein as well as wholly synthetic scaffolds comprising, for example, a biocompatible polymer. See, for example, Korndorfer et al., 2003, Proteins: Structure, Function, and Bioinformatics, Volume 53, Issue 1:121-129; Roque et al., 2004, Biotechnol. Prog. 20:639-654. In addition, peptide antibody mimetics ("PAMs") can be used, as well as scaffolds based on antibody mimetics utilizing fibronection components as a scaffold.

An antigen binding protein can have, for example, the structure of a naturally occurring immunoglobulin. An "immunoglobulin" is a tetrameric molecule. In a naturally occurring immunoglobulin, each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light/heavy chain pair form the antibody binding site such that an intact immunoglobulin has two binding sites.

Naturally occurring immunoglobulin chains exhibit the same general structure of relatively conserved framework regions (FR) joined by three hypervariable regions, also called complementarity determining regions or CDRs. From N-terminus to C-terminus, both light and heavy chains comprise the domains FR1, CDR1, FR2, CDR2, FR3, CDR3 and FR4. The assignment of amino acids to each domain is in accordance with the definitions of Kabat et al. in Sequences of Proteins of Immunological Interest, 5th Ed., US Dept. of Health and Human Services, PHS, NIH, NIH Publication no. 91-3242, 1991.

An "antibody" refers to an intact immunoglobulin or to an antigen binding portion thereof that competes with the intact antibody for specific binding, unless otherwise specified. Antigen binding portions may be produced by recombinant DNA techniques or by enzymatic or chemical cleavage of intact antibodies. Antigen binding portions include, *inter alia,* Fab, Fab', F(ab')₂, Fv, domain antibodies (dAbs), and complementarity determining region (CDR) fragments, single-chain antibodies (scFv), chimeric antibodies, diabodies, triabodies, tetrabodies, and polypeptides that contain at least a portion of an immunoglobulin that is sufficient to confer specific antigen binding to the polypeptide.

A Fab fragment is a monovalent fragment having the V_{L}, V_{H}, C_{L} and C_{H}1 domains; a F(ab')₂ fragment is a bivalent fragment having two Fab fragments linked by a disulfide bridge at the hinge region; a Fd fragment has the V_{H} and C_{H}1 domains; an Fv fragment has the V_{L} and V_{H} domains of a single arm of an antibody; and a dAb fragment has a V_{H} domain, a V_{L} domain, or an antigen-binding fragment of a V_{H} or V_{L} domain (US Pat. No. 6,846,634, 6,696,245, US App. Pub. No. 05/0202512, 04/0202995, 04/0038291, 04/0009507, 03/0039958, Ward et al., Nature 341:544-546, 1989).

A single-chain antibody (scFv) is an antibody in which a V_{L} and a V_{H} region are joined via a linker (*e.g*., a synthetic sequence of amino acid residues) to form a continuous protein chain wherein the linker is long enough to allow the protein chain to fold back on itself and form a monovalent antigen binding site (see, *e.g.,* Bird et al., 1988, Science 242:423-26 and Huston et al., 1988, Proc. Natl. Acad. Sci. USA 85:5879-83). Diabodies are bivalent antibodies comprising two polypeptide chains, wherein each polypeptide chain comprises V_{H} and V_{L} domains joined by a linker that is too short to allow for pairing between two domains on the same chain, thus allowing each domain to pair with a complementary domain on another polypeptide chain (see, *e.g.,* Holliger et al., 1993, Proc. Natl. Acad. Sci. USA 90:6444-48, and Poljak et al., 1994, Structure 2:1121-23). If the two polypeptide chains of a diabody are identical, then a diabody resulting from their pairing will have two identical antigen binding sites. Polypeptide chains having different sequences can be used to make a diabody with two different antigen binding sites. Similarly, tribodies and tetrabodies are antibodies comprising three and four polypeptide chains, respectively, and forming three and four antigen binding sites, respectively, which can be the same or different.

Complementarity determining regions (CDRs) and framework regions (FR) of a given antibody may be identified using the system described by Kabat et al. in Sequences of Proteins of Immunological Interest, 5th Ed., US Dept. of Health and Human Services, PHS, NIH, NIH Publication no. 91-3242, 1991. One or more CDRs may be incorporated into a molecule either covalently or noncovalently to make it an antigen binding protein. An antigen binding protein may incorporate the CDR(s) as part of a larger polypeptide chain, may covalently link the CDR(s) to another polypeptide chain, or may incorporate the CDR(s) noncovalently. The CDRs permit the antigen binding protein to specifically bind to a particular antigen of interest.

An antigen binding protein may have one or more binding sites. If there is more than one binding site, the binding sites may be identical to one another or may be different. For example, a naturally occurring human immunoglobulin typically has two identical binding sites, while a "bispecific" or "bifunctional" antibody has two different binding sites.

The term "human antibody" includes all antibodies that have one or more variable and constant regions derived from human immunoglobulin sequences. All of the variable and constant domains may be derived from human immunoglobulin sequences (a fully human antibody). These antibodies may be prepared in a variety of ways, examples of which are described below, including through the immunization with an antigen of interest of a mouse that is genetically modified to express antibodies derived from human heavy and/or light chain-encoding genes.

A humanized antibody has a sequence that differs from the sequence of an antibody derived from a non-human species by one or more amino acid substitutions, deletions, and/or additions, such that the humanized antibody is less likely to induce an immune response, and/or induces a less severe immune response, as compared to the non-human species antibody, when it is administered to a human subject. Certain amino acids in the framework and constant domains of the heavy and/or light chains of the non-human species antibody may be mutated to produce the humanized antibody. The constant domain(s) from a human antibody may be fused to the variable domain(s) of a non-human species. One or more amino acid residues in one or more CDR sequences of a non-human antibody may be changed to reduce the likely immunogenicity of the non-human antibody when it is administered to a human subject, wherein the changed amino acid residues either are not critical for immunospecific binding of the antibody to its antigen, or the changes to the amino acid sequence that are made are conservative changes, such that the binding of the humanized antibody to the antigen is not significantly worse than the binding of the non-human antibody to the antigen. Examples of how to make humanized antibodies may be found in U.S. Pat. Nos. 6,054,297, 5,886,152 and 5,877,293.

The term "chimeric antibody" refers to an antibody that contains one or more regions from one antibody and one or more regions from one or more other antibodies. One or more of the CDRs may be derived from a human anti-IGF-1R antibody. All of the CDRs may be derived from a human anti-IGF-1R antibody. The CDRs from more than one human anti-IGF-1R antibodies may be mixed and matched in a chimeric antibody. For instance, a chimeric antibody may comprise a CDR1 from the light chain of a first human anti-IGF-1R antibody, a CDR2 and a CDR3 from the light chain of a second human anti-IGF-I R antibody, and the CDRs from the heavy chain from a third anti-IGF-1R antibody. Further, the framework regions may be derived from one of the same anti-IGF-1R antibodies, from one or more different antibodies, such as a human antibody, or from a humanized antibody. In one example of a chimeric antibody, a portion of the heavy and/or light chain is identical with, homologous to, or derived from an antibody from a particular species or belonging to a particular antibody class or subclass, while the remainder of the chain(s) is/are identical with, homologous to, or derived from an antibody (- ies) from another species or belonging to another antibody class or subclass. Also included are fragments of such antibodies that exhibit the desired biological activity (*i.e.,* the ability to specifically bind IGF-1R). See, *e.g.*, U.S. Patent No. 4,816,567 and Morrison, 1985, Science 229:1202-07.

A "neutralizing antibody" or "an inhibitory antibody" is an antibody that inhibits the binding of IGF-1R to IGF-I and/or IGF-2 when an excess of the anti-IGF-1R antibody reduces the amount of IGF-1and/or IGF-2 bound to IGF-1R by at least about 20% using the assay described in Example 9. The antibody may reduce the amount of IGF-I and/or IGF-2 bound to IGF-1R by at least 30%, 40%, 50%, 60%, 70%, 75%, 80%, 85%, 90%, 95%, 97%, 99%, and 99.9%.

An "activating antibody" is an antibody that activates IGF-1R by at least about 20% when added to a cell, tissue or organism expressing IGF-1R, where "100% activation" is the level of activation achieved under physiological conditions by the same molar amount of IGF-1and/or IGF-2. The antibody may activate IGF-1R activity by at least 30%, 40%, 50%, 60%, 70%, 80%, 90%, 100%, 125%, 150%, 175%, 200%, 250%, 300%, 350%, 400%, 450%, 500%, 750%, or 1000%.

Fragments or analogs of antibodies can be readily prepared by those of ordinary skill in the art following the teachings of this specification and using techniques well-known in the art. Preferred amino- and carboxy-termini of fragments or analogs occur near boundaries of functional domains. Structural and functional domains can be identified by comparison of the nucleotide and/or amino acid sequence data to public or proprietary sequence databases. Computerized comparison methods can be used to identify sequence motifs or predicted protein conformation domains that occur in other proteins of known structure and/or function. Methods to identify protein sequences that fold into a known three-dimensional structure are known. See, *e.g.,* Bowie et al., 1991, Science 253:164.

A "CDR grafted antibody" is an antibody comprising one or more CDRs derived from an antibody of a particular species or isotype and the framework of another antibody of the same or different species or isotype.

A "multi-specific antibody" is an antibody that recognizes more than one epitope on one or more antigens. A subclass of this type of antibody is a "bi-specific antibody" which recognizes two distinct epitopes on the same or different antigens.

An antigen binding protein "specifically binds" to an antigen (*e.g.,* human IGF-1R) if it binds to the antigen with a dissociation constant of 1 nanomolar or less.

An "antigen binding domain," "antigen binding region," or "antigen binding site" is a portion of an antigen binding protein that contains amino acid residues (or other moieties) that interact with an antigen and contribute to the antigen binding protein's specificity and affinity for the antigen. For an antibody that specifically binds to its antigen, this will include at least part of at least one of its CDR domains.

An "epitope" is the portion of a molecule that is bound by an antigen binding protein (e.g., by an antibody). An epitope can comprise non-contiguous portions of the molecule (*e.g.,* in a polypeptide, amino acid residues that are not contiguous in the polypeptide's primary sequence but that, in the context of the polypeptide's tertiary and quaternary structure, are near enough to each other to be bound by an antigen binding protein).

The "percent identity" of two polynucleotide or two polypeptide sequences is determined by comparing the sequences using the GAP computer program (a part of the GCG Wisconsin Package, version 10.3 (Accelrys, San Diego, CA)) using its default parameters.

The terms "polynucleotide," "oligonucleotide" and "nucleic acid" are used interchangeably throughout and include DNA molecules (*e.g*., cDNA or genomic DNA), RNA molecules (*e.g*., mRNA), analogs of the DNA or RNA generated using nucleotide analogs (*e.g*., peptide nucleic acids and non-naturally occurring nucleotide analogs), and hybrids thereof. The nucleic acid molecule can be single-stranded or double-stranded. The nucleic acid molecules described herein may comprise a contiguous open reading frame encoding an antibody, or a fragment, derivative, mutein, or variant thereof.

Two single-stranded polynucleotides are "the complement" of each other if their sequences can be aligned in an anti-parallel orientiation such that every nucleotide in one polynucleotide is opposite its complementary nucleotide in the other polynucleotide, without the introduction of gaps, and without unpaired nucleotides at the 5' or the 3' end of either sequence. A polynucleotide is "complementary" to another polynucleotide if the two polynucleotides can hybridize to one another under moderately stringent conditions. Thus, a. polynucleotide can be complementary to another polynucleotide without being its complement.

A "vector" is a nucleic acid that can be used to introduce another nucleic acid linked to it into a cell. One type of vector is a "plasmid," which refers to a linear or circular double stranded DNA molecule into which additional nucleic acid segments can be ligated. Another type of vector is a viral vector (*e.g*., replication defective retroviruses, adenoviruses and adeno-associated viruses), wherein additional DNA segments can be introduced into the viral genome. Certain vectors are capable of autonomous replication in a host cell into which they are introduced (*e.g*., bacterial vectors comprising a bacterial origin of replication and episomal mammalian vectors). Other vectors (*e.g.,* non-episomal mammalian vectors) are integrated into the genome of a host cell upon introduction into the host cell, and thereby are replicated along with the host genome. An "expression vector" is a type of vector that can direct the expression of a chosen polynucleotide.

A nucleotide sequence is "operably linked" to a regulatory sequence if the regulatory sequence affects the expression (*e.g*., the level, timing, or location of expression) of the nucleotide sequence. A "regulatory sequence" is a nucleic acid that affects the expression (*e.g.,* the level, timing, or location of expression) of a nucleic acid to which it is operably linked. The regulatory sequence can, for example, exert its effects directly on the regulated nucleic acid, or through the action of one or more other molecules (*e.g*., polypeptides that bind to the regulatory sequence and/or the nucleic acid). Examples of regulatory sequences include promoters, enhancers and other expression control elements (*e.g*., polyadenylation signals). Further examples of regulatory sequences are described in, for example, Goeddel, 1990, Gene Expression Technology: Methods in Enzymology 185, Academic Press, San Diego, CA and Baron et al., 1995, Nucleic Acids Res. 23:3605-06.

A "host cell" is a cell that can be used to express a nucleic acid. A host cell can be a prokaryote, for example, *E. coli,* or it can be a eukaryote, for example, a single-celled eukaryote (*e.g.,* a yeast or other fungus), a plant cell (*e.g.,* a tobacco or tomato plant cell), an animal cell (*e.g.,* a human cell, a monkey cell, a hamster cell, a rat cell, a mouse cell, or an insect cell) or a hybridoma. Examples of host cells include the COS-7 line of monkey kidney cells (ATCC CRL 1651) (see Gluzman et al., 1981, Cell 23:175), L cells, C127 cells, 3T3 cells (ATCC CCL 163), Chinese hamster ovary (CHO) cells or their derivatives such as Veggie CHO and related cell lines which grow in serum-free media (see Rasmussen et al., 1998, Cytotechnology 28:31) or CHO strain DX-B11, which is deficient in DHFR (see Urlaub et al., 1980, Proc. Natl. Acad. Sci. USA 77:4216-20), HeLa cells, BHK (ATCC CRL 10) cell lines, the CV1/EBNA cell line derived from the African green monkey kidney cell line CV1 (ATCC CCL 70) (see McMahan et al., 1991, EMBO J. 10:2821), human embryonic kidney cells such as 293, 293 EBNA or MSR 293, human epidermal A431 cells, human Colo205 cells, other transformed primate cell lines, normal diploid cells, cell strains derived from in vitro culture of primary tissue, primary explants, HL-60, U937, HaK or Jurkat cells. Typically, a host cell is a cultured cell that can be transformed or transfected with a polypeptide-encoding nucleic acid, which can then be expressed in the host cell. The phrase "recombinant host cell" can be used to denote a host cell that has been transformed or transfected with a nucleic acid to be expressed. A host cell also can be a cell that comprises the nucleic acid but does not express it at a desired level unless a regulatory sequence is introduced into the host cell such that it becomes operably linked with the nucleic acid. It is understood that the term host cell refers not only to the particular subject cell but to the progeny or potential progeny of such a cell. Because certain modifications may occur in succeeding generations due to, *e.g.,* mutation or environmental influence, such progeny may not, in fact, be identical to the parent cell, but are still included within the scope of the term as used herein.

### IGF-1R

IGF-1R is a transmembrane receptor tyrosine kinase (Blume-Jensen et al., 2001, Nature 411:355-65). The human IGF-1R is synthesized as a 1367 amino acid precursor polypeptide that includes a 30 amino acid signal peptide removed during translocation into the endoplasmic reticulum (Swiss-Prot: P08069). The IGF-1R proreceptor is glycosylated and cleaved by a protease at positions 708-711 (counting from the first amino acid following the signal peptide sequence) during maturation in the ER-golgi resulting in the formation of an α-chain (1-707) and a β-chain (712-1337) that remain linked by disulfide bonds (Bhaumick et al., 1981, Proc Natl Acad Sci USA 78:4279-83, Chernausek et al., 1981, Biochemistry 20:7345-50, Jacobs et al., 1983, Proc Natl Acad Sci USA 80:1228-31, LeBon et al., 1986, J Biol Chem 261:7685-89, Elleman, et al., 2000, Biochem J 347:771-79). The predominant form of the IGF-1R (and INSR) that exists on the cell-surface is a proteolytically processed and glycosylated (αβ)₂ dimer joined covalently by one or more disulfide bonds.

The extracellular portion of the IGF-1R consists of the α-chain and 191 amino acids of the β-chain (712-905). The receptor contains a single transmembrane spanning sequence (906-929) and a 408-residue cytoplasmic domain that includes a functional tyrosine kinase (Rubin et al., 1983, Nature 305:438-440). Comparative sequence analysis has revealed that the IGF-1R is composed of 11 distinct structural motifs (reviewed by Adams et al., 2000, Cell Mol Life Sci 57:1050-93, Marino-Buslje et al., 1998, FEBS Ltrs 441:331-36, Ward et al., 2001, BMC Bioinformatics 2:4). The N-terminal half of the extracellular domain contains two homologous domains referred to as L1 (1-151) and L2 (299-461) (Ward *et al.,* 2001, *supra*) separated by a cysteine-rich (CR) region (152-298) consisting of several structural modules with disulfide linkages that align with repeating units present in the TNF receptor and laminin (Ward et al., 1995, Proteins 22:141-53). The crystal structure of the L1-CR-L2 domain has been solved (Garrett et al., 1998, Nature 394:395-99). The L2 domain is followed by three fibronectin type III domains (Marino-Buslje *et al.,* 1998, *supra,* Mulhern et al., 1998, Trends Biochem Sci 23:465-66, Ward et al., 1999, Growth Factors 16:315-22). The first FnIII domain (FnIII-1, 461-579) is 118 amino acids in length. The second FnIII domain (FnIII-2, 580-798) is disrupted by a major insert sequence (ID) of about 120 amino acids in length. The ID domain includes a furin protease cleavage site that separates the α and β chains of the mature receptor. The third FnIII domain (FnIII-3) is located entirely in the β-chain (799-901) terminating several residues before the transmembrane sequence. The catalytic domain of the IGF-1R tyrosine kinase is located between amino acids positions 973-1229, and its structure has been solved (Favelyukis et al., 2001, Nature Structural Biol 8:1058-63, Pautsch et al., 2001, Structure 9:955-65). The kinase is flanked by two regulatory regions, the juxtamembrane region (930-972) and a 108 amino acid C-terminal tail (1220-1337) (Surmacz et al., 1995, Experimental Cell Res 218:370-80, Hongo et al., 1996, Oncogene 12:1231-38). The two regulatory regions contain tyrosine residues that serve as docking sites for signal transducing proteins when phosphorylated by the activated IGF-1R tyrosine kinase (reviewed by Baserga (ed.), 1998 The IGF-1 Receptor in Normal and Abnormal Growth, Hormones and Growth Factors in Development and Neoplasia, Wiley-Liss, Inc., Adams et al., 2000, Cell Mol Life Sci 57:1050-93).

The IGF-1R amino acid sequence is about 70% identical to the insulin receptor (INSR; Swiss-Prot: P06213). The highest homology between the receptors is located in the tyrosine kinase domain (84%); the lowest identity is in the CR region and the C-terminus. The IGF-1R is also highly related (∼ 55% identical) to the insulin related receptor (IRR; Swiss-Prot: P14616).

Human IGF-1R can be activated by the insulin-like growth factors, IGF-1and IGF-2 and insulin (INS) (Hill et al., 1985, Pediatric Research 19:879-86). IGF-1and IGF-2 are encoded nonallelic genes (Brissenden et al., 1984, Nature 310: 781-8, Bell et al., 1985, Proceedings of the National Academy of Sciences of the United States of America 82: 6450-54), and both genes express alternative proteins related by differential RNA splicing and protein processing. The most common and well-studied mature forms of IGF-1 and IGF-2 are respectively 70 and 67 amino acids in length (Jansen et al., 1983, Nature 306:609-11, Dull et al., 1984, Nature 310: 777-81). These proteins (and their isoforms) are identical at 11/21 positions to the insulin A-peptide, and identical at 12/30 positions with the insulin B-peptide.

Both IGF-1and IGF-2 are secreted in large quantities by the liver under tight regulation by growth hormone secreted by the pituitary. In response to growth hormone (GH) which is in turn regulated by somatostatin (SMS) and GH releasing hormone. IGF-binding proteins (IGFBPs) are also secreted by the liver. IGF-1 and IGF-2 are both bound in circulation and sequestered in an inactive form by a series of seven IGF binding proteins (IGFBP 1-7) that bind to IGF1 and IGF2 with an affinity that is in the same order of magnitude as IGF-1R. Therefore IGF-1 and IGF-2 are only active when unbound and "bioavailable." These binding proteins are also secreted by the liver and numerous other tissues and serve a number of functions with respect to regulating levels of free IGFs and in turn activity of IGF-1R in tumor tissue. IGFBP-3 provides most of the IGF binding capacity of serum and greatly prolongs the circulating half-life of the IGFs while competing with receptor for its ligands. The ternary complex of IGF- 1or IGF-2 and IGFBP-3, together with the acid labile subunit, is stable until dissociation through proteolysis of IGFBP-3 by proteolytic enzymes that are expressed at sites of IGF ligand activity. The functions of the other six IGFBPs are not well characterized apart from there IGF-1and IGF-2 binding activities.

IGF-1R is expressed in all cells types in the normal adult animal except for liver hepatocytes and mature B-cells. Human blood plasma contains high concentrations of IGF-1 and IGF-2, and IGF-1can be detected in most tissues. The receptor is an integral component of the physiological mechanism controlling organ size and homeostasis. Without being bound to a particular theory, the "Somatomedin Hypothesis" states that Growth Hormone (GH) mediated somatic growth that occurs during childhood and adolescence is dependent on the endocrine form of IGF-1 that is mainly produced and secreted by the liver (Daughaday, 2000, Pediatric Nephrology 14: 537-40). The synthesis of hepatic IGF-1is stimulated by GH release in the pituitary in response to hypothalamic GHRH (GH releasing hormone). The serum concentration of IGF-1increases over 100 fold between ages 5-15 in humans. The bioavailability of IGF-1 is regulated by IGF binding protein 3 (IGFBP3) with approximately 99% of the growth factor compartmentalized in the bound state. Primary IGF-1deficiency arising form partial gene deletions, and secondary IGF-1deficiency resulting from defects in GH production or signaling are not lethal (Woods, 1999, IGF Deficiency in Contemporary Endocrinology: The IGF System, R. a. R. Rosenfeld, C. Jr. Totowa, ed.s, Humana Press, NJ: 651-74). The affected individuals exhibit growth retardation at birth, grow slowly and can face certain CNS abnormalities.

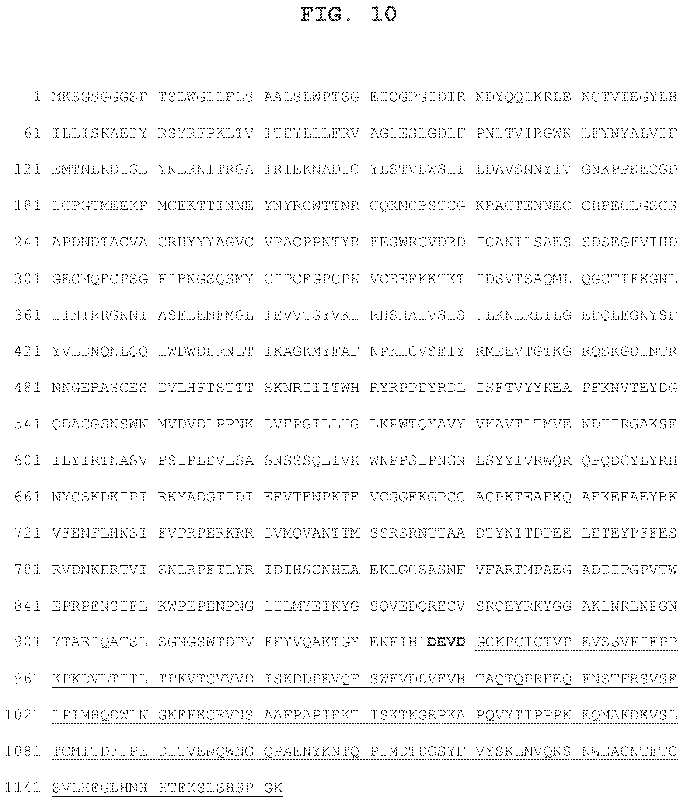

IGF-1R signaling promotes cell growth and survival through the IRS adapter protein-dependent activation of the PI3Kinase/Akt pathway. IGF-1R transmits a signal to its major substrates, IRS-1 through IRS-4 and the Shc proteins (Blakesley et al., 1999, IGF-1 receptor function: transducing the IGF-1 signal into intracellular events in The IGF System, R. G. a. R. Rosenfeld, Jr. C.T. Totowa, ed.s, Humana Press, NJ: 143-63). This results in activation of the Ras/Raf/MAP kinase and PI3 Kinase/Akt signaling pathways. However, induction of Akt-mediated cell survival via IRS is the dominant pathway response upon IGF stimulation of most cells. See Figure 10.

### Antigen binding proteins

Herein described are antigen binding proteins (*e.g*., antibodies, antibody fragments, antibody derivatives, antibody muteins, and antibody variants), that bind to IGF-1R, *e.g.,* human IGF-1R.

Antigen binding proteins include antigen binding proteins that inhibit a biological activity of IGF-1R. Examples of such biological activities include binding a signaling molecule (*e.g*., IGF-1and/or IGF-2), and transducing a signal in response to binding a signaling molecule.

Different antigen binding proteins may bind to different domains or epitopes of IGF-1R or act by different mechanisms of action. Examples include but are not limited to antigen binding proteins that interfere with binding of IGF-1and/or IGF-2 to IGF-1R or that inhibit signal transduction. The site of action may be, for example, intracellular (*e.g*., by interfering with an intracellular signaling cascade) or extracellular. An antigen binding protein need not completely inhibit an IGF-1and/or IGF-2 induced activity; rather, antigen binding proteins that reduce a particular activity of IGF-1and/or IGF-2 are contemplated for use as well. (Discussions herein of particular mechanisms of action for IGF-1R-binding antigen binding proteins in treating particular diseases are illustrative only, and the methods presented herein are not bound thereby.)

It has been observed that IGF-1 and IGF-2 each exhibits biphasic binding to IGF-1R. High affinity binding has been reported to have a K_{D} in the range of 0.2 nM; high affinity binding, about ten fold higher. Herein described is an IGF-1R inhibitor that inhibits both the high and low affinity binding of IGF-1 and/or IGF-2 to IGF-R. It has been suggested that the high affinity binding, rather than the low affinity binding, of IGF-1and/or IGF-2 to IGF-1R is required for the conformation change that activates the tyrosine kinase activity of IGF-1R. Thus, the IGF-1R inhibitor preferentially may inhibit the high affinity binding of IGF-1 and/or IGF-2 to IGF-1R as compared to the low affinity binding.

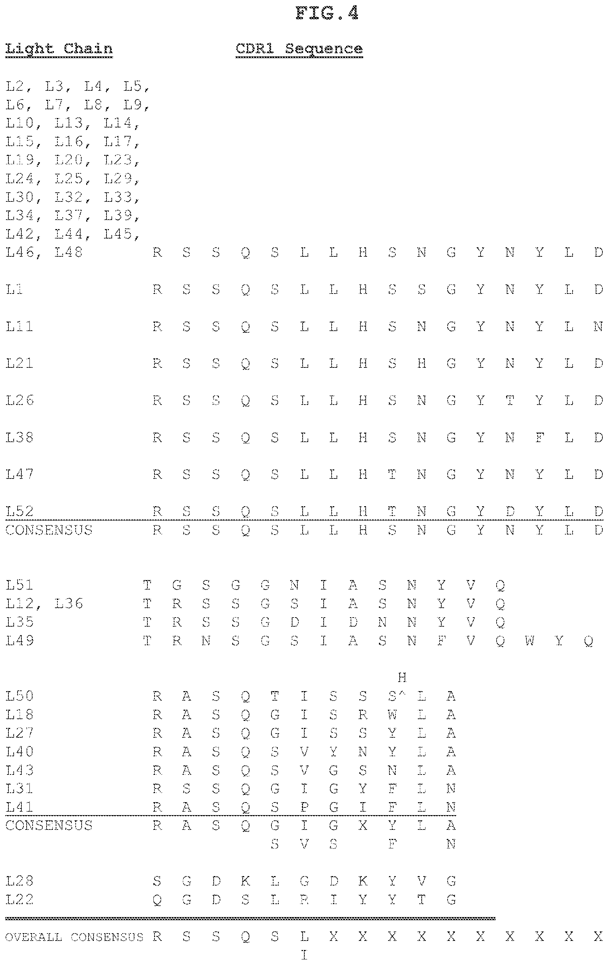

Also herein described are antigen binding proteins that comprise a light chain variable region selected from the group consisting of L1 through L52 and/or a heavy chain variable region selected from the group consisting of H1 through H52, and fragments, derivatives, muteins, and variants thereof (see Figures 2 and 3). Such an antigen binding protein can be denoted using the nomenclature "LxHy", wherein "x" corresponds to the number of the light chain variable region and "y" corresponds to the number of the heavy chain variable region as they are labeled in Figures 2 and 3. For example, L2H1 refers to an antigen binding protein with a light chain variable region comprising the amino acid sequence of L2 and a heavy chain variable region comprising the amino acid sequence of H1, as shown in Figures 2 and 3. Figures 2 and 3 also indicate the location of the CDR and framework regions of each of these variable domain sequences. The CDR regions of each light and heavy chain also are grouped by type and by sequence similarity in Figures 4 through 9. Antigen binding proteins include, for example, antigen binding proteins having a combination of light chain and heavy chain variable domains selected from the group of combinations consisting of L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51^{,} and L52H52^{.}

Herein described is an antigen binding protein comprising a light chain variable domain comprising a sequence of amino acids that differs from the sequence of a light chain variable domain selected from the group consisting of L1 through L52 only at 15, 14, 13, 12, 11, 10, 9, 8, 7, 6, 5, 4, 3, 2, or 1 residues, wherein each such sequence difference is independently either a deletion, insertion, or substitution of one amino acid residue. The light-chain variable domain may comprise a sequence of amino acids that is at least 70%, 75%, 80%, 85%, 90%, 95%, 97%, or 99% identical to the sequence of a light chain variable domain selected from the group consisting of L1 through L52. The light chain variable domain may comprise a sequence of amino acids that is encoded by a nucleotide sequence that is at least 70%, 75%, 80%, 85%, 90%, 95%, 97%, or 99% identical to a nucleotide sequence that encodes a light chain variable domain selected from the group consisting of L1 through L52. The light chain variable domain may comprise a sequence of amino acids that is encoded by a polynucleotide that hybridizes under moderately stringent conditions to the complement of a polynucleotide that encodes a light chain variable domain selected from the group consisting of L1 through L52. The light chain variable domain may comprise a sequence of amino acids that is encoded by a polynucleotide that hybridizes under moderately stringent conditions to the complement of a polynucleotide that encodes a light chain variable domain selected from the group consisting of L1 through L52. The light chain variable domain may comprise a sequence of amino acids that is encoded by a polynucleotide that hybridizes under moderately stringent conditions to a complement of a light chain polynucleotide selected from Figure 1.

Herein described is an antigen binding protein comprising a heavy chain variable domain comprising a sequence of amino acids that differs from the sequence of a heavy chain variable domain selected from the group consisting of H1 through H52 only at 15, 14, 13, 12, 11, 10, 9, 8, 7, 6, 5, 4, 3, 2, or 1 residue(s), wherein each such sequence difference is independently either a deletion, insertion, or substitution of one amino acid residue. The heavy chain variable domain may comprise a sequence of amino acids that is at least 70%, 75%, 80%, 85%, 90%, 95%, 97%, or 99% identical to the sequence of a heavy chain variable domain selected from the group consisting of H1 through H52. The heavy chain variable domain may comprise a sequence of amino acids that is encoded by a nucleotide sequence that is at least 70%, 75%, 80%, 85%, 90%, 95%, 97%, or 99% identical to a nucleotide sequence that encodes a heavy chain variable domain selected from the group consisting of H1 through H52. The heavy chain variable domain may comprise a sequence of amino acids that is encoded by a polynucleotide that hybridizes under moderately stringent conditions to the complement of a polynucleotide that encodes a heavy chain variable domain selected from the group consisting of H1 through H52. The heavy chain variable domain may comprise a sequence of amino acids that is encoded by a polynucleotide that hybridizes under moderately stringent conditions to the complement of a polynucleotide that encodes a heavy chain variable domain selected from the group consisting of H1 through H52. The heavy chain variable domain may comprise a sequence of amino acids that is encoded by a polynucleotide that hybridizes under moderately stringent conditions to a complement of a heavy chain polynucleotide selected from Figure 1.

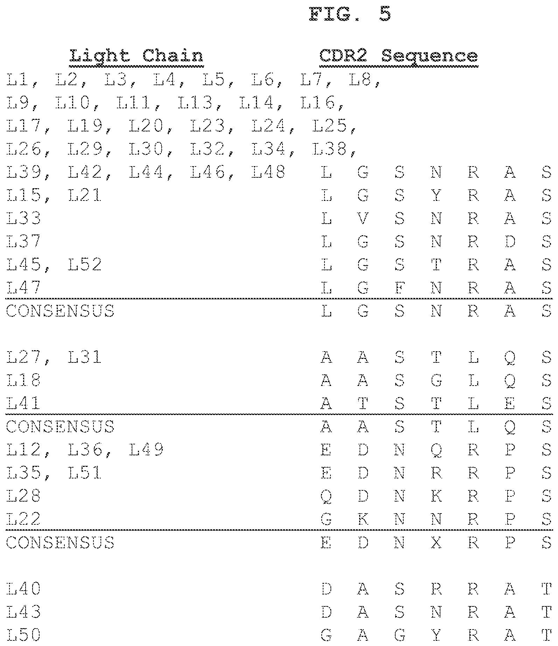

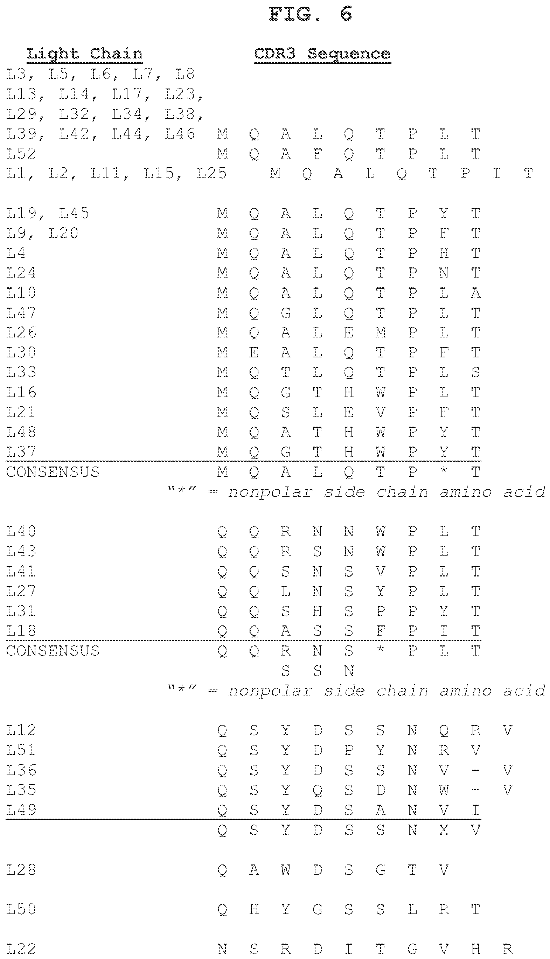

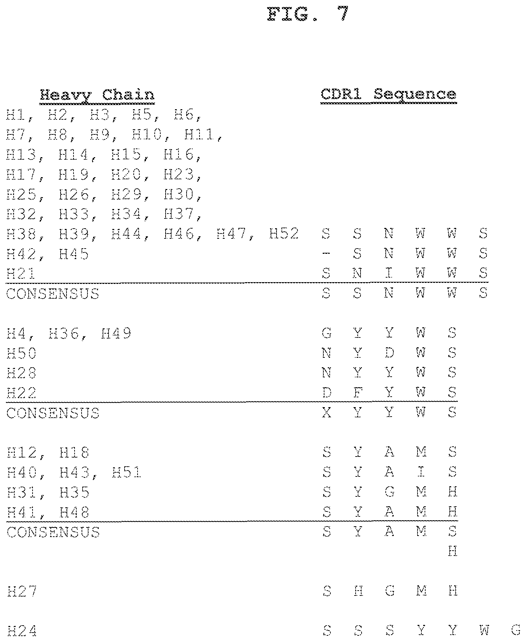

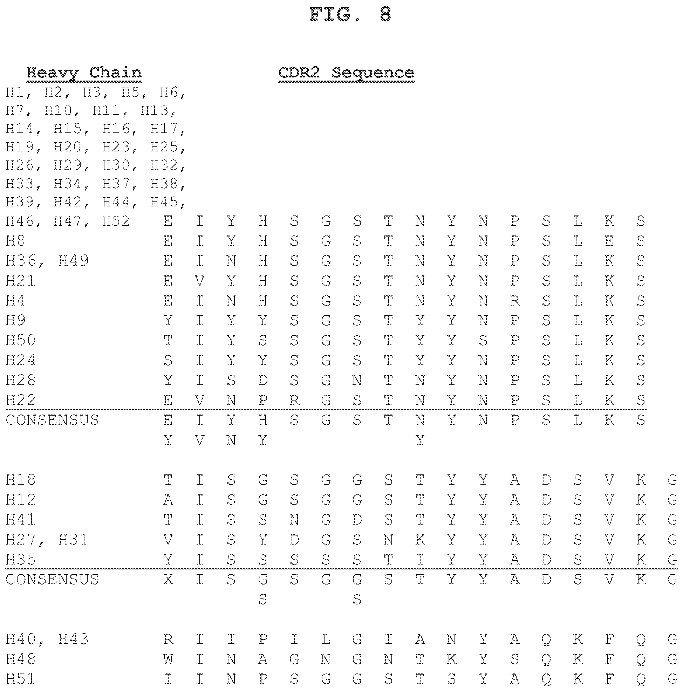

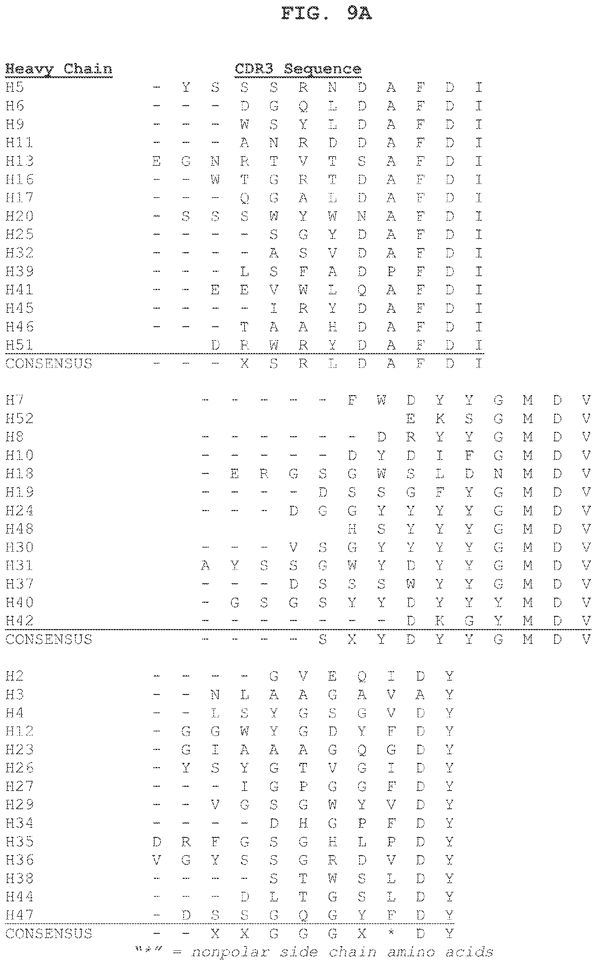

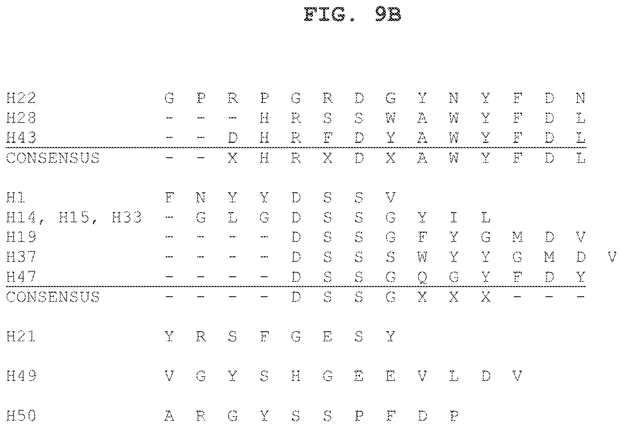

Particular antigen binding proteins described herein comprise one or more amino acid sequences that are identical to the amino acid sequences of one or more of the CDRs and/or FRs illustrated in Figures 2 through 9. The antigen binding protein may comprise a light chain CDR1 sequence illustrated in Figure 4. The antigen binding protein may comprise a light chain CDR2 sequence illustrated in Figure 5. The antigen binding protein may comprise a light chain CDR3 sequence illustrated in Figure 6. The antigen binding protein may comprise a heavy chain CDR1 sequence illustrated in Figure 7. The antigen binding protein may comprise a heavy chain CDR2 sequence illustrated in Figure 8. The antigen binding protein may comprise a heavy chain CDR3 sequence illustrated in Figure 9. The antigen binding protein may comprise a light chain FR1 sequence illustrated in Figure 2. The antigen binding protein may comprise a light chain FR2 sequence illustrated in Figure 2. The antigen binding protein may comprise a light chain FR3 sequence illustrated in Figure 2. The antigen binding protein may comprise a light chain FR4 sequence illustrated in Figure 2. The antigen binding protein may comprise a heavy chain FR1 sequence illustrated in Figure 3. The antigen binding protein may comprise a heavy chain FR2 sequence illustrated in Figure 3. The antigen binding protein may comprise a heavy chain FR3 sequence illustrated in Figure 3. The antigen binding protein may comprise a heavy chain FR4 sequence illustrated in Figure 3.

Herein described is an antigen binding protein that comprises one or more CDR sequences that differ from a CDR sequence shown in Figures 2 through 9 by no more than 5, 4, 3, 2, or 1 amino acid residues.

Also herein described is an antigen binding protein that comprises at least one CDR from L1-L52 and/or H1-H52, as shown in Figures 2 through 9, and at least one CDR sequence from an anti-IGF-1R antibody described in US Pat. App. Pub. Nos. 03/0235582, 04/0228859, 04/0265307, 04/0886503, 05/0008642, 05/0084906, 05/0186203, 05/0244408, PCT Pub. Nos. WO 03/059951, WO 03/100008, WO 04/071529A2, WO 04/083248, WO 04/087756, WO 05/016967, WO 05/016970, or WO 05/058967 wherein the antigen binding protein binds to IGF-1 receptor. The antigen binding protein may comprise 2, 3, 4, or 5 CDR sequences from L1-L52 and/or H1-H52, as shown in Figures 2 through 9. The antigen binding protein may comprise 2, 3, 4, or 5 CDR sequences from an anti-IGF-1R antibody described in US Pat. App. Pub. Nos. 03/0235582, 04/0228859, 04/0265307, 04/0886503, 05/0008642, 05/0084906, 05/0186203, 05/0244408, PCT Pub. Nos. WO 03/059951, WO 03/100008, WO 04/071529A2, WO 04/083248, WO 04/087756, WO 05/016967, WO 05/016970, or WO 05/058967. At least one of the antigen binding protein's CDR3 sequences may be a CDR3 sequence from L1-L52 and/or H1-H52, as shown in Figures 2, 3, 6 and 9. The antigen binding protein's light chain CDR3 sequence may be a light chain CDR3 sequence from L1-L52 as shown in Figures 2 and 6 and the antigen binding protein's heavy chain CDR3 sequence is a heavy chain sequence from H1-H52 as shown in Figures 3 and 9. The antigen binding protein may comprise 1, 2, 3, 4, or 5 CDR sequences that each independently differs by 6, 5, 4, 3, 2, 1, or 0 single amino acid additions, substitutions, and/or deletions from a CDR sequence of L1-L52 and/or H1-H52, and the antigen binding protein further comprises 1, 2, 3, 4, or 5 CDR sequences that each independently differs by 6, 5, 4, 3, 2, 1, or 0 single amino acid additions, substitutions, and/or deletions from a CDR sequence of US Pat. App. Pub. Nos. 03/0235582, 04/0228859, 04/0265307, 04/0886503, 05/0008642, 05/0084906, 05/0186203, 05/0244408, PCT Pub. Nos. WO 03/059951, WO 03/100008, WO 04/071529A2, WO 04/083248, WO 04/087756, WO 05/016967, WO 05/016970, or WO 05/058967. The CDR sequence(s) may be from US Pat. App. Pub. Nos. 03/0235582, 04/0228859, 04/0265307, 04/0886503, 05/0008642_{;} 05/0084906, 05/0186203, 05/0244408, PCT Pub. Nos. WO 03/059951, WO 03/100008, WO 04/071529A2, WO 04/083248, WO 04/087756, WO 05/016967, WO 05/016970, or WO 05/058967. The CDR sequence(s) may be from (an) antibody(-ies) that bind(s) to the L2 portion of the extracellular domain of IGF-1 receptor. The antigen binding protein may not comprise a light chain CDR3 sequence and/or a heavy chain CDR3 sequence from an anti-IGF-1R antibody from US Pat. App. Pub. Nos. 03/0235582, 04/0228859, 04/0265307, 04/0886503, 05/0008642, 05/0084906, 05/0186203, 05/0244408, PCT Pub. Nos. WO 03/059951, WO 03/100008, WO 04/071529A2, WO 04/083248, WO 04/087756, WO 05/016967, WO 05/016970,or. WO 05/058967.

Herein described is an antigen binding protein that comprises a light chain CDR1 comprising the sequence RSSQSLLHX₁X₂GYNX₃LX₄ (SEQ ID NO:236), wherein X₁ is a serine or a threonine residue, X₂ is an asparagine, serine, or histidine residue, X₃ is a tyrosine or a phenylalanine residue, and X₄ is an aspartate or an asparagine residue. The light chain CDR1 may comprise the sequence TRSSGX_{I}IX₂X₃NYVQ (SEQ ID NO:237), wherein X₁ is a serine or an aspartate residue, X₂ is an alanine or an aspartate residue, and X₃ is a serine or an asparagine residue. The light chain CDR1 may comprise the sequence RASQX₁X₂X₃X₄X₅LX₆ (SEQ ID NO:238), wherein X₁ is a glycine or a serine residue, X₂ is an isoleucine, valine, or proline residue, and X₃ is a serine, glycine, or tyrosine residue, X₄ is any amino acid residue, X₅ is a phenylalanine, tyrosine, asparagine, or tryptophan residue, and X₆ is an alanine or an asparagine residue. X₂ may be an isoleucine or valine residue, X₃ a glycine or serine residue, X₄ an arginine, serine, asparagine, serine, tyrosine, or isoleucine residue, and X₅ a phenylalanine or a tyrosine residue.

Described herein is an antigen binding protein that comprises a light chain CDR2 comprising the sequence LX₁X₂X₃RX₄S (SEQ ID NO:239), wherein X₁ is a glycine or a valine residue, X₂ is a serine or a phenylalanine residue, X₃ is an asparagine, tyrosine, or threonine residue, and X₄ is an alanine or an aspartate residue. The CDR2 may comprise the sequence AX₁SX₂LX₃S (SEQ ID NO:240), wherein X₁ is an alanine or a threonine residue, X₂ is a threonine or a glycine residue, and X₃ is a glutamine or a glutamate residue. The CDR2 may comprise the sequence X₁X₂NX₃RPS (SEQ ID NO:241), wherein X₁ is a glutamate, glutamine, or glycine residue, X₂ is an aspartate or lysine residue, and X₃ is any amino acid residue.

Herein described is an antigen binding protein that comprises a light chain CDR3 comprising the sequence MX₁X₂X₃X₄X₅PX₆X₇ (SEQ ID NO:242), wherein X₁ is a glutamine or glutamate residue, X₂ is an alanine, glycine, serine, or threonine residue, X₃ is a leucine or threonine residue, X₄ is a glutamine, glutamate, or histidine residue, X₅ is a threonine, tryptophan, methionine, or valine residue, X₆ is a nonpolar side chain residue, and X₇ is a threonine, serine, or alanine residue. The CDR3 may comprise the sequence QQX₁X₂X₃X₄PXₛT (SEQ ID NO:243), wherein X₁ is an arginine, serine, leucine, or alanine residue, X₂ is an asparagine, serine, or histidine residue, X₃ is a serine or an asparagine residue, X₄ is a nonpolar side chain residue, and X₅ is a leucine, isoleucine, tyrosine, or tryptophan residue. The CDR3 may comprise the sequence QSYX₁SX₂NX₃X₄V (SEQ ID NO:244), wherein X₁ is an aspartate or a glutamine residue, X₂ is a serine or an aspartate residue, X₃ is a glutamine, valine, or tryptophan residue, and X₄ is an arginine residue or no residue.

Herein described is an antigen binding protein that comprises a heavy chain CDR1 comprising the sequence X₁X₂X₃WWS (SEQ ID NO:245), wherein X₁ is a serine residue or no residue, X₂ is a serine or asparagine residue, and X₃ is an asparagine residue and an isoleucine residue. The heavy chain CDR1 may comprise the sequence X₁X₂YWS (SEQ ID NO:246), wherein X₁ is a glycine, asparagine, or aspartate residue, and X₂ is a tyrosine or phenylalanine residue. The heavy chain CDR1 may comprise the sequence SYX₁X₂X₃ (SEQ ID NO:247), wherein X₁ is an alanine or glycine residue, X₂ is a methionine or isoleucine residue, and X₃ is a serine or histidine residue.

Also herein described is an antigen binding protein that comprises a heavy chain CDR2 comprising the sequence X₁X₂X₃X₄X₅GX₆TX₇YNPSLX₈S (SEQ ID NO:248), wherein X₁ is a glutamate, tyrosine, or serine residue, X₂ is a isoleucine or valine residue, X₃ is a tyrosine, asparagine, or serine residue, X₄ is a histidine, tyrosine, aspartate, or proline residue, X₅ is a serine or arginine residue, X₆ is a serine or asparagine residue, X₇ is an asparagine or tyrosine residue, and X₈ is a lysine or glutamate residue. The heavy chain CDR2 may comprise the sequence X₁ISX₂X₃X₄X₅X₆X₇YYADSVKG (SEQ ID NO:249), wherein X₁ is a threonine, alanine, valine, or tyrosine residue, X₂ is a glycine, serine, or tyrosine residue, X₃ is a serine, asparagine, or aspartate residue, X₄ is a glycine or serine residue, X₅ is a glycine, serine, or aspartate residue, X₆ is a serine, threonine, or asparagine residue, and X₇ is a threonine, lysine, or isoleucine residue.

Herein described is an antigen binding protein that comprises a heavy chain CDR3 comprising the sequence X₁X₂X₃X₄X₅X₆X₇X₈X₉FDI (SEQ ID NO:250), wherein X₁ is a glutamate residue or no residue, X₂ is tyrosine, glycine, or serine residue or no residue, X₃ is a serine, asparagine, tryptophan, or glutamate residue, or no residue, X₄ is a serine, aspartate, tryptophan, alanine, arginine, threonine, glutamine, leucine, or glutamate residue, or no residue, X₅ is a serine, glycine, asparagine, threonine, tryptophan, alanine, valine, or isoleucine residue, X₆ is an arginine, glutamine, tyrosine, valine, alanine, glycine, serine, phenylalanine, or tryptophan residue, X₇ is a leucine, asparagine, aspartate, threonine, tryptophan, tyrosine, valine, alanine, or histidine residue, X₈ is an aspartate, serine, asparagine, or glutamine residue, and X₉ is an alanine or a proline residue. The heavy chain CDR3 may comprise the sequence X₁X₂X₃X₄X₅X₆X₇X₈X₉X₁₀X₁₁MDV (SEQ ID NO:251), wherein X₁ is an alanine residue, or no residue, X₂ is a glutamate, tyrosine, or glycine residue, or no residue, X₃ is a serine or arginine residue, or no residue, X₄ is an aspartate, glycine, serine, or valine residue, or no residue, X₅ is a serine, glycine, or aspartate residue, or no residue, X₆ is a glycine, phenylalanine, aspartate, serine, tryptophan, or tyrosine residue, or no residue, X₇ is a tyrosine, tryptophan, serine, or aspartate residue, or no residue, X₈ is an aspartate, arginine, serine, glycine, tyrosine, or tryptophan residue, X₉ is a tyrosine, isoleucine, leucine, phenylalanine, or lysine residue, X₁₀ is a tyrosine, phenylalanine, aspartate, or glycine residue, and X₁₁ is a glycine, tyrosine, or asparagine residue. The heavy chain CDR3 may comprise the sequence X₁X₂X₃X₄X₅X₆X₇X₈X₉X₁₀Y (SEQ ID NO:252), wherein X₁ is an aspartate or valine residue, or no residue, X₂ is a glycine, tyrosine, arginine, or aspartate residue, or no residue, X₃ is an asparagine, leucine, glycine, isoleucine, serine, valine, phenylalanine, or tyrosine residue, or no residue, X₄ is a leucine, serine, tryptophan, alanine, tyrosine, isoleucine, glycine, or aspartate residue, or no residue, X₅ is a glycine, alanine, tyrosine, serine, aspartate, or leucine residue, X₆ is a valine, alanine, glycine, threonine, proline, histidine, or glutamine residue, X₇ is a glutamate, glycine, serine, aspartate, glycine, valine, tryptophan, histidine, or arginine residue, X₈ is a glutamine, alanine, glycine, tyrosine, proline, leucine, aspartate, or serine residue, X₉ is a nonpolar side chain residue, and X₁₀ is an aspartate or alanine residue. The heavy chain CDR3 may comprise the sequence X₁X₂X₃X₄X₅X₆X₇X₈X₉X₁₀YFDX₁₁ (SEQ ID NO:253), wherein X₁ is a glycine residue, or no residue, X₂ is a proline residue, or no residue, X₃ is an arginine or aspartate residue, or no residue, X₄ is a histidine or proline residue, X₅ is an arginine or glycine residue, X₆ is an arginine, serine, or phenylalanine residue, X₇ is an aspartate or serine residue, X₈ is a glycine, tryptophan, or tyrosine residue, X₉ is a tyrosine or alanine residue, X₁₀ is an asparagine or tryptophan residue, and X₁₁ is an asparagine or leucine residue. The heavy chain CDR3 may comprise the sequence X₁X₂X₃X₄DSSX₅X₆X₇XgX₉X₁₀X₁₁X₁₂ (SEQ ID NO:254), wherein X₁ is a phenylalanine residue, or no residue, X₂ is an asparagine or glycine residue, or no residue, X₃ is a tyrosine or a leucine residue, or no residue, X₄ is a tyrosine or glycine residue, or no residue, X₅ is a glycine, serine, or valine residue, X₆ is a tyrosine, phenylalanine, tryptophan, or glutamine residue, or no residue, X₇ is a tyrosine, glycine, or isoleucine residue, or no residue, X₈ is a tyrosine, leucine, or glycine residue, or no residue, X₉ is a methionine, glycine, or phenylalanine residue, or no residue, X₁₀ is an aspartate or methionine residue, or no residue, X₁₁ is a valine, aspartate, or tyrosine residue, or no residue, and X₁₂ is a valine residue, or no residue.

Also described herein is an isolated antigen binding protein, comprising either: a. a light chain CDR3 comprising a sequence selected from the group consisting of: i. a light chain CDR3 sequence selected from the group consisting of the light chain CDR3 sequences of L1-L52 as shown in Figure 6; ii. MQALQTPZT; iii. QQ(R/S)(N/S)(S/N)ZPLT; and iv. QSYDSSNXJV; b. a heavy chain CDR3 comprising a sequence selected from the group consisting of: i. a heavy chain CDR3 sequence that differs by no more than a total of three amino acid additions, substitutions, or deletions from a CDR3 sequence selected from the group consisting of the heavy chain CDR3 sequences of H1-H52 as shown in Figure 9; ii. SRLDAFDI; iii. SXYDYYGMDV; iv. HRXDXAWYFDL; and v. DSSG; or c. the light chain CDR3 sequence of (a) and the heavy chain CDR3 sequence of (b); wherein amino acid residue symbols enclosed in parentheses identify alternative residues for the same position in a sequence, each X is independently any amino acid residue, each Z is independently a glycine residue, an alanine residue, a valine residue, a leucine residue, an isoleucine residue, a proline residue, a phenylalanine residue, a methionine residue, a tryptophan residue, or a cysteine residue, each J is independently a glutamine residue, an arginine residue, a valine residue, or a tryptophan residue, and the antigen binding protein binds to human IGF-1R.

The nucleotide sequences of Figure 1, or the amino acid sequences of Figures 2 through 9, can be altered, for example, by random mutagenesis or by site-directed mutagenesis (*e.g.,* oligonucleotide-directed site-specific mutagenesis) to create an altered polynucleotide comprising one or more particular nucleotide substitutions, deletions, or insertions as compared to the non-mutated polynucleotide. Examples of techniques for making such alterations are described in Walder et al., 1986, Gene 42:133; Bauer et al. 1985, Gene 37:73; Craik, BioTechniques, January 1985, 12-19; Smith et al., 1981, Genetic Engineering: Principles and Methods, Plenum Press; and U.S. Patent Nos. 4,518,584 and 4,737,462. These and other methods can be used to make, for example, derivatives of anti-IGF-1R antibodies that have a desired property, for example, increased affinity, avidity, or specificity for IGF-1R, increased activity or stability *in vivo* or *in vitro,* or reduced *in vivo* side-effects as compared to the underivatized antibody.

Other derivatives of anti- IGF-1R antibodies include covalent or aggregative conjugates of anti-IGF-1R antibodies, or fragments thereof, with other proteins or polypeptides, such as by expression of recombinant fusion proteins comprising heterologous polypeptides fused to the N-terminus or C-terminus of an anti- IGF-1R antibody polypeptide. For example, the conjugated peptide may be a heterologous signal (or leader) polypeptide, *e.g.,* the yeast alpha-factor leader, or a peptide such as an epitope tag. Antigen binding protein-containing fusion proteins can comprise peptides added to facilitate purification or identification of antigen binding protein (*e.g.*, poly-His). An antigen binding protein also can be linked to the FLAG peptide Asp-Tyr-Lys-Asp-Asp-Asp-Asp-Lys (DYKDDDDK) (SEQ ID NO:255) as described in Hopp et al., Bio/Technology 6:1204, 1988, and U.S. Patent 5,011,912. The FLAG peptide is highly antigenic and provides an epitope reversibly bound by a specific monoclonal antibody (mAb), enabling rapid assay and facile purification of expressed recombinant protein. Reagents useful for preparing fusion proteins in which the FLAG peptide is fused to a given polypeptide are commercially available (Sigma, St. Louis, MO).

Oligomers that contain one or more antigen binding proteins may be employed as IGF-1R antagonists. Oligomers may be in the form of covalently-linked or non-covalently-linked dimers, trimers, or higher oligomers. Oligomers comprising two or more antigen binding protein are contemplated for use, with one example being a homodimer. Other oligomers include heterodimers, homotrimers, heterotrimers, homotetramers, heterotetramers, *etc.*

Oligomers may comprise multiple antigen binding proteins joined *via* covalent or non-covalent interactions between peptide moieties fused to the antigen binding proteins. Such peptides may be peptide linkers (spacers), or peptides that have the property of promoting oligomerization. Leucine zippers and certain polypeptides derived from antibodies are among the peptides that can promote oligomerization of antigen binding proteins attached thereto, as described in more detail below.

The oligomers may compris from two to four antigen binding proteins. The antigen binding proteins of the oligomer may be in any form, such as any of the forms described above, e.g., variants or fragments. The oligomers may comprise antigen binding proteins that have IGF-1R binding activity.

An oligomer may be prepared using polypeptides derived from immunoglobulins. Preparation of fusion proteins comprising certain heterologous polypeptides fused to various portions of antibody-derived polypeptides (including the Fc domain) has been described, *e.g.,* by Ashkenazi et al., 1991, PNAS USA 88:10535; Byrn et al., 1990, Nature 344:677; and Hollenbaugh et al., 1992 "Construction of Immunoglobulin Fusion Proteins", in Current Protocols in Immunology, Suppl. 4, pages 10.19.1 - 10.19.11.

Herein described is a dimer comprising two fusion proteins created by fusing an IGF-1R binding fragment of an anti- IGF-1R antibody to the Fc region of an antibody. The dimer can be made by, for example, inserting a gene fusion encoding the fusion protein into an appropriate expression vector, expressing the gene fusion in host cells transformed with the recombinant expression vector, and allowing the expressed fusion protein to assemble much like antibody molecules, whereupon interchain disulfide bonds form between the Fc moieties to yield the dimer.

The term "Fc polypeptide" as used herein includes native and mutein forms of polypeptides derived from the Fc region of an antibody. Truncated forms of such polypeptides containing the hinge region that promotes dimerization also are included. Fusion proteins comprising Fc moieties (and oligomers formed therefrom) offer the advantage of facile purification by affinity chromatography over Protein A or Protein G columns.

One suitable Fc polypeptide, described in PCT application WO 93/10151 is a single chain polypeptide extending from the N-terminal hinge region to the native C-terminus of the Fc region of a human IgG1 antibody. Another useful Fc polypeptide is the Fc mutein described in U.S. Patent 5,457,035 and in Baum et al., 1994, EMBO J. 13:3992-4001. The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors.

The variable portion of the heavy and/or light chains of an anti-IGF-1R antibody may be substituted for the variable portion of an antibody heavy and/or light chain.

Alternatively, the oligomer is a fusion protein comprising multiple antigen binding proteins, with or without peptide linkers (spacer peptides). Among the suitable peptide linkers are those described in U.S. Patents 4,751,180 and 4,935,233.

Another method for preparing oligomeric antigen binding proteins involves use of a leucine zipper. Leucine zipper domains are peptides that promote oligomerization of the proteins in which they are found. Leucine zippers were originally identified in several DNA-binding proteins (Landschulz et al., 1988, Science 240:1759), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al, 1994, FEBS Letters 344:191. The use of a modified leucine zipper that allows for stable trimerization of a heterologous protein fused thereto is described in Fanslow et al., 1994, Semin. Immunol. 6:267-78. In one approach, recombinant fusion proteins comprising an anti- IGF-1R antibody fragment or derivative fused to a leucine zipper peptide are expressed in suitable host cells, and the soluble oligomeric anti- IGF-1R antibody fragments or derivatives that form are recovered from the culture supernatant.

Herein described are antigen binding proteins that interfere with the binding of IGF-1 and/or IGF-2 to an IGF-1R. Such antigen binding proteins can be made against IGF-1R, or a fragment, variant or derivative thereof, and screened in conventional assays for the ability to interfere with binding of IGF-1 and/or IGF-2 to IGF-1R. Examples of suitable assays are assays that test the antigen binding proteins for the ability to inhibit binding of IGF-1 and/or IGF-2 to cells expressing IGF-1R, or that test antigen binding proteins for the ability to reduce a biological or cellular response that results from the binding of IGF-1 and/or IGF-2 to cell surface IGF-1R receptors.

Also herein described is an antigen binding protein that blocks the binding of IGF-1 and/or IGF-2 to IGF-1R but does not significantly block the binding of insulin to insulin receptor (INS-R). The antigen binding protein may not bind to INS-R. The antigen binding protein may bind to the INS-R with such a low affinity that it does not effectively block the binding of insulin to INS-R. The antigen binding protein may bind to INS-R, but antigen binding protein-bound INS-R may still bind to insulin. The antigen binding protein's selectivity for IGF-1R may be at least 50 times greater than its selectivity for insulin receptor. The selectivity of the antigen binding protein may be more than 100 times greater than its selectivity for insulin receptor.

Herein describeed is an antigen binding protein that demonstrates species selectivity. The antigen binding protein may bind to one or more mammalian IGF-1R, for example, to human IGF-1R and one or more of mouse, rat, guinea pig, hamster, gerbil, cat, rabbit, dog, goat, sheep, cow, horse, camel, and non-human primate IGF-1R. The antigen binding protein may bind to one or more primate IGF-1R, for example, to human IGF-1R and one or more of cynomologous, marmoset, rhesus, and chimpanzee IGF-1R. The antigen binding protein may bind specifically to human, cynomologous, marmoset, rhesus, or chimpanzee IGF-1R. The antigen binding protein may not bind to one or more of mouse, rat, guinea pig, hamster, gerbil, cat, rabbit, dog, goat, sheep, cow, horse, camel, and non-human primate IGF-1R. The antigen binding protein may not bind to a New World monkey species such as a marmoset. The antigen binding protein may not exhibit specific binding to any naturally occurring protein other than IGF-1R. The antigen binding protein may not exhibit specific binding to any naturally occurring protein other than mammalian IGF-1R. The antigen binding protein may not exhibit specific binding to any naturally occurring protein other than primate IGF-1R. The antigen binding protein may not exhibit specific binding to any naturally occurring protein other than human IGF-1R. The antigen binding protein specifically may bind to mouse, rat, cynomolgus monkey, and human IGF-1R. The antigen binding protein specifically may bind to mouse, rat, cynomolgus monkey, and human IGF-1R with a similar binding affinity. In antigen binding protein may block binding of human IGF-1 and IGF-2 with mouse, rat, cynomolgus monkey, and human IGF-1R. The antigen binding protein may block binding of human IGF-1 and IGF-2 with mouse, rat, cynomolgus monkey, and human IGF-1R with similar Kᵢ. The antigen binding protein may block binding of human IGF-1 and IGF-2 with mouse, rat, cynomolgus monkey, and human IGF-1R with a Kᵢ of between about 0.57 and about 0.61 nM.

One may determine the selectivity of an antigen binding protein for an IGF-1R using methods well known in the art and following the teachings of the specification. For example, one may determine the selectivity using Western blot, FACS, ELISA or RIA.

Herein described is an IGF-1R binding antigen binding protein (for example, an anti-IGF-1R antibody), that has one or more of the following characteristics: binds to both human and murine IGF-1R, inhibits the binding of both IGF-1 and IGF-2 to human IGF-1R, inhibits the binding of both IGF-1 and IGF-2 to murine IGF-1R, preferentially inhibits the high affinity binding of IGF-1 and/or of IGF-2 to IGF-1R, binds to the L2 domain of IGF-1R, causes relatively little down-regulation of cell-surface expressed IGF-1R after 17 hours of exposure (as compared to MAB391 (R&D systems, Minneapolis, MN); e.g., amount of IGF-1R is reduced by less than 20%), causes a level of down-regulation of cell-surface expressed IGF-1R on Colo-205 or MiaPaCa-2 xenograft tumor cells in mice as MAB391 after four weeks of once weekly doses of 200 micrograms.

Antigen-binding fragments of antigen binding proteins may be produced by conventional techniques. Examples of such fragments include, but are not limited to, Fab and F(ab')₂ fragments. Antibody fragments and derivatives produced by genetic engineering techniques also are herein described.

Also herein described are chimeric antibodies, *e.g.,* humanized versions of non-human (*e.g.*, murine) monoclonal antibodies. Such humanized antibodies may be prepared by known techniques, and offer the advantage of reduced immunogenicity when the antibodies are administered to humans. A humanized monoclonal antibody may comprise the variable domain of a murine antibody (or all or part of the antigen binding site thereof) and a constant domain derived from a human antibody. Alternatively, a humanized antibody fragment may comprise the antigen binding site of a murine monoclonal antibody and a variable domain fragment (lacking the antigen-binding site) derived from a human antibody. Procedures for the production of chimeric and further engineered monoclonal antibodies include those described in Riechmann et al., 1988, Nature 332:323, Liu et al., 1987, Proc. Nat. Acad. Sci. USA 84:3439, Larrick et al., 1989, Bio/Technology 7:934, and Winter et al., 1993, TIPS 14:139. The chimeric antibody is a CDR grafted antibody. Techniques for humanizing antibodies are discussed in, *e.g.,* U.S. Pat. App. No. 10/194,975 (published February 27, 2003), U.S. Pat. No.s 5,869,619, 5,225,539, 5,821,337, 5,859,205, Padlan et al., 1995, FASEB J. 9:133-39, and Tamura et al., 2000, J. Immunol. 164:1432-41.

Procedures have been developed for generating human or partially human antibodies in non-human animals. For example, mice in which one or more endogenous immunoglobulin genes have been inactivated by various means have been prepared. Human immunoglobulin genes have been introduced into the mice to replace the inactivated mouse genes. Antibodies produced in the animal incorporate human immunoglobulin polypeptide chains encoded by the human genetic material introduced into the animal. A non-human animal, such as a transgenic mouse, may be immunized with an IGF-1R polypeptide, such that antibodies directed against the IGF-1R polypeptide are generated in the animal. One example of a suitable immunogen is a soluble human IGF-1R, such as a polypeptide comprising the extracellular domain of the protein of Figure 10, or other immunogenic fragment of the protein of Figure 10. Examples of techniques for production and use of transgenic animals for the production of human or partially human antibodies are described in U.S. Patents 5,814,318, 5,569,825, and 5,545,806, Davis et al., 2003, Production of human antibodies from transgenic mice in Lo, ed. Antibody Engineering: Methods and Protocols, Humana Press, NJ:191-200, Kellermann et al., 2002, Curr Opin Biotechnol. 13:593-97, Russel et al., 2000, Infect Immun. 68:1820-26, Gallo et al., 2000, Eur J Immun. 30:534-40, Davis et al., 1999, Cancer Metastasis Rev. 18:421-25, Green, 1999, J Immunol Methods. 231:11-23, Jakobovits, 1998, Advanced Drug Delivery Reviews 31:33-42, Green et al., 1998, J Exp Med. 188:483-95, Jakobovits A, 1998, Exp. Opin. Invest. Drugs. 7:607-14, Tsuda et al., 1997, Genomics. 42:413-21, Mendez et al., 1997, Nat Genet. 15:146-56, Jakobovits, 1994, Curr Biol. 4:761-63, Arbones et al., 1994, Immunity. 1:247-60, Green et al., 1994, Nat Genet. 7:13-21, Jakobovits et al., 1993, Nature. 362:255-58, Jakobovits et al., 1993, Proc Natl Acad Sci U S A. 90:2551-55. Chen, J., M. Trounstine, F. W. Alt, F. Young, C. Kurahara, J. Loring, D. Huszar. "Immunoglobulin gene rearrangement in B cell deficient mice generated by targeted deletion of the JH locus." International Immunology 5 (1993): 647-656, Choi et al., 1993, Nature Genetics 4: 117-23, Fishwild et al., 1996, Nature Biotechnology 14: 845-51, Harding et al., 1995, Annals of the New York Academy of Sciences, Lonberg et al., 1994, Nature 368: 856-59, Lonberg, 1994, Transgenic Approaches to Human Monoclonal Antibodies in Handbook of Experimental Pharmacology 113: 49-101, Lonberg et al., 1995, Internal Review of Immunology 13: 65-93, Neuberger, 1996, Nature Biotechnology 14: 826, Taylor et al., 1992, Nucleic Acids Research 20: 6287-95, Taylor et al., 1994, International Immunology 6: 579-91, Tomizuka et al., 1997, Nature Genetics 16: 133-43, Tomizuka et al., 2000, Proceedings of the National Academy of Sciences USA 97: 722-27, Tuaillon et al., 1993, Proceedings of the National Academy of Sciences USA 90: 3720-24, and Tuaillon. et al., 1994, Journal of Immunology 152: 2912-20.

Herein described are monoclonal antibodies that bind to IGF-1R. Monoclonal antibodies may be produced using any technique known in the art, *e.g.,* by immortalizing spleen cells harvested from the transgenic animal after completion of the immunization schedule. The spleen cells can be immortalized using any technique known in the art, *e.g.,* by fusing them with myeloma cells to produce hybridomas. Myeloma cells for use in hybridoma-producing fusion procedures preferably are non-antibody-producing, have high fusion efficiency, and enzyme deficiencies that render them incapable of growing in certain selective media which support the growth of only the desired fused cells (hybridomas). Examples of suitable cell lines for use in mouse fusions include Sp-20, P3-X63/Ag8, P3-X63-Ag8.653, NS1/1.Ag 4 1, Sp210-Ag14, FO, NSO/U, MPC-11, MPC11-X45-GTG 1.7 and S194/5XX0 Bul; examples of cell lines used in rat fusions include R210.RCY3, Y3-Ag 1.2.3, IR983F and 4B210. Other cell lines useful for cell fusions are U-266, GM1500-GRG2, LICR-LON-HMy2 and UC729-6.

A hybridoma cell line may be produced by immunizing an animal (*e.g.*, a transgenic animal having human immunoglobulin sequences) with an IGF-1R immunogen; harvesting spleen cells from the immunized animal; fusing the harvested spleen cells to a myeloma cell line, thereby generating hybridoma cells; establishing hybridoma cell lines from the hybridoma cells, and identifying a hybridoma cell line that produces an antibody that binds an IGF-1R polypeptide.

Monoclonal antibodies secreted by a hybridoma cell line can be purified using any technique known in the art. Hybridomas or mAbs may be further screened to identify mAbs with particular properties, such as the ability to block an IGF-1 and/or IGF-2 induced activity. Examples of such screens are provided in the examples below.

Molecular evolution of the complementarity determining regions (CDRs) in the center of the antibody binding site also has been used to isolate antibodies with increased affinity, for example, antibodies having increased affinity for c-erbB-2, as described by Schier et al., 1996, J. Mol. Biol. 263:551. Accordingly, such techniques are useful in preparing antibodies to IGF-1R.

Antigen binding proteins directed against an IGF-1R can be used, for example, in assays to detect the presence of IGF-1R polypeptides, either *in vitro* or *in vivo.* The antigen binding proteins also may be employed in purifying IGF-1R proteins by immunoaffinity chromatography. Those antigen binding proteins that additionally can block binding of IGF-1 and/or IGF-2 to IGF-1R may be used to inhibit a biological activity that results from such binding. Blocking antigen binding proteins can be used in the methods described herein.

Such antigen binding proteins that function as IGF-1 and/or IGF-2 antagonists may be employed in treating any IGF-1 and/or IGF-2-induced condition, including but not limited to cancer. A human anti- IGF-1R monoclonal antibody generated by procedures involving immunization of transgenic mice may be employed in treating such conditions.

Antigen binding proteins may be employed in an *in vitro* procedure, or administered *in vivo* to inhibit an IGF-1and/or IGF-2-induced biological activity. Disorders caused or exacerbated (directly or indirectly) by the interaction of IGF-1and/or IGF-2 with cell surface IGF-1R, examples of which are described above, thus may be treated. Herein described is a therapeutic method comprising *in vivo* administration of an IGF-1 and/or IGF-2 blocking antigen binding protein to a mammal in need thereof in an amount effective for reducing an IGF-1 and/or IGF-2-induced biological activity.

Antigen binding proteins include partially human and fully human monoclonal antibodies that inhibit a biological activity of IGF-1and also inhibit a biological activity of IGF-2. A human monoclonal antibody may at least partially block binding of IGF-1 and of IGF-2 to a cell that expresses human IGF-1R. The antibodies may be generated by immunizing a transgenic mouse with an IGF-1R immunogen. The immunogen may be a human IGF-1R polypeptide (e.g., a soluble fragment comprising all or part of the IGF-1R extracellular domain). Hybridoma cell lines derived from such immunized mice, wherein the hybridoma secretes a monoclonal antibody that binds IGF-1R, also are described herein.

Although human, partially human, or humanized antibodies will be suitable for many applications, particularly those involving administration of the antibody to a human subject, other types of antigen binding proteins will be suitable for certain applications. The non-human antibodies described herein can be, for example, derived from any antibody-producing animal, such as mouse, rat, rabbit, goat, donkey, or non-human primate (such as monkey (*e.g.,* cynomologous or rhesus monkey) or ape (*e.g.,* chimpanzee)). Non-human antibodies can be used, for example, in *in vitro* and cell-culture based applications, or any other application where an immune response to the antibody does not occur, is insignificant, can be prevented, is not a concern, or is desired. A non-human antibody may be administered to a non-human subject. The non-human antibody may not elicit an immune response in the non-human subject. The non-human antibody may be from the same species as the non-human subject, e.g., a mouse antibody may be is administered to a mouse. An antibody from a particular species can be made by, for example, immunizing an animal of that species with the desired immunogen (e.g., a soluble IGF-1R polypeptide) or using an artificial system for generating antibodies of that species *(e.g.,* a bacterial or phage display-based system for generating antibodies of a particular species), or by converting an antibody from one species into an antibody from another species by replacing, e.g., the constant region of the antibody with a constant region from the other species, or by replacing one or more amino acid residues of the antibody so that it more closely resembles the sequence of an antibody from the other species. The antibody may be a chimeric antibody comprising amino acid sequences derived from antibodies from two or more different species.

Antigen binding proteins may be prepared by any of a number of conventional techniques. For example, they may be purified from cells that naturally express them (e.g., an antibody can be purified from a hybridoma that produces it), or produced in recombinant expression systems, using any technique known in the art. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, NY, (1988).

Any expression system known in the art can be used to make the recombinant polypeptides. In general, host cells are transformed with a recombinant expression vector that comprises DNA encoding a desired polypeptide. Among the host cells that may be employed are prokaryotes, yeast or higher eukaryotic cells. Prokaryotes include gram negative or gram positive organisms, for example *E. coli* or bacilli. Higher eukaryotic cells include insect cells and established cell lines of mammalian origin. Examples of suitable mammalian host cell lines include the COS-7 line of monkey kidney cells (ATCC CRL 1651) (Gluzman et al., 1981, Cell 23:175), L cells, 293 cells, C127 cells, 3T3 cells (ATCC CCL 163), Chinese hamster ovary (CHO) cells, HeLa cells, BFFK (ATCC CRL 10) cell lines, and the CVI/EBNA cell line derived from the African green monkey kidney cell line CVI (ATCC CCL 70) as described by McMahan et al., 1991, EMBO J. 10: 2821. Appropriate cloning and expression vectors for use with bacterial, fungal, yeast, and mammalian cellular hosts are described by Pouwels et al. (Cloning Vectors: A Laboratory Manual, Elsevier, New York, 1985).

The transformed cells can be cultured under conditions that promote expression of the polypeptide, and the polypeptide recovered by conventional protein purification procedures. One such purification procedure includes the use of affinity chromatography, e.g., over a matrix having all or a portion (*e.g.*, the extracellular domain) of IGF-1R bound thereto. Polypeptides contemplated for use herein include substantially homogeneous recombinant mammalian anti- IGF-1R antibody polypeptides substantially free of contaminating endogenous materials.

Antigen binding proteins may be prepared, and screened for desired properties, by any of a number of known techniques. Certain of the techniques involve isolating a nucleic acid encoding a polypeptide chain (or portion thereof) of an antigen binding protein of interest (e.g., an anti-IGF-1R antibody), and manipulating the nucleic acid through recombinant DNA technology. The nucleic acid may be fused to another nucleic acid of interest, or altered (*e.g.*, by mutagenesis or other conventional techniques) to add, delete, or substitute one or more amino acid residues, for example.

Described herein are antigen-binding fragments of an anti-IGF-1R antibody. Such fragments can consist entirely of antibody-derived sequences or can comprise additional sequences. Examples of antigen-binding fragments include Fab, F(ab')2, single chain antibodies, diabodies, triabodies, tetrabodies, and domain antibodies. Other examples are provided in Lunde et al., 2002, Biochem. Soc. Trans. 30:500-06.

Single chain antibodies may be formed by linking heavy and light chain variable domain (Fv region) fragments via an amino acid bridge (short peptide linker), resulting in a single polypeptide chain. Such single-chain Fvs (scFvs) have been prepared by fusing DNA encoding a peptide linker between DNAs encoding the two variable domain polypeptides (V_{L} and V_{H}). The resulting polypeptides can fold back on themselves to form antigen-binding monomers, or they can form multimers (*e.g.*, dimers, trimers, or tetramers), depending on the length of a flexible linker between the two variable domains (Kortt et al., 1997, Prot. Eng. 10:423; Kortt et al., 2001, Biomol. Eng. 18:95-108). By combining different V_{L} and V_{H}-comprising polypeptides, one can form multimeric scFvs that bind to different epitopes (Kriangkum et al., 2001, Biomol. Eng. 18:31-40). Techniques developed for the production of single chain antibodies include those described in U.S. Patent No. 4,946,778; Bird, 1988, Science 242:423; Huston et al., 1988, Proc. Natl. Acad. Sci. USA 85:5879; Ward et al., 1989, Nature 334:544, de Graaf et al., 2002, Methods Mol Biol. 178:379-87. Single chain antibodies derived from antibodies described include, but are not limited to, scFvs comprising the variable domain combinations L1H, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, and L52H52).

Antigen binding proteins (e.g., antibodies, antibody fragments, and antibody derivatives) can comprise any constant region known in the art. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, *e.g.,* a human alpha-, delta-, epsilon-, gamma-, or mu-type heavy chain constant region. The light or heavy chain constant region may be a fragment, derivative, variant, or mutein of a naturally occurring constant region.

Techniques are known for deriving an antibody of a different subclass or isotype from an antibody of interest, *i.e.,* subclass switching. Thus, IgG antibodies may be derived from an IgM antibody, for example, and *vice versa.* Such techniques allow the preparation of new antibodies that possess the antigen-binding properties of a given antibody (the parent antibody), but also exhibit biological properties associated with an antibody isotype or subclass different from that of the parent antibody. Recombinant DNA techniques may be employed. Cloned DNA encoding particular antibody polypeptides may be employed in such procedures, *e.g.*, DNA encoding the constant domain of an antibody of the desired isotype. See also Lantto et al., 2002, Methods Mol. Biol. 178:303-16.

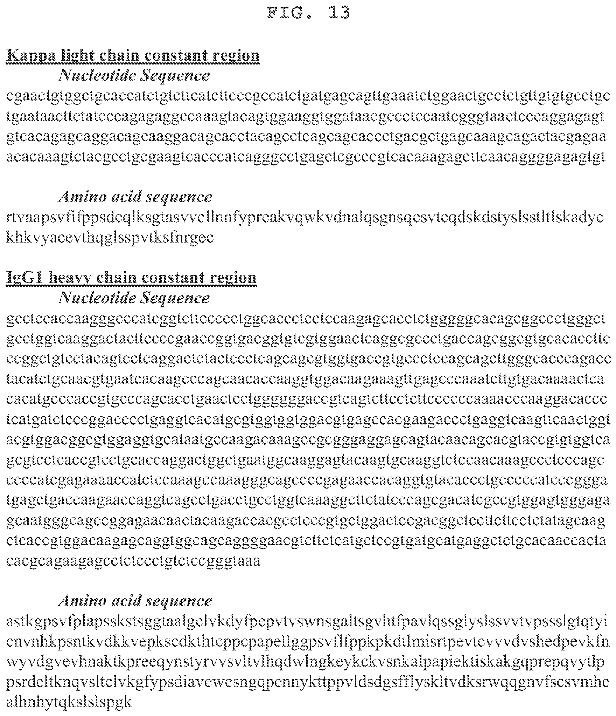

An antigen binding protein may comprise the IgG1 heavy chain domain of Figure 13 or a fragment of the IgG1 heavy chain domain of Figure 13. An antigen binding protein may comprise the kappa light chain constant chain region of Figure 13 or a fragment of the kappa light chain constant region of Figure 13. An antigen binding protein may comprise both the IgG1 heavy chain domain, or a fragment thereof, of Figure 13 and the kappa light chain domain, or a fragment thereof, of Figure 13.

Accordingly, the antigen binding proteins of described herein include those comprising, for example, the variable domain combinations L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, and L52H52, having a desired isotype (for example, IgA, IgG1, IgG2, IgG3, IgG4, IgM, IgE, and IgD) as well as Fab or F(ab')₂ fragments thereof. Moreover, if an IgG4 is desired, it may also be desired to introduce a point mutation (CPSCP -> CPPCP) in the hinge region as described in Bloom et al., 1997, Protein Science 6:407, to alleviate a tendency to form intra-H chain disulfide bonds that can lead to heterogeneity in the IgG4 antibodies.

An example of an antigen binding protein is the IgG1 antibody AMG 479, also known as ganitumab. Ganitumab has the light and heavy chain variable domains of L16H16.

Moreover, techniques for deriving antigen binding proteins having different properties (*i.e.,* varying affinities for the antigen to which they bind) are also known. One such technique, referred to as chain shuffling, involves displaying immunoglobulin variable domain gene repertoires on the surface of filamentous bacteriophage, often referred to as phage display. Chain shuffling has been used to prepare high affinity antibodies to the hapten 2-phenyloxazol-5-one, as described by Marks et al., 1992, BioTechnology, 10:779.

Antigen binding proteins may have a binding affinity (Kₐ) for IGF-1R of at least 10⁶, measured as described in the Examples. In other embodiments, the antigen binding proteins exhibit a Kₐ of at least 10⁷, at least 108, at least 10⁹, or at least 10¹⁰.

Herein described is an antigen binding protein that has a low dissociation rate from IGF-1R. e.g., the antigen binding protein has a K_{off} of 1x10⁻⁴ _{S}⁻¹ or lower, the K_{off} is 5x 10⁻⁵ s⁻¹ or lower or the K_{off} is substantially the same as an antibody having a combination of light chain and heavy chain variable domain sequences selected from the group of combinations consisting of L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51 , and L52H52. The antigen binding protein may bind to IGF-1R with substantially the same K_{off} as an antibody that comprises one or more CDRs from an antibody having a combination of light chain and heavy chain variable domain sequences selected from the group of combinations consisting of L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, and L52H52. The antigen binding protein may bind to IGF-1R with substantially the same K_{off} as an antibody that comprises one of the amino acid sequences illustrated in Figures 2 through 9. The antigen binding protein may bind to IGF-1R with substantially the same K_{off} as an antibody that comprises one or more CDRs from an antibody that comprises one of the amino acid sequences illustrated in Figures 2 through 9.

Herein described is an antigen binding protein that binds to the L2 domain of human IGF-1R. Antigen binding proteins that bind to the L2 domain can be made using any technique known in the art. For example, such antigen binding proteins can be isolated using the full-length IGF-1R polypeptide (e.g., in a membrane-bound preparation), a soluble extracellular domain fragment of IGF-1R (an example of which is provided in Example 1), or a smaller fragment of the IGF-1R extracellular domain comprising or consisting of the L2 domain (examples of which are provided in Example 10). Antigen binding proteins so isolated can be screened to determine their binding specificity using any method known in the art (an example of which is provided in Example 10).

Also herein described is an antigen binding protein that binds to human IGF-1R expressed on the surface of a cell and, when so bound, inhibits IGF-1R signaling activity in the cell without causing a significant reduction in the amount of IGF-1R on the surface of the cell. Any method for determining or estimating the amount of IGF-1R on the surface and/or in the interior of the cell can be used. Herein described is an antigen binding protein that binds to the L2 domain of a human IGF-1R expressed on the surface of a cell and, when so bound, inhibits IGF-1R signaling activity in the cell without significantly increasing the rate of internalization of the IGF-1R from the surface of the cell. Binding of the antigen binding protein to the IGF-1R-expressing cell may cause less than about 75%, 50%, 40%, 30%, 20%, 15%, 10%, 5%, 1%, or 0.1% of the cell-surface IGF-1R to be internalized. Binding of the antigen binding protein to the IGF-1R-expressing cell may cause a gradual reduction in the amount of IGF-1R on the cell surface such that within a few hours of contacting the cell with the antigen binding protein, little or no decrease in cell surface IGF-1R is detected, but, after several days or weeks of exposure of the cell to the antigen binding protein, a marked decrease in cell surface IGF-1R is detected.

Herein described is an antigen binding protein having a half-life of at least one day *in vitro* or *in vivo* (*e.g.,* when administered to a human subject). The antigen binding protein may have a half-life of at least three days. The antigen binding protein may have a half-life of four days or longer, or a half-life of eight days or longer. The antigen binding protein may be derivatized or modified such that it has a longer half-life as compared to the underivatized or unmodified antigen binding protein. The antigen binding protein may contain one or more point mutations to increase serum half life, such as described in WO 00/09560, published Feb.24, 2000.

Herein described are multi-specific antigen binding proteins, for example, bispecific antigen binding protein, e.g., antigen binding protein that bind to two different epitopes of IGF-1R, or to an epitope of IGF-1R and an epitope of another molecule, via two different antigen binding sites or regions. Moreover, bispecific antigen binding protein as disclosed herein can comprise an IGF-1R binding site from one of the herein-described antibodies and a second IGF-1R binding region from another of the herein-described antibodies. Alternatively, a bispecific antigen binding protein may comprise an antigen binding site from one of the herein described antibodies and a second antigen binding site from another IGF-1R antibody that is known in the art, or from an antibody that is prepared by known methods or the methods described herein.

Numerous methods of preparing bispecific antibodies are known in the art, and discussed in US Patent Application 09/839,632, filed April 20, 2001. Such methods include the use of hybrid-hybridomas as described by Milstein et al., 1983, Nature 305:537, and others (U.S. Patent 4,474,893, U.S. Patent 6,106,833), and chemical coupling of antibody fragments (Brennan et al., 1985, Science 229:81; Glennie et al., 1987, J. Immunol. 139:2367; U.S. Patent 6,010,902). Moreover, bispecific antibodies can be produced via recombinant means, for example by using leucine zipper moieties *(i.e.,* from the Fos and Jun proteins, which preferentially form heterodimers; Kostelny et al., 1992, J. Immnol. 148:1547) or other lock and key interactive domain structures as described in U.S. Patent 5,582,996. Additional useful techniques include those described in Kortt *et al.*, 1997, *supra;* U.S. Patent 5,959,083; and U.S. Patent 5,807,706.

The antigen binding protein may comprise a derivative of an antibody. The derivatized antibody can comprise any molecule or substance that imparts a desired property to the antibody, such as increased half-life in a particular use. The derivatized antibody can comprise, for example, a detectable (or labeling) moiety (*e.g.,* a radioactive, colorimetric, antigenic or enzymatic molecule, a detecable bead (such as a magnetic or electrodense (*e.g*., gold) bead), or a molecule that binds to another molecule (*e.g.,* biotin or streptavidin)), a therapeutic or diagnostic moiety (*e.g.,* a radioactive, cytotoxic, or pharmaceutically active moiety), or a molecule that increases the suitability of the antibody for a particular use (*e.g.,* administration to a subject, such as a human subject, or other *in vivo* or *in vitro* uses). Examples of molecules that can be used to derivatize an antibody include albumin (*e.g*., human serum albumin) and polyethylene glycol (PEG). Albumin-linked and PEGylated derivatives of antibodies can be prepared using techniques well known in the art. The antibody may be conjugated or otherwise linked to transthyretin (TTR) or a TTR variant. The TTR or TTR variant can be chemically modified with, for example, a chemical selected from the group consisting of dextran, poly(n-vinyl pyurrolidone), polyethylene glycols, propropylene glycol homopolymers, polypropylene oxide/ethylene oxide co-polymers, polyoxyethylated polyols and polyvinyl alcohols. US Pat. App. No. 20030195154.

Herein described are methods of screening for a molecule that binds to IGF-1R using the antigen binding proteins described herein. Any suitable screening technique can be used. An IGF-1R molecule, or a fragment thereof to which an antigen binding protein binds, may be contacted with an antigen binding protein herein described and with another molecule, wherein the other molecule binds to IGF-1R if it reduces the binding of the antigen binding protein to IGF-1R. Binding of the antigen binding protein can be detected using any suitable method, *e.g.,* an ELISA. Detection of binding of the antigen binding protein to IGF-1R can be simplified by detectably labeling the antigen binding protein, as discussed above. The IGF-1R-binding molecule may be further analyzed to determine whether it inhibits IGF-1R, IGF-1, and/or IGF-2-mediated signaling.

### Nucleic acids

Herein described are isolated nucleic acid molecules. The nucleic acids comprise, for example, polynucleotides that encode all or part of an antigen binding protein , for example, one or both chains of an antibody or a fragment, derivative, mutein, or variant thereof, polynucleotides sufficient for use as hybridization probes, PCR primers or sequencing primers for identifying, analyzing, mutating or amplifying a polynucleotide encoding a polypeptide, anti-sense nucleic acids for inhibiting expression of a polynucleotide, and complementary sequences of the foregoing. The nucleic acids can be any length. They can be, for example, 5, 10, 15, 20, 25, 30, 35, 40, 45, 50, 75, 100, 125, 150, 175, 200, 250, 300, 350, 400, 450, 500, 750, 1,000, 1,500, 3,000, 5,000 or more nucleotides in length, and/or can comprise one or more additional sequences, for example, regulatory sequences, and/or be part of a larger nucleic acid, for example, a vector. The nucleic acids can be single-stranded or double-stranded and can comprise RNA and/or DNA nucleotides, and artificial variants thereof (*e.g.,* peptide nucleic acids).

Nucleic acids encoding antibody polypeptides (*e.g*., heavy or light chain, variable domain only, or full length) may be isolated from B-cells of mice that have been immunized with IGF-1R. The nucleic acid may be isolated by conventional procedures such as polymerase chain reaction (PCR).

Figure 1 provides nucleic acid sequences encoding the variable regions of the heavy and light chain variable regions shown in Figures 2 and 3. The skilled artisan will appreciate that, due to the degeneracy of the genetic code, each of the polypeptide sequences in Figures 2 through 9 also is encoded by a large number of other nucleic acid sequences. Herein described are each degenerate nucleotide sequence encoding each antigen binding protein herein described.

Herein described are nucleic acids that hybridize to other nucleic acids (*e.g.,* nucleic acids comprising a nucleotide sequence of Figure 1) under particular hybridization conditions. Methods for hybridizing nucleic acids are well-known in the art. See, *e.g.,* Current Protocols in Molecular Biology, John Wiley & Sons, N.Y. (1989), 6.3.1-6.3.6. As defined herein, a moderately stringent hybridization condition uses a prewashing solution containing 5X sodium chloride/sodium citrate (SSC), 0.5% SDS, 1.0 mM EDTA (pH 8.0), hybridization buffer of about 50% formamide, 6X SSC, and a hybridization temperature of 55° C (or other similar hybridization solutions, such as one containing about 50% formamide, with a hybridization temperature of 42° C), and washing conditions of 60° C, in 0.5X SSC, 0.1% SDS. A stringent hybridization condition hybridizes in 6X SSC at 45° C, followed by one or more washes in 0.1X SSC, 0.2% SDS at 68° C. Furthermore, one of skill in the art can manipulate the hybridization and/or washing conditions to increase or decrease the stringency of hybridization such that nucleic acids comprising nucleotide sequences that are at least 65, 70, 75, 80, 85, 90, 95, 98 or 99% identical to each other typically remain hybridized to each other. The basic parameters affecting the choice of hybridization conditions and guidance for devising suitable conditions are set forth by, for example, Sambrook, Fritsch, and Maniatis (1989, Molecular Cloning: A Laboratory Manual, Cold Spring Harbor Laboratory Press, Cold Spring Harbor, N.Y., chapters 9 and 11; and Current Protocols in Molecular Biology, 1995, Ausubel et al., eds., John Wiley & Sons, Inc., sections 2.10 and 6.3-6.4), and can be readily determined by those having ordinary skill in the art based on, for example, the length and/or base composition of the DNA.

Changes can be introduced by mutation into a nucleic acid, thereby leading to changes in the amino acid sequence of a polypeptide (*e.g.,* an antigen binding protein) that it encodes. Mutations can be introduced using any technique known in the art. One or more particular amino acid residues may be changed using, for example, a site-directed mutagenesis protocol. One or more randomly selected residues may be changed using, for example, a random mutagenesis protocol. However it is made, a mutant polypeptide can be expressed and screened for a desired property (*e.g.,* binding to IGF-1R or blocking the binding of IGF-1 and/or IGF-2 to IGF-1R).

Mutations can be introduced into a nucleic acid without significantly altering the biological activity of a polypeptide that it encodes. For example, one can make nucleotide substitutions leading to amino acid substitutions at non-essential amino acid residues. A nucleotide sequence provided in Figure 1, or a desired fragment, variant, or derivative thereof, may be mutated such that it encodes an amino acid sequence comprising one or more deletions or substitutions of amino acid residues that are shown in Figures 2 through 9 to be residues where two or more sequences differ. The mutagenesis may insert an amino acid adjacent to one or more amino acid residues shown in Figures 2 through 9 to be residues where two or more sequences differ. Alternatively, one or more mutations can be introduced into a nucleic acid that selectively change the biological activity (*e.g.,* binding of IGF-1R, inhibiting IGF-1 and/or IGF-2, *etc*.) of a polypeptide that it encodes. For example, the mutation can quantitatively or qualitatively change the biological activity. Examples of quantitative changes include increasing, reducing or eliminating the activity. Examples of qualitative·changes include changing the antigen specificity of an antigen binding protein.

Herein also described are nucleic acid molecules that are suitable for use as primers or hybridization probes for the detection of nucleic acid sequences. A nucleic acid molecule can comprise only a portion of a nucleic acid sequence encoding a full-length polypeptide, for example, a fragment that can be used as a probe or primer or a fragment encoding an active portion (*e.g.,* an IGF-1R binding portion) of a polypeptide.

Probes based on the sequence of a nucleic acid can be used to detect the nucleic acid or similar nucleic acids, for example, transcripts encoding a polypeptide. The probe can comprise a label group, *e.g.,* a radioisotope, a fluorescent compound, an enzyme, or an enzyme co-factor. Such probes can be used to identify a cell that expresses the polypeptide.

Herein described are vectors comprising a nucleic acid encoding a polypeptide or a portion thereof. Examples of vectors include, but are not limited to, plasmids, viral vectors, non-episomal mammalian vectors and expression vectors, for example, recombinant expression vectors.

The recombinant expression vectors can comprise a nucleic acid in a form suitable for expression of the nucleic acid in a host cell. The recombinant expression vectors include one or more regulatory sequences, selected on the basis of the host cells to be used for expression; which is operably linked to the nucleic acid sequence to be expressed. Regulatory sequences include those that direct constitutive expression of a nucleotide sequence in many types of host cells (*e.g*., SV40 early gene enhancer, Rous sarcoma virus promoter and cytomegalovirus promoter), those that direct expression of the nucleotide sequence only in certain host cells (*e.g.,* tissue-specific regulatory sequences, see Voss et al., 1986, Trends Biochem. Sci. 11:287, Maniatis et al., 1987, Science 236:1237), and those that direct inducible expression of a nucleotide sequence in response to particular treatment or condition (*e.g*., the metallothionin promoter in mammalian cells and the tet-responsive and/or streptomycin responsive promoter in both prokaryotic and eukaryotic systems (see *id*.). It will be appreciated by those skilled in the art that the design of the expression vector can depend on such factors as the choice of the host cell to be transformed, the level of expression of protein desired, *etc.* The expression vectors can be introduced into host cells to thereby produce proteins or peptides, including fusion proteins or peptides, encoded by nucleic acids as described herein.

Also herein described are host cells into which a recombinant expression vector has been introduced. A host cell can be any prokaryotic cell (for example, *E. coli*) or eukaryotic cell (for example, yeast, insect, or mammalian cells (*e.g*., CHO cells)). Vector DNA can be introduced into prokaryotic or eukaryotic cells via conventional transformation or transfection techniques. For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.,* for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (*e.g*., cells that have incorporated the selectable marker gene will survive, while the other cells die), among other methods.

### Indications

Herein described are methods of treating a subject. The method can, for example, have a generally salubrious effect on the subject, *e.g.,* it can increase the subject's expected longevity. Alternatively, the method can, for example, treat, prevent, cure, relieve, or ameliorate ("treat") a disease, disorder, condition, or illness ("a condition"). Among the conditions to be treated are conditions characterized by inappropriate expression or activity of IGF-1, IGF-2, and/or IGF-1R. In some such conditions, the expression or activity level is too high, and the treatment comprises administering an IGF-1R antagonist as described herein. In other such conditions, the expression or activity level is too low, and the treatment comprises administering an IGF-1R agonist as described herein.

One example of a type of condition that can be treated using the methods and compositions described herein is a condition that involves cell growth, for example, a cancerous condition. Thus, herein described are compositions and methods for treating a cancerous condition. The cancerous condition can be any cancerous condition that can be treated using the compositions comprised herein, for example, IGF-1R antagonizing antigen binding proteins such as anti-IGF-1R antibodies, antibody fragments, or antibody derivatives. Examples of cancerous conditions include, for example, Acute Lymphoblastic Leukemia, Adrenocortical Carcinoma, AIDS-Related Cancers, AIDS-Related Lymphoma, Anal Cancer, Childhood Cerebellar Astrocytoma, Childhood Cerebral Astrocytoma, Basal Cell Carcinoma, Extrahepatic Bile Duct Cancer, Bladder Cancer, Osteosarcoma/Malignant Fibrous Histiocytoma Bone Cancer, Brain Tumors (e.g., Brain Stem Glioma, Cerebellar Astrocytoma, Cerebral Astrocytoma/Malignant Glioma, Ependymoma, Medulloblastoma, Supratentorial Primitive Neuroectodermal Tumors, Visual Pathway and Hypothalamic Glioma), Breast Cancer, Bronchial Adenomas/Carcinoids, Burkitt's Lymphoma, Carcinoid Tumor, Gastrointestinal Carcinoid Tumor, Carcinoma of Unknown Primary, Primary Central Nervous System, Cerebellar Astrocytoma, Cerebral Astrocytoma/Malignant Glioma, Cervical Cancer, Childhood Cancers, Chronic Lymphocytic Leukemia, Chronic Myelogenous Leukemia, Chronic Myeloproliferative Disorders, Colon Cancer, Colorectal Cancer, Cutaneous T-Cell Lymphoma, Endometrial Cancer, Ependymoma, Esophageal Cancer, Ewing's Family of Tumors, Extracranial Germ Cell Tumor, Extragonadal Germ Cell Tumor, Extrahepatic Bile Duct Cancer, Intraocular Melanoma Eye Cancer, Retinoblastoma Eye Cancer, Gallbladder Cancer, Gastric (Stomach) Cancer, Gastrointestinal Carcinoid Tumor, Germ Cell Tumors (e.g., Extracranial, Extragonadal, and Ovarian), Gestational Trophoblastic Tumor, Glioma (e.g., Adult, Childhood Brain Stem, Childhood Cerebral Astrocytoma, Childhood Visual Pathway and Hypothalamic), Hairy Cell Leukemia, Head and Neck Cancer, Hepatocellular (Liver) Cancer, Hodgkin's Lymphoma, Hypopharyngeal Cancer, Hypothalamic and Visual Pathway Glioma, Intraocular Melanoma, Islet Cell Carcinoma (Endocrine Pancreas), Kaposi's Sarcoma, Kidney (Renal Cell) Cancer, Laryngeal Cancer, Leukemia (e.g., Acute Lymphoblastic, Acute Myeloid, Chronic Lymphocytic, Chronic Myelogenous, and Hairy Cell), Lip and Oral Cavity Cancer, Liver Cancer, Non-Small Cell Lung Cancer, Small Cell Lung Cancer, Lymphoma (e.g., AIDS-Related, Burkitt's, Cutaneous T-Cell, Hodgkin's, Non-Hodgkin's, and Primary Central Nervous System), Waldenström's Macroglobulinemia, Malignant Fibrous Histiocytoma of Bone/Osteosarcoma, Medulloblastoma, Melanoma, Intraocular (Eye) Melanoma, Merkel Cell Carcinoma, Mesothelioma, Metastatic Squamous Neck Cancer with Occult Primary, Multiple Endocrine Neoplasia Syndrome, Multiple Myeloma/Plasma Cell Neoplasm, Mycosis Fungoides, Myelodysplastic Syndromes, Myelodysplastic/Myeloproliferative Diseases, Myelogenous Leukemia, Chronic Myeloid Leukemia, Multiple Myeloma, Chronic Myeloproliferative Disorders, Nasal Cavity and Paranasal Sinus Cancer, Nasopharyngeal Cancer, Neuroblastoma, Oral Cancer, Oropharyngeal Cancer, Osteosarcoma/Malignant Fibrous Histiocytoma of Bone, Ovarian Cancer, Ovarian Epithelial Cancer, Ovarian Germ Cell Tumor, Ovarian Low Malignant Potential Tumor, Pancreatic Cancer, Islet Cell Pancreatic Cancer, Paranasal Sinus and Nasal Cavity Cancer, Parathyroid Cancer, Penile Cancer, Pheochromocytoma, Pineoblastoma, Pituitary Tumor, Plasma Cell Neoplasm/Multiple Myeloma, Pleuropulmonary Blastoma, Primary Central Nervous System Lymphoma, Prostate Cancer, Rectal Cancer, Renal Cell (Kidney) Cancer, Renal Pelvis and Ureter Transitional Cell Cancer, Retinoblastoma, Rhabdomyosarcoma, Salivary Gland Cancer, Soft Tissue Sarcoma, Uterine Sarcoma, Sezary Syndrome, non-Melanoma Skin Cancer, Merkel Cell Skin Carcinoma, Small Intestine Cancer, Soft Tissue Sarcoma, Squamous Cell Carcinoma, Cutaneous T-Cell Lymphoma, Testicular Cancer, Thymoma, Thymic Carcinoma, Thyroid Cancer, Gestational Trophoblastic Tumor, Carcinoma of Unknown Primary Site, Cancer of Unknown Primary Site, Urethral Cancer, Endometrial Uterine Cancer, Uterine Sarcoma, Vaginal Cancer, Visual Pathway and Hypothalamic Glioma, Vulvar Cancer, Waldenström's Macroglobulinemia, and Wilms' Tumor.

Four different groups have studied a total of 425 breast cancers, mostly ductal in origin, and 48 normal tissues or benign specimens by radioimmunoassay ("RIA") or immunohistochemistry ("IHC") (Papa et al., 1993, Cancer Research 53: 3736-40, Happerfield et al., 1997, Journal of Pathology 183: 412-17; Ellis et al., 1998, Breast Cancer Research & Treatment 52: 175-84, Lee et al., 1998, Breast Cancer Research & Treatment 47: 295-302, Schnarr et al., 2000, International Journal of Cancer 89: 506-13). These studies suggest that elevated IGF-1R expression, on the order of 5-10 fold, is associated with favorable prognosis and biomarkers (ER+ PR+), suggesting that estrogen and IGF cooperate in the maintenance or progression of well differentiated tumor. Similarly, estrogen has been shown to be essential for the growth and survival of the ER+ MCF-7 breast cancer cell line, and in this context IGF-1R is up-regulated by estrogen treatment (reviewed in Ellis et al., 1998, Breast Cancer Research & Treatment 52: 175-84). Thus, herein described is a method of treating breast cancer in a subject in need of such treatment, comprising administering to the subject an effective amount of an IGF-1R antagonist as described herein. The method may further comprise administering a hormone inhibitor, *e.g.,* an estrogen inhibitor.

A retrospective IGF-1R IHC analysis has been reported for a collection of 12 colonic adenomas, 36 primary colorectal adenocarcinomas and 27 corresponding metastases, and 34 adjacent normal tissues (Hakam et al., 1999, Human Pathology. 30: 1128-33). The frequency of moderate to strong IHC staining appeared to dramatically increase with higher stage and tumor grade (0% normal vs. 93 % metastases). The results are consistent with RNA analysis by RNAse protection assay ("RPA") (Freier et al., 1999, Gut 44: 704-08). Herein described is a method of treating colon cancer in a subject in need of such treatment, comprising administering to the subject an effective amount of an IGF-1R antagonist as described herein.

High plasma IGF-1 and reduced IGFbp3 in men 40-80 years old is associated with increased prostate cancer risk (Chan et al., 1998, Science 279: 563-6). High IGF-1 is associated with a risk of other cancers including breast (Hankinson et al., 1998, Lancet 351: 1393-96), colon (Ma et al., 1999, Journal of the National Cancer Institute 91: 620-25) and lung (Yu et al., 1999, Journal of the National Cancer Institute 91: 151-56). In transgenic mouse models, tumor incidence is increased by IGF-1 overexpression in diverse locations (Bol et al., 1997, Oncogene 14: 1725-34; DiGiovanni et al., 2000, Cancer Research 60: 1561-70; DiGiovanni et al., 2000, Proceedings of the National Academy of Sciences of the United States of America 97: 3455-60, Hadsell et al., 2000, Oncogene 19: 889-98). These mouse studies point to a role for both serum and stromal produced IGF-1. Herein described is a method of treating a subject in need of such treatment, comprising administering to the subject an effective amount of an antagonist of IGF-1 R as described herein, wherein the antagonist inhibits the activation of IGF-1R by IGF-1. The subject may have cancer or a tumor. The cancer may be prostate, breast, colon or lung cancer.

It has been observed that bone is the major source of IGF-1 in the body. Herein described are compositions and methods for inhibiting IGF-1R in a bone of a subject. An IGF-1R inhibitor may be administered to a subject that has, or is at risk for developing, a tumor in a bone. The tumor can be, for example, a primary tumor or a metastatic tumor. The treatment optionally further comprises administering to the subject one or more additional therapeutic and/or palliative treatments, for example, an anti-tumor treatment (*e.g*., chemotherapy, radiation therapy, or anti-hormone therapy) or a treatment that inhibits bone turnover (*e.g.,* denosumab (Amgen Inc., Thousand Oaks, CA)).

IGF-2 is overexpressed in a variety of tumors and stromal tissues. IGF-2 levels appear especially high (as much as 40 fold) in primary liver cancers (Cariani et al., 1988, Cancer Research 48: 6844-49) and adenocarcinoma of the colon (Freier et al., 1999, Gut 44: 704-08). Many of the overgrowth disorders are associated with an increased incidence of childhood tumors. Five to ten percent of individuals with either the prenatal growth disorder Beckwith-Weidmann Syndrome (BWS) or hemihyperplasia develop tumors such as nephroblastoma, adrenal carcinoma, and neuroblastoma (reviewed by Morison et al., 1998, Molecular Medicine Today 4: 110-05). The tumor-predisposing factor in these children appears to be the mosaic loss of maternal IGF-2 gene imprinting, or duplication of the paternal chromosomal arm (11p) that carries IGF-2. Both alterations would increase the level of IGF-2 expression. IGF-2 overexpression as a result of mosaic uniparental disomy or loss of IGF-2 imprinting has also been detected in Wilms tumors. Growth disorders are not observed in these children even though the IGF-2 gene alterations also occur in some normal tissues, perhaps reflecting the tissue distribution of the affected cells. Imprinting of the maternal IGF-2 gene also occurs in mice, and the effects of IGF-2 overexpression are consistent with the human situation (Cariani et al., 1991, Journal of Hepatology 13: 220-26, Schirmacher et al., 1992, Cancer Research 52: 2549-56; Harris et al., 1998, Oncogene 16: 203-09). The incidence of tumors and organomegaly increases in mice that transgenically express excess IGF-2 (Christofori et al., 1994, Nature 369: 414-18, Ward et al., 1994, Proceedings of the National Academy of Sciences of the United States of America 91: 10365-9, Wolf et al., 1994, Endocrinology 135: 1877-86, Bates et al., 1995, British Journal of Cancer 72: 1189-93, Hassan et al., 2000, Cancer Research 60: 1070-76). Local IGF-2 overexpression increases the spontaneous appearance of prostate, mammary, intestinal, liver and epidermal tumors. Plasma specific expression using liver promoters elevate hepatocellular carcinomas and lymphoma. Herein described is a method of treating a subject in need of such treatment, comprising administering to the subject an effective amount of an antagonist of IGF-1R as described herein, wherein the antagonist inhibits the activation of IGF-1R by IGF-2. The subject may have cancer or a tumor. The subject may have liver cancer, adenocarcinoma of the colon, Beckwith-Weidmann Syndrome, hemihyperplasia, nephroblastoma, adrenal carcinoma, neuroblastoma, mosaic loss of maternal IGF-2 gene imprinting, duplication of the paternal chromosomal arm (11p), increased IGF-2 expression, a tumor (*e.g.,* a prostate, mammary, intestinal, liver, epidermal, or Wilms tumor), organomegaly, hepatocellular carcinoma, or lymphoma.

Herein described are methods of preventing or inhibiting a cancer from spreading to another part of the body, or of treating a cancer that has spread to another part of the body. e.g., the cancer has spread to a regional lymph node. The cancer may be metastatic. The primary tumor can be any kind of tumor, for example, an adenocarcinoma tumor (*e.g*., a prostate adenocarcinoma tumor, a breast carcinoma tumor, or a renal cell carcinoma tumor), a non-small cell or small cell lung cancer tumor, a thyroid cancer tumor, *etc.* The site of the metastatic tumor can be anywhere in the body. It can be, for example, in bone, the lymph system, lung, brain, eye, skin, pancrease, or liver. A subject having a tumor disease may be treated with an effective amount of an IGF-1 R inhibiting composition described herein such that the primary tumor is prevented from metastasizing. A subject having a primary tumor may be treated with an effective amount of an IGF-1R inhibiting composition described herein such that the primary tumor is inhibited from metastasizing. A subject having a metastatic tumor may be treated with an effective amount of an IGF-1R inhibiting composition such that growth or spreading of the secondary tumor is inhibited. A subject having a metastatic tumor may be treated with an effective amount of an IGF-1 R inhibiting composition such that the secondary tumor is reduced in size. The primary tumor may be an adenocarcinoma tumor, a non-small cell lung tumor, a small cell lung tumor, or a thyroid cancer. The metastatic tumor may be in a bone. A metastatic tumor may be prevented or inhibited from forming in a bone. The method may comprise treating the subject with an IGF-1R inhibiting composition and one or more other treatments (e.g., a treatment that kills or inhibits the growth of cancer cells, such as radiation, hormonal therapy, or chemotherapy, or a treatment that inhibits the turnover of bone, such as denosumab). The one or more other treatments can include, for example the standard of care for the subject's particular condition and/or palliative care.

Without being bound to any particular theory, tumor cells appear to depend on the PI3 Kinase/Akt signaling pathway to resist the apoptosis-inducing activity of chemotherapeutics, radiation, and anti-hormone therapy. Thus, herein described are methods of treating a subject in need of such treatment comprising administering to the subject an IGF-1R antagonist and a chemotherapeutic, radiation, and/or an anti-hormone therapy. This concept has been validated experimentally in cell culture models and rodent tumor models by antisense and dominant negative mutations (reviewed by Baserga et al., 1997, Biochimica et Biophysica Acta 1332: F105-26, Baserga, 2000, Oncogene 19: 5574-81). The chemotherapeutic agents may be selected from the group consisting of mitotic inhibitors, alkylating agents, anti-metabolites, intercalating antibiotics, growth factor inhibitors, cell cycle inhibitors, enzymes, topoisomerase inhibitors, anti-survival agents, biological response modifiers, anti-hormones, e.g. anti-androgens, and anti-angiogenesis agents.

One example of a chemotherapeutic agent that can be administered in combination with an IGF-1 receptor inhibitor described herein is CPT-11. CPT-11 (Irinotecan hydorchloride trihydrate) is a semi synthetic, water soluble derivative of camptothecin, a plant alkaloid. CPT-11 and an associated metabolite called SN38 inhibit topoisomerase 1 (TOPO1). This enzyme introduces reversible single-strand breaks in DNA that allow unwinding and permit DNA replication to proceed. Inhibition of TOPO1 prevents religation of single-strand breaks after DNA replication resulting in greatly increased chromosomal fragmentation. This DNA damage promotes cell death by apoptosis through the action of p53 and other systems that monitor genome integrity: The cytotoxic effect of CPT-1 1 is generally limited to cells that are replicating DNA (S-Phase). Quiescent cells are largely unaffected.

Herein described is treating a subject in need thereof with an effective amount of an IGF-1R antagonist and with an effective amount of an apoptosis-inducing agent.

An anti-angiogenesis agent, such as an MMP-2 (matrix-metalloproteinase 2) inhibitor, an MMP-9 (matrix-metalloproteinase 9) inhibitor, and/or a COX-II (cyclooxygenase II) inhibitor, may be used in conjunction with a compound described herein. Examples of useful COX-II inhibitors include CELEBREX™ (alecoxib), BEXTRA™ (valdecoxib), and VIOXX™ (rofecoxib). Examples of useful matrix metalloproteinase inhibitors are described in WO 96/33172 (published Oct. 24, 1996), WO 96/27583 (published Mar. 7, 1996), European Patent Application No. 97304971.1 (filed Jul. 8, 1997), European Patent Application No. 99308617.2 (filed Oct. 29, 1999), WO 98/07697 (published Feb. 26, 1998), WO 98/03516 (published Jan. 29, 1998), WO 98/34918 (published Aug. 13, 1998), WO 98/34915 (published Aug. 13, 1998), WO 98/33768 (published Aug. 6, 1998), WO 98/30566 (published Jul. 16, 1998), European Patent Publication 606,046 (published Jul. 13, 1994), European Patent Publication 931,788 (published Jul. 28, 1999), WO 90/05719 (published May 31, 1990), WO 99/52910 (published Oct. 21, 1999), WO 99/52889 (published Oct. 21, 1999), WO 99/29667 (published Jun. 17, 1999), PCT International Application No. PCT/IB98/01113 (filed Jul. 21, 1998), European Patent Application No. 99302232.1 (filed Mar. 25, 1999), Great Britain patent application number 9912961.1 (filed Jun. 3, 1999), U.S. Provisional Application No. 60/148,464 (filed Aug. 12, 1999), U.S. Pat. No. 5,863,949 (issued Jan. 26, 1999), U.S. Pat. No. 5,861,510 (issued Jan. 19, 1999), and European Patent Publication 780,386 (published Jun. 25, 1997). The MMP inhibitor may be one that does not demonstrate arthralgia. The MMP inhibit may selectively inhibit MMP-2 and/or MMP-9 relative to other matrix-metalloproteinases (*i.e.,* MMP-1, MMP-3, MMP-4, MMP-5, MMP-6, MMP-7, MMP-8, MMP-10, MMP-11, MMP-12, and MMP-13). Some specific examples of MMP inhibitors useful are AG-3340, RO 32-3555, RS 13-0830, and the compounds recited in the following list: 3-[[4-(4-fluoro-phenoxy)-benzene-sulfonyl]-(1-hydroxycarbamoyl-cyclopentyl)-amino]-propionic acid; 3-exo-3-[4-(4-fluoro-phenoxy)-benzenesulfonylamino]-8-oxa-bicyclo[3.2.1]o-ctane-3-carboxylic acid hydroxyamide; (2R, 3R) 1-[4-(2-chloro-4-fluoro-ben- zyloxy)-benzenesulfonyl]-3-hydroxy-3-methyl-piperidine-2-carboxylic acid hydroxyamide; 4-[4-(4-fluoro-phenoxy)-benzenesulfonylamino]-tetrahydro-py-ran-4-carboxylic acid hydroxyamide; 3-[[4-(4-fluoro-phenoxy)-benzenesulfon- yl]-(1-hydroxycarbamoyl-cyclobutyl)-amino]-propionic acid; 4-[4-(4-chloro-phenoxy)-benzenesulfonylamino]-tetrahydro-pyran-4-carboxyl-ic acid hydroxyamide; (R) 3-[4-(4-chloro-phenoxy)-benzenesulfonylamino]-te- trahydro-pyran-3-carboxylic acid hydroxyamide; (2R, 3R) 1-[4-(4-fluoro-2-methyl-benzyloxy)-benzenesulfonyl]-3-hydroxy-3-methyl-pi- peridine-2-carboxylic acid hydroxyamide; 3-[[4-(4-fluoro-phenoxy)-benzenesulfonyl]-(1-hydroxycarbamoyl-1-methyl-ethyl)-amino]-propionic acid; 3-[[4-(4-fluoro-phenoxy)-benzenesulfonyl]-(4-hydroxycarbamoyl-tetrahydro--pyran-4-yl)-amino]-propionic acid; 3-exo-3-[4-(4-chloro-phenoxy)-benzenesu-lfonylamino]-8-oxa-icyclo[3.2.1]octane-3-carboxylic acid hydroxyamide; 3-endo-3-[4-(4-fluoro-phenoxy)-benzenesulfonylamino]-8-oxa-icyclo[3.2.1]octane-3-carboxylic acid hydroxyamide; and (R) 3-[4-(4-fluoro-phenoxy)-b-enzenesulfonylamino]-tetrahydro-furan-3-carboxylic acid hydroxyamide; and pharmaceutically acceptable salts, solvates, derivatives, and other preparations of the compounds.

Sporadic mutations that inactivate the PETN gene product occur relatively frequently in most human cancers (Yamada et al., 2001, J Cell Sci 114:2375-82, Hill et al., 2002, Pharmacol Therapeut 93:243-51). Loss of PTEN causes the Akt phosphorylated state to persist through loss of the ability to down-regulate stimulatory signals originating from IGF-1R and other sources. The status of the p53 tumor suppressor also influences the activity of the IGF-1R signaling system. In the ground state, the basal or constitutive transcription of IGF-1R is repressed by p53 via an indirect mechanism. Activation of Akt promotes the phosphorylation of mdm2, which then binds the p53 tumor suppressor and promotes its degradation (Mayo et al., 2002, TIBS 27:462-67), resulting in increased IGF-1R expression. A similar outcome is observed when p53 is inactivated by mutation. When transiently expressed in Saos-2 (a human osteosarcoma cell line) and RD (a rhabdomyosarcoma cell line), wild-type p53 is able to suppress the activity of a cotransfected IGF-1R promoter construct, whereas tumor-derived, mutant versions of p53 have no effect. It has been proposed that the increased level of IGF-1R promotes the resistance to apoptosis associated with p53 loss in malignant cells (Werner et al., 2000, Cell Mol Life Sci 57:932-42). Herein described is a method of treating a cancerous condition in a subject in need of such treatment comprising administering to the subject an effective amount of an IGF-1R antagonist as described herein, wherein the cancerous condition is characterized by cells that have a reduced expression or activity of p53.

The WT1 (Wilms kidney tumor suppressor 1 protein) also has been shown to bind and repress the IGF-1R promoter. Thus, Herein described is a method of treating a cancerous condition in a subject in need of such treatment comprising administering to the subject an effective amount of an IGF-1R antagonist as described herein wherein the cancerous condition is characterized by a reduced expression or activity of WT1.

The proliferation of normal fibroblasts has been shown to require, under defined culture conditions, the combined action of IGF and a stromal growth factor (e.g. PDGF, EGF) to ramp-up Ras/Raf/Map Kinase and promote cell cycle entry (the G0 to G1 transition). Fibroblasts derived from IGF-1R (-/-) mice do not respond to growth factor alone, or most oncogenes (*e.g.* oncogenic Ras) that activate the Ras/Raf/Map Kinase pathway. Herein described is a method of treating a subject in need of such treatment comprising administering to the subject an IGF-1R antagonist as described herein and an agent that targets a growth factor and/or a growth factor receptor, such as a growth factor receptor tyrosine kinase, *e.g.,* the EGFR, HER-2, bcr-abl, VEGFR, Kit, raf, mTOR, CDK1/2, VEGFR2, PKCβ, Mek, and/or KDR. Examples of molecules that target such growth factors and/or receptors include panitumumab (Abgenix, Fremont, CA/Amgen, Thousand Oaks, CA), HERCEPTIN™ (Genentech, South San Francisco, CA), GLEEVEC™ (Novartis, East Hanover, NJ), IRESSA™ (AstraZeneca, Wilmington, DE), ERBITUX™, (ImClone, New York, NY), AVASTIN™, (Genentech), PTK787 (Novartis), SU11248 (Pfizer, New York, NY), TARCEVA™ (OSI Pharmaceuticals, Melville, NY), 43-9006 (Bayer, West Haven, CT), CCI-779 (Wyeth, Madison, NJ), RAD001 (Novartis), BMS-387032 (Bristol-Myers Squibb, New York, NY), IMC-1C11 (ImClone), LY333531 (Eli Lilly, Indianapolis, IN), PD 184352 (Pfizer), 2C4 (Genentech), and GW2016 (GlaxoSmithKline, Research Triangle Park, NC).

The role of IGF-1R in hematological malignancies has been reviewed by (Novak et al., 2003, Insulin-Like Growth Factors and Hematological Malignancies in Insulin-Like Growth Factors, LeRoith et al., ed.s, Landes Bioscience). A functional role for the IGF-1R in hematopoietic malignancies is demonstrated by, for example, the ability of IGF-1R monoclonal antibodies to block transformed cell growth in culture. IGF-I has been found to enhance growth of freshly isolated human acute myelogenous leukemia and acute lymphoblastic leukemia blasts. With respect to T cell malignancies, IGF-I has been shown to influence the growth of murine lymphoma cells bearing a pre-T cell phenotype and, immature and mature primary human T lineage acute lymphoblastic leukemia cells were found to express high numbers of IGF-1R. Thus, herein described are methods of treating a hematological malignancy in a subject in need thereof comprising administering to the subject an antagonist of IGF-1R as described herein. The malignancy may be an acute myelogenous leukemia, an acute lymphoblastic leukemia, or a T cell malignancy.

Herein described are methods of identifying subjects who are more likely to benefit from treatment using the compositions and/or methods of treatment described herein. Such methods can enable a caregiver to better tailor a therapeutic regimen to a particular subject's needs and reduce the likelihood of an ineffective or counterproductive course of treatment. Herein described is a method of determining whether a subject is a candidate for treatment using a composition or method as described herein comprising determining whether a target cell type in the subject expresses IGF-1R, wherein if the target cell type expresses IGF-1R, then the subject is a candidate for treatment. The method may comprise determining the approximate average number of IGF-1R molecules per target cell, wherein 10², 10³, 10⁴, 10⁵, or 10⁶ IGF-1R per cell indicates that the subject is a candidate for treatment. The approximate average number of IGF-1R molecules per target cell can be determined using any technique known in the art, for example, by staining a sample comprising cells of the target cell type with an IGF-1R binding molecule, and detecting the amount of IGF-1R binding molecule bound to the sample, where the amount of IGF-1R binding molecule detected is proportional to the average number of IGF-1R molecules in the sample. The method may comprise comparing the approximate average number of IGF-1R molecules per target cell to a reference standard, wherein if the approximate average number of IGF-1R molecules per target cell is greater than the reference standard, then the subject is more likely to benefit from treatment using the compositions and/or methods of treatment described herein. The target cell type may be cancerous cell type which may be a colon cancer cell type, a breast cancer cell type, an NSCLC cell type, or a leukemic cell type.

A subject who is a candidate for treatment may be identified by detecting IGF-1 and/or IGF-2 in the target cell type, or in the stratum of the target cell type. The target cell type may be a cancerous cell type such as a colon cancer cell type, a breast cancer cell type, an NSCLC cell type, or a leukemic cell type.

A subject who is a candidate for treatment may be identified by detecting activity of IGF-1R-mediated signaling in the target cell type *(e.g.,* a tumor or other cancerous tissue), wherein IGF-1R-mediated signaling in the target cell type indicates that the subject is a candidate for treatment. Examples of molecules that can be monitored for IGF-1R-dependent changes are shown in Figure 10, such as molecules in the PI3/Akt pathway, *e.g.,* IGF-1R, IRS adapters, Akt, *etc.* Such molecules can be monitored for, for example, a change in phosphorylation status, *e.g.,* an increase in phosphorylation. Phosphospecific antibodies that recognize the activated forms of these protein markers are highly developed, and these reagents have proven to be reliable for immunoblot detection in experimental systems.

Herein described are methods and compositions for determining whether a tissue in a subject (for example, a tumor tissue or other cancerous tissue in the subject) has a molecular marker that identifies the subject as being more likely or less likely to respond favorably to treatment using the therapeutic methods and compositions described herein. Any such molecular marker can be used. The molecular marker may be a chromosomal abnormality (for example, in tumor-derived tissue), such as a chromosomal abnormality involving the EWS gene and a transcription factor. The molecular marker may be a EWS-FLI chromosomal translocation in a tumor or other cancerous tissue. Such translocations can be detected using any method known in the art (see, for example, Giovannini et al., 1994, J Clin Invest. 94:489-96; Delattre et al., 1994, NEJM 331:294-99; and Zoubek et al., 1994, Br J Cancer 70:908-13. Examples of such detection methods include cytological analysis, fluorescent *in situ* hybridization (FISH), sequence analysis of a EWS-FLI hybrid gene, detection and/or quantification of a transcriptional product of a EWS-FLI hybrid gene (using, *e.g.,* a PCR-based technique such as RT-PCR, or a hybridization based technique such as *in situ* hybridization or a northern blot), detection and/or quantification of a polypeptide product of a EWS-FLI hybrid gene (using, *e.g.,* an antibody-based technique such as *in situ* staining or a western blot), detection and/or quantification of a molecule or an activity associated with a EWS-FLI hybrid gene product, detection and/or quantification of a molecule or an activity dependent upon an activity of a EWS-FLI hybrid gene product, or detection and/or quantification of a molecule or an activity affected by an activity of a EWS-FLI hybrid gene product. Detection of a EWS-FLI hybrid gene product (*e.g.,* a product of transcription or of translation) in a tumor or other cancerous tissue may indicate that the tumor or cancerous tissue is more likely to respond to treatment using an anti-IGF-l receptor inhibitor, or another inhibitor of signaling through the IGF-1 receptor signaling pathway, than a tumor or other cancerous tissue in which a EWS-FLI hybrid gene product is not detected. A sample derived from a tumor or other cancerous tissue containing a EWS-FLI chromosomal translocation may be tested to determine whether it expresses a EWS-FLI hybrid gene product. Detection of the EWS-FLI hybrid gene product indicates that the tumor or cancerous tissue is more likely to respond to treatment using an anti-IGF-1 receptor treatment or another inhibitor of signaling through the IGF-1 receptor signaling pathway.

The molecular marker may be a mutation in a signaling molecule, for example, in a kinase. The mutation can, for example, increase the activity of the signaling molecule, decrease the activity of the signaling molecule, and/or alter the ligand specificity, substrate specificity, timing, or location of the activity of the signaling molecule. The signaling molecule may be a RAS, and the mutation may be an activating mutation. RAS mutations are found in about one third of all human tumors. Examples of activating RAS mutations include mutations to codons 12, 13, and 61. Other examples of activating RAS mutations include mutations in codons 10, 11, 15, 18, and 22. Other types of mutations or other changes can also cause an inappropriate increase in signaling through a RAS molecule. Examples of such other types of changes include gene amplification, overexpression, or upstream activation of a RAS pathway, *e.g*., approximately 40% of esophageal adenocarcinomas have an amplified KRAS gene, resulting in increased KRAS signaling; high levels of RAS activity are found in about half of all breast cancer tumors and are associated with expression of epidermal growth factor and HER-2, yet RAS mutations are rare in these tumors. Herein described are methods for identifying subjects with elevated RAS activity as being more likely to respond favorably to treatment using an inhibitor of IGF-1 receptor signaling, and/or of treating such subjects with an inhibitor of IGF-1 receptor signaling.

It may be determined whether a subject has an activating KRAS mutation in at least some cells of at least one tumor, wherein the presence of the activating KRAS mutation indicates that the subject is more likely to respond to treatment of the tumor using an inhibitor of IGF-1 receptor signaling. The activating KRAS mutation can be any known in the art, for example, one affecting codon 10, 11, 12, 13, 15, 18, 22, 59, 61, and 63, such as G12C, G12D, G12E, and G12V. KRAS mutations are the most prevalent type of RAS mutations found in human tumors. Many tumor types are known to comprise activating KRAS mutations, including tumors of the pancreas (72-90% of which have an activating KRAS mutation), colon or rectum (32-57%), lung (15-50%), endometrium (5-50%), gallbladder (14-38%), and testes (9-12%), and multiple myeloma tumors (16-33%). Friday et al., 2005, Biochim Biophys Acta 1756:127-44. Described herein are methods and compositions for detecting KRAS mutations in at least some cells of a tumor in a subject, and/or treating the subject with an inhibitor of IGF-1 receptor signaling. The subject may have a tumor of the pancreas, colon, rectum, lung, endometrium, gallbladder, or testes, or a multiple myeloma tumors.

A tumor that has a wild-type allele of KRAS may be treated with an IGF-1 receptor inhibitor. The tumor may also be treated with an EGF receptor inhibitor, such as panitumumab or cetuximab. The tumor may have been previously treated with an EGF receptor inhibitor, such as panitumumab or cetuximab, and is now treated with both an EGF receptor inhibitor (either the same EGF receptor inhibitor previously used, or another) and an IGF-1 receptor inhibitor. The treated tumor may be colorectal tumor.

It may be determined whether some fraction of cells taken from a tumor in a subject has reduced PTEN activity, wherein reduced PTEN activity indicates that the tumor is less likely to respond to inhibition of IGF-1 receptor signaling. The reduction in PTEN activity can be detected using any suitable method. For example, expression levels can be detected using a method that detects PTEN RNA levels (*e.g.,* via a hybridization-based method such as Northern Blot or *in situ* hybridization), protein levels *(e.g.,* using a detecable PTEN-binding agent, such as a detectably labled anti-PTEN antibody), or PTEN enzymatic activity (*e.g*., by measuring PTEN activity directly or indirectly through its effects on other molecules, or by detecting mutations that cause a reduction of PTEN activity, such as partial or complete loss-of-function mutations in PTEN, for example PTEN D331G). *See, e.g.,* Teng et al., 1997, Cancer Res 57:5221-25; Bonneau et al., 2000, Human Mutation 16:109-22.

Herein described are compositions, kits, and methods for determining whether a patient is a good candidate for treatment with regimen that reduces IGF-1 and/or IGF-2-dependent signaling. It is understood in the art that it is desirable to determine whether a patient is a good candidate to begin a treatment regimen (or to continue with a treatment regimen already begun) as this allows the patient and medical professional to make better informed and more effective treatment decisions. It is particularly desirable to distinguish better from worse candidates for a treatment regimen for diseases that are aggressive or deadly, such as pancreatic cancer. The treatment regimen can target one or more molecules upstream or downstream of IGF-1 and or IGF-2, or target either or both of IGF-1 and IGF-2 themselves. Any such treatment known in the art or described herein can be used. The treatment may target IGF-1R or IGF-1R/insulin receptor hybrids. Examples of such treatments include small molecules (such as OSI-906, linsitinib, BMS-754807, INSM-18, XL228, AXL1717, BMS-536924, NVP-ADW742, GSK621659A, GSK1838705A, A-928605, AZD4253, TAE226, or AG1024) and anti-IGF-1R antibodies (such as ganitumab, AMG 479, figitumumab, CP-751,871, cixutumumab, IMC-A12, dalotuzumab, MK0646, RG1507, robatumumab, SCH 717454, AVE-1642a, MEDI-573, BIIB022, rhuMab IGFR, L1 HI, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, or L52H52, or fragments or derivatives thereof).

Also herein described is a method of determining whether a cancerous condition in a patient is likely to respond to a treatment that reduces signaling mediated by IGF-1 or IGF-2, comprising determining the concentration of a circulating biomarker in the patient's serum, wherein the amount of the circulating biomarker is predictive of a response to the treatment. Examples of such markers include total IGF-1, free IGF-1, total IGF-2, free IGF-2, IGFBP-1, IGFBP-2, IGFBP-3, IGFBP-4, IGFBP-5, IGFBP-6, and IGFBP-7. A patient may be a better candidate for treatment if the patient's IGFBP-1 and/or IGFBP-2 levels are lower than average for patients with the same condition. A patient may be a better candidate for treatment if the patient's total IGF-1, free IGF-1, total IGF-2, free IGF-2, IGFBP-3, and/or IGFBP-4 is higher than average for patients with the same condition.

Levels of two, three, four, or more of these markers may be determined. Taken together, these two or more markers are used as a composite marker. A composite marker can include, for example, the relative quantities of total IGF-1, free IGF-1, total IGF-2, free IGF-2, peptides that include the IGF-2 protein sequence, IGFBP-1, IGFBP-2, IGFBP-3, IGFBP-4, IGFBP-5, IGFBP-6 and/or IGFBP-7. Examples of specific composite markers include, for example, ratios or quantitative differences between IGF-1 and IGF-2, IGF-1 and IGFBP-1, IGF-1 and IGFBP-2, IGF-1 and IGFBP-3, IGF-1 and IGFBP-4, IGF-1 and IGFBP-5, IGF-1 and IGFBP-6, IGF-1 and IGFBP-7, IGF-2 and IGFBP-1, IGF-2 and IGFBP-2, IGF-2 and IGFBP-3, IGF-2 and IGFBP-4, IGF-2 and IGFBP-5, IGF-2 and IGFBP-6, IGF-2 and IGFBP-7, and between the various binding proteins e.g. IGFBP-2 and IGFBP-3.

Any method of determining the concentration of a circulating biomarker can be used. A detectable molecule that binds to the circulating biomarker may be used. Examples of such molecules include proteins, such as antibodies or fragments of antibodies, optionally coupled to a detectable moiety.

Examples of commercially available kits for detecting IGF-1 include the ELISA kits from Enzo Life Sciences (Farmingdale, NY), Diagnostic Systems Laboratories, Inc. (Webster, TX), Abnova Corporation (Taipei City, Taiwan), Antigenix America Inc. (Huntington Station, NY), ARP American Research Products, Inc. (Waltham, MA), BioVendor Laboratory Medicine, Inc. (Candler, NC), Cell Sciences (Canton, MA), DRG International, Inc. (Mountainside, NJ), EIAab & USCNLIFE(Wuhan EIAab Science Co.,Ltd) (Wuhan, China), GenWay Biotech, Inc. (San Diego, CA), IBL International GmbH (Hamburg, Germany), R&D Systems (Minneapolis, MN), and Raybiotech, Inc. (Norcross, GA).

Examples of commercially available kits for detecting IGF-2 include the ELISA kits from Antigenix America Inc. (Huntington Station, NY), BioVendor Laboratory Medicine, Inc. (Candler, NC), and Diagnostic Systems Laboratories, Inc. (Webster, TX).

Examples of commercially available kits for detecting IGFBP-1 include the ELISA kits from R&D Systems (Minnneapolis, MN), BioVendor Laboratory Medicine, Inc. (Candler, NC), Diagnostic Systems Laboratories, Inc. (Webster, TX), Cell Sciences (Canton, MA), and Raybiotech, Inc. (Norcross, GA).

Examples of commercially available kits for detecting IGFBP-2 include the ELISA kits from R&D Systems (Minnneapolis, MN), BioVendor Laboratory Medicine, Inc. (Candler, NC), Diagnostic Systems Laboratories, Inc. (Webster, TX), Cell Sciences (Canton, MA), and Raybiotech, Inc. (Norcross, GA).

Examples of commercially available kits for detecting IGFBP-3 include the ELISA kits from R&D Systems (Minnneapolis, MN), BioVendor Laboratory Medicine, Inc. (Candler, NC), Diagnostic Systems Laboratories, Inc. (Webster, TX), Cell Sciences (Canton, MA), and Raybiotech, Inc. (Norcross, GA), EIAab & USCNLIFE(Wuhan EIAab Science Co., Ltd) (Wuhan, China), Abnova Corporation (Taipei City, Taiwan), and Antigenix America Inc. (Huntington Station, NY).

Examples of commercially available kits for detecting IGFBP-4 include the ELISA kits from R&D Systems (Minnneapolis, MN), Cell Sciences (Canton, MA), Diagnostic Systems Laboratories, Inc. (Webster, TX), and Raybiotech, Inc. (Norcross, GA).

Examples of commercially available kits for detecting IGFBP-5 include the ELISA kits from R&D Systems (Minnneapolis, MN), Antigenix America Inc. (Huntington Station, NY), and Raybiotech, Inc. (Norcross, GA).

Examples of commercially available kits for detecting IGFBP-6 include the ELISA kits from R&D Systems (Minnneapolis, MN) and Raybiotech, Inc. (Norcross, GA).

Herein described are compositions, kits, and methods for determining whether to modulate or discontinue treatment with regimen that reduces IGF-1 and/or IGF-2-dependent signaling. The treatment regimen can target one or more molecules upstream or downstream of IGF-1 and or IGF-2, or target either or both of IGF-1 and IGF-2 themselves. Any such treatment known in the art or described herein can be used. The treatment may target IGF-1R and/or IGF-1R/insulin receptor hybrids (Soos et al., 1993, Biochem J 290:419-26; Benyoucef et al., 2007, Biochem J 403:603-13). Examples of such treatments include small molecules (such as OSI-906, linsitinib, BMS-754807, INSM-18, XL228, AXL1717, BMS-536924, NVP-ADW742, GSK621659A, GSK1838705A, A-928605, AZD4253, TAE226, or AG1024) and anti-IGF-1R antibodies (such as ganitumab, AMG 479, figitumumab, CP-751,871, cixutumumab, IMC-A12, dalotuzumab, MK0646, RG1507, robatumumab, SCH 717454, AVE-1642a, MEDI-573, BIIB022, rhuMab IGFR, L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, or L52H52, or fragments or derivatives thereof).

The patient's exposure to the treatment may be determined. If the patient's exposure is below the mean exposure for patient's receiving the same treatment regimen for the same cancerous condition, the treatment may be discontinued. If the patient's exposure is below the mean exposure for patient's receiving the same treatment regimen for the same cancerous condition, the dose or frequency of the treatment is increased.

The foregoing methods can be combined with other biomarkers, patient stratification methods, and efficacy-monitoring methods relating to IGF-1 and/or IGF-2-mediated signaling inhibition, for example, those described in US 2010/0184125, US 2010/0316639, US 2009/0092596, US 2009/093488, WO 2010/146059, WO 2009/079587, EP 1 828 249, EP 2 241 634, WO 2010/119126, US 2010/0240665, WO 2010/022268, WO 2010/048123, WO 2010/048123, WO 2010/138908, WO 2007/035744, WO 2008/115470, WO 2008/144345, WO 2011/053779, WO 2012/006681, US Pat. No. 7,129,040, US Pat No. 7,217,795, WO 2011/087868, WO 2009/106566, WO 2011/133668, US 2009/0258365, WO 2011/066200, EP 1 926 996, US 2011/0217309, US 2011/0275644, US Pat. No. 7,939,272, US Pat. No. 8,048,621, US Pat. No. 8,062,838, WO 2011/109572, WO 2011/109584, WO 2011/083391, US 2011/0060605, WO 2011/031861, EP 1 828 249, EP 2 032 989, EP 2 281 841, EP2 283 831, WO 2009/079587, WO 2012/000763, US 2011/0052667, US 2011/0262453, and WO 2009/120767.

The compositions and/or methods described herein also can be used, for example, in cosmetic treatments, in veterinary treatments, to increase longevity, to treat reproductive defects, and to treat a variety of growth related disorders.

### Therapeutic methods

Certain methods described herein comprise administering to a subject an inhibitor of IGF-1 R-mediated signaling. Any treatment that results in a reduction of an activity or signal mediated by IGF-1 R can be used. Examples of such treatments are provided in Sachdev et al., 2007, Mol Cancer Ther. 6:1-12. The treatment may comprise administering to the subject a substance that reduces an activity mediated by IGF-1R. Examples of such substances include, but are not limited to, antibodies (including fragments and derivatives thereof), peptibodies, and AVIMERS™ (Amgen, Inc., Thousand Oaks, CA) that bind to IGF-1R, IGF-1, or IGF-2, soluble, IGF-1- and/or IGF-2-binding derivatives of IGF-1R, small molecules that bind to IGF-1R, IGF-1, IGF-2, IRS1, SHC, GRB2, SOS1, PI3K, SHP2, or any other molecule that acts in the IGF-1R signaling cascade, IGF-1 or IGF-2 binding proteins (and derivatives thereof), inhibitory nucleic acids (such as siRNA) and derivatives thereof (including peptide nucleic acids). Non-limiting examples of such molecules can be found in, for example, US Pat. No. 7,329,7347 (published February 12, 2008),173,005 (issued February 6, 2007), 7,071,300 (issued July 4, 2006), 7,020,563 (issued March 28, 2006), 6875741 (issued April 5, 2005); US Pat. App. Pub. No. 07/0299010 (published December 27, 2007), 07/0265189 (published November 15, 2007), 07/0135340 (published June 14, 2007), 07/0129399 (published June 7, 2007), 07/0004634 A1 (published January 4, 2007), 05/0282761 A1 (published December 22, 2005), 05/0054638 A1 (published March 10, 2005), 04/0023887 A1 (published February 5, 2004), 03/0236190 A1 (published December 25, 2003), 03/0195147 A1 (published October 16, 2003); PCT Pub. No. WO 07/099171 (published September 7, 2007), WO 07/099166 (published September 7, 2007), 07/031745 (published March 22, 2007), WO 07/029106 (published March 15, 2007), WO 07/029107 (published March 15, 2007), WO 07/004060 (published January 11, 2007), WO 06/074057 A2 (published July 13, 2006), WO 06/069202 A2 (published June 29, 2006), WO 06/017443 A2 (published February 16, 2006), WO 06/012422 A1 (published February 2, 2006), WO 06/009962 A2 (published January 26, 2006), WO 06/009950 A2 (published January 26, 2006), WO 06/009947 A2 (published January 26, 2006), WO 06/009933 A2 (published January 26, 2006), WO 05/097800 A1 (October 20, 2005), WO 05/082415 A2 (published September 9, 2005), WO 05/037836 A2 (published April 28, 2005), WO 03/070911 A2 (published August 28, 2003), WO 99/28347 A2 (published June 10, 1999); European Pat. No. EP 1 732 898 B1 (published January 23, 2008), EP 0 737 248 B1 (published November 14, 2007), European Pat. App. No. EP 1 496 935 A2 (published January 19, 2005) and EP 1 432 433 A2 (published June 30, 2004), and D'ambrosio et al., 1996, Cancer Res. 56:4013-20. Specific examples of such molecules include OSI-906 (OSI Pharmaceuticals, Melvilee, NY), BMS 536924 (Wittman et al., 2005, J Med Chem. 48:5639-43; Bristol Myers Squibb, New York, NY), XL228 (Exelexis, South San Francisco, CA), INSM-18, NDGA, and rhIGFBP-3 (Insmed, Inc., Richmond, VA; Breuhahn et al, 2002006, Curr Cancer Ther Rev. 2:157-67; Youngren et al., 2005, Breast Cancer Res Treatment 94:37-46; US Pat. No. 6,608,108).

Any suitable anti-IGF-1R antibody, antibody fragment, or antibody derivative can be used in the methods described herein. The antibody, antibody fragment, or antibody derivative may bind to the extracellular domain of IGF-1R, or compete for binding to IGF-R with IGF-1 and/or IGF-2. The antibody, antibody fragment, or antibody derivative, when bound to IGF-1R, may reduce the amount of IGF-1 and/or IGF-2 that binds to the IGF-1R. The antibody, antibody fragment, or antibody derivative may bind to the L1 subdomain of the IGF-1R extracellular domain. The antibody, antibody fragment, or antibody derivative may bind to the CR subdomain of the IGF-1 R extracellular domain, bind to the L2 subdomain of the IGF-1R extracellular domain, bind to the FnIII1 subdomain of the IGF-1R extracellular domain, bind to the FnIII2-ID subdomain of the IGF-1R extracellular domain, or bind to the FnIII subdomain of the IGF-1R extracellular domain. (The IGF-1R extracellular subdomains are defined in Example 12, below.) The antibody, antibody fragment, or antibody derivative may bind to more than one IGF-1R extracellular domain. Non-limiting examples of anti-IGF-1R antibodies that can be used in the methods described herein include each of the antibodies identified herein as L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20, H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, and L52H52, and IGF-1R-binding fragments and derivatives thereof. Other non-limiting examples of anti-IGF-1R antibodies for use in the methods described herein include those described in US Pat. App. Pub. No. 06/0040358 (published February 23, 2006), 05/0008642 (published January 13, 2005), 04/0228859 (published November 18, 2004), e.g., antibody 1A (DSMZ Deposit No. DSM ACC 2586), antibody 8 (DSMZ Deposit No. DSM ACC 2589), antibody 23 (DSMZ Deposit No. DSM ACC 2588) and antibody 18 as described therein; PCT Pub. No. WO 06/138729 (published December 28, 2006), WO 05/016970 (published February 24, 2005), and Lu et al., 2004, J Biol Chem. 279:2856-65, *e.g.,* antibodies 2F8, A12, and IMC-A12 as described therein; PCT Pub. No. WO 07/012614 (published February 1, 2007), WO 07/000328 (published January 4, 2007), WO 06/013472 (published February 9, 2006), 05/058967 (published June 30, 2005), 03/059951 (published July 24, 2003), US Pat. App. Pub. No. 05/0084906 (published April 21, 2005), e.g., antibody 7C10, chimaeric antibody C7C10, antibody h7C10, antibody 7H2M, chimaeric antibody *7C10, antibody GM 607, humanized antibody 7C10 version 1, humanized antibody 7C10 version 2, humanized antibody 7C10 version 3, and antibody 7H2HM, as described therein; US Pat. App. Pub. No. 05/0249728 (published November 10, 2005), 05/0186203 (published August 25, 2005), 04/0265307 (published December 30, 2004), 03/0235582 (published December 25, 2003), Maloney et al., 2003, Cancer Res. 63:5073-83, *e.g.,* antibody EM164, resurfaced EM164, humanized EM164, huEM164 v1.0, huEM164 vl.1, huEM164 vl.2, and huEM164 vl.3, as described therein; US Pat. No. 7,037,498 (issued May 2, 2006), US Pat. App. No. 05/0244408 (published November 30, 2005), 04/0086503 (published May 6, 2004), Cohen, et al., 2005, Clinical Cancer Res. 11:2063-73, *e.g.,* antibody CP-751,871, each of the antibodies produced by the hybridomas having the ATCC accession numbers PTA-2792, PTA-2788, PTA-2790, PTA-2791, PTA-2789, PTA-2793, and antibodies 2.12.1, 2.13.2, 2.14.3, 3.1.1, 4.9.2, and 4.17.3, as described therein; US Pat. App. No. 05/0136063 (published June 23, 2005), 04/0018191 (published January 29, 2004), e.g. antibody 19D12 and an antibody comprising a heavy chain encoded by a polynucleotide in plasmid 15H12/19D12 HCA (γ4), deposited at the ATCC under number PTA-5214, and a light chain encoded by a polynucleotide in plasmid 15H12/19D12 LCF (κ), deposited at the ATCC under number PTA-5220, as described therein; US Pat. App. No. 04/0202655 (published October 14, 2004), e.g., antibodies PINT-6A1, PINT-7A2, PINT-7A4, PINT-7A5, PINT-7A6, PINT-8A1, PINT-9A2, PINT-11A1, PINT-11A2, PINT-11A3, PINT-11A4, PINT-11A5, PINT-11A7, PINT-11A12, PINT-12A1, PINT-12A2, PINT-12A3, PINT-12A4, and PINT-12A5, as described therein; US Pat. App. No. 07/0243194 (published October 18, 2007), e.g., antibodies M13-C06, M14-G11, M14-C03, M14-B01, M12-E01, and M12-G04, and antibodies produced by hybridomas P2A7.3E11, 20C8.3B8, P1A2.2B11, 20D8.24B11, P1E2.3B12, and P1G10.2B8. Also suitable for use are antibodies, antibody fragments, or antibody derivatives that compete for binding to IGF-1 receptor with one of the aforementioned antibodies. The antibody, antibody fragment, or antibody derivative may bind to the same epitope as one of the aforementioned antibodies, or to an epitope that overlaps with the epitope of one of the aforementioned antibodies.

Methods described herein may involve contacting endogenous IGF-1R with an IGF-1R binding antigen binding protein, *e.g*., via administration to a subject or in an *ex vivo* procedure.

The term "treatment" encompasses alleviation or prevention of at least one symptom or other aspect of a disorder, or reduction of disease severity, and the like. A treatment need not effect a complete cure, or eradicate every symptom or manifestation of a disease, to constitute a viable therapy. As is recognized in the pertinent field, drugs or other treatments employed as therapeutic agents may reduce the severity of a given disease state, but need not abolish every manifestation of the disease to be regarded as therapeutically useful. Similarly, a prophylactically administered treatment need not be completely effective in preventing the onset of a condition in order to constitute a viable prophylactic agent. Simply reducing the impact of a disease (for example, by reducing the number or severity of its symptoms, by delaying the onset of the condition, by accelerating the reduction of symptoms, by increasing the effectiveness of another treatment, or by producing another beneficial effect), or reducing the likelihood that the disease will occur or worsen in a subject, is sufficient. Therapeutically useful treatments also include treatments that are effective in some patients, but not in others. A method may comprise administering to a patient an IGF-1R antagonist in an amount and for a time sufficient to induce a sustained improvement over baseline of an indicator that reflects the severity of the particular disorder.

The progress of a course of treatment can be monitored or measured using any suitable technique. For treating a tumor, such techniques include detecting the size, or change in size, of the tumor. The size of the tumor can be measured by its length, circumference, volume, *etc.,* as determined or estimated using any suitable technique, including direct observation, radiological techniques, and the like. Progress of the treatment may be monitored using the RECIST techniques and criteria (Therasse et al. 2000, J Natl Cancer Inst. 92:205-16). Progress of the treatment can also be monitored in other ways, for example, by determining the relative health or vigor of the tumor tissue, *e.g*., by measuring the tumor's uptake of glucose using a PET scan, or by monitoring an aspect of the tumor that is correlated with the health or vigor of the tumor tissue, or with the effectiveness of the treatment. Examples of such aspects of the tumor include expression levels of particular genes or proteins, phosphorylation states or other post-translational modifications of particular proteins, and the like.

As is understood in the pertinent field, pharmaceutical compositions comprising the molecules described herein are administered to a subject in a manner appropriate to the indication. Pharmaceutical compositions may be administered by any suitable technique, including but not limited to parenterally, topically, or by inhalation. If injected, the pharmaceutical composition can be administered, for example, via intra-articular, intravenous, intramuscular, intralesional, intraperitoneal or subcutaneous routes, by bolus injection, or continuous infusion. Localized administration, *e.g*. at a site of disease or injury is contemplated, as are transdermal delivery and sustained release from implants. Delivery by inhalation includes, for example, nasal or oral inhalation, use of a nebulizer, inhalation of the antagonist in aerosol form, and the like. Other alternatives include eyedrops; oral preparations including pills, syrups, lozenges or chewing gum; and topical preparations such as lotions, gels, sprays, and ointments.

Use of pharmaceutical compositions in *ex vivo* procedures also is possible. For example, a patient's blood or other bodily fluid may be contacted with an inhibitor of IGF-1 R signaling *ex vivo.* The inhibitor may be bound to a suitable insoluble matrix or solid support material.

IGF-1R signaling inhibitors described herein can be administered in the form of a composition comprising one or more additional components such as a physiologically acceptable carrier, excipient or diluent. Optionally, the composition additionally comprises one or more physiologically active agents, for example, a second IGF-1 R signaling inhibitor, an anti-angiogenic substance, a chemotherapeutic substance, an analgesic substance, *etc.* The composition may comprise one, two, three, four, five, or six physiologically active agents in addition to an IGF-1R binding antigen binding protein

The pharmaceutical composition may comprise an inhibitor of IGF-1R signaling together with one or more substances selected from the group consisting of a buffer, an antioxidant such as ascorbic acid, a low molecular weight polypeptide (such as those having fewer than 10 amino acids), a protein, an amino acid, a carbohydrate such as glucose, sucrose or dextrins, a chelating agent such as EDTA, glutathione, a stabilizer, and an excipient. Neutral buffered saline or saline mixed with conspecific serum albumin are examples of appropriate diluents. In accordance with appropriate industry standards, preservatives such as benzyl alcohol may also be added. The composition may be formulated as a lyophilizate using appropriate excipient solutions (*e.g*., sucrose) as diluents. Suitable components are nontoxic to recipients at the dosages and concentrations employed. Further examples of components that may be employed in pharmaceutical formulations are presented in Remington's Pharmaceutical Sciences, 16th Ed. (1980) and 20th Ed. (2000), Mack Publishing Company, Easton, PA.

Kits for use by medical practitioners include an IGF-1 receptor-inhibiting substance and a label or other instructions for use in treating any of the conditions discussed herein. The kit may include a sterile preparation of one or more inhibitors of IGF-1R signaling, which may be in the form of a composition as disclosed above, and may be in one or more vials.

Dosages and the frequency of administration may vary according to such factors as the route of administration, the particular antigen binding proteins employed, the nature and severity of the disease to be treated, whether the condition is acute or chronic, and the size and general condition of the subject. Appropriate dosages can be determined by procedures known in the pertinent art, *e.g.* in clinical trials that may involve dose escalation studies. "Intermittent dosing" refers to methods of administering to a subject a therapeutic compound (for example, an inhibitor of IGF-1R signaling) in multiple doses, wherein there is an interval of time between administration of a particular dose and any subsequent dose. Any schedule of dosing can be used so long as it is therapeutically effective or otherwise medically justified. The interval between consecutive doses can be very short, on the order of seconds or minutes, or longer, on the order of hours, days, weeks, months, or even years. The interval can be the same between every dose, for example, one dose per week or month, or it can vary from dose to dose. Likewise, the amount of the therapeutically active compound (e.g., an inhibitor of IGF-1R signaling or chemotherapeutic agent) can vary from dose to dose. The period between consecutive doses and the amount of a therapeutically active substance in each dose are selected to keep a pharmacodynamic or pharmacokinetic parameter of interest (for example, serum concentration of said substance or percent reduction in IGF-1R signaling activity) within a desired range. The interval between doses and the amount of therapeutically active substance may vary according to other criteria (for example, subject's objective or subjective response to the course of treatment).

The IGF-1R signal inhibiting substance described herein is administered over a period of at least a month or more, *e.g.,* for one, two, or three months, six months, a year, for several years, or even indefinitely. For treating chronic conditions, long-term treatment is generally most effective. However, for treating acute conditions, administration for shorter periods, e.g. from one to six weeks, may be sufficient. In general, the IGF-1R signal inhibiting substance is administered until the patient manifests a medically relevant or desirable degree of improvement over baseline for the chosen indicator or indicators.

An IGF-1R inhibiting substance may be administered at a dosage of from about 1 ng of antigen binding protein per kg of subject's mass per dose ("1ng/kg/dose") to about 50 mg/kg/dose, more preferably from about 1 mg/kg/dose to about 30 mg/kg/dose, e.g.. from about 10 mg/kg/dose to about 20 mg/kg/dose, to a subject. The IGF-1R inhibiting substance may be administered to adults one time per month, once every two weeks, once per week, two times per week, or three or more times per week, to treat an IGF-1 and/or IGF-2 mediated disease, condition or disorder, *e.g*., a medical disorder disclosed herein. If injected, the effective amount of IGF-1R inhibiting substance per adult dose may range from 1-20 mg/m², and preferably is about 5-12 mg/m². Alternatively, a flat dose may be administered; the amount may range from 5-100 mg/dose. One range for a flat dose is about 20-30 mg per dose. A flat dose of 25 mg/dose may be repeatedly administered by injection. If a route of administration other than injection is used, the dose is appropriately adjusted in accordance with standard medical practices. One example of a therapeutic regimen involves injecting a dose of about 20-30 mg of IGF-1R inhibiting substance from one to three times per week over a period of at least three weeks, though treatment for longer periods may be necessary to induce the desired degree of improvement. For pediatric subjects (age 4-17), one exemplary suitable regimen involves the subcutaneous injection of 0.4 mg/kg, up to a maximum dose of 25 mg of IGF-1R inhibiting substance administered two or three times per week.

Subcutaneous injection of from 0.5 mg to 500 mg, e.g., from 50 to 300 mg, of an antigen binding protein may be administered once or twice per week. Alternative pulmonary administration (*e.g.*, by nebulizer) of 3 or more mg of IGF-1R inhibiting substance may be given.

Other examples of therapeutic regimens comprise subcutaneous or intravenous administration of a dose of 1, 3, 5, 6, 7, 8, 9, 10, 11, 12, 15, 20, 25, 30, 35, 40, 45, 50, 75, 100, 125, 150, 175, 200, 250, 300, 400, or 500 milligrams of an IGF-1R inhibitor per kilogram body mass of the subject (mg/kg). The dose can be administered once to the subject, or more than once at a certain interval, for example, once a day, three times a week, twice a week, once a week, three times a month, twice a month, once a month, once every two months, once every three months, once every six months, or once a year. The duration of the treatment, and any changes to the dose and/or frequency of treatment, can be altered or varied during the course of treatment in order to meet the particular needs of the subject.

An antigen binding protein may be administered to the subject in an amount and for a time sufficient to induce an improvement, preferably a sustained improvement, in at least one indicator that reflects the severity of the disorder that is being treated. Various indicators that reflect the extent of the subject's illness, disease or condition may be assessed for determining whether the amount and time of the treatment is sufficient. Such indicators include, for example, clinically recognized indicators of disease severity, symptoms, or manifestations of the disorder in question. An improvement may be considered to be sustained if the subject exhibits the improvement on at least two occasions separated by two to four weeks. The degree of improvement generally is determined by a physician, who may make this determination based on signs, symptoms, biopsies, or other test results, and who may also employ questionnaires that are administered to the subject, such as quality-of-life questionnaires developed for a given disease. An improvement in a subject's condition can be one that is, for example, detected, measured, or quantified by a physician or other health care provider using any appropriate technique. Such techniques include, but are not limited to, observing the subject, testing the subject or a sample taken from the subject, and collecting from the subject, directly or indirectly, the subject's impressions of the subject's condition. Such impressions can relate to any aspect of the subject's health or well-being, particularly those aspects that are affected directly or indirectly by subject's tumor disease. Examples of such aspects include, but are not limited to, pain, discomfort, sleep, appetite, thirst, mobility, strength, flexibility, and mental state.

Elevated levels of IGF-1 and/or IGF-2 are associated with a number of disorders, including, for example, cancer (e.g., lung, prostate, breast and colon cancers), and acromegaly and other overgrowth disorders (e.g., constitutionally tall children). Subjects with a given disorder may be screened, to identify those individuals who have elevated IGF-1 and/or IGF-2 levels, thereby identifying the subjects who may benefit most from treatment with an IGF-1R signaling inhibitor. Thus, treatment methods described herein optionally comprise a first step of measuring a subject's IGF-1 and/or IGF-2 levels. An antigen binding protein may be administered to a subject in whom IGF-1 and/or IGF-2 levels are elevated above a normal or a desirable level.

A subject's levels of IGF-1 and/or IGF-2 may be monitored before, during and/or after treatment with an antigen binding protein, to detect changes, if any, in their levels. For some disorders, the incidence of elevated IGF-1 and/or IGF-2 levels may vary according to such factors as the stage of the disease or the particular form of the disease. Known techniques may be employed for measuring IGF-1 and/or IGF-2 levels, *e.g.,* in a subject's serum. IGF-1 and/or IGF-2 levels in blood samples may be measured using any suitable technique, for example, ELISA.

The use of an antigen binding protein and one or more additional IGF-1R antagonists may be involved for example, two or more antigen binding proteins, or an antigen binding protein and one or more other IGF-1R antagonists. Antigen binding protein may be administered alone or in combination with other agents useful for treating the condition with which the patient is afflicted. Examples of such agents include both proteinaceous and non-proteinaceous drugs. When multiple therapeutics are co-administered, dosages may be adjusted accordingly, as is recognized in the pertinent art. "Coadministration" and combination therapy are not limited to simultaneous administration, but also include treatment regimens in which an antigen binding protein is administered at least once during a course of treatment that involves administering at least one other therapeutic agent to the patient.

Examples of other agents that may be co-administered with an antigen binding protein are other antigen binding proteins or therapeutic polypeptides that are chosen according to the particular condition to be treated. Alternatively, non-proteinaceous drugs that are useful in treating one of the particular conditions discussed above may be co-administered with an IGF-1R antagonist.

### Combination therapy

Herein described is a method of treating a subject with an IGF-1R inhibiting antigen binding protein and one or more other treatments. Such a combination therapy may achieve synergy or an additive effect by, for example, attacking multiple sites or molecular targets in a tumor. Types of combination therapies that can be used include inhibiting or activating (as appropriate) multiple nodes in a single disease-related pathway, multiple pathways in a target cell, and multiple cell types within a target tissue (e.g., within a tumor). For example, an IGF-1R inhibitor can be combined with a treatment that inhibits IGF-1, promotes apoptosis, inhibits angiogenesis, or inhibits macrophage. A targeted agent, that, when used by itself, fails to elicit a therapeutically desired effect, could be used to, for example, sensitize cancer cells or augment treatment effect of other agents. An IGF-1R inhibitor may be used in combination with a cytotoxic drug or other targeted agent that induces apoptosis. An IGF-1R inhibitor may be used in combination with one or more agents that inhibit different targets that are involved in cell survival (*e.g*., PKB, mTOR), different receptor tyrosine kinases (*e.g*., ErbB1, ErbB2, c-Met, c-kit), or different cell types (*e.g*., KDR inhibitors, c-fms). An IGF-1R inhibitor is added to the existing standard of care for a particular condition. Examples of therapeutic agents include, but are not limited to, gemcitabine, taxol, taxotere, and CPT-11.

A combination therapy method may comprise administering to the subject two, three, four, five, six or more of the IGF-1R agonists or antagonists described herein. The method may comprise administering to the subject two or more treatments that together inhibit or activate (directly or indirectly) IGF-1R-mediated signal transduction. Examples of such methods include using combinations of two or more IGF-1 R inhibiting antigen binding progeins, of an IGF-1 R inhibiting antigen binding protein and one or more other IGF-1, IGF-2, and/or IGF-1R agonists or antagonists (*e.g.*, IGF-1 and/or IGF-2 binding polypeptides, IGF-1R binding polypeptides, IGF-1 and/or IGF-2 derivatives, anti-IGF-1 and/or IGF-2 antibodies, anti-sense nucleic acids against IGF-1, IGF-2, and/or IGF-1R, or other molecules that bind to IGF-1, IGF-2, and/or IGF-1R polypeptides or nucleic acids), or of an IGF-1R inhibiting antigen binding protein and one or more other treatments (*e.g*., surgery, ultrasound, radiotherapy, chemotherapy, or treatment with another anti-cancer agent), as described, for example, in US Pat. No. 5,473,054 (issued Dec. 5, 1995), 6,051,593 (issued April 18, 2000), 6,084,085 (issued July 4, 2000), 6,506,763 (issued Jan. 14, 2003), US Pat. App. Pub. No.s 03/0092631 (published May 15, 2003), 03/0165502 (published Sept. 4, 2003), 03/0235582 (published Dec. 25, 2003), 04/0886503 (published May 6, 2004), 05/0272637 (published Dec. 8, 2005), PCT Pub. Ser. No.s WO 99/60023 (published Nov. 25, 1999), WO 02/053596 (published July 11, 2002), WO 02/072780 (published Sept. 19, 2002), WO 03/027246 (published March 3, 2003), WO 03/020698 (published March 13, 2003), WO 03/059951 (published July 24, 2003), WO 03/100008 (published Dec. 4, 2003), WO 03/106621 (published Dec. 24, 2003), WO 04/071529 (published August 26, 2004), WO 04/083248 (published Sept. 30, 2004), WO 04/087756 (published Oct. 14, 2004), WO 05/112969 (published Dec. 1, 2005), Kull et al., 1983, J Biol Chem 258:6561-66, Flier et al., 1986, Proc Natl Acad Sci USA 83:664-668, Conover et al., 1987, J Cell Physiol 133:560-66, Rohlik et al., 1987, Biochem Biophys Res Comm 149:276-81, Arteaga et al., 1989, J Clinical Investigation 84:1418-23, Arteaga et al., 1989, Cancer Res 49:6237-41, Gansler et al., 1989, American J Pathol 135:961-66, Gustafson et al., 1990, J Biol Chem 265:18663-67, Steele-Perkins et al., 1990, Biochem Biophys Res Comm 171:1244-51, Cullen et al., 1992, Mol Endocrinol 6:91-100, Soos et al., 1992, J Biol Chem 267:12955-63, Xiong et al., 1992, Proc Natl Acad Sci USA 89:5356-60, Brunner et al., 1993, Euro J Cancer 29A:562-69, Furlanetto et al., 1993, Cancer Res 53:2522-26, Li et al., 1993, Biochem Biophys Res Comm 196:92-98, Kalebic et al., 1994, Cancer Res 54:5531-34, Lahm et al., 1994, Intl J Cancer 58:452-59, Zia et al., 1996, J Cell Biochem Supp 24:269-75, Jansson et al., 1997, J Biol Chem 272:8189-97, Scotlandi et al., 1998, Cancer Res 58:4127-31, Logie *et al.,* 1999, Li et al., 2000, Cancer Immunol Immunotherapy 49:243-52, J Mol Endocrinol 23:23-32, De Meyts et al., 2002, Nature Reviews 1:769-83, Hailey et al., 2002, Mol Cancer Therapeutics 1:1349-53, Maloney et al., 2003, Cancer Research 63:5073-83, Burtrum et al., 2003, Cancer Research 63:8912-21, and Karavitaki et al., 2004, Hormones 3:27-36, may be employed in methods and compositions described herein. Furthermore, one or more anti-IGF-1R antibodies or antibody derivatives can be used in combination with one or more molecules or other treatments, wherein the other molecule(s) and/or treatment(s) do not directly bind to or affect IGF-1R, IGF-1, or IGF-2, but which combination is effective for treating or preventing a condition, such as cancer or an overgrowth disorder (*e.g*., acromegaly). One or more of the molecule(s) and/or treatment(s) may treat or prevent a condition that is caused by one or more of the other molecule(s) or treatment(s) in the course of therapy, *e.g*., nausea, fatigue, alopecia, cachexia, insomnia, *etc.* In every case where a combination of molecules and/or other treatments is used, the individual molecule(s) and/or treatment(s) can be administered in any order, over any length of time, which is effective, *e.g*., simultaneously, consecutively, or alternately. The method of treatment may comprise completing a first course of treatment with one molecule or other treatment before beginning a second course of treatment. The length of time between the end of the first course of treatment and beginning of the second course of treatment can be any length of time that allows the total course of therapy to be effective, *e.g*., seconds, minutes, hours, days, weeks, months, or even years.

The method may comprise administering one or more of the IGF-1R antagonists described herein and one or more other treatments (*e.g*., a therapeutic or palliative treatment), for example, anti-cancer treatments (such as surgery, ultrasound, radiotherapy, chemotherapy, or treatment with another anti-cancer agent). Where a method comprises administering more than one treatment to a subject, it is to be understood that the order, timing, number, concentration, and volume of the administrations is limited only by the medical requirements and limitations of the treatment, *i.e.,* two treatments can be administered to the subject, *e.g*., simultaneously, consecutively, alternately, or according to any other regimen. Examples of agents that can be administered in combination with the IGF-1R antagonists described herein include, but are not limited to, neutrophil-boosting agents, irinothecan, SN-38, gemcitabine, herstatin, or an IGF-1R-binding herstatin derivative (as described, for example, in US Pat. App. No. 05/0272637), AVASTIN® (Genentech, South San Francisco, CA), HERCEPTIN® (Genentech), RITUXAN® (Genentech), ARIMIDEX® (AstraZeneca, Wilmington, DE), IRESSA® (AstraZeneca), BEXXAR® (Corixa, Seattle, WA), ZEVALIN® (Biogen Idec, Cambridge, MA), ERBITUX® (Imclone Systems Inc., New York, NY), GEMZAR® (Eli Lilly and Co., Indianapolis, IN), CAMPTOSAR® (Pfizer, New York, NY), GLEEVEC® (Novartis), SU-11248 (Pfizer), BMS-354825 (Bristol-Myers Squibb), VECTIBIX™ (Abgenix, Fremont, CA/Amgen Inc., Thousand Oaks, CA), and denosumab (Amgen Inc., Thousand Oaks, CA).

Herein described is a combination therapy for treating a tumor disease comprising administering to a subject an inhibitor of IGF-1 receptor signaling before, during, or after treatment of the subject with an inhibitor of RAS signaling, *e.g.,* an inhibitor of KRAS, NRAS, or HRAS. Any inhibitor of RAS activity can be used. Examples of types of RAS inhibitors include antisense oligonucleotides, RNA interference, inhibition of RAS post-translational modification or processing (e.g., farnesyltransferase inhibitors (FTIs), such as CAAX peptidomimetics like FTI-276 and FTI-277, and non-peptidomimetics like tipifarnib (R115777), lonafarnib (SCH663366), and BMS-214662)), geranylgeranyltransferase inhibitors (GGTIs), combination FTI/GGTIs, inhibitors of RAS proteolytic cleavage, methylation, or palmitoylation, immunological approaches (*e.g*., vaccination against an activated RAS mutant), mutant RAS peptide inhibitors, and inhibitors of downstream RAS effectors such as Raf kinase (*e.g.,* BAY 43-9006), MEK (*e.g.,* CI-1040, PD0325901, and ARRY-142886), and mTOR (*e.g*., rapamycin, CCI-779, RAD001, and . AP23573). See Friday et al., 2005, Biochim Biophys Acta 1756:127-44.

The following examples, both actual and prophetic, are provided for the purpose of illustrating specific embodiments or features of the instant invention and do not limit its scope.

### EXAMPLE 1: Preparation of Antibodies

This example demonstrates a method of preparing antibodies recognizing the IGF-1 receptor.
IGF-1 receptor polypeptides may be employed as immunogens in generating monoclonal antibodies by conventional techniques. It is recognized that polypeptides in various forms may be employed as immunogens, e.g., full length proteins, fragments thereof, fusion proteins thereof such as Fc fusions, cells expressing the recombinant protein on the cell surface, *etc.*

To summarize an example of such a procedure, an IGF-1R immunogen emulsified in complete Freund's adjuvant is injected subcutaneously into Lewis rats, in amounts ranging from 10-100 µl. Three weeks later, the immunized animals are boosted with additional immunogen emulsified in incomplete Freund's adjuvant and boosted every three weeks thereafter. Serum samples are periodically taken by retro-orbital bleeding or tail-tip excision for testing by dot-blot assay, ELISA (enzyme-linked immunosorbent assay), or inhibition of binding of ¹²⁵I-IGF-1 or ¹²⁵I-IGF-2 to extracts of IGF-1R-expressing cells. Following detection of an appropriate antibody titer, positive animals are given a final intravenous injection of antigen in saline. Three to four days later, the animals are sacrificed, splenocytes harvested, and fused to the murine myeloma cell line AG8653. The resulting hybridoma cell lines are plated in multiple microtiter plates in a HAT selective medium (hypoxanthine, aminopterin, and thymidine) to inhibit proliferation of non-fused cells, myeloma hybrids, and spleen cell hybrids.

Hybridoma clones thus generated are screened for reactivity with IGF-1R. Initial screening of hybridoma supernatants utilizes an antibody capture and binding of partially purified ¹²⁵I-IGF-1 receptor. Hybridomas that are positive in this screening method are tested by a modified antibody capture to detect hybridoma cells lines that are producing blocking antibody. Hybridomas that secrete a monoclonal antibody capable of inhibiting ¹²⁵I-IGF-1 binding to cells expressing IGF-1R are thus detected. Such hydridomas then are injected into the peritoneal cavities of nude mice to produce ascites containing high concentrations (>1 mg/ml) of anti-IGF-I R monoclonal antibody. The resulting monoclonal antibodies may be purified by ammonium sulfate precipitation followed by gel exclusion chromatography, and/or affinity chromatography based on binding of antibody to Protein G.

Similar methods can be used to generate human antibodies in transgenic mice. See, *e.g.,* Chen et al., 1993, Internat. Immunol. 5: 647-56; Chen et al., 1993, EMBO J. 12: 821-30; Choi et al., 1993, Nature Genetics 4: 117-23; Fishwild et al., 1996, Nature Biotech. 14: 845-51; Harding et al., 1995, Annals New York Acad. Sci.; Lonberg et al., 1994, Nature 368: 856-59; Lonberg, 1994, Handbook Exper.1 Pharmacol. 113: 49-101; Lonberg et al., 1995, Internal Rev. Immunol. 13: 65-93; Morrison, 1994, Nature 368: 812-13; Neuberger, 1996, Nature Biotech. 14: 826; Taylor et al., 1992, Nuc. Acids Res. 20: 6287-95; Taylor et al., 1994, Internat. Immunol. 6: 579-91; Tomizuka et al., 1997, Nature Genetics 16: 133-43; Tomizuka et al., 2000, Proc. Nat. Acad. Sci. USA 97: 722-27; Tuaillon et al., 1993, Proc. Nat. Acad. Sci. USA 90: 3720-24; Tuaillon et al., 1994, J. Immunol. 152: 2912-20; Russel et al., 2000, Infection and Immunity April 2000: 1820-26; Gallo et al., 2000, Eur. J. Immunol. 30: 534-40; Davis et al., 1999, Cancer Metastasis Rev. 18:421-25; Green, 1999, J. Immunol. Methods 231:11-23; Jakobovits, 1998, Advanced Drug Delivery Rev. 31:33-42; Green et al., 1998, J. Exp. Med. 188: 483-95; Jakobovits, 1998, Exp. Opin. Invest. Drugs 7: 607-14; Tsuda et al., 1997, Genomics 42: 413-21; Mendez et al., 1997, Nature Genetics 15: 146-56; Jakobovits, 1996, Weir's Handbook of Experimental Immunology, The Integrated Immune System Vol. IV, 194.1-194.7; Mendez et al.,1995, Genomics 26: 294-307; Jakobovits, 1994, Current Biol. 4: 761-63; Arbones, 1994, Immunity 1: 247-60; Green et al., 1994, Nature Genetics 7: 13-21; Jakobovits et al., 1993, Nature 362: 255-58; Jakobovits et al., 1993,. Proc. Nat. Acad. Sci. USA 90: 2551-55.

### EXAMPLE 2: Isolation of Human IGF-1R(ECD)-C3-muIgG1

This example provides a method of making a soluble fragment of IGF-1R useful for raising antibodies.

### Cloning of pDSRα:huIGF-1R(ECD)-C3-muIgG1Fc

Primers 2830-36: 5' AGCAAGCTTCCACCATGAAGTCTGGCTCCGGAGGAGG 3' SEQ ID NO:256) and 2830-38: 5' ATTTGTCGACTTCGTCCAGATGGATGAAGTTTTCAT 3', SEQ ID NO:257) were used to amplify the human IGF-1R extracellular domain (1-906) cDNA sequence. The primers included a Kozak translation initiation sequence (underlined above) preceding the start codon, restriction sites for subsequent subcloning, and a caspace-3 site, which is inserted next to the extracellular domain C-terminus. PCR was performed on a PerkinElmer 2400 (PerkinElmer, Torrance, CA) under the following conditions: 1 cycle at 95° C for 2 min, 23 cycles at 95° C for 30 sec, 58.5° C for 30 sec, and 72° C for 3 min, and 1 cycle at 72° C for 10 min. Final reaction conditions were 1X *pfu* TURBO® buffer (Stratagene, La Jolla, CA), 200 µM dNTPs, 2 µM each primer, 5 U *pfu* TURBO® (Stratagene) and 1 ng template DNA. The PCR product was purified using a Clontech Nucleospin Column (Clontech, Palo Alto, CA) according to the manufacturers instructions, digested with *Hin*d III and *Sal* I (Roche, Indianapolis, IN) and gel purified. The human IGF-1R insert was ligated into *Hind* III/*Sal* I digested pDSRα-muIgG1. Integrity of the insert was confirmed by DNA sequencing. The sequence of the protein encoded by the resulting open reading frame (IGF-1R-C3-muFc) is shown in Figure 10. The final expression vector, pDSRα:huIGF1R(ECD)-C3-muIgG1Fc, is described in Table 1.

**Table 1**

| **pDSRα:huIGF1R(ECD)-C3-muIgG1Fc** | |
|---|---|
| Plasmid Base | |
| Pair Number: | |
| 11-3496 | HuIGF 1R (Caspase 3 site)-muIgG1 Fc |
| | |
| | |
| 3507 to 4391 | A transcription termination/polyadenylation signal from the α-subunit of the bovine pituitary glycoprotein hormone (α-FSH) (Goodwin et al., 1983, Nucleic Acids Res. 11:6873-82; Genbank Accession Number X00004) |
| 4600 to 5163 | A mouse dihydrofolate reductase (DHFR) minigene containing the endogenous mouse DHFR promoter, the cDNA coding sequences, and the DHFR transcription termination/polyadenylation signals (Gasser et al., 1982, Proc. Natl. Acad. Sci. U. S. A. 79:6522-6; Nunberg et al., 1980, Cell 19:355-64; Setzer et al., 1982, J. Biol. Chem. 257:5143-7; McGrogan et al., 1985, J. Biol. Chem. 260:2307-14) |
| 6389 to 7246 | pBR322 sequences containing the ampicillin resistance marker gene and the origin for replication of the plasmid in *E. coli* (Genbank Accession Number J01749) |
| 7459 to 7802 | An SV40 early promoter, enhancer and origin of replication (Takebe et al., 1988, Mol. Cell Biol. 8:466-72, Genbank Accession Number J02400) |
| 7809 to 8065 | A translational enhancer element from the HTLV-1 LTR domain (Seiki et al., 1983, Proc. Natl. Acad. Sci. U. S. A. 80:3618-22, Genbank Accession Number J02029) |
| 8109 to 8205 | An intron from the SV40 16S, 19S splice donor/acceptor signals (Okayama and Berg, 1983. Mol. Cell Biol. 3:280-9, Genbank Accession Number J02400) |

### Expression of hu IGF-1R(ECD)-C3-muIgG1Fc

Fifteen micrograms of linearized expression vector pDSRα:huIGF1R(ECD)-C3-muIgG1Fc was transfected into AM-1/D CHOd- cells using LT1 lipofection reagent (PanVera Corp., Madison, WI), and cells cultured under conditions to allow expression and secretion of protein into the cell media. Twenty-four colonies were selected after 10-14 days on DHFR selection medium (Dulbecco's Modified Eagles Medium (Invitrogen) supplemented with 10% dialyzed fetal bovine serum, 1x penicillin-streptomycin (Invitrogen)) and expression levels evaluated by western blot. To perform this assay, 0.5 ml of serum free medium was added to a single well confluent cells cultured in a 24 well plate (Falcon). The conditioned medium was recovered after 48hr. Samples for western blotting were run in 10% Tris-glycine gel (Novex), and blotted on 0.45 µm Nitrocellulose membrane (Invitrogen), using the Mini Trans-Blot cell (Biorad). The blotted membranes were incubated with rabbit anti-mouse IgG Fc antibody, conjugated with Horseradish Peroxidase (Pierce). The clone expressing the highest level of IGF-1R(ECD)-C3-muIgG1Fc was expanded in DHFR selection medium and 2 x 10⁷ cells were inoculated into 50 roller bottles each (Corning) in 250 ml of high-glucose DMEM (Invitrogen), 10% dialyzed FBS (Invitrogen), 1x glutamine (Invitrogen), 1x Non essential amino acids (Invitrogen), 1x sodium pyruvate (Invitrogen). Medium was gassed with 10% CO₂/balance air for 5 seconds before capping the roller bottle. Roller bottles were kept at 37° C on roller racks spinning at 0.75 rpm.

When cells reached approximately 85-90% confluency (after approximately 5-6 days in culture), growth medium was discarded, cells washed with 100 ml PBS and 200 ml production medium was added (50 % DMEM (Invitrogen)/ 50 % F12 (Invitrogen), 1x glutamine (Invitrogen), 1x non-essential amino acids (Invitrogen), 1x sodium pyruvate (Invitrogen), 1.5% DMSO (Sigma)). The conditioned medium was harvested and replaced at one week intervals. The resulting 30 liters of conditioned medium were filtered through a 0.45 µm cellulose acetate filter (Corning, Acton, MA).

### Purification of hu IGF-1R(ECD)-C3-muIgG1Fc

The resulting filtrate from the conditioned medium was concentrated 20-fold using a spiral-wound cartridge (molecular weight cut-off = 10 kDa), then diluted 1:1 with 3 M KCl, 1 M glycine, pH 9.0 to bring the final salt concentration to 1.5 M KCl, 0.5 M glycine, pH 9.0. This sample was applied to a rProtein A-Sepharose column (Amersham Pharmacia Biotech, Uppsala, Sweden) which had been equilibrated in 1.5 M KCl, 0.5 M glycine, pH 9.0. The column was washed with 40 column volumes of the same buffer, then eluted with 20 column volumes of 0.1 M glycine-HCl, pH 2.8. Five-mL fractions were collected and immediately neutralized with 1 mL of 1 M Tris-HCl, pH 7.5. Fractions containing huIGF1R(ECD)-C3-muIgGFc were identified by SDS-PAGE, pooled, and dialyzed against phosphate-buffered saline. The yield was 2.4 mg/L of conditioned medium. The major protein species detected were the mature α and β chains and murine Fc, each of which appeared to be properly glycosylated based on their elevated and heterogeneous molecular weights. Unprocessed IGF-1R(ECD), as well as glycosylated but not proteolytically cleaved IGF-1R(CED), was also present in the preparation. The shift in bands to higher molecular weights under non-reducing conditions indicates that disulfide linkages joined the α □ and β chains. Amino-terminal sequencing of the final product indicated that 60% of the protein was correctly processed between the α- and β-chains of IGF-1R(ECD), while 40% remained unprocessed.

### EXAMPLE 3: Isolation of Human INSR(ECD)-muIgG1

This example presents a method of cloning and expressing a soluble fragment of the human insulin receptor.

### Cloning of pDSRα:huINSR(ECD)-muIgG1Fc

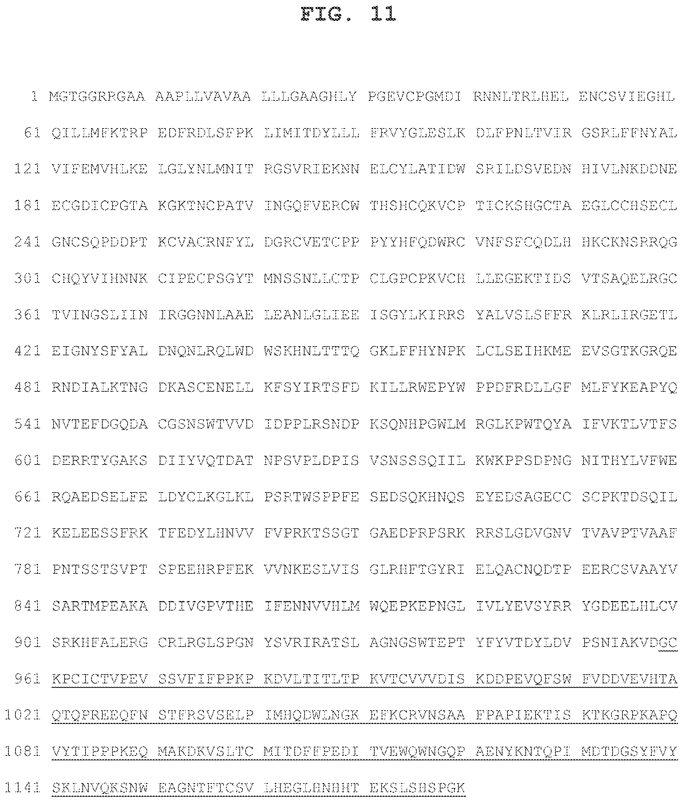

Primers 2830-40: 5' AGCAAGCTTCCACCATGGGCACCGGGGGCCGG 3' SEQ ID NO:259 (*Hind* III site underlined) and 2830-41: 5' ATTTGTCGACTTTTGCAATATTTGACGGGACGTCTAA 3' SEQ ID NO:260 (*Sal* I site underlined) were used to amplify the human INSR extracellular domain (1-929) from and INSR parental plamid encoding the B form of the INSR splice variant (Ullrich et al., 1985, Nature 313:756-61; Ebina et al., 1985, Cell 40:747-58). The primers included a Kozak translation initiation sequence preceding the start codon and restriction sites for subsequent sub-cloning. PCR was performed on a PerkinElmer 2400 under the following conditions: 1 cycle at 95° C for 2 min, 32 cycles at 95° C for 30 sec, 58.5° C for 30 sec, and 72° C for 3 min, and 1 cycle at 72° C for 10 min. Final reaction conditions were 1X *pfu* TURBO® buffer, 200 µM dNTPs, 2 µM each primer, 5 U *pfu* TURBO® (Stratagene) and 10 ng template DNA. The PCR product was purified using a NUCLEOSPIN® Column (BD Biosciences Clontech, Palo Alto, CA) according to the manufacturer's instructions, digested with *Hind* III and *Sal* I (Roche), and gel purified prior to ligation into *Hind* III/*Sal* I digested pDSRα-muIgG1. The integrity of the insert was confirmed by DNA sequencing. The protein sequence of the INSR-muFc is shown in Figure 11. The final expression vector is described in Table 2.

**Table 2**

| Plasmid Base | |
|---|---|
| Pair Number: | |
| 11-3550 | HuINSR-muIgG1Fc |
| | |
| | |
| 3557 to 4441 | A transcription termination/polyadenylation signal from the α-subunit of the bovine pituitary glycoprotein hormone (α-FSH) (Goodwin et al., 1983, Nucleic Acids Res. 11:6873-82; Genbank Accession Number X00004) |
| 4446 to 5586 | A mouse dihydrofolate reductase (DHFR) minigene containing the endogenous mouse DHFR promoter, the cDNA coding sequences, and the DHFR transcription termination/polyadenylation signals (Gasser et al., 1982, Proc. Natl. Acad. Sci. U. S. A. 79:6522-6; Nunberg et al., 1980, Cell 19:355-64; Setzer et al., 1982, J. Biol. Chem. 257:5143-7; McGrogan et al., 1985, J. Biol. Chem. 260:2307-14) |
| 5594 to 6241 | pBR322 sequences containing the ampicillin resistance marker gene and the origin for replication of the plasmid in *E. coli* (Genbank Accession Number J01749) |
| 7513 to 7856 | An SV40 early promoter, enhancer and origin of replication *(*Takebe et a/., 1988, Mol. Cell Biol. 8:466-72, Genbank Accession Number J02400) |
| 7863 to 8119 | A translational enhancer element from the HTLV-1 LTR domain (Seiki et al., 1983, Proc. Natl. Acad. Sci. U. S. A. 80:3618-22, Genbank Accession Number J02029) |
| 8163 to 8259 | An intron from the SV40 16S, 19S splice donor/acceptor signals (Okayama and Berg, 1983. Mol. Cell Biol. 3:280-9, Genbank Accession Number J02400) |

### Expression of hu INSR(ECD)C3-muIgG1Fc

AM-1/D CHOd- cells were transfected with 15 µm of linearized expression vector pDSRa:huINSR(ECD) -muIgG1Fc using FUGENE™ 6 lipofection reagent (Roche Diagnostics Corp., Indianapolis, IN), then cultured under conditions to allow expression and secretion of protein into the cell medium. Colonies were selected and analyzed as described above.

### Purification of hu INSR(ECD)-C3-muIgG1Fc

The filtered conditioned medium containing huINSR(ECD)-muIgGFc was concentrated 17-fold using a spiral-wound cartridge (molecular weight cut-off = 10 kDa), then diluted 1:1 with 3 M KCl, 1 M glycine, pH 9.0 to bring the final salt concentration to 1.5 M KCl, 0.5 M glycine, pH 9.0. This sample was applied to a rProtein A-Sepharose column (Pharmacia) which had been equilibrated in 1.5 M KCl, 0.5 M glycine, pH 9.0. The column was washed with 40 column volumes of the same buffer, then eluted with 20 column volumes of 0.1 M glycine-HCl, pH 2.8. Five-mL fractions were collected and immediately neutralized with 1-mL of 1 M Tris-HCl, pH 7.5. Fractions containing huINSR(ECD)-muIgGFc were identified by SDS-PAGE, pooled, and dialyzed against phosphate-buffered saline. The yield was 0.9 mg/L of conditioned medium. The major protein species were the mature α and β chains and murine Fc. Each of these species appeared to be properly glycosylated based on its elevated and heterogeneous molecular weight. Unprocessed INSR (ECD) as well as glycosylated but not proteolytically cleaved INSR (CED) also was present in the preparation. The shift in bands to higher molecular weights under non-reducing conditions indicated that disulfide linkages joined the α □ and β chains. Amino-terminal sequencing of the final product indicated that 87% of the protein was correctly processed between the α- and β-chains of INSR(ECD), while 13% remained unprocessed.

### EXAMPLE 3: Initial Screen for Anti-IGF-IR phage Fab

This example provides a method of identifying anti-IGF-1R antibodies.

A Target Quest Q Fab library ("the TQ library"; Target Quest, Maastricht, the Netherlands), which was constructed using peripheral blood lymphocytes from four healthy donors and splenic lymphocytes from one patient with gastric carcinoma, was obtained. The library diversity was 3.7 x 10¹⁰ clones, containing 3x10⁹ heavy chains. The source, screening methods, and characterization of the library have been published (de Haard et al, 1999, J Biol Chem 274:18218-30). Dynabeads (200 µl) M-450 Uncoated (catalog # 140.02, Dynal, Lake Success, NY) were washed 3 times with PBS, resuspended in 200 µlof IGF1R(ECD)-C3-mFc to a concentration of 0.5 µM in PBS, and incubated at 4° C on a rotator overnight. The IGF-1R(ECD)-C3-mFc coated beads were washed 3x with 1 ml of 2% non-fat dry milk (M) in PBS (2% MPBS), and then blocked with 1 ml of 2% MPBS at room temperature for 1 hour. In parallel, 750 µl of the TQ library (4x10¹² pfu) was preblocked by mixing with 250 µl 8% MPBS at room temperature for 30 minutes to 1 hour. 500 µl of blocked beads were transferred into another microfuge tube and separated from the blocking solution on a magnetic separator. The preblocked phage mixture was added to the blocked beads and incubated for 90 minutes on a rotator at room temperature. Bead-bound phage were separated from the unbound phage, and then washed 6x with 1ml 2% MPBS/0.1% Tween 20, 6x with 1ml PBS/0.1% Tween 20, 2x with PBS with a change of tubes between different wash solutions. Bound phage was eluted with 1 ml of 0.1M TEA (pH11) for 10 minutes, then immediately separated from the beads and neutralized with 0.5 ml of 1 M Tris.HCl. The eluted phage pool was mixed with 4 ml 2x YT broth (10 g yeast extract, 16 g bacto-tryptone, 5 g NaCl per liter of water) and 5 ml of TG1 bacterial culture (O.D.₅₉₀ about 0.5) in a 50-ml conical tube. The infection mixture was incubate at 37° C in an incubator for 30 min., then centrifuged at 3500 rpm for 20 min. The cell pellet was resuspended in 1500 µl 2xYT-CG broth and 300 µl were spread on each of five 2xYT-CG (2x YT broth containing 100 µg/ml carbenicillin and 2% glucose) plates. After 20 hours of incubation at 30° C, 4 ml of 2x YT-AG were added to each plate and the cells were recovered with cell scraper from the plates. This step was repeated three times. A small portion of the recovered cells was used for phage rescue (see below). The remaining cell suspension was centrifuged at 3500 rpm for 20 min. The cell pellet was suspended into an amount of 50% glycerol roughly half the volume of the pellet size and stored at -80° C.

In order to rescue phage, the plated-amplified cell suspension was used to inoculate 40 ml of 2x YT-CG to an OD₅₉₀ of about 0.05. The culture was incubated at 37° C on a shaker to OD₅₉₀ 0.5. The log phase culture was infected with M13KO7 helper phage (GIBCO BRL, Gaithersburg, MD, catalog # 18311-019, 1.1 x 10¹¹ pfu/ml) at M.O.I. 20 followed by incubation at 37° C for 30 min. The infected cells were centrifuged at 4000 rpm for 20 min. The cell pellet was re-suspended in 200 ml of 2xYT-CK (100 µg/ml carbenicillin and 40 µg/ml kanamycin) and transferred to two 250-ml flasks and incubated at 30° C with shaking at 270 rpm for 20 hours. The over-night culture was centrifuged at 4000 rpm for 20 min to removal cell debris. The centrifugation was repeated to ensure the removal of cell debris. About 1/5 volume of PEG solution (20% PEG 8000, 2.5 M NaCl) was added to the supernatant to precipitate the phage particles. The mixture was incubated on ice for at least 1 hour, followed by centrifugation at 4000 rpm for 20 min to collect the precipitated phage particles. The phage pellet was re-suspended into 1 ml of PBS and transferred to a microfuge tube. The phage suspension was left on ice for 1 hour to allow complete suspension of phage particles, and clarified by centrifugation at 14,000 rpm for 2 min to remove the residual cell debris. Phage precipitation step was repeated. The final phage pellet was suspended into PBS after clarification. The rescued phage suspension was used in the next round of selection.

Four rounds of selection were performed that included alterations of various standard binding parameters. The second round of selection was identical to the first round of selection. Variations in input phage number and elution reagent were introduced in rounds three and four. For the round three selection, 5x10¹¹ pfu of phages were selected and bound phages were eluted either with 1 µM IGF-1 (catalog # 13769, Sigma, St. Louis, MO) or with a 1 µM concentration of a chimeric αIR3-huFc antibody to yield two round-three pools, TQ4-3IS and TQ4-3CA. Round four selection was carried out on rescued phage pools from both round three pools. Two rounds of negative selection with mouse IgG Fc-coated DYNABEADS® (Dynal Biotech, Oslo, Norway) were included to remove mouse Fc binders prior to actual IGF-1R selection. The incubation time for negative selection was 30 minutes each. 3.78x10¹¹ pfu of TQ4-3IS pool and 3.75x10¹² pfu of TQ4-3CA pool were selected separately. Bound phage were eluted with 1 µM IGF-2 (catalog # 12526, Sigma, St. Louis, MO) to yield two round-4 pools, TQ4-4ISI2 and TQ4-4CAI2. The sequence of about 96-192 phage DNA inserts was determined at each elution step.

In some cases, a secondary screen was done. Phagemid DNA mixtures of the total TQ library, and the selected phage amplified after several rounds of selection against IGF-1 R, were prepared using a DNA Maxiprep kit according to the manufacturer's instructions (Qiagen, Valencia, CA). All four DNA preparations were digested with *Asc* I and *Eco*R I (New England Biolab, Beverly, MA). The resulting two *Aisc* I/*Eco*R I fragments were separated on preparative 0.5% agarose gels. The 2.1 kb fragments containing heavy chains were gel purified from the IGF-1R selected phage. The 3.9 kb fragments containing the light chains and pCES1 vector portion were gel purified from the total TQ library DNA. The 2.1 kb fragments were ligated to the 3.9 kb fragments from the DNA sample of TQ library in 3:1 ratio. The ligated DNA was precipitated and used to transform TG1 cells by electroporation. The library size of the resulted light chain shuffled secondary library was 8.8x10⁸. After sequencing 96 randomly picked clones, 76 unique light chain sequences were obtained, indicating that the attempt to shuffle light chains was successful.

The binding, washing and elution condition for screening the light chain shuffle library were essentially the same as decribed for the intial screen. However, several variations were included to increase selection pressure for amplification of IGF-1R binders with higher affinities, especially those with significantly slower off-rates. These parameters were: higher number of input phage (2-2.7 x10¹³ pfu), smaller bead volume (100 µl for round one, 50 µl for round two, and 25 µl for round three), and extended specific elution time up to 20 hours. Elution buffers were 0.1 M TEA for round one (RD1), 1 µM IGF-1 in 0.4% MPBS for RD2 and 1 µM IGF-1 or IGF-2 in 0.4% MPBS for RD3. In RD2 and RD3, binders that were eluted in 15 min or 2 hours were discarded. Elution was continued and eluted phages were collected after 8-10 hours and again after 20 hours.

### Phage Fab ELISA Screen

In 96-well 2-ml deep-well blocks, 480 µl/well 2xYT-CG broth was inoculated with 20 µl of overnight cultures of the individual clones, then incubated at 37° C, 300 rpm for 3 hours. To each well, 50 µl of 1:3 diluted M13KO7 helper phage were added to infect the cells. The block was incubated at 37° C without shaking for 30 minutes, and then shaken gently for another 30 minutes at 150 rpm. The block was centrifuged at 3600 rpm for 20 minutes to pellet the infected cells. The cell pellet in each well was suspended into 480 µl of 2xYT-CK (2xYT broth containing 100 µg/ml carbenicillin and 40 µg/ml kanamycin), and incubated at 30° C overnight for about 20 hours. The cell debris was separated by centrifugation at 3600 rpm for 20 minutes. The rescued phage supernatant was used in the phage ELISA to check for IGF-1R-specific, INSR-cross reactive, or mouse Fc binding of individual clones.

Three sets of Nunc MaxiSorb Immunoplates were coated with 100 µl/well of IGF-1R-C3-mFc at 5 µg/ml, INSR-mFc at 5 µg/ml, or mouse IgG1 (catalog # 010-0103, Rockland, Gilbertsville, PA) at 2 µg/ml in PBS, respectively, at 4° C overnight. The coated plates were washed 3x with 300 µl/wel] of PBS. The washed plates were blocked with 300 µl/well 2% MPBS at room temperature for one hour. Meanwhile, rescued phages of individual clones were pre-blocked by mixing 170 µl of rescued phage with 170 µl of 4% MPBS. The blocked plates were washed 5x with 300 µl/well TBST (TBS: 10 mM Tris-HCl, pH 7.5, 1 mM EDTA, 150 mM NaCl; Tween-20. 0.1%). 100 µl/well of pre-blocked phage dilutions were distributed to each set of coated plate, which were incubated at room temperature on a rocker for 90 minutes. The plates were washed 5x with 300 µl/well TBST. 100 µl/well of anti-M13-HRP in 2% MPBS (1:3000 dilution, catalog number 27-9421-01, Amersham Pharmacia Biotech) were distributed, and plates were incubated at room temperature on rocker for one hour. The plates were washed 5x with 300 µl/well TBST. 100 µl/well of the substrate 1-Step™ ABTS (Pierce Biotechnology, Rockford, IL, catalog number 37615) were added. Plates were incubated for one hour. OD₄₀₅ was measured for signal detection.

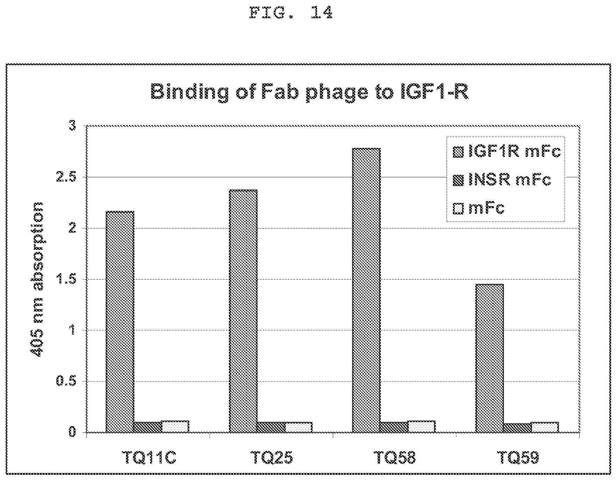

The phage displayed antibodies exhibited essentially no crossreactivity with the insulin receptor and murine Fc domain. The signal observed in the IGF-1R ELISA is therefore specific for the IGF-1R extracellular domain. Results from similar assays for four of the phage-displayed antibodies are shown in Figure 14.

The DNA inserts of IGF-1R positive, INSR and mu IgG1 negative, clones were sequenced. Fifty-two unique Fab sequences were identified, having the following combinations of light chain and heavy chain variable domain sequences: L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20, H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, and L52H52, wherein "Lx" indicates light chain variable domain number "x" and "Hx" indicates heavy chain variable domain number "x." Figure 1 presents the polynucleotide sequences of each of these light and heavy variable domains. Figures 2 and 3 present the corresponding amino acid sequences.

### EXAMPLE 4: Subcloning of V_{H} and V_{L} into IgG1 expression vectors

This example presents a method of subcloning the previously identified variable domain sequences into an IgG1 expression vector.

### Construction of pDSRα20 and pDSRα20:hIgG1C_{H}

The pDSRα20:hIgG1C_{H} expression vector (WO 90/14363) was a derivative of pDSR19:hIgG1C_{H} (*see* U.S. Provisional Patent Application No. 60/370,407, filed April 5, 2002, "Human Anti-OPGL Neutralizing Antibodies As Selective OPGL Pathway Inhibitors," ). The pDSRα19:hIgG1C_{H} plasmid encoded a rat variable region/human constant region IgG1 (rVh/hCh1). The plasmid was constructed by the three-piece ligation of *Xba* I and *BsmB* I terminated rat antibody variable region PCR product, the human IgG1 constant region (C_{H1}, hinge, C_{H2} and C_{H3} domains) derived by *Sal* I cleavage and gel isolation of the *BsmB* I and *Sal* I fragment from the linear plasmid pDSRα19:hIgG1 C_{H} (*Hind* III and *BsmB* I ends) and a linearized pDSRα19 with *Xba* I and *Sal* I ends. pDSRα20 was produced by changing nucleotide 2563 in pDSRα19 from a guanosine to an adenosine by site directed mutagenesis. The heavy chain expression vector, pDSRα20:hIgG1C_{H} rat variable region/human constant region IgG1 (rVh/hCh1), is 6163 base pairs and contains the 7 functional regions described in Table 3.

**Table 3**

| Plasmid Base | |
|---|---|
| Pair Number: | |
| 2 to 881 | A transcription termination/polyadenylation signal from the α-subunit of the bovine pituitary glycoprotein hormone (α-FSH) (Goodwin et al., 1983, Nucleic Acids Res. 11:6873-82; Genbank Accession Number X00004) |
| 882 to 2027 | A mouse dihydrofolate reductase (DHFR) minigene containing the endogenous mouse DHFR promoter, the cDNA coding sequences, and the DHFR transcription termination/polyadenylation signals (Gasser et al., 1982, Proc. Natl. Acad. Sci. U. S. A. 79:6522-6; Nunberg et al., 1980, Cell 19:355-64; Setzer et al., 1982, J. Biol. Chem. 257:5143-7; McGrogan et al., 1985, J. Biol. Chem. 260:2307-14) |
| 2031 to 3947 | pBR322 sequences containing the ampicillin resistance marker gene and the origin for replication of the plasmid in *E. coli* (Genbank Accession Number J01749) |
| 3949 to 4292 | An SV40 early promoter, enhancer and origin of replication (Takebe et al., 1988, Mol. Cell Biol. 8:466-72, Genbank Accession Number J02400) |
| 4299 to 4565 | A translational enhancer element from the HTLV-1 LTR domain (Seiki et al., 1983, Proc. Natl. Acad. Sci. U. S. A. 80:3618-22, Genbank Accession Number J02029) |
| 4574 to 4730 | An intron from the SV40 16S, 19S splice donor/acceptor signals (Okayama and Berg, 1983. Mol. Cell Biol. 3:280-9, Genbank Accession Number J02400) |
| 4755 to 6158 | The rVh/hCh1 heavy chain cDNA between the *Xba*I and *Sal*I sites. This heavy chain fragment sequence is shown below (SEQ ID NO: 262) with the sequences of the restriction sites underlined: |
| | *Xba*I |
| | |
| | *Bsm*B1 |
| | |
| | |
| | *Sal*I |
| | AATGATAAGTCGAC |

The linear plasmid pDSRα20:hIgG1C_{H} was prepared by digesting the pDSR20: rat variable region/human constant region IgG1 plasmid with the restriction enzymes *Xba* I and *BsmB* I to remove the rat variable region and purified using a QIAquick Gel Extraction kit. The linear plasmid pDSRα20:hIgG1C_{H} containing the 1.0 kbp human IgG1 constant region domain was used to accept anti-IGF-1R variable heavy chain coding sequences.

### Construction of the anti-IGF-1R IgG1 Heavy Chain Expression Clones

The sequence coding for the anti-IGF-1R variable region of the heavy chains was amplified from phagemid DNA with complementary oligonucleotide primers. Primers for polymerase chain reaction (PCR) were designed to incorporate a *Hind* III site, *Xba* I site, Kozak sequence (CCACC) and signal sequence (translated peptide is MDMRVPAQLLGLLLLWLRGARC; SEQ ID NO:263) onto the 5' end of the variable region, while a *BsmB* I site was added onto the 3' end of the PCR product. The PCR products were digested with *Xba* I and *BsmB* I, and then cloned into the *Xba I-Bsm*B I linear pDSRα20:hIgG1C_{H} expression vector containing the human IgG1 constant region (Figure 13). The final expression vectors contained the seven functional regions described in Table 4.

**Table 4**

| Plasmid Base | |
|---|---|
| Pair Number: | |
| 2 to 881 | A transcription termination/polyadenylation signal from the α-subunit of the bovine pituitary glycoprotein hormone (α-FSH) (Goodwin et al., 1983, Nucleic Acids Res. 11:6873-82; Genbank Accession Number X00004) |
| 882 to 2027 | A mouse dihydrofolate reductase (DHFR) minigene containing the endogenous mouse DHFR promoter, the cDNA coding sequences, and the DHFR transcription termination/polyadenylation signals (Gasser et al., 1982, Proc. Natl. Acad. Sci. U. S. A. 79:6522-6; Nunberg et al., 1980, Cell 19:355-64; Setzer et al., 1982, J. Biol. Chem. 257:5143-7; McGrogan et al., 1985, J. Biol. Chem. 260:2307-14) |
| 2031 to 3947 | pBR322 sequences containing the ampicillin resistance marker gene and the origin for replication of the plasmid in *E. coli* (Genbank Accession Number J01749) |
| 3949 to 4292 | An SV40 early promoter, enhancer and origin of replication (Takebe et al., 1988, Mol. Cell Biol. 8:466-72, Genbank Accession Number J02400) |
| 4299 to 4565 | A translational enhancer element from the HTLV-1 LTR domain (Seiki et al., 1983, Proc. Natl. Acad. Sci. U. S. A. 80:3618-22, Genbank Accession Number J02029) |
| 4574 to 4730 | An intron from the SV40 16S, 19S splice donor/acceptor signals (Okayama and Berg, 1983. Mol. Cell Biol. 3:280-9, Genbank Accession Number J02400) |
| 4755 to 6185 | The heavy chain IgG1 cDNA between the *Xba*I and *Sai*I sites |

### Construction of the anti-IGF-1R IgG1 Variable Chain Expression Clones.

The light chains encoded in anti-IGF-1R phage were either kappa or lambda class. They were cloned using one of two approaches. Complementary primers were designed to add a *Hind* III site, an *Xba* I site, Kozak sequence (CCACC) and signal sequence (translated peptide is MDMRVPAQLLGLLLLWLRGARC, SEQ ID NO:264) were added to the 5' end of the coding region. Those chains that had error-free coding regions were cloned as full-length products. The full-length light chains were cloned as *Xba* I and *Sal* I fragments into the expression vector pDSRα20. The final expression vectors contained the seven functional regions described in Table 5.

**Table 5**

| Plasmid Base | |
|---|---|
| Pair Number: | |
| 2 to 881 | A transcription termination/polyadenylation signal from the α-subunit of the bovine pituitary glycoprotein hormone (α-FSH) (Goodwin et al., 1983, Nucleic Acids Res. 11:6873-82; Genbank Accession Number X00004) |
| 882 to 2027 | A mouse dihydrofolate reductase (DHFR) minigene containing the endogenous mouse DHFR promoter, the cDNA coding sequences, and the DHFR transcription termination/polyadenylation signals (Gasser et al, 1982, Proc. Natl. Acad. Sci. U. S. A. 79:6522-6; Nunberg et al., 1980, Cell 19:355-64; Setzer et al., 1982, J. Biol. Chem. 257:5143-7; McGrogan et al., 1985, J. Biol. Chem. 260:2307-14) |
| 2031 to 3947 | pBR322 sequences containing the ampicillin resistance marker gene and the origin for replication of the plasmid in *E. coli* (Genbank Accession Number J01749) |
| 3949 to 4292 | An SV40 early promoter, enhancer and origin of replication (Takebe et al., 1988, Mol. Cell Biol. 8:466-72, Genbank Accession Number J02400) |
| 4299 to 4565 | A translational enhancer element from the HTLV-1 LTR domain (Seiki et al., 1983, Proc. Natl. Acad. Sci. U. S. A. 80:3618-22, Genbank Accession Number J02029) |
| 4574 to 4730 | An intron from the SV40 16S, 19S splice donor/acceptor signals (Okayama and Berg, 1983, Mol. Cell Biol. 3:280-9, Genbank Accession Number J02400) |
| 4755 to 5485 | The kappa light chain cDNA between the *Xba*I and *Sai*I sites |

Some kappa clones had errors in their constant regions when compared to natural human constant region sequence. To eliminate these discrepancies, the kappa variable region was amplified with a primer that would introduce an *Xba* I site into the 5' end and a *BsmB* I site into the 3' end. This fragment was then ligated along with a human kappa constant region (Figure 13) with a compatible *BsmB* I on the 5' end and a 3'*Sal* I ends into pDSRα20 with *Xba* I and *Sal* I ends.

### EXAMPLE 5: Transient Expression of Antibodies

This example provides a method of transiently expressing anti-IGF-1R antibodies.

The antibodies were expressed transiently in serum-free suspension adapted 293T cells. All transfections were performed as 250 mL cultures. Briefly, 1.25 x 10⁸ cells (5.0 x 10⁵ cells/mL x 250 mL) were centrifuged at 2,500 RPM for 10 minutes at 4° C to remove the conditioned medium. The cells were resuspended in serum-free DMEM and centrifuged again at 2,500 RPM for 10 minutes at 4° C. After aspirating the wash solution, the cells were resuspended in growth medium [DMEM/F12 (3:1) + 1x Insulin-Transferrin-Selenium Supplement + 1X Pen Strep Glut + 2mM L-Glutamine + 20 mM HEPES + 0.01% Pluronic F68] in a 500 mL spinner flask culture. The spinner flask culture was maintained on magnetic stir plate at 125 RPM which was placed in a humidified incubator maintained at 37° C and 5% CO₂. The plasmid DNA was incubated with the transfection reagent in a 50 mL conical tube. The DNA-transfection reagent complex was prepared in 5% of the final culture volume in serum-free DMEM. One microgram of plasmid DNA per milliliter of culture was first added to serum-free DMEM, followed by 1µl X-TremeGene RO-1539/mL culture. The complexes were incubated at room temperature for approximately 30 minutes and then added to the cells in the spinner flask. The transfection/expression was performed for 7 days, after which the conditioned medium was harvested by centrifugation at 4,000 RPM for 60 minutes at 4° C.

If the initial transfection failed to yield the required 100 µg purified antibody, those clones were re-expressed in roller bottles. These transfections used 293T adherent cells grown and maintained in DMEM supplemented with 5% FBS + 1x Non-Essential Amino Acids + 1x Pen Strep Glut + 1x Sodium Pyruvate. Approximately, 4-5 x 10⁷ 293T cells were seeded in a 850 cm² roller bottles overnight. The previously seeded cells were then transfected the following day using FUGENE™ 6 transfection reagent. The DNA - transfection reagent mixture was prepared in approximately in 6.75 mL serum-free DMEM. 675 µl FUGENE™ 6 transfection reagent was first added, followed by 112.5 µg plasmid DNA. The complex was incubated at room temperature for 30 minutes. The entire mixture was then added to a roller bottle. The roller bottle was infused with a 5% CO₂ gas mixture, capped tightly and placed in a 37° C incubator on a roller rack rotating at 0.35 RPM. The transfection was performed for 24 hours after which the medium was replaced with 100 mL DMEM + 1X Insulin-Transferrin-Selenium Supplement + 1X Pen Strep Glu + 1X Non-Essential Amino Acids + 1X Sodium Pyruvate. Typically, 2-3 harvests (100ml) were obtained from each roller bottle at a 48 hr interval. The harvested serum-free conditioned medium was pooled together and centrifuged at 4,000 RPM for 30 minutes at 4° C.

### EXAMPLE 6: Anti-IGF-IR Antibody Small-scale Purification

This example provides a method of purifying anti-IGF-1R antibodies on a small scale.

Conditioned medium was filtered through a 0.45 µm cellulose acetate filter and concentrated approximately 8-fold using a Vivaflow 200 50 K tangential flow membrane (Vivascience, Goettingen, Germany). rProtein A SEPHAROSE™ Fast Flow resin (Amersham Biosciences, Piscataway, NJ) was washed with phosphate buffered saline (2.7 mM potassium chloride, 138 mM sodium chloride, 1.5 mM potassium phosphate, and 8.1 mM sodium phosphate, pH 7.4) (PBS) four times then directly applied to the concentrated media. The amount of resin used was based on antibody concentration determined by ELISA where 1 µl of resin was used per 5 µg antibody. The medium was incubated overnight at 4° C with gentle agitation. The resin was centrifuged at 500 g for 10 min. at 4° C. The supernatant was decanted as the unbound fraction. The resin was washed with PBS four times for one minute at room temperature with gentle agitation, each time collecting the resin by centrifugation at 500 g for 10 min. at 4° C. The antibody was eluted by incubating the resin with 1.5 volumes of 0.1 M glycine pH 3.0 for 10 min. at room temperature. The resin was centrifuged at 500 g for 10 min. at 4° C and the supernatant decanted as eluted antibody. The elution step described above was repeated for a total of three elutions; each time the eluted material was neutralized with 0.04 volumes of 1.0 M tris-HCl, pH 9.2. The sample was filtered through a 0.2 µm cellulose acetate filter. Protein concentration was determined by the Bradford method using the Bio-Rad Protein Assay (Bio-Rad Laboratories, Hercules, CA) as per the supplied instructions using Human IgG (Sigma-Aldrich, St. Louis, MO) as a standard. The sample was compared to a Human IgG1, K standard (Sigma-Aldrich, St. Louis, MO) using a 4-20% tris-glycine SDS polyacrylamide gel (SDS-PAGE) gel stained with Coomassie brilliant blue dye. No contaminating protein was visible in these preparations.

### EXAMPLE 7: Isolation of Stable CHO Clones Expressing Antibodies

This example provides a method for isolating stable CHO cell lines expressing anti-IGF-1 R antibodies.

Stable expression of TQ11C, TQ25, TQ 58 and TQ59 IgG1 was achieved by co-transfection of AM1-D CHO cells (U.S. Pat. No. 6,210,924) with pDSRα20 heavy and light chian IgG1 expression constructs. The plasmid transfections were performed using LF2000 (Invitrogen, Carlsbad, CA) according to the manufacturer's instructions. Briefly, 4 x 106AM1-D CHO cells were plated 24 hours prior to transfection, in 100 mm diameter FALCON™ plastic petri dishes (BD Falcon, Franklin Lakes, NJ) in 10 ml of Dulbecco's Modified Eagles Medium (Invitrogen) supplemented with 5% fetal bovine serum, 1x penicillin-streptomycin and glutamine (Invitrogen), non-essential amino acids (Invitrogen), sodium pyruvate, and HT (0.1 mM sodiumhypoxanthine, 16 nM thymidine; Invitrogen). Approximately 15 mg of each pDSRα21 - light chain and heavy chain plasmid DNA were linearized using *Pvu* I (New England Biolabs) and diluted in 2 ml of OPTI-MEM® (Invitrogen). The diluted plasmids were mixed with 75 µl of LIPOFECTAMINE™ 2000 (LF2000; GIBCO/BRL) diluted in 2 ml of OPTI-MEM® and the mixture was incubated for 20 min at room temperature. The following day fresh growth medium was added. The cells were cultured in complete growth medium for 48 hours, then plated in HT- selection medium in 1:20 and 1:50 dilutions. Approximately 2 weeks after transfection, 12-24 visible colonies were picked into 24-well plates, using the sterile cloning discs (RPI). The clones expressing the highest level of TQ11C, TQ25, TQ58 and TQ59 IgG1 were identified by western immunoblot analysis. To perform this assay, 0.5 ml of serum free medium was added to a single-well confluent cells cultured in a 24 well plate (BD Falcon). The conditioned medium was recovered after 24 hr, and 10 µl of CM was mixed with an equal volume of loading buffer to run a 10% Tris-Glycine polyacrylamide protein gel (Invitrogen). The gel was transferred to a 0.45 µm pore size nitrocellulose membrane (Invitrogen), and western blot analysis was done using 1:1000 dilution of goat anti-human IgG Fc ImmunoPure antibody (Pierce Biotechnology, Inc., Rockford, IL) and ECL as detection agent.

### EXAMPLE 8: Mid-scale Expression of Antibodies

This example provides a method of expressing anti IGF-1 R antibodies expressed by stable CHO cell lines.

The CHO cell lines made according to Example 7 were expanded to T-175 tissue culture flasks (Falcon) for scale-up expression. A confluent T175 flask (approximately 2-3 x 107 cells) was used to seed 3 - 850 cm2 roller bottles (Corning Life Sciences, Acton, MA), and three confluent roller bottles (approximately 1-2 x 108 cells per roller bottle) were used to seed 30 rollers in 250 ml of high-glucose DMEM (Invitrogen), 10% dialyzed FBS (Invitrogen), 1x glutamine (Invitrogen), 1x non-essential amino acids (Invitrogen), 1x sodium pyruvate (Invitrogen). Medium was infused with 10% CO₂/balance air for 5 seconds before capping the roller bottle. Roller bottles were incubated at 37° C on roller racks spinning at 0.75 rpm.

When cells reached approximately 85-90% confluency (approximately 5-6 days in culture), the growth medium was discarded, the cells were washed with 100 ml PBS, and 200 ml production medium was added (50% DMEM (Invitrogen)/ 50% F12 (Invitrogen), 1x glutamine (Invitrogen), 1x non-essential amino acids (Invitrogen), 1x sodium pyruvate (Invitrogen), 1.5% DMSO (Sigma). Conditioned medium was harvested every seven days for a total of four harvests.

Conditioned medium was filtered through a 0.45 µm cellulose acetate filter and concentrated approximately 10-fold using a Sartorius Sartocon Slice Disposable 30 K tangential flow membrane (Sartorius AG, Goettingen, Germany). The concentrated material was applied to a 10 ml rProtein A Sepharose column at 4° C and the flowthrough was collected as the unbound fraction. The column was washed with four column volumes of PBS. The bound sample was eluted with approximately four column volumes of 0.1 M glycine pH 3.0. The eluate peak was collected and neutralized with 0.04 volumes of 1.0 M tris-HCl, pH 9.2. The eluate was dialyzed against 150 volumes of PBS overnight at 4° C. The sample was filtered through a 0.2 µm cellulose acetate filter and protein concentration was measured by determining the absorbance at 280nm using an extinction coefficient of 14,000 M-1. The sample was compared to a Human IgG1, K standard (Sigma-Aldrich, St. Louis, Missouri, USA) using a 4-20% tris-glycine SDS-PAGE gel stained with Coomassie brilliant blue stain. Endotoxin levels in each antibody prepration was determined using the Pyrotell Limulus Amebocyte Lysate Assay (Associates of Cape Cod, Inc., Falmouth, Ma) as per the supplied instructions.

### EXAMPLE 9: ORIGEN® Dose Response Competition Assays

This example provides methods for testing the ability of an antibody to block ligand binding to IGF-1R.

An ORIGEN® binding assay was used to determine whether TQ11C, TQ25, TQ 58 and TQ59 IgG1 antibodies could block ligand binding to IGF-1R using procedures provided by the manufacturer (Igen, Inc., Gaithersburg, MD). To label IGF-1 and IGF-2 with ruthenium, lyophilized proteins were dissolved into PBS to give a 1.0 mg/ml solution. Label (ORI-TAG-NHS ester from Igen, Cat # 110034) was added to the protein at a molar ratio of 5:1 (label: protein) from a label stock of 5 mg/ml in DMSO. The mixture was incubated at room temperature (20-22° □C) for 1 hr in the dark then treated with 20 µl 2M glycine for 10 min at room temperature. The labeled protein was separated from the free label by application to an Amersham Biosciences NAP-5 column (Amersham Biosciences, Piscataway, NJ) equilibrated in PBS and 0.33 ml fractions collected. The protein concentration of the fractions was determined by Micro BCA Protein Assay (Pierce Biotechnology, Inc., Rockford, IL). Fractions two and three contained significant protein and were combined. The amount of incorporated ruthenium label was assessed using the following formula: ruthenium tris-bipyridyl compound (Ru(bpy)₃²⁺) labeling of IGF-1 and IGF-2.

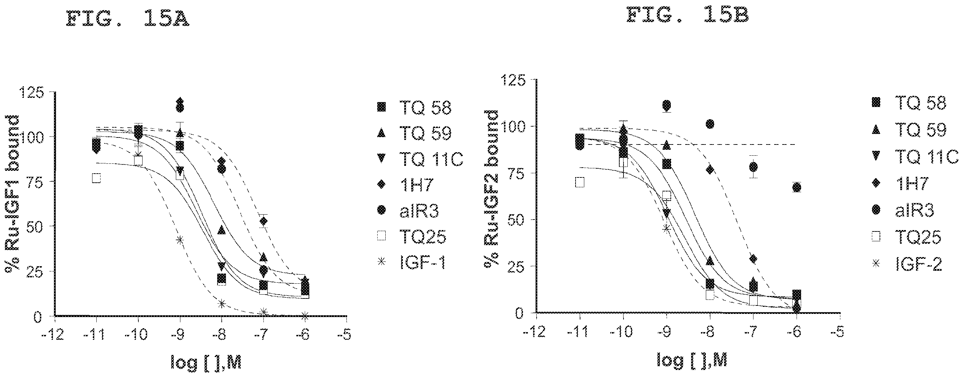

Dynal M450 paramagnetic beads coated with sheep anti-mouse IgG was used as the solid support phase for the IGF-1R(ECD)-C3-muFc. The M450 beads were prepared for receptor loading by washing three times with assay buffer containing 1x PBS, 0.05% TWEEN™ 20 (ICI Americas, Inc., Wilmington DE) 0.1% BSA, 0.01% sodium azide. The IGF-1R(ECD)-C3-muFc was bound for 1 hr at a ratio of 50 ng receptor per 1 x 10⁶ M450 beads in a volume of 25 µl assay buffer. To generate dose response data, the antibodies or unlabeled IGF-1 and IGF-2 factors were added at increasing concentrations (10⁻¹¹M to 10⁻⁶M) simultaneously with 1 nM Ru-IGF-1 or 2 nM Ru-IGF-2. The final reaction volume was 100 µl. After incubation at room temperature in the dark for 2 hr, an M8 Analyzer (Igen) was used to remove free ruthenium labeled ligand and determine the amount of ligand bound to receptor. The data were expressed as the percent of total ligand bound minus background remaining after competition with excess unlabeled growth IGF1 or IGF-2. Competition curves were generated with GraphPad Prism software (GraphPad Software, San Diego, CA) using a single component equilibirium model. Essentially all (> 98%) binding was competed with excess unlabeled growth factors. The positive control antibodies in the binding analysis were the murine anti-IGF-1R antibodies αIR3 (Calbiochem, San Diego, CA) or MAB391 (R&D systems, Minneapolis, MN), 24-57 (Biocarta, San Diego, CA) and 1H7 (Santa Cruz Biotechnology, Inc., Santa Cruz, CA). The negative control antibody was an anti-CD20 antibody. Ligand competition data are shown in Figure 15. The Ki and maximum inhibition values observed for IGF-1 and IGF-2 binding reactions are listed in Table 6.

**Table 6**

| Antibody | IGF-1 | | IGF-2 | |
|---|---|---|---|---|
| | Ki (nM)¹ | Max (%)² | Ki (nM)¹ | Max (%)² |
| TQ11C | 0.6 | 84 | 0.3 | 91 |
| TQ25 | 0.8 | 88 | 0.8 | 94 |
| TQ58 | 0.8 | 91 | 0.8 | 91 |
| TQ59 | 1.5 | 79 | 1.4 | 91 |
| 1H7 | 16.0 | 89 | 13.1 | 99 |
| αIR3 | 5.3 | 91 | No Inhibition | |

| | | | | |
|---|---|---|---|---|
| ¹ Ki of inhibition. ² Maximum level of inhibition at 1 µM antibody concentration. | | | | |

### EXAMPLE 10: SPA Dose Response Competition Assay

This example presents a scintillation proximity assay (SPA) for assesessing the effect of antibodies on the interaction of insulin (INS) with the insulin receptor (INSR) and of IGF-1 and IGF-2 to IGF-1R.

IGF-1R binding reactions for TQ11C, TQ25, TQ 58 and TQ59 IgG1 antibodies contained 1x PBS, 0,05% TWEEN® 20 (Mallinkrodt), 0.1% BSA (EM Science, Gibbstown, NJ), 50 ng IGF-1R(ECD)-C3-muFc, 500 ug SPA PVT anti-mouse IgG fluoromicrospheres (Amersham) and ¹²⁵I-labeled IGF-1 or IGF-2 obtained from Amersham at a final concentration of 0.64 nM. The total reaction volume was 100 µl. The INSR binding reactions were identical except they contained 50 ng INSR(ECD)-muFc and 0.64 nM ¹²⁵I-INS (Amersham). Receptor was loaded onto SPA PVT microspheres for 1h at room temperature prior to assembly of the binding reactions. To generate dose response data, antibodies or unlabeled growth factors were added at increasing concentrations (10⁻¹¹ M to 10⁻⁶M) simultaneously with ¹²⁵I-labeled growth factors. Essentially all binding was competed with excess unlabeled growth factors. The receptor-independent background, caused by random γ stimulation of the SPT PVT microspheres, was less than 0.5% of the input ¹²⁵I cpm. The data were expressed as the percent of total ligand bound minus background remaining after competition with excess unlabeled growth IGF1 or IGF-2. Competition curves were generated with GraphPad Prism software using a single component equilibrium model.

### EXAMPLE 11: Antibody Binding to IGF-1R

This example provides a method of detecting the binding of an anti-IGF-1R antibody to IGF-1R.

BIACORE® 2000, sensor chip CM5, surfactant P20, HBS-EP (10mM HEPES, 0.15M NaCl, 3.4mM EDTA, 0.005% P20, pH 7.4), amine coupling kit, 10mM acetate pH 4.5 and 10mM glycine pH 1.5 all were purchased from BIACore, Inc. (Piscataway, NJ). Phosphate-buffered saline (PBS, 1X, no calcium chloride, no magnesium chloride) was from Gibco. Bovine serum albumin (BSA, fraction V, IgG free) was from Sigma. Recombinant Protein G ("rProtein G") was from Pierce Biotechnology.

Immobilization of rProtein G and IGF-1R-C3-muFc to the sensor chip surface was performed according to manufacturer's instructions, using a continuous flow of 10mM HEPES, 0.15M NaCl, 3.4mM EDTA, 0.005% P20, pH 7.4 (HBS-EP buffer). Briefly, carboxyl groups on the sensor chips's surfaces were activated by injecting 60 µl of a mixture containing 0.2 M N-ethyl-N'-(dimethylaminopropyl)carbodiimide (EDC) and 0.05 M N-hydroxysuccinimide (NHS). Specific surfaces were obtained by injecting rProtein A (Pierce) or IGF-1R-C3-mFc diluted in 10mM acetate, pH 4.5 at concentrations between 20 and 50 µg/ml. Excess reactive groups on the surfaces were deactivated by injecting 60 µl of 1 M ethanolamine. Final immobilized levels were 5,000-6,000 resonance units (RU) for the Protein G surfaces, and -7,800 RU for the IGF-1R-mFc surfaces. A blank, mock-coupled reference surface was also prepared on the IGF-1R-mFc sensor chip.

The kinetic analysis of the interaction between IGF-1R-mFc and antibodies was performed as follows. Antibodies as well as a positive control antibody (anti-IR3-CDR-human-mouse chimera) were diluted in PBS + 0.005% P20 + 0.1 mg/ml BSA and injected over the Protein G surfaces to capture the antibodies. IGF-1R-mFc was diluted in PBS + 0.005% P20 + 0.1 mg/ml BSA from 500nM to 3.9nM, and each concentration was injected over the captured antibody surfaces, as well as over a blank Protein G surface for background subtraction. After a 10 minute dissociation, each surface was regenerated by injecting 10mM glycine, pH 1.5. Kinetic analysis of the resulting sensorgrams was performed using BIAEvaluation, v. 3.2 (BIACore, Inc.).

A solution affinity analysis was done by incubating two different concentrations (0.2nM and 1nM) of antibody with varying concentrations (0.01 nM to 50nM) of IGF-1R-mFc in PBS + 0.005% P-20 + 0.1 mg/ml BSA. Incubations were done at room temperature for at least five hours to allow samples to reach equilibrium. Samples were then injected over the immobilized IGF-1R-mFc surface. After the sample injection, the surfaces were regenerated by injecting 25 µl 8mM glycine, pH 1.5. The binding signal obtained is proportional to the free antibody in solution at equilibrium. The dissociation equilibrium constant (K_{D}) was obtained from nonlinear regression analysis of the competition curves using a dual-curve one-site homogeneous binding model (KinExA software v. 2.3, Sapidyne Instruments Inc., Boise ID). The data are shown in Table 7

**Table 7**

| Antibody | kₒₐ (l/Ms) | K_{d} (l/s) | Kd (kₐ/k_{d}) Kinetic Method | Kd Equilibrium Method |
|---|---|---|---|---|
| TQ11C | 6.0 x 10⁴ | 6.7 x 10⁻⁵ | 1.1 nM | 0.3 nM |
| TQ25 | 4.4 x 10⁴ | <<5 x 10⁻⁵ | | 0.10 nM |
| TQ58 | 1.1 x 10⁵ | 2.8 x 10⁻⁵ | 0.25 nM | 0.25 nM |
| TQ59 | 6.9 x 10⁴ | 2.1 x 10⁻⁴ | 3.0 nM | 0.30 nM |

### EXAMPLE 12: Epitope Mapping Avidin-Fusion proteins

This example provides a method of determining the epitope of IGF-1 R bound by an anti-IGF-1R antibody.

The subdomains of IGF-1R bound by antibodies TQ11C, TQ25, TQ58, and TQ59 were determined using avidin-IGF-1R fusion proteins. To express each protein the coding DNA sequences of the complete IGF-1R(ECD) was cloned into the expression vector pCep4-avidin-C such that chicken avidin sequence is joined to the C-terminus of the expressed IGF-1R protein. The ECD coding sequence (1-932) was PCR amplified from a parental IGF-1R plasmid using PCR primers 2804-25: 5' GCAAGCTTGGGAGAAATCTGCGGGCCAG 3' SEQ ID NO:265 and 2826-68: 5' ATTGCGGCCGCTTCATATCCTGTTTTGGCCTG 3' SEQ ID NO:266

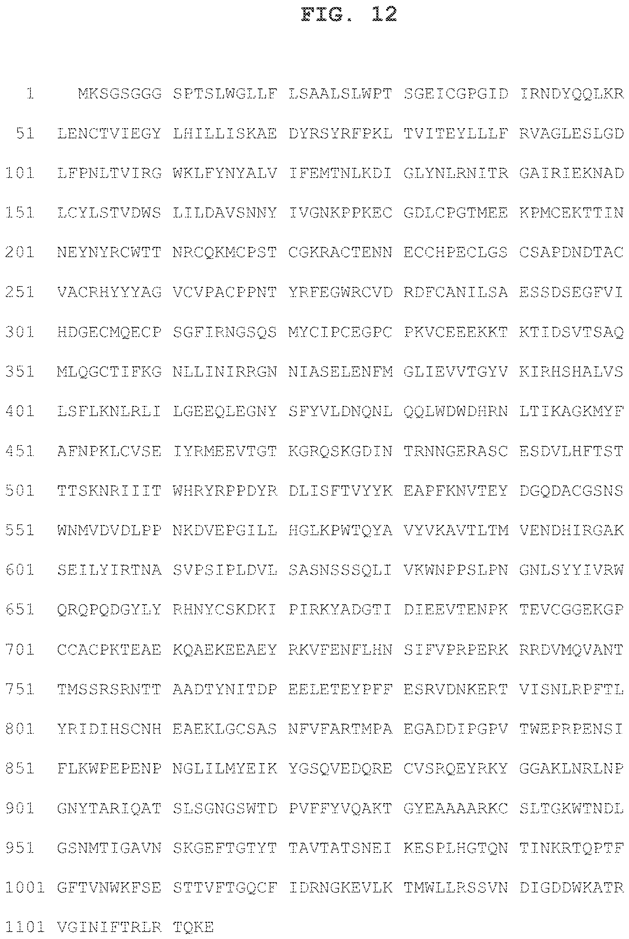

The primers include a 5' *Hind* III site and a 3' *Not* I site for cloning into pCep4avidin-C. The amino acid sequence of the avidin-human IGF-1R(ECD) fusion protein is shown in Figure 12. The IGF-1R subdomains constructs used for epitope mapping included: L1 (1-151), CR (152-298), L2 (299-461), FnIII-1 (461-579), FnIII-2/ID (580-798), FnIII-3 (799-901), L1+CR+L2 (1-461), and L1+CR (1-298). The amino acid coordinates of the IGF-1R subdomain represented in each expression plasmid are given in parenthesis. The coding sequence of each domain was PCR amplified from a parental IGF1R cDNA clone using the following primer pairs:
L1:
   2804-25: (SEQ ID NO:265)
   2804-19:
      5' ATTGCGGCCGCCCCACATTCCTTTGGGGGC 3'SEQ ID NO:267
CR:
   2804-38:
      5' AGCAAGCTTGGACCTGTGTCCAGGGACC 3' SEQ ID NO:268
   2804-20:
      5' ATTGCGGCCGCGCAAGGACCTTCACAAGGG 3' SEQ ID NO:269
L2:
   2804-39:
      5' AGCAAGCTTGCCGAAGGTCTGTGAGGAAG 3' SEQ ID NO:270
   2804-23:
      5' ATTGCGGCCGCACTTTCACAGGAGGCTCTC 3' SEQ ID NO:271
FnIII-1:
   2808-08:
      5' AGCAAGCTTGGACGTCCTGCATTTCACCTC 3' SEQ ID NO:272
   2804-52:
      5' ATTGCGGCCGCGGTGCGAATGTACAAGATCTC 3' SEQ ID NO:273
FnIII-2+ID:
   2804-41:
      5' AGCAAGCTTGAATGCTTCAGTTCCTTCCATTC 3' SEQ ID NO:274
   2804-51:
      5' ATTGCGGCCGCAGTCCTTGCAAAGACGAAGTTG 3' SEQ ID NO:275
FnIII-3:
   2804-42:
      5' AGCAAGCTTGATGCCCGCAGAAGGAGCAG 3' SEQ ID NO:276
   2804-50:
      5' ATTGCGGCCGCTTTAATGGCCACTCTGGTTTC 3' SEQ ID NO:277
L1+CR+L2:
   2804-25:
      5' AGCAAGCTTGGGAGAAATCTGCGGGCCAG 3' SEQ ID NO:278
   2804-23 (SEQ ID NO:272)
L1+CR:
   2804-25: AGC AAG CTT GGG AGA AAT CTG CGG GCC AG (SEQ ID NO:279)
   2804-20 (SEQ ID NO:270)

The primers included *Hind* III and *Not* I site for cloning as described for the IGF-1R (ECD). The IGF-1R subdomains were cloned into the expression vector pCep4avidin-N such that chicken avidin sequence (with endogenous signal sequence) is joined to the N-terminus of the expressed IGF-1R proteins.
Expression of each avidin-fusion protein was achieved by transient transfection of human 293-EBNA cells (Invitrogen) in roller bottles cultures. The cells were grown and maintained in DMEM supplemented with 5% FBS + 1x Non-Essential Amino Acids + 1x Pen Strep Glut + 1x Sodium Pyruvate. Approximately 4-5 x 10⁷ 293-EBNA cells were seeded in 850 cm² roller bottles overnight. The previously seeded cells were then transfected with pCep4-avidin plasmid DNA the following day using FUGENE™ 6 transfection reagent. The DNA - transfection reagent mixture was prepared in approximately in 6.75 mL serum-free DMEM. 675 µl FUGENE™ 6 transfection reagent was first added, followed by 112.5 µg plasmid DNA. The complex was incubated at room temperature for 30 minutes. The entire mixture was then added to a roller bottle. The roller bottle was gassed with a 5% CO₂ gas mixture, capped tightly and placed in a 37° C incubator on a roller rack rotating at 0.35 RPM. The transfection was performed for 24 hours after which the medium was replaced with 100 mL DMEM + 1X Insulin-Transferrin-Selenium Supplement + 1X Pen Strep Glu + 1X Non-Essential Amino Acids + 1X Sodium Pyruvate. Harvest of the condition medium and replacement with fresh medium occurred 48 hr intervals (2-3 cycles). The harvested serum-free conditioned medium was pooled together and clarified by centrifugation at 10,000 x g for 30 minutes at 4° C.

The concentration of avidin-fusion in each conditioned medium was determined using a quantitative FACS based method. The avidin fusion protein in 200 µl of conditioned medium was captured by incubation for 2 hr at room temperature with 5 µl (∼3.5 x 10⁵) of biotin coated polystyrene beads (Spherotech, Inc., Libertyville, IL). The conditioned medium was removed by three cycles of centrifugation and resuspension of the avidin-coated beads in PBS containing 0.5% BSA (BPBS). The avidin-beads were stained with 1 µg/ml of goat FITC-labeled anti-avidin antibody (Vector Lab Burlingame, CA) in 1ml BPBS. After 0.5 hr incubation antibody-beads complexes were collected by centrifugation at 1800 rpm for 5 min and the pellet was washed three times. The FITC fluorescence was detected with a FACSCAN (Beckton Dickson Bioscience, Franklin Lakes, NJ). The signal was converted to protein mass using a standard curve derived with recombinant avidin. For epitope mapping the biotin-beads were loaded with 50-100 ng avidin-fusion protein per ∼3.5 x 10⁵ beads of beads by incubation with the appropriate amount (1-20 ml) of conditioned medium. The loaded beads were washed extensively and resuspended in 1ml BPBS. For all experiment the biotin-beads were blocked with 10% BSA in PBS prior to loading fusion protein.

*Method 1, One Color Assay:* Biotin-coated polystyrene beads loaded with IGF-1R (ECD) and IGF-1R subdomain fusion proteins were mixed with 1 µg of anti-IGF-1R antibody in 1 ml of BPBS. After incubation for 1 hr at room temperature, 4 ml washing buffer was added and the antibody-beads complexes were collected by centrifugation for 5 min at 750g. The pellet was washed 3 times by resuspension in 4 ml of BPBS. The antibody bound to avidin-bead complexes was detected by treatment with 0.5 µg/ml Phycoerythrin-(PE) labeled goat anti-human F(ab')2 (Southern Biotech Associates, Inc., Birmingham, AL) in 1 ml BPBS. Tested antibodies were found to bind to the avidin-fusion protein containing the complete IGF-1R ECD and the L2 domain. Binding to L1, CR or FnIII-1 was not detected in this experiment. A relatively weak reaction was also observed with the L1 domain.

*Method 2, Two color assay:* To simultaneously monitor the amounts of anti-IGF-1R monoclonal antibody and avidin-fusion bound to biotin-beads, FITC-labeled anti-avidin antibody was included (1 µg/ml) was included in the binding reaction in combination with 0.5 µg/ml PE-labeled goat anti-human IgG1. The beads were prepared for FACSCAN analysis as described for the one color assay.

*Method 3, Antibody Competition:* To prepare for labeling with fluorescein the antibodies were dialyzed or resuspended at a concentration of 1 mg/ml in PBS (pH 8.5). Label ([6-fluorescein-5- (and-6)-carboxamido] hexanoic acid, succinimidyl ester 5(6)-SFX] mixed isomers from Molecular Probes (Eugene, OR, Cat. No. F2181) was added to the protein at a molar ratio 9.5:1 (label: protein) from a label stock of 5mg/ml in DMSO. The mixture was incubated at 4° □C overnight in the dark. The labeled antibody was separated from the free label by dialysis in PBS. The FITC/ antibody ratios obtained ranged from 3 to 8. For each competition experiment, a binding reaction was assembled that contained a 50 fold excess (10-50 µg/ml) of unlabeled competitor antibody, 3.5 x 10⁵ biotin beads coated with avidin fusion protein in BPBS. The FITC-labeled antibody (1 µg/ml) was added after a 30 min preincubation. The process followed the one color method from this point forward.

Each of the four tested antibodies binds to the IGF-1R L2 domain, as shown in Table 8. However, the precise amino acid contacts of each antibody in the IGF-1R L2 domain may differ.

**Table 8**

| Antibody | L1¹ | CR¹ | L2¹ | FnIII-1¹ | ECD^{1,2} |
|---|---|---|---|---|---|
| TQ11C | No | No | Yes | No | Yes |
| TQ25 | No | No | Yes | No | Yes |
| TQ58 | Yes | No | Yes | No | Yes |
| TQ59 | No | No | Yes | No | Yes |

| | | | | | |
|---|---|---|---|---|---|
| ¹ Epitope mapping was performed with avidin-IGF-1R fusion proteins containing the indicated human IGF-1R regions. ² The ECD fusion contains L1+CR+L2+FnIII-1+FnIII-2+ID+FnIII-3. | | | | | |

### EXAMPLE 13: Antibody Binding to Cell-Surface IGF-1R

This example provides a method for detecting the binding of an anti-IGF-1R antibody to cell-surface expressed IGF-1R.

The ability of antibodies TQ11C, TQ25, TQ58, and TQ59 to bind to human IGF-1R displayed on the cell surface was evaluated using Balb/C 3T3 fibroblasts and MCF-7 human breast cancer cells engineered to overexpress the human IGF-1R receptor at a level of ∼3-4 x 10⁵ molecules per cell. A Balb/C 3T3 cell line that stably overexpresses the human IGF-1R (∼3 x 10⁵ receptors per cell) was derived using with a retroviral vector essentially as described by Pietrzkowski et al., 1992, Cell Growth Differentiation 3:199-205. MCF-7 breast cancer cells that overproduce huIGF-1R were transfected with a pcDNA3.1 expression vector (Invitrogen Corp.). Zeocin resistant cells that express a high level of hu IGF-1R (∼4 x 10⁵ receptors per cell) were expanded after selection by FACS using anti-IGF-1R monoclonal antibody αIR3 and an PE-labeled goat anti murine IgG antibody (Caltag Laboratories, Burlingame, CA). The process of selection and expansion was repeated four times.

IGF-1R Receptor antibody staining and receptor expression was monitored by FACS as follows: the cells were released from T175 flasks (Corning) by washing 2 times with excess PBS (Ca/Mg free) followed by treatment with 5 ml of Cell Dissociation Buffer (Sigma) for 10 min at room temperature. The cells were collected by centrifugation and washed two times by resuspending them in PBS and centrifugation. For primary antibody staining, 1 µg of antibody was added to 10⁶ cells resuspended in 100 µl PBS plus 0.5% BSA (BPBS) and the cells were incubated at 4° ^{□}C for 1.5 hr. The cells were collected by centrifugation and washed twice with BPBS to remove unbound primary antibody. The cells were resuspended in 100 µl of BPBS and incubated with 1 µg of FITC-labeled goat anti-human F(ab')2 (Southern Biotechnology Associates, Inc., Birmingham, AL) at 4°□C for 30 minutes. After washing to remove unbound FITC secondary antibody, the cells were resuspended in 1 ml of PBS+ 0.5% BSA and FITC cell fluorescence was detected with a FACSCAN (Beckton Dickson Bioscience, Franklin Lakes, NJ). The fluorescence levels were converted to absolute receptor levels using Quantum microbead (Bangs Laboratories, Inc., Fishers, IN) with predetermined IgG1 binding capacity to generate a standard curve. Data reduction was performed with QuickCal v2.1 software (Verity Software House, Topsham, ME) provided by the manufacturer.

The peak fluorescent intensity of anti-1GF-1R antibody labeling of the IGF-1R overexpressors was increased 10-20 fold relative to parental Balb/C 3T3 and MCF-7 cells for each of the tested antibodies. This is the result predicted for an antibody that specifically binds IGF-1R. Background fluorescence of cells treated with no antibodies or FITC-labeled secondary alone were insignificant.

### EXAMPLE 14: Inhibition of IGF-1R

This example presents methods of detecting inhibition of IGF-1R by anti-IGF-I R antibodies.

### 32D hu IGF-1R+IRS-1 Cell Inhibition

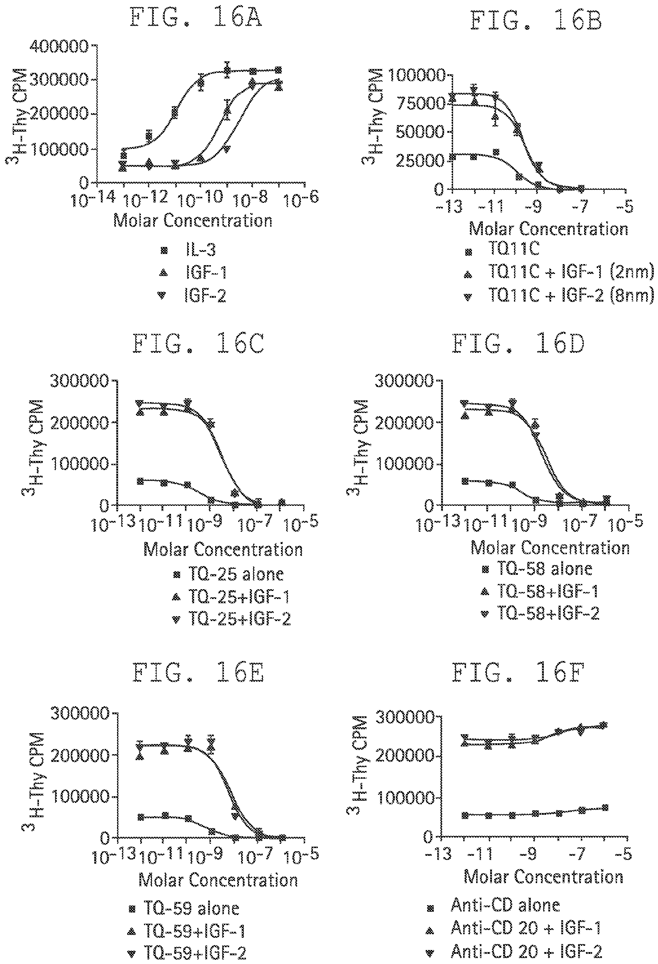

Murine 32D cells that coexpress the human IGF-1R receptor (20K per cell) and human IRS-1 have proven to be a effective system to examine the molecular components IGF-1R signaling Valentinis et al., 1999, J Biol Chem 274:12423-30. Normal 32D cells express relatively low levels of the murine orthologs of these two gene products. 32D cell normally required IL3 for growth and survival. IGF-1 or IGF-2 can replace IL3 in 32D huIGF-1R+IRS-1 cells as shown in Figure 16, panel A. The EC₅₀ to the IGF-1 dose response curve was about 0.5 nM, whereas the IGF-2 EC₅₀ (2.8 nM) is about six fold higher reflecting weaker affinity of IGF-2 for IGF-1R. To assess the ability of the antibodies TQ11C, TQ25, TQ58, and TQ59 to block IGF-1 or IGF-2 stimulation, 96-well microtitre plates were seeded with 30,000 32D hu IGF-1R+IRS-1 cells per well in a volume of 200 µl of RPMI (Gibco/BRL) containing 5% fetal bovine serum (Gibco/BRL) and 1x penicillin, streptomycin, glutamine (Giboco/BRL) and increasing concentrations of antibody (10⁻¹²M to 10⁻⁶M) or no antibody. IGF-1 (2 nM), IGF-2 (8 nM) or nothing was added after 1 hr preincubation with antibody. ³H-thymidine (1 µCi per well) was added at 27 hr post-antibody addition. The cells were harvested 21 hr later, and incorporation of ³H- thymidine into DNA was determined for each sample. The assays were performed in triplicate. An anti-CD20 antibody was used as a negative control. Each of antibodies TQ11C, TQ25, TQ58, and TQ59 was able to completely block the IGF-1 and IGF-2 mediated stimulation of the 32D cells. The reduction of background proliferation in the absence of added IGF-1 and IGF-2 is due to the inhibition of serum IGF-1 and IGF-2. The binding data were analyzed using GraphPad PRIZM™ software. The data are shown in Figure 16.

### Balb/C 3T3 hu IGF-1R Cell Inhibition

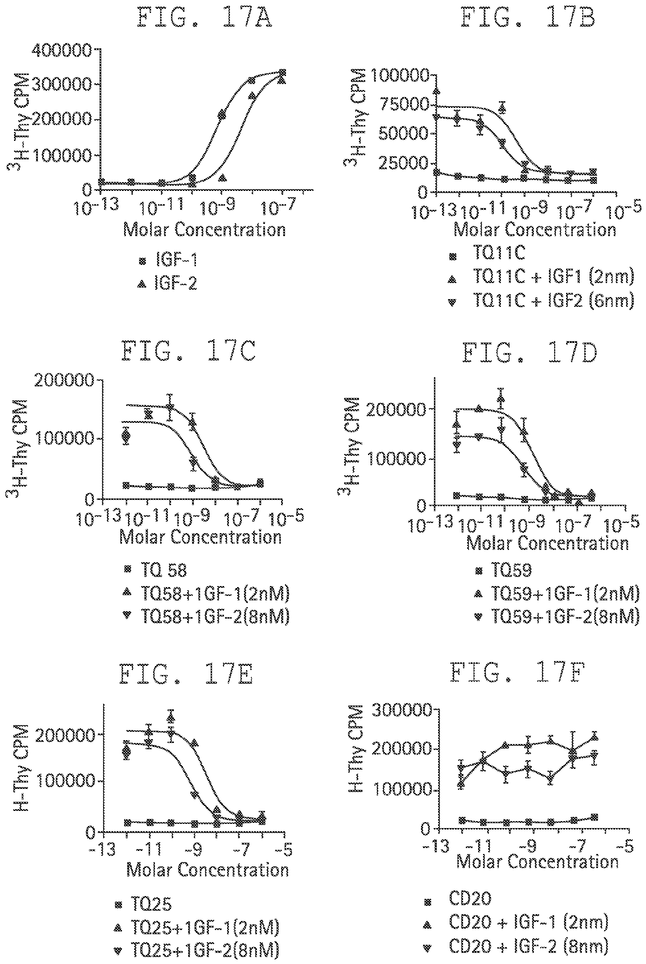

IGF-1 greatly stimulates the incorporation of ³H-thymidine by serum-starved cultures of mouse embryonic fibroblasts (Balb/C 3T3 or NIH 3T3) that overexpress IGF-IR (∼1 x 10⁶ IGF1R per cell). Kato et al., 1993, J Biol Chem 268:2655-61; Pietrzkowski et al., 1992, Cell Growth Differentiation 3:199-205. This phenomenon is recapitulated with both IGF-1 and IGF-2 in a Balb/C 3T3 cell line hu IGF-1R overexpressor. Both growth factors stimulated ³H-thymidine incorporation by about 20-fold. The EC₅₀ of the IGF-1 dose response curve was about 0.7 nM, whereas the IGF-2 EC₅₀ (4.4 nM) is sevenfold higher, indicating a weaker affinity of IGF-2 for IGF-1R. To assess the ability of a given antibody to block IGF-1 or IGF-2 stimulation, 96-well microtitre plates were seeded with 10,000 cells per well in a volume of 200 µl of DMEM (Gibco/BRL) containing 10% calf serum (Gibco/BRL) and 1x penicillin, streptomycin, glutamine (Giboco/BRL). After overnight incubation when the cells were about 80% confluent the growth medium was replaced with 100 µl DMEM containing 0.1% BSA after washing once with 200 µl PBS. Antibodies at increasing concentrations (10⁻¹²M to 10⁻⁶M), or no antibody, were added at 24 hr post-serum starvation. IGF-1 (2 nM), IGF-2 (8 nM) and ³H-thymindine (1 µCi per well) were added after a 1 hr preincubation with antibody. The cells were harvested 24 hr later, and incorporation of ³H- thymidine into DNA was determined for each sample. The assays were performed in triplicate. Each tested antibody was able to completely block the IGF-1 and IGF-2 mediated stimulation of Balb/C 3T3 cells, as shown in Figure 17. An anti-CD20 antibody was used as a negative control ("CD20" in Figure 17).

### EXAMPLE 15: Treatment of cancer in humans with an anti-IGF-1R antibody

This example demonstrates that inhibition of the IGF-1R pathway is effective for treating a variety of types of tumors in human subjects.

Human subjects were selected for treatment in a First in Human Phase 1 clinical trial with a fully-human anti-human IGF-1 receptor IgG1 monoclonal antibody comprising the light chain variable domain identified herein as L16 and the heavy chain variable domain identified herein as H16 ("Study Drug"), as shown in Table 9.

**Table 9**

| **Cohort #1 (1mg/Kg)** | |
|---|---|
| **Subject #5** | |
| Diagnosis | Thymus |
| Baseline TM (cm) | 10 |
| Antibody per Dose (mg) | 92.5 |
| Dosed at Days | 1, 15, 29 |
| Day 50 Tumor (cm) | 10.4 (+4%) |

| | |
|---|---|
| **Subject #8** | |
| Diagnosis | Unknown |
| Baseline TM (cm) | 18.5 |
| Antibody per Dose (mg) | 84.1 |
| Dosed at Days | 1, 15, 29, 57, 71, 85, 99, 113, 127, 141, 155 |
| Day 50 Tumor (cm) | 18.2 (-2%) |
| Day 106 Tumor (cm) | 18.9 (+2%) |
| Day 162 Tumor (cm) | 23.2 (+25%) |

| | |
|---|---|
| **Subject #7** | |
| Diagnosis | Adenoid |
| Baseline TM (cm) | 31.1 |
| Antibody per Dose (mg) | 60 |
| Dosed at Days | 1, 15, 29, 57, 71, 85 |
| Day 50 Tumor (cm) | 30.9(-1%) |

| **Cohort #2 (3mg/Kg)** | |
|---|---|
| **Subject #1** | |
| Diagnosis | Nerve Sheath |
| Baseline TM (cm) | 1.1 |
| Antibody per Dose (mg) | 208 |
| Dosed at Days | 1, 15, 29 |
| Day 50 Tumor (cm) | 1.4 (+27%) |

| | |
|---|---|
| **Subject #11** | |
| Diagnosis | Carcinoid |
| Baseline TM (cm) | 13.1 |
| Antibody per Dose (mg) | Week 1-35: 207 Week 39 and on: 828 |
| Dosed at Days | 1, 15, 29, 57, 85, 99, 120, 134, 148, 162, 176, 190, 204, 218, 232, 246, 260, 274, 296, 308, 331 |
| Day 50 Tumor (cm) | 14 (+7%) |
| Day 106 Tumor (cm) | 11 (-16%) |
| Day 169 Tumor (cm) | 10.6 (-19%) |
| Day 225 Tumor (cm) | 8.4 (-36%) |
| Day 281 Tumor (cm) | 8.2 (-37%) |
| Day 338 Tumor (cm) | 6.8 (-48%) |

| **Cohort #3 (10mg/Kg)** | |
|---|---|
| **Subject #2** | |
| Diagnosis | Prostate |
| Baseline TM (cm) | 15.6 |
| Antibody per Dose (mg) | 790 |
| Dosed at Days | 1, 15, 29 |
| Day 50 Tumor (cm) | 18.8 (+21%) |

| | |
|---|---|
| **Subject #6** | |
| Diagnosis | Melanoma |
| Baseline TM (cm) | 28.1 |
| Antibody per Dose (mg) | 854.5 |
| Dosed at Days | 1, 15, 29, 57, 71 |
| Day 50 Tumor (cm) | 28.4 (+1%) |

| | |
|---|---|
| **Subject #4** | |
| Diagnosis | Colorectal |
| Baseline TM (cm) | 42.2 |
| Antibody per Dose (mg) | 895 |
| Dosed at Days | 1, 15, 29 |
| Day 50 Tumor (cm) | 45.3 (+7%) |

| **Cohort #4 (20mg/Kg)** | |
|---|---|
| **Subject #3** | |
| Diagnosis | Ovarian |
| Baseline TM (cm) | 15.9 |
| Antibody per Dose (mg) | 2118 |
| Dosed at Days | 1, 15, 29 |
| Day 50 Tumor (cm) | 18.6 (+17%) |

| | |
|---|---|
| **Subject #9** | |
| Diagnosis | Breast |
| Baseline TM (cm) | 4.8 |
| Antibody per Dose (mg) | 1570 |
| Dosed at Days | 1, 15, 29, 57, 71 |
| Day 50 Tumor (cm) | 4.7 (-2%) |

| **Cohort #5 (12mg/Kg)** | |
|---|---|
| **Subject #12** | |
| Diagnosis | Ewing's |
| Baseline TM (cm) | 9.8 |
| Antibody per Dose (mg) | 1190 |
| Dosed at Days | 1, 15, 29, 57, 71 |
| Day 50 Tumor (cm) | 2.2 (-78%) |
| Day 85 Tumor (cm) . | 0.0 (-100%) |

| **Cohort #6 (20mg/Kg)** | |
|---|---|
| **Subject #10** | |
| Diagnosis | Adenoid R eye |
| Baseline TM (cm) | 38.7 |
| Antibody per Dose (mg) | 1763.6 |
| Dosed at Days | 1, 15, 29, 57, 71 |
| Day 50 Tumor (cm) | 35.2 (-9%) |

Prior to being selected for the study, each subject had failed available conventional treatments for his or her particular tumor disease, if such treatments were available, and was receiving only supportive care.

Each subject was assigned to one of six dosing cohorts. Subjects in any given cohort each received the same dose of the Study Drug intravenously. Dosing between cohorts ranged from 1 to 20 milligrams of Study Drug per kilogram of subject's body mass (mg/kg), as shown in Table 9. The Study Drug was formulated at 30 mg/ml in 10mM acetate, pH 5.2, 5.0% w/v sorbitol, and 0.004% w/v Polysorbate 20. During the course of treatment, the subjects received the Study Drug as their only anti-tumor treatment. The subjects also received individualized palliative care, as appropriate, to reduce the severity of their symptoms.

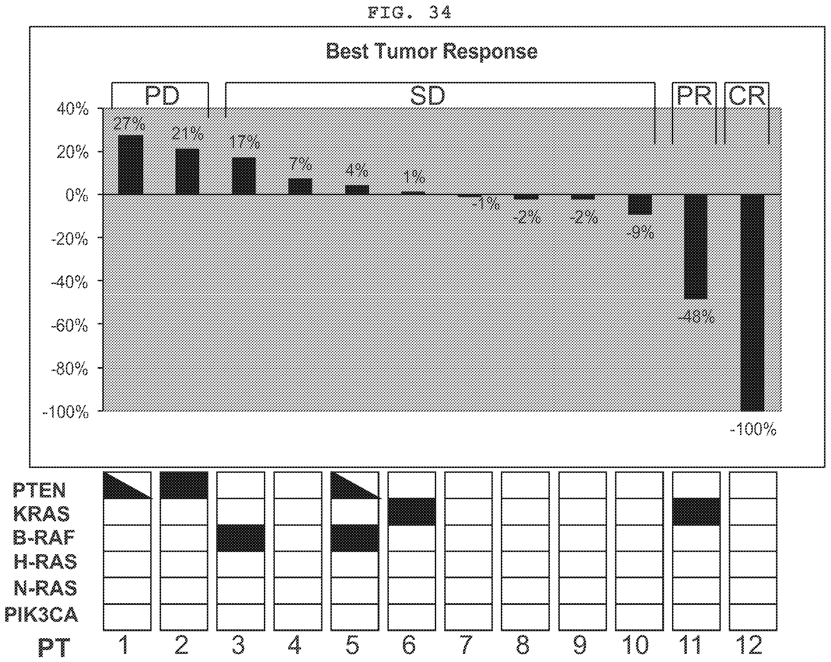

Response to treatment was assessed using the Response Evaluation Criteria in Solid Tumors (RECIST) criteria as described in Therasse et al. 2000, J Natl Cancer Inst. 92:205-16. Briefly, prior to administration of the first dose, each subject was given a computerized tomography (CT) scan to determine the length of the largest measurable tumor along its longest diameter ("Baseline TM (cm)" in Table 9). CT scans were used to measure the same tumors along the same diameter at certain points after initiation of treatment ("Day X Tumor (cm)" in Table 9). Each such measurement was compared to the baseline tumor measurement for the same subject to calculate the percent increase or decrease in tumor size. As shown in Figure 34 and in Table 9, two of the subjects showed a reduction in tumor size of at least 30%. One of these subjects was classified as a partial responder (PR) according to RECIST. The other had a 100% reduction in tumor dimension and so was classified as a complete responder (CR) according to RECIST. Eight other subjects had as a best response either a reduction of tumor size of less than 30% or an increase of less than 20%, and so are classified as having stable disease (SD) using RECIST criteria (note that one of these subject's had as a best response an initial 2% reduction in tumor size, but that subsequently the tumor showed and overall increase in size of 25%). Each of these subjects (except the CR subject, discussed below) eventually showed disease progression and was taken off of study. The remaining two subjects had RECIST tumor measurements that increased by more than 20%, indicating a best response of progressive disease (PD).

The CR subject had classical Ewing's sarcoma (characterized by a EWS-FLI genetic translocation; see, *e.g.,* Dagher et al., 2001, J Pediatr Hematol Oncol. 23:221-24; Morishita et al., 2001, Mol Biotechnol. 18:97-104) that had formed large metastatic tumors in the lungs, making breathing difficult, particularly while lying prone. The subject was resistant to multiple prior chemotherapy regimens, including 1) adriamycin and cytoxan, 2) ifosphamide and vincristine, 3) topotecan and vincristine, 4) taxotere, and 5) gemcitabine. The subject received a first dose of 12mg/kg of anti-IGF-1R antibody. The subject experienced significant symptomatic relief within two days of receiving the first dose of the Study Drug, allowing him to comfortably sleep in a prone position for the first time in several months. The subject subsequently received three doses of 12 mg/kg at 14 day intervals. Fifty days after the first injection, a CT scan of the subject showed a decrease in tumor size from the baseline measurement of 9.8 cm to 2.2 cm, or 78%, using RECIST. At day 50, the subject was also given a PET scan, which showed no detectable uptake of labeled glucose, indicating that most or all of the remaining tumor tissue was dead. At day 85, the subject underwent a CT scan that showed a complete resolution of tumor from the pre-treatment diameter of 9.8 cm to 0 cm. The subject continued to receive 12 mg/kg of the Study Drug at 14 day intervals and at day 434 still had a CR according to RECIST.

The PR subject had a mid gut carcinoid tumor and achieved a partial response after 33 weeks in the trial with a RECIST tumor dimension decrease from 13.1 to 6.8 cm, or 48%. The subject continued to receive 3mg/kg of the Study Drug at 14 day intervals and showed a maximum RECIST tumor dimension reduction of 63%. At day 655, the subject was discovered to have new bone metastases and was taken off of the study.

Some subjects exhibited grade 3 or 4 thrombocytopenia. In every case where thrombocytopenia was detected, it resolved spontaneously with cessation or interruption of dosing. There were no cases of spontaneous bleeding noted in these subjects.

Additional patients were treated on this study who also had diagnoses of either Ewing's sarcoma or Desmoplastic Small Round Cell Tumors. Each of these subjects had had multiple prior cytotoxic chemotherapy regimens and had subsequently shown progression. Twelve such subjects received either 12 mg/kg (n=6) or 20 mg/kg (n = 6) of the Study Drug at two week intervals. Table 10 shows the results for the study.

**Table 10**

| **Subject Number** | **Study Drug Dose** | **Translocation** | **Study Status** | **PET D8** | **Best Response** |
|---|---|---|---|---|---|
| 1 | 20 mg/kg | N/A | Off at day 127 | -32% | SD |
| 2 | 12 mg/kg | N/A | Off at day 114 | -10% | SD |
| 3 | 20 mg/kg | N/A | Off at day 79 | -57% | N/A |
| 4 | 20 mg/kg | N/A | Off at day 58 | -60% | PD |
| 5 | 12 mg/kg | N/A | Off at day 57 | +16% | PD |
| 6 | 12 mg/kg | N/A | Off at day 48 | +10% | PD |
| 7 | 20 mg/kg | Negative | Off at day 43 | +11% | PD |
| 8 | 12 mg/kg | N/A | Off at day 39 | +25% | PD |
| 9 | 20 mg/kg | "EWS-FLI" | Off at day 37 | -11% | PD |
| 10 | 12 mg/kg | Negative | Off at day 35 | +1% | PD |
| 11 | 20 mg/kg | N/A | Off at day 34 | -35% | PD |
| 12 | 12 mg/kg | "EWS-FLI" | Off at day 23 | -12% | PD |

Two subjects were classified as having a best response of SD using RECIST criteria. One of them showed a reduction in tumor metabolic activity of 32%, the other of 10%, on day 8 according to a PET scan. A third subject achieved a PR according to RECIST and a 57% reduction in metabolic activity on day 8. The tumors in all three subjects subsequently progressed, and so the subjects were taken off of the study. The remaining subjects all showed progressive disease as a best response and were taken off of the study, although several of them showed reductions in metabolic activity on day 8 of between 11% and 35%.

The tumor genotypes of the three best responders were not available. However, two of the subjects who showed a reduction in metabolic activity on day 8 (but whose best RECIST response was PD) were found to contain the EWS-FLI translocation. Two other subjects who showed a best RECIST response of PD, and who showed no change or a slight increase in tumor metabolic activity on day 8, were found to not have the translocation.

Another study was done in subjects with carcinoid tumors. Five subjects were given either 6 (n = 1) or 20 mg/kg (n = 4) of the Study Drug at two week intervals. The results are shown in Table 11.

**Table 11**

| **Subject Number** | **Study Drug Dose** (mg/kg) | **Study Status** | **RECIST** | **Best Response** |
|---|---|---|---|---|
| 1 | 20 | Off at day 288 | -32% | PR |
| 2 | 20 | Continued past day 378 | -20% | SD |
| 3 | 20 | Continued past day 282 | -2% | SD |
| 4 | 6 | Off at day 112 | N/A | SD |
| 5 | 20 | Off at day 191 | -5% | SD |

Each of the subjects was enrolled in the study after having tried and failed other treatments. Subject 1 showed a best response of PR (32% reduction in tumor size according to RECIST criteria). The remaining subjects showed best responses of SD, with between a 2% and 20% reduction in tumor size according to RECIST criteria.

Subjects 2 and 3 remained on the study past day 378 and day 282, respectively. Subject 1 was removed from the study on day 288 after showing progressive disease. Subject 4 was removed from the study on day 112 for noncompliance. Subject 5 was removed from the study on day 191 after developing a pulmonary embolus.

Another study was done in subjects with colorectal cancer (CRC). Seven subjects were each given 6 mg/kg of panitumumab (a human anti-EGF receptor antibody) and either 6 (n = 3) or 12 mg/kg (n = 4) of the Study Drug at two week intervals. The results are shown in Table 12.

**Table 12**

| **Subject Number** | **Panitumumab Dose** (mg/kg) | **Study Drug Dose** (mg/kg) | **Study Status** | **Prior EGFR** | **Best WHO Response** | **Wk 8 change (WHO)** | **Wk 8 change (RECIST)** |
|---|---|---|---|---|---|---|---|
| 1 | 6 | 6 | Off at day 99 | Yes | SD | N/A | N/A |
| 2 | 6 | 6 | Off at day 113 | Yes | SD | -39% | -27% |
| 3 | 6 | 6 | Off at day 58 | - | PD | N/A | N/A |
| 4 | 6 | 12 | Off at day 168 | Yes | SD | -19% | -5% |
| 5 | 6 | 12 | Continued Past Day 191 | Yes | PR | -54% | -36% |
| 6 | 6 | 12 | Off at day 7 | Yes | PD** | | |
| 7 | 6 | 12 | Off at day 57 | No | PD*** | -7% | +1% |

| | | | | | | | |
|---|---|---|---|---|---|---|---|
| * "Yes" indicates subject previously treated with EGF receptor inhibitor ** Newly discovered brain metastases at day 7 *** Progression of non-index lesions at day 57 | | | | | | | |

All of the subjects had advanced solid malignancies refractory to standard therapy. In table 12, "Yes" in the "Prior EGFR" column means that the subject had previously been treated with an anti-EGF receptor antibody (either panitumumab or cetuximab). "Best WHO Response" and "Wk 8 CT change (WHO)" refer to tumor assessments done using WHO criteria (Miller et al., 1981, Cancer 47:207-14).

Subject 5 showed a best WHO response of PR. The tumors of subject 5, who experienced a best WHO response of PR and who continued on the study past day 191, were found to have a wild-type allele of KRAS. Before beginning the study, subject 5 had failed four prior chemotherapy regimens and five cycles of irinotecan and cetuximab.

Three subjects with non-CRC tumors also received 6 mg/kg panitumumab and 6 mg/kg of Study Drug and had their best responses evaluated according to WHO criteria. None of these subjects had previously been treated with an EGF receptor inhibitor. A first subject with a thyroid tumor showed a best response of progressive disease and was removed from the study on day 55. This subject was prediabetic prior to participation in the study, with a fasting glucose level of 113 mg/dL, and experienced a dose limiting toxicity of Grade 3 hyperglycemia. A second subject with a GE Junction tumor had a best response of stable disease and was removed from the study on day 114. A third subject with a pancreatic tumor had a best response of stable disease and was removed from the study on day 106.

Another study was done using Study Drug in combination with gemcitabine treatment in subjects with a variety of tumor types. Eleven subjects were each given three doses of gemcitabine at 1000 mg/kg every four weeks and were also given Study Drug at either 6 (n = 6) or 12 mg/kg (n = 5) every 2 weeks. The results are shown in Table 13.

**Table 13**

| **Subject Number** | **Study Drug Dose** (mg/kg) | **Diagnosis** | **Study Status** | **DLT** | **Best WHO Response** |
|---|---|---|---|---|---|
| 1 | 6 | Ovarian | Off at day 157 | No | SD |
| 2 | 6 | Ovarian | Off at day 126 | No | SD |
| 3 | 6 | Lung | Off at day 53 | Yes* | PD |
| 4 | 6 | Carcinoid | Off at day 112 | No | SD |
| 5 | 6 | Lung | Off at day 56 | No | SD |
| 6 | 6 | Head and Neck | Off at day 123 | No | SD |
| 7 | 12 | Colon | Off at day 106 | No | SD |
| 8 | 12 | Breast | Off at day 184 | No | SD |
| 9 | 12 | Colon | Continued past day 116 | No | SD |
| 10 | 12 | Prostate | Continued past day 114 | No | SD |
| 11 | 12 | Gallbladder | Continued past day 47 | No | N/A |

| | | | | | |
|---|---|---|---|---|---|
| * Grade 4 Neutropenia on day 8 | | | | | |

All but one evaluated subject had a best response according to WHO criteria of stable disease. Subject 3 had a best response of progressive disease, and also showed a dose limiting toxicity ("DLT" in Table 13) of Grade 4 neutropenia on day 8.

### EXAMPLE 16: Correlation of Molecular Markers with Response to Inhibition of IGF-1 Receptor Signaling

This example demonstrates that molecular markers can be used to determine whether a subject is more likely or less likely to respond to an anti-tumor treatment comprising an inhibitor of IGF-1 receptor signaling.

The presence or absence of certain biomarkers was found to correlate with the response of subjects to treatment with an inhibitor of IGF-1 receptor signaling. Of the subjects listed in Table 9, both of the subjects with disease progression (PD) after eight weeks of treatment exhibited a reduction of PTEN expression (complete loss of PTEN expression in 10% of the tumor cells observed in one subject, complete loss of PTEN in 5% of tumor cells in the other subject) as assessed by immunohistochemical staining of archival formalin fixed paraffin embedded tumor sections by a contract laboratory (Ventana Medical Systems, Tucson, AZ), as shown in Figure 34. PTEN expression was completely eliminated (absent in 100% of tumor cells) in one subject with stable disease (this subject exhibited a 4% increase in his tumor RECIST measurement). PTEN loss was not observed in either subject who had a PR or a CR to treatment with the anti-IGF-1R antibody.

The subject showing a complete loss of PTEN expression in 5% of tumor cells also was found to have a PTEN loss of function mutation (D331G).

An activating mutation of the gene encoding KRAS that changed the glycine normally found at codon twelve to a cysteine (*i.e.,* KRAS G12C) was observed in the PR subject with the mid gut carcinoid tumor and in another subject with metastatic melanoma who had stable disease after eight weeks of treatment (RECIST 1% increased).

To further define the relationship between PTEN genotype and responsiveness to treatment with an anti-IGF-1 receptor inhibitor, six human tumor cell lines were identified that display negative PTEN status. Their sensitivity to an anti-IGF-1R antibody was tested *in vivo* in a mouse xenograft model. The cell lines used were PC-3 and LnCap (prostate), U-87MG (Glioblastoma), Cal-51 (Breast), 786-0 (Kidney), and Colo-320 (Colon/carcinoid). Five million cells of each of these cell lines were injected subcutaneously in the left flank of 4-6 week old female athymic nude mice. When the average tumor size reached approximately 200-220 mm³, mice were randomly assigned into groups (10 mice/group). Therapy with anti-IGF-1R antibody ("Antibody") at three doses (30, 100, or 300 µg/dose), or human IgG1 control ("Control"; 300 µg/dose) started on randomization day and continued until the end of each study. Administration of Antibody orControl occurred twice per week, intraperitoneally. Tumor volume and body weight of each animal were measured twice per week using calipers and an analytical scale, respectively. Data were gathered as mean +/standard error. Cell lines were considered responsive to Antibody if a statistically significant decrease in tumor volume was measured between any dose group and the Control group. For the statistical analysis, repeated measures ANOVA (RMANOVA), post-hoc Scheffe, was employed. Results are shown in Table 14. Xenograft data showed that none of the six PTEN null models studied was sensitive to Antibody. In contrast, all sensitive xenograft models displayed wild-type PTEN status. These data support the clinical observations and support the use of PTEN status as a negative stratification marker for treatment with IGF-1R inhibitors.

**Table 14**

| Cell Line | PTEN Status | p53 Status | Tumor Type | Antibody p<0.05 TGI |
|---|---|---|---|---|
| Colo 205 | WT | Mut | Colon | Yes |
| DLD-1 | WT | Mut | Colon | Yes |
| BT-474 | WT | Mut | Breast | Yes |
| BxPC-3 | WT | Mut | Pancreas | Yes |
| MiaPaCa | WT | Mut | Pancreas | Yes |
| SJSA-1 | WT | mdm2 | Osteosarcoma | Yes |
| U-87MG | Null | Wt | GMB | No |
| Cal-51 | Null | Wt | BBC | No |
| PC-3 | Null | Mut | Prostate | No |
| LnCap | Null | Wt | Prostate | No |
| Cal-51 | Null | Mut | Breast | No |
| 786-O | Null | Mut | Kidney | No |
| Colo-320 | Null | Mut | Colon/Carcinoid | No |

### EXAMPLE 17: A Placebo-Controlled, Randomized Phase 2 Study of Conatumumab (AMG 655) or Ganitumab (AMG 479) or Placebo Plus Gemcitabine in Patients with Metastatic Pancreatic Cancer

This example demonstrates that ganitumab increased median overall survival and progress free survival in a Phase 2 clinical study in metastatic pancreatic cancer patients.

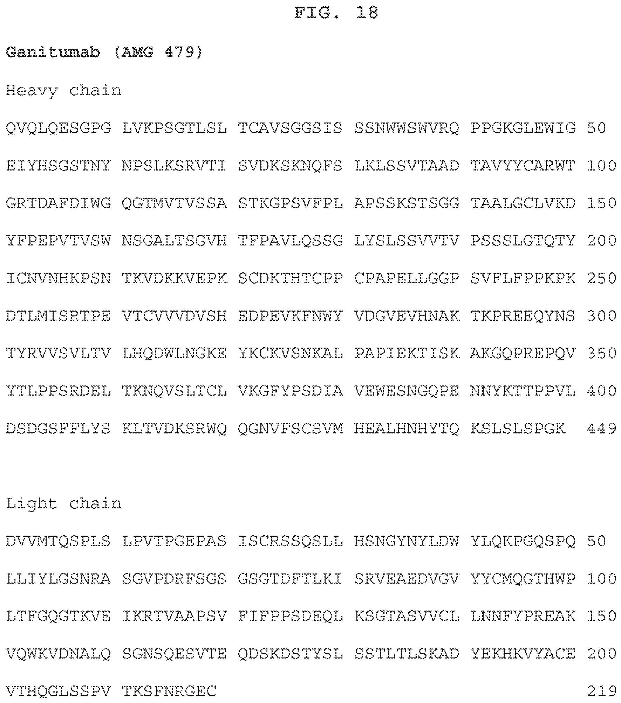

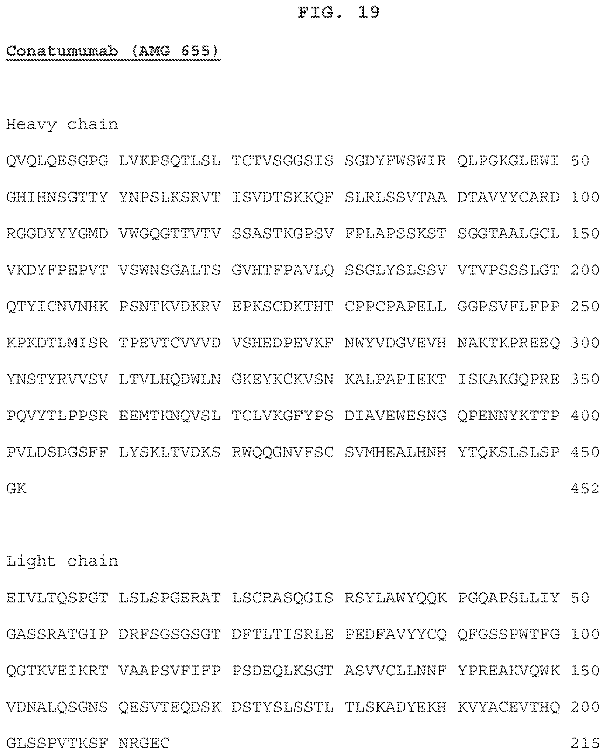

A three-arm, placebo-controlled, randomized phase 2 study was conducted to evaluate ganitumab (Figure 18) plus gemcitabine or conatumumab (Figure 19) plus gemcitabine versus placebo plus gemcitabine in metastatic pancreatic cancer patients.

The primary endpoint was six month overall survival rate. Secondary endpoints were response rate (by RECIST; Investigator defined, no central review), progression-free survival (PFS, investigator defined), overall survival (OS), incidence of adverse events and laboratory abnormalities, incidence of anti-ganitumab or anti-conatumumab antibodies, pharmacokinetics of ganitumab and conatumumab, and dose intensity of gemcitabine when combined with ganitumab, conatumumab, or placebo.

Key inclusion criteria were histologically or cytologically documented metastatic adenocarcinoma of the pancreas, age eighteen years or older, ECOG PS 0 or 1, adequate hematologic, hepatic, renal, and coagulation function, amylase and lipase ≤ 2.0 x ULN, adequately controlled type 1 or 2 diabetes (amended during protocol to fasting blood glucose < 160 mg/mL only), fasting blood glucose ≤ 160 mg/mL, HbA1c < 8%, and written informed consent.

Key exclusion criteria were uncontrolled cardiac disease and prior chemotherapy or radiation in the adjuvant or metastatic setting.

The study was designed as an estimation study with a planned sample size of 120 patients (40 patients/arm); the actual number of patients was greater as shown in Table 15. As designed (estimation, not hypothesis-testing), the trial had 80% power to detect a difference between a 6-month OS rate of 45% for placebo, 69% for conatumumab or ganitumab (target improvement, 10%).

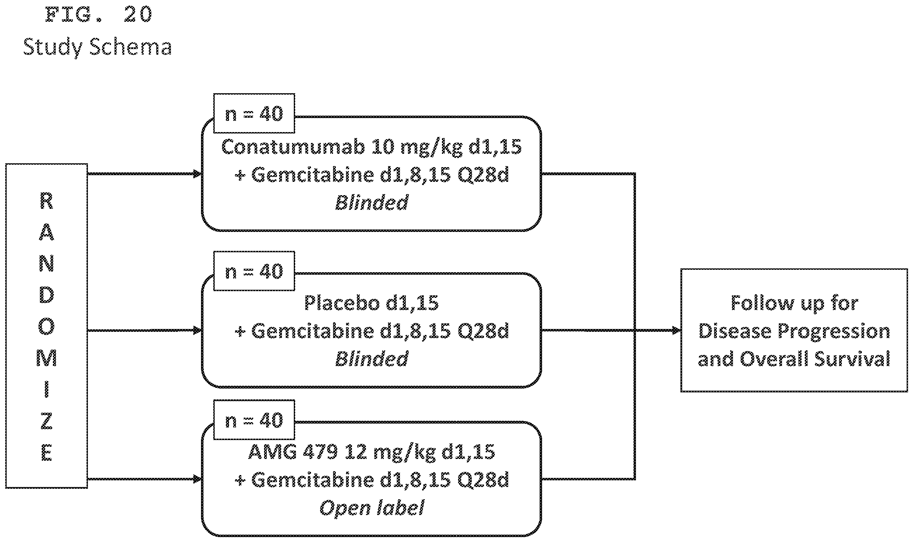

The study schema is shown in Figure 20.

**Baseline Demographics are shown in Table 15:**

| | **Conatumumab 10 mg/kg (Q2W)** | **Ganitumab 12 mg/kg (Q2W)** | **Placebo** |
|---|---|---|---|
| | **(N = 41)** | **(N = 42)** | **(N = 42)** |
| **Sex - n (%)** | | | |
| **Male** | **24 (59)** | **25 (60)** | **26 (62)** |
| **Female** | **17 (41)** | **17 (40)** | **16 (38)** |

| **Race/ethnicity - n (%)** | | | |
|---|---|---|---|
| **White or Caucasian** | **32 (78)** | **35 (83)** | **37 (88)** |
| **Black or African American** | **3 (7)** | **3 (7)** | **3 (7)** |
| **Hispanic or Latino** | **5 (12)** | **4 (10)** | **2 (5)** |
| **Asian** | **1 (2)** | **0 (0)** | **0 (0)** |

| **Age (years) at Randomization** | | | |
|---|---|---|---|
| **Mean** | **62.3** | **62.1** | **62.6** |
| **Median** | **61.0** | **66.0** | **61.0** |
| **Min, Max** | **45, 80** | **37, 82** | **43, 82** |

| **ECOG performance status at enrollment - n (%)** | | | |
|---|---|---|---|
| **0** | **17 (41)** | **19 (45)** | **16 (38)** |
| **1** | **24 (59)** | **23 (55)** | **26 (62)** |

**Lesion sites at baseline are described in Table 16:**

| | **Conatumumab 10 mg/kg (Q2W) + gemcitabine** | **AMG 479 12 mg/kg (Q2W) + gemcitabine** | **Placebo + gemcitabine** | **All Subjects** |
|---|---|---|---|---|
| | **(N = 41)** | **(N = 42)** | **(N = 42)** | **(N = 125)** |
| **Lesions in the Pancreas - n (%)** | | | | |
| **Head** | **11(27)** | **12(29)** | **12(29)** | **35(28)** |
| **Body/Tail** | **7(17)** | **9(21)** | **6(14)** | **22(18)** |
| **Neck** | **1(2)** | **2(5)** | **3(7)** | **6(5)** |
| **NOS** | **13(32)** | **12(29)** | **15(36)** | **40(32)** |
| | | | | |

| **Lesions outside the Pancreas - n (%)** | | | | |
|---|---|---|---|---|
| **Liver** | **27(66)** | **29(69)** | **33(79)** | **89(71)** |
| **Lung** | **19(46)** | **14(33)** | **13(31)** | **46(37)** |
| **Lymph nodes** | **21(51)** | **17(40)** | **18(43)** | **56(45)** |
| **Abdomen** | **3(7)** | **4(10)** | **8(19)** | **15(12)** |
| **Other** | **2(5)** | **1(2)** | **0(0)** | **3(2)** |

**Lesion sizes at baseline are described in Table 17:**

| | **Conatumumab 10 mg/kg (Q2W) + gemcitabine** | **Ganitumab 12 mg/kg (Q2W) + gemcitabine** | **Placebo + gemcitabine** |
|---|---|---|---|
| | **(N = 41)** | **(N = 42)** | **(N = 42)** |
| **Sum of the longest diameters (SLD) of target lesions at baseline (mm)** | | | |
| **n** | **38** | **39** | **40** |
| **Mean** | **98.83** | **85.26** | **108.14** |
| **SD** | **61.87** | **45.71** | **69.81** |
| **Median** | **73.00** | **77.00** | **97.00** |
| **Q1, Q3** | **59.00, 122.00** | **51.20, 97.00** | **58.00, 144.50** |
| **Min, Max** | **30.0, 284.0** | **21.0, 218.0** | **24.0, 395.0** |
| **Number of patients with at least one lesion > 5 cm, n(%)** | **13 (32)** | **12 (29)** | **17 (40)** |
| **Number of patients with liver metastases - n (%)** | **27 (66)** | **29 (69)** | **33 (79)** |

**Efficacy was determined as overall survival (OS) and progression-free survival (PFS). The OS and PFS results suggested improvements in both metrics that were robust after adjusting for baseline covariates (age, gender, ECOG PS status, liver metastases, and tumor sum of longest diameter). Results are summarized in Table 18:**

| | Conatumumab + Gemcitabine | Ganitumab + Gemcitabline | Placebo + Gemcitabine |
|---|---|---|---|
| 6-Month OS Rate (95% CI)^{a} | 59% (42, 73) | 57% (41, 70) | 50% (33, 64) |
| 12-Month OS Rate (95% CI) | 20% (9, 34) | 39% (25, 54) | 23% (12, 38) |
| Median PFS, Months (95% CI) | 4.0 (3.3, 5.0) | 5.1 (2.8, 5.8) | 2.1 (1.9, 3.3) |
| Stratified HR (95% CI) | 0.65 (0.41, 1.05) | 0.65 (0.41, 1.04) | |
| P-value | 0.08 | 0.07 | |
| Median OS, Months (95% CI) | 7.5 (4.8, 10.0) | 8.7 (5.3, 12.2) | 5.9 (4.1, 9.7) |
| Stratified HR (95% CI) | 0.87 (0.53, 1.43) | 0.67 (0.41, 1.12) | |
| P-value | 0.59 | 0.12 | |

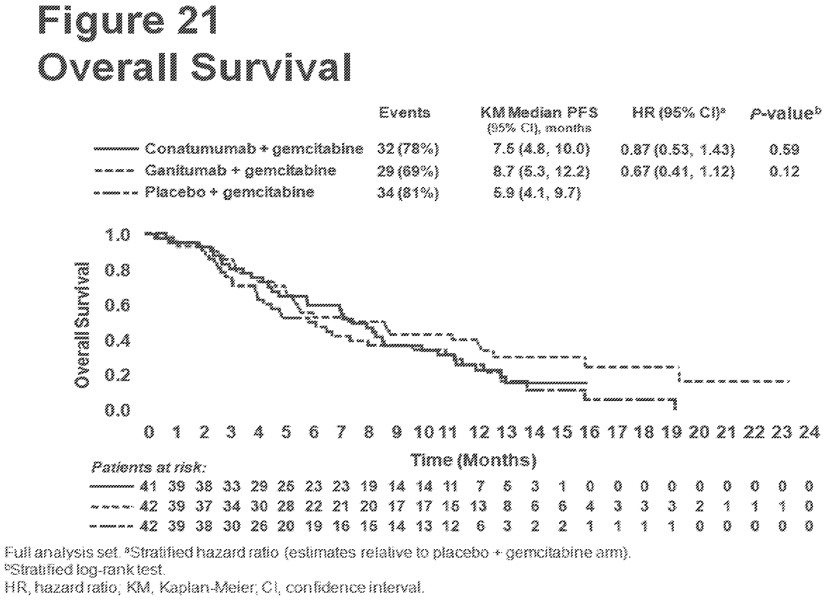

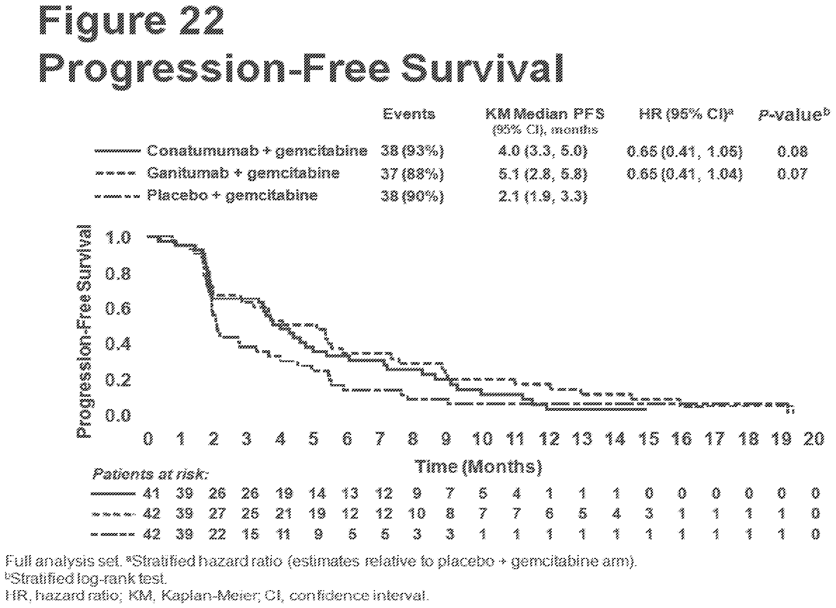

Overall survival data are presented graphically in Figure 21. Progression free survival data are presented graphically in Figure 22.

**The best overall tumor response data are presented in Table 19:**

| | Conatumumab + Gemcitabine | Ganitumab + Gemcitabine | Placebo + Gemcitabine | All Patients |
|---|---|---|---|---|
| | (N = 41) | (N = 42) | (N = 42) | (N = 125) |
| Number of Patients with Measurable Disease at Baseline | 38 | 39 | 40 | 117 |
| Best Overall Response Assessment | - n(%) | | | |
| Confirmed CR | 0 (0) | 0 (0) | 0 (0) | 0 (0) |
| Confirmed PR | 1 (3) | 4 (10) | 1 (3) | 6 (5) |
| Unconfirmed | 2 (5) | 0 (0) | 3 (8) | 5 (4) |
| PR SD (Includes Unconfirmed PR) | 22 (58) | 16 (41) | 15 (38) | 53 (45) |
| PD | 12 (32) | 13 (33) | 18 (45) | 43 (37) |
| Unknown | 3 (8) | 6 (15) | 6 (15) | 15 (13) |

**Grade 3-5 adverse events occurring in at least 5% of patients are presented in Table 20:**

| | Conatumumab + Gemcitabine | Ganitumab + Gemcitabine | Placebo + Gemcitabine | All Pts |
|---|---|---|---|---|
| n(%) | (N = 41) | (N = 40) | (N = 40) | (N = 121) |
| Number of Patients | | | | |
| Reporting ≥ 1 AE | 36 (88) | 34 (85) | 27 (68) | 97 (80) |

| Hematologic | | | | |
|---|---|---|---|---|
| Neutropenia | 9 (22) | 7 (18) | 5 (13) | 21 (17) |
| Thrombocytopenia | 7 (17) | 6 (15) | 3 (8) | 16 (13) |
| Anemia | 4 (10) | 4 (10) | 1 (3) | 9 (7) |

| Non-Hematologic | | | | |
|---|---|---|---|---|
| Abdominal pain | 7 (17) | 3 (8) | 5 (13) | 15 (12) |
| Fatigue | 5 (12) | 5 (13) | 2 (5) | 12 (10) |
| Alanine Aminotransferase Increased | 2 (5) | 6 (15) | 3 (8) | 11 (9) |
| Aspartate Aminotransferase Increased | 3 (7) | 4 (10) | 2 (5) | 9 (7) |
| Hyperglycemia | 1 (2) | 7 (18) | 1 (3) | 9 (7) |
| Deep Vein Thrombosis | 4 (10) | 2 (5) | 1 (3) | 7 (6) |
| Hyperbilirubinemia | 2 (5) | 3 (8) | 2 (5) | 7 (6) |
| Hypotension | 3 (7) | 3 (8) | 0 (0) | 6 (5) |
| Pulmonary Embolism | 1 (2) | 2 (5) | 3 (8) | 6 (5) |
| Grade 5 AE | 7 (17)^{a} | 4 (10)^{b} | 1 (3)^{c} | 12 (10) |

| | | | | |
|---|---|---|---|---|
| ^{a} One GI perforation, one GI hemorrhage, one hepatic failure in a patient with liver metastases, 4 PD; One acute renal failure, one hemorrhage, two PD; ^{c}One GI hemorrhage | | | | |

**Drug exposure data are presented in Table 21:**

| Median (Range) | | Conatumumab + Gemcitabine (N = 41) | Ganitumab + Gemcitabine (N = 40) | Placebo + Gemcitabine (N = 40) |
|---|---|---|---|---|
| Number of Cycles | | 4 (1 to 17) | 4 (1 to 17) | 2 (1 to 11) |
| Number of Infusions | | | | |
| | of IP Per Patient | 7 (1 to 31) | 6 (1 to 32) | 4 (1 to 21) |
| Number of Infusions | | | | |
| | of Gemcitabine | | | |
| | Per Patient | 10 (1 to 39) | 9 (2 to 47) | 6 (2 to 31) |
| Average Dose | | | | |
| | of IP Delivered, | | | |
| | mg/kg/infusion | 10.1 (8.2 to 10.7) | 12.1 (9.2 to 12.7) | 0 |
| Relative Dose Intensity | | | | |
| | of Gemcitabine^{a} | 0.70 (0.3 to 1.0) | 0.80 (0.4 to 1.0) | 0.78 (0.1 to 1.0) |
| Time on IP, Months | | 3.38 (0.3 to 14.8) | 3.56 (0.3 to 15.4) | 1.94 (0.5, 10.6) |

| | | | | |
|---|---|---|---|---|
| ^{a}Ratio of cumulative dose of drug : protocol specified cumulative dose over the specified period. IP, investigational product (conatumumab or ganitumab) | | | | |

The results show evidence of activity for ganitumab with trends across several efficacy parameters in metastatic pancreatic cancer, including higher 6-month OS rate (the study's primary endpoint; 57% vs 50%), higher 12-month OS (39% vs 24%), longer PFS (5.1 vs. 2.1 months, HR 0.65), longer OS (8.7 vs. 5.9 months, HR 0.67), and higher rates of SD + PR (51% vs 41%). The study also shows evidence of activity for conatumumab, including higher 6-month OS rate (the study's primary endpoint; 59% vs. 50%), longer PFS (4.0 vs. 2.1 months, HR 0.65), and higher rates of SD + PR (61% vs 41%). Both drug combinations were well-tolerated.

### EXAMPLE 18: Exposure-Response Analysis for Ganitumab (AMG 479) in Combination with Gemcitabine to Treat Metastatic Pancreatic Cancer

Pooled data from 37 patients with metastatic pancreatic cancer (mPC; 35 from the Phase 2 trial described in Example 17 and two from a first-in-human study) and 62 patients with advanced non-pancreatic solid tumors were used to construct a population PK model for ganitumab.

**Table 22**

| | Ganitumab Dose | | | PK Sampling | No. of PK |
|---|---|---|---|---|---|
| Study | (No of Patients) | Tumor type | Study Drug(s) | Frequency Samples | |
| **Phase 1** | | | | | |
| Ganitumab monotherapy IV Q2W2 | 1 mg/kg (n=3) | Non-pancreatic, n=51 | Ganitumab alone | PK profiles (0-336 hour) for 1st and 3rd doses; sampled pre- and end of infusion in specified cycles | 914 |
| | 3 mg/kg (n=3) | Pancreatic, n=2 | | | |
| | 10 mg/kg (n=3) | (1 received 12 mg/kg; 1 received 20 mg/kg) | | | |
| | 12 mg/kg (n=21) | | | | |
| | 20 mg/kg (n=23) | | | | |
| Ganitumab + gemcitabine IV Q2W3 | 6 mg/kg (n=8) | Non-pancreatic, n=11 | Ganitumab + gemcitabine | PK profiles (0-336 hour) for 3rd dose; sampled pre- and end of infusion in specified cycles | 117 |
| | 12 mg/kg (n=3) | | | | |

| **Phase 2** | | | | | |
|---|---|---|---|---|---|
| Ganitumab + gemcitabine IV Q2W1 | 12 mg/kg (n=35)^{a} | Pancreatic, n=35 | Ganitumab + gemcitabine | PK profiles (0-336 hour) for 1st dose; sampled pre- and end of infusion in specified cycles | 196 |

| | | | | | |
|---|---|---|---|---|---|
| ^{a}35 out of 40 patients who received ganitumab in the Phase 2 study had ganitumab concentration data. | | | | | |

Eighty patients with mPC who received either ganitumab plus gemcitabine or placebo plus gemcitabine were included in the exposure-response analysis.

A population PK model was constructed with a two-compartment disposition structural model with non-linear mixed effects modeling (NONMEM).

Univariate analysis was performed to illustrate the effect of exposure of patients to ganitumab which was calculated as the integral of the exposure curve over time (AUCₛₛ). When patients receiving ganitumab were stratified based on exposure (into a high exposure group where AUCₛₛ was greater than or equal to the median value (19.2 mg•h/mL) and a low exposure group where AUCₛₛ was less than the median value), Kaplan-Meier plots for OS and PFS for the high and low exposure groups were used to evaluate the effect of exposure on OS and PFS using Cox proportional hazard models. Multivariate analysis was performed to evaluate the effect of exposure on OS and PFS adjusting for potential confounding factors using Cox proportional hazard models with a forward selection technique. All baseline factors were initial candidates in the forward selection model. Only the most statistically significant variables were added to the final model after the forward selection process.

Baseline factors for the multivariate analysis are listed in Table 23:

| **Demographics** |
|---|
| Height, Weight*, Gender, Age, BSA |

| **Baseline Lab Values/Vital Signs** |
|---|
| Bilirubin, SGOT, SGPT, alkaline phosphatase, serum creatinine,* creatinine clearance, BUN, albumin,* fasting glucose, platelets, ANC, WBC, lymphocytes, systolic/diastolic BP |

| **Baseline Prognostic Factors** |
|---|
| ECOG PS, Liver metastasis, Years of diagnosis, Baseline tumor size, CA 19-9 |

| |
|---|
| *Covariate affecting PK Abbreviations: BSA, body surface area; SGOT, serum glutamic-oxaloacetic transaminase; SGPT, serum glutamic pyruvic transaminase; BUN, blood urea nitrogen; ANC, absolute neutrophil count; WBC, white blood cell; ECOG PS, Eastern Cooperative Oncology Group Performance Status. |

The effect of exposure on the incidence of selected adverse events (hearing and vestibular disorders, hepatic disorders, hyperglycemia, infusion reaction, neutropenia, rash and skin-related toxicity, thrombocytopenia, and venous embolic and thrombotic events) was summarized with descriptive statistics.

The effect of exposure on changes in selected laboratory values (aspartate transaminase, alanine transaminase, fasting glucose, total neutrophils, and platelets) was assessed by linear regression.

The AUCₛₛ distribution and PFS profiles for 12 and 20 mg/kg ganitumab Q2W were evaluated with Monte Carlo simulations.

Ganitumab PK parameters from the final model are shown in Table 24:

| Population PK | | | Inter- |
|---|---|---|---|
| Individual | | | |
| Parameters (%) | Tumor Type | Typical Values | variability |
| CL (L/h) | Pancreatic | 0.0481•(WT/74.55)^{0.984}•(ALB/38)^{-0.859}•(CR/0.8)^{-0.394} | 26.19% |
| | Non-Pancreatic | 0.0283•(WT/74.55)^{0.984}•(ALB/38)^{-0.859}•(CR/0.8)^{-0.394} | |
| Vc (L) | Pancreatic | 5.13•(WT/74.55)^{0.559} | 21.66% |
| | Non-Pancreatic | 3.85•(WT/74.55)^{0.559} | |
| Q (L/h) | | 0.0261 | 43.70% |
| Vp (L) | | 3.41 | 60.25% |

| **Error Model** | | | |
|---|---|---|---|
| Proportional Error (%) | | 16.43% | NA |

| | | | |
|---|---|---|---|
| ALB, albumin (g/L); CL, clearance; CR, serum creatinine (mg/dL); NA, not applicable; Q, inter-compartmental clearance; Vc, central volume of distribution; Vp, peripheral volume of distribution; WT, weight (kg). | | | |

Faster clearance (CL) and larger central volume of distribution (Vc) were observed in patients with mPC resulting in lower ganitumab exposure. Tumor type (pancreatic versus non-pancreatic) and baseline body weight, albumin, and serum creatinine were identified as statistically significant covariates of ganitumab PK parameters. Gemcitabine coadministration was not a significant covariate of ganitumab PK parameters.

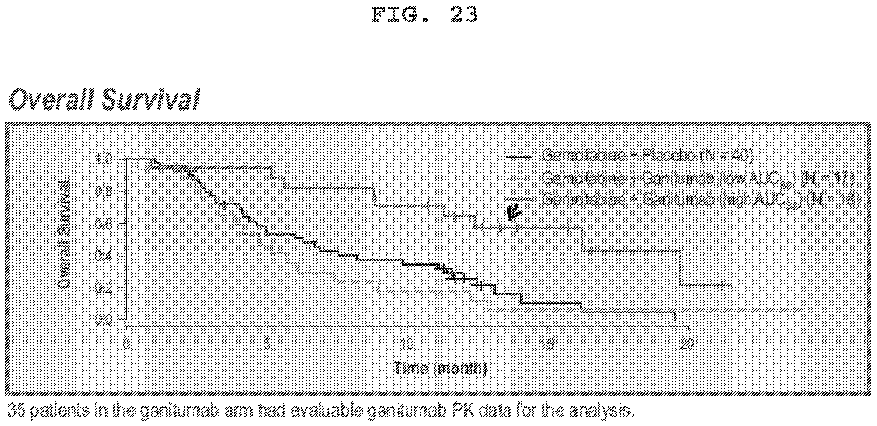

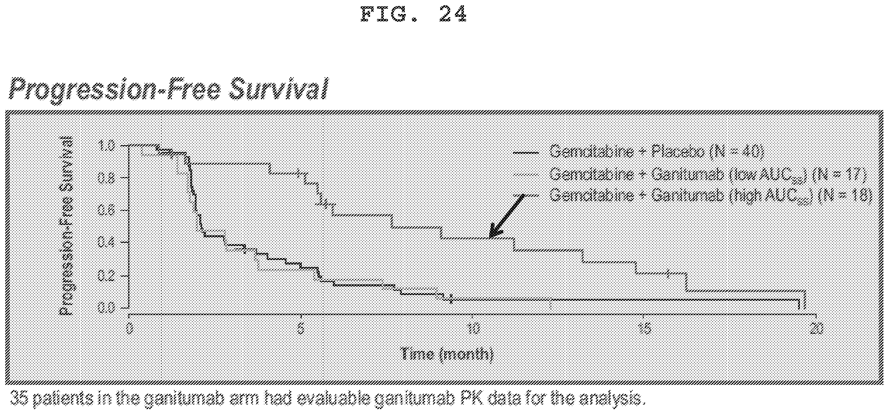

**The effect of ganitumab exposure on overall survival and progression free survival is shown in Figure 23, Figure 24 and in Table 25:**

| Clinical | Exposure Ganitumab Only | | Ganitumab and Placebo | |
|---|---|---|---|---|
| Endpoints Parameter | *HR (95% CI) | P-value | HR (95% CI) | P-value |
| PFS | logAUCₛₛ | 0.0611 (0.0160, 0.2327) | <0001 0.8322 (0.7111, 0.9741) | 0.0222 |
| OS | logAUCₛₛ | 0.0421 (0.0089, 0.1983) | <.0001 0.8288 (0.6978, 0.9845) | 0.0325 |

| | | | | |
|---|---|---|---|---|
| *HR = Cox proportional hazard ratio for a survival event (PFS or OS) for one unit increment in AUCₛₛ (natural log scale). Results from the ganitumab arm and the placebo (logAUCₛₛ = 0) plus ganitumab arms are presented. | | | | |

The association between exposure and OS and PFS was significant whether or not data from the placebo arm (assigned an AUCss of zero) were included. Imbalances in baseline prognostic factors between the high and low exposure groups may confound the exposure-PFS and exposure-OS relationships. Therefore, multivariate analyses and sensitivity analyses were performed to confirm these relationships.

**The effect of baseline factors on progression free survival and overall survival is shown in Table 26:**

| | | Parameter | | Chi- | | | |
|---|---|---|---|---|---|---|---|
| Variable | DF | Estimate | SE | ·Square | p-value | HR | 95% CI |
| **Effect on Progression Free Survival** | | | | | | | |
| Log AUCss | 1 | -1.9986 | 0.7762 | 6.6302 | 0.0100 | 0.1355 | 0.0296, 0.6204 |
| Baseline ALP | 1 | 0.0035 | 0.0018 | 3.9088 | 0.0480 | 1.0035 | 1.0000, 1.0071 |
| Baseline CrCl | 1 | 0.0130 | 0.0051 | 6.5334 | 0.0106 | 1.0130 | 1.0030, 1.0232 |

| **Effect on Overall Survival** | | | | | | | |
|---|---|---|---|---|---|---|---|
| Log AUCₛₛ | 1 | -2.4742 | 0.8840 | 7.8335 | 0.0051 | 0.0842 | 0.0149, 0.4764 |
| Baseline ALP | 1 | 0.0064 | 0.0019 | 11.8663 | 0.0006 | 1.0064 | 1.0028, 1.0101 |

| | | | | | | | |
|---|---|---|---|---|---|---|---|
| Data are from the ganitumab-treated arm. ALP, alkaline phosphatase; C, confidence interval; CrCl, creatine clearance; AUCss, steady state AUC; HR, hazard ratio; SE, standard error. DF, degree of freedom. | | | | | | | |

Higher exposure and lower baseline ALP were associated with longer OS and PFS; lower baseline creatine clearance (CrCl) was associated with longer PFS. The effect of exposure on efficacy remained after adjusting for potential baseline factors.

In general, the incidence of AEs was not dependent on exposure to ganitumab. The incidences of grade 3 / 4 hyperglycemia and thrombocytopenia were higher in the high-exposure group; the incidence of grade 3 / 4 hepatic disorders was higher in the low exposure group. Grade 5 AEs included three patients in the low-exposure group (2 disease progression, 1 hemorrhage), one in the high-exposure group (acute renal failure; patient had prior history of IV contrast-induced renal insufficiency), and one in the placebo arm (gastrointestinal hemorrhage). There was no strong evidence of an association between exposure and selected laboratory values.

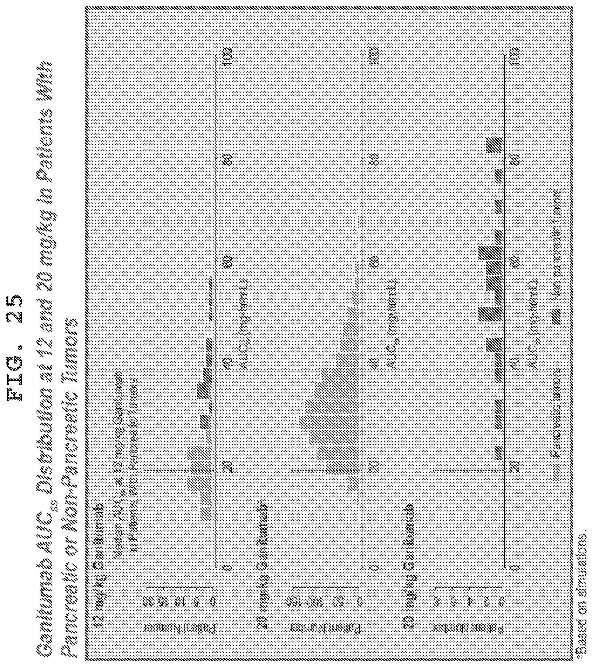

Ganitumab AUCₛₛ distributions at 12 and 20 mg/kg in patients with pancreatic or non-pancreatic tumors (observed and predicted by simulation) are shown in Figure 25.

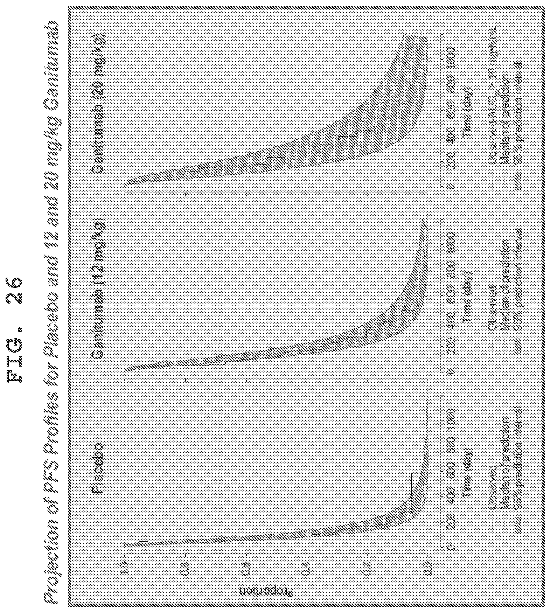

Projections of PFS profiles for placebo and 12 and 20 mg/kg ganitumab are shown in Figure 26. The projected median PFS times in the placebo and 12 and 20 mg/kg ganitumab dose groups were 2.6, 4.7, and 6.7 months, respectively, indicating that a higher dose of ganitumab may further improve PFS.

In summary, ganitumab exposure among patients with mPC was -40% lower than that observed among patients with other tumor types. An effect of gemcitabine on ganitumab PK was not evident. PFS and OS were longer among patients in the high exposure group. The relationship between exposure and survival remained after adjusting for baseline prognostic factors. There was no strong association between exposure and the incidence of most adverse events.

### EXAMPLE 19: Identification of Biomarkers

To ascertain whether bioavailable ligand concentrations predict response to ganitumab treatment, the relationship between baseline levels of IGF-1 (free and total), IGF-1 (total), and the major binding proteins (IGFBP-1, -2, -3, -4) and overall survival was carried out for the clinical study described in Example 17.

Descriptive statistics on the baseline levels of circulating biomarkers (free IGF-1, total IGF-1, IGF-II and IGFBP1-4) are summarized in Table 27:

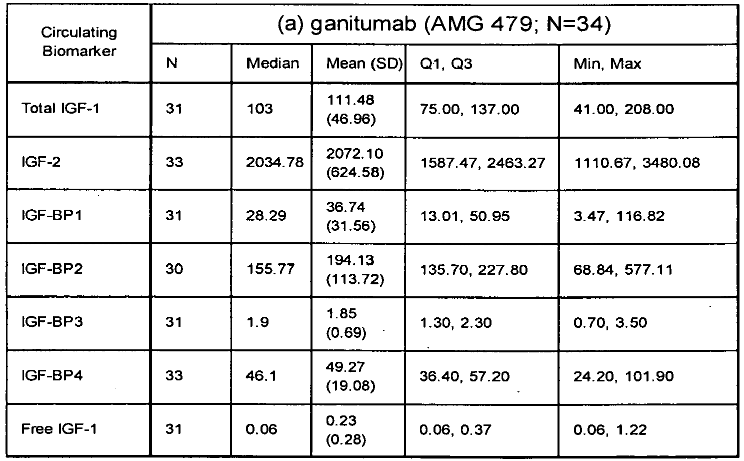

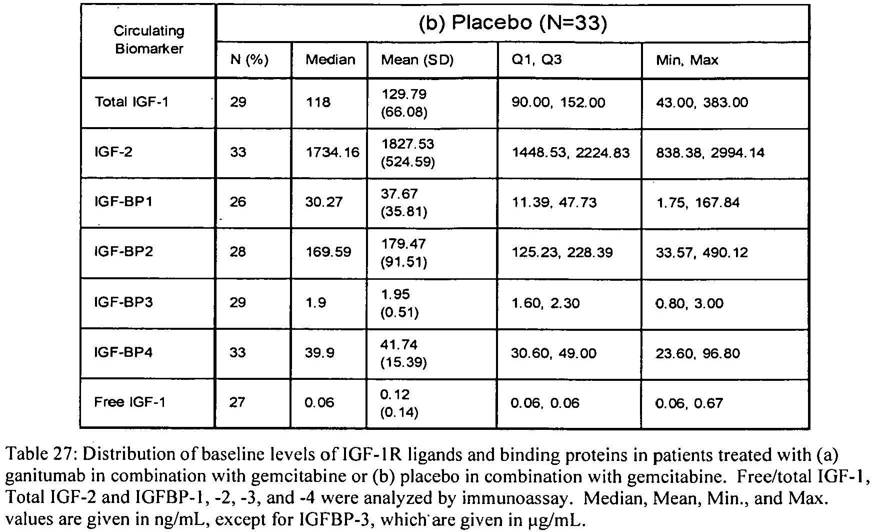

To establish the relationship between levels of these baseline markers and overall survival, a Cox proportional hazard model was used to calculate hazard ratios (HR) using normalized log transformed marker concentrations. This analysis tests the risk of death associated with a unit increase in the normalized log baseline biomarker and this risk is expressed as a hazard ratio. Because of the small sample size, it is difficult to dissect prognostic associations from relationships that are modified by treatment with ganitumab relative to placebo, i.e., predictive. This is because the addition of treatment effect adds a variable to the analysis that compromises the power of the test masking significant predictive relationships that may exist. However even a prognostic relationship identified in this analysis is important because it informs the hypothesized relationship between circulating bioavailable ligands and the adaptation of the tumor to IGF-1R dependent progression, and in turn sensitivity to inhibitors that block IGF-1 R or downstream signaling pathways.

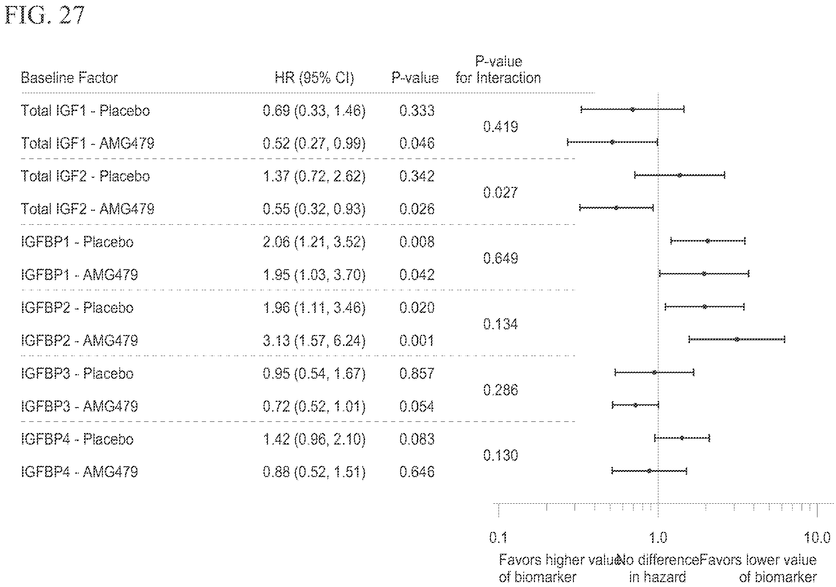

Cox model data are tabulated and represented graphically as a Forest plot in Figure 27. With the caveat described above, the hazard ratio (HR), with its corresponding 95% CI and P-value, was estimated independently for the ganitumab and placebo arms and the significance modification of biomarker effect with treatment status was tested using both ganitumab and placebo arms. The significance is expressed as a P-value for interaction. Although this interaction analysis may be overly stringent, any marker that is associated with a significant P-value for interaction is likely to be highly predictive of ganitumab response.

As shown in Figure 27, in the ganitumab (AMG 479) arm significant relationships between overall survival and baseline levels of total IGF-1, IGF-2, IGFBP-1, IGFBP-2 and IGFBP-3 were observed suggesting that circulating components of the IGF system are associated with tumor progression in pancreatic cancer. This relationship can be either prognostic or predictive of ganitumab treatment. To test the latter, these relationships were tested in the placebo treatment arm. In this case, significant relationships were observed for IGFBP-1 and IGFBP-2. The significance of the difference between arms, i.e., the specificity for ganitumab treatment (or predictive value) was calculated by testing the interaction with treatment status. Using the conventional threshold p-value of 0.05, the treatment effect is significant for IGF-2 suggesting that baseline levels of this marker are predictive of response to ganitumab. However, given the small sample size, a less stringent p-value threshold can be used. If a more reasonable p-value cut-off 0.15 is used, IGFBP-2 and IGFBP-4 each reaches significance as well. Without being bound to any particular theory, the association of these markers with OS in pancreatic cancer supports the hypothesis that pancreatic tumors are adapted to using IGF-1R for growth and survival and that circulating levels of IGF-1R ligand and the binding proteins that regulate bio-availability of these ligands are important determinants of tumor progression and survival. The interaction term between baseline biomarker and treatment is significant for some, but not all, of these markers; however, the small sample size of the study and minor differences of markers, which may have a prognostic relationship as well as a predictive relationship, between arms means that this test may miss predictive relationships.

To further test this hypothesis, median values of each of the markers from the overall population (i.e., both treatment and placebo arms) were used as a threshold and the predictive value for OS between the resulting high and low biomarker defined subgroups was established by comparing each sub-group in the ganitumab arm with the corresponding group from the placebo arm. This allows a direct test of the predictive value of using these markers in a dichotomized way to predict OS in pancreatic cancer patients that are treated with inhibitors of IGF-1R.

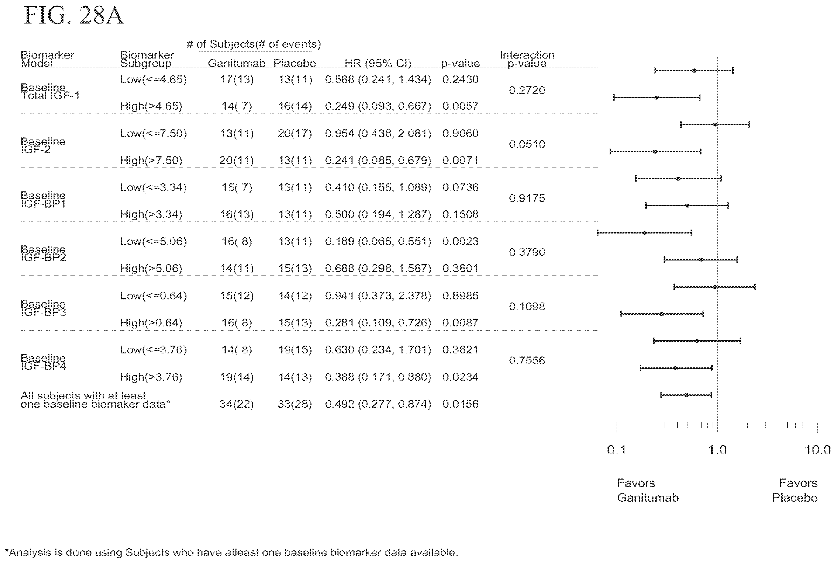

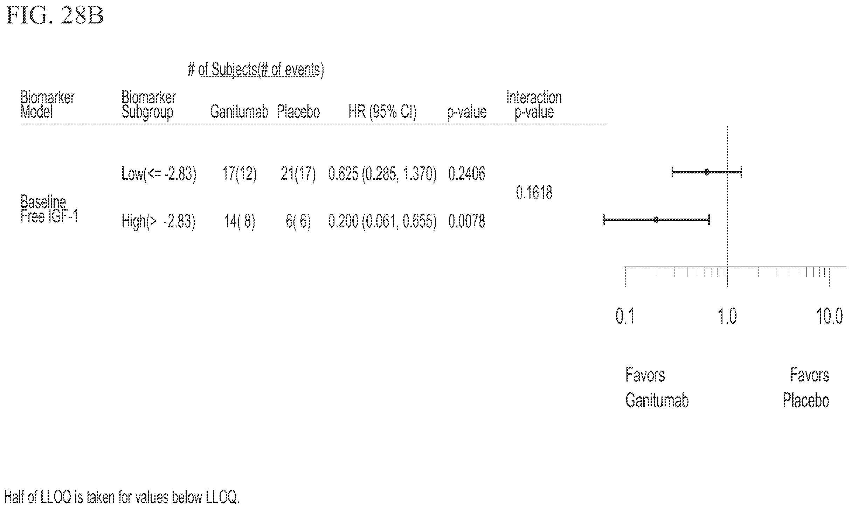

The biomarker defined sub-group analysis is shown in Table 28 and Figure 28 with the HR and associated P-values for comparisons of ganitumab treatment effect on OS in the biomarker defined low and high sub-groups. Low and significant HR for ganitumab versus placebo was observed in low sub-groups, but not in high sub-groups, for baseline IGFBP-2, suggesting that levels below median may be predictive for response to ganitumab. In contrast, low and significant HR were observed in high sub-groups, but not low sub-groups, for total IGF-1, IGF-2 and IGFBP-3, suggesting that levels above median may be predictive for response to ganitumab.

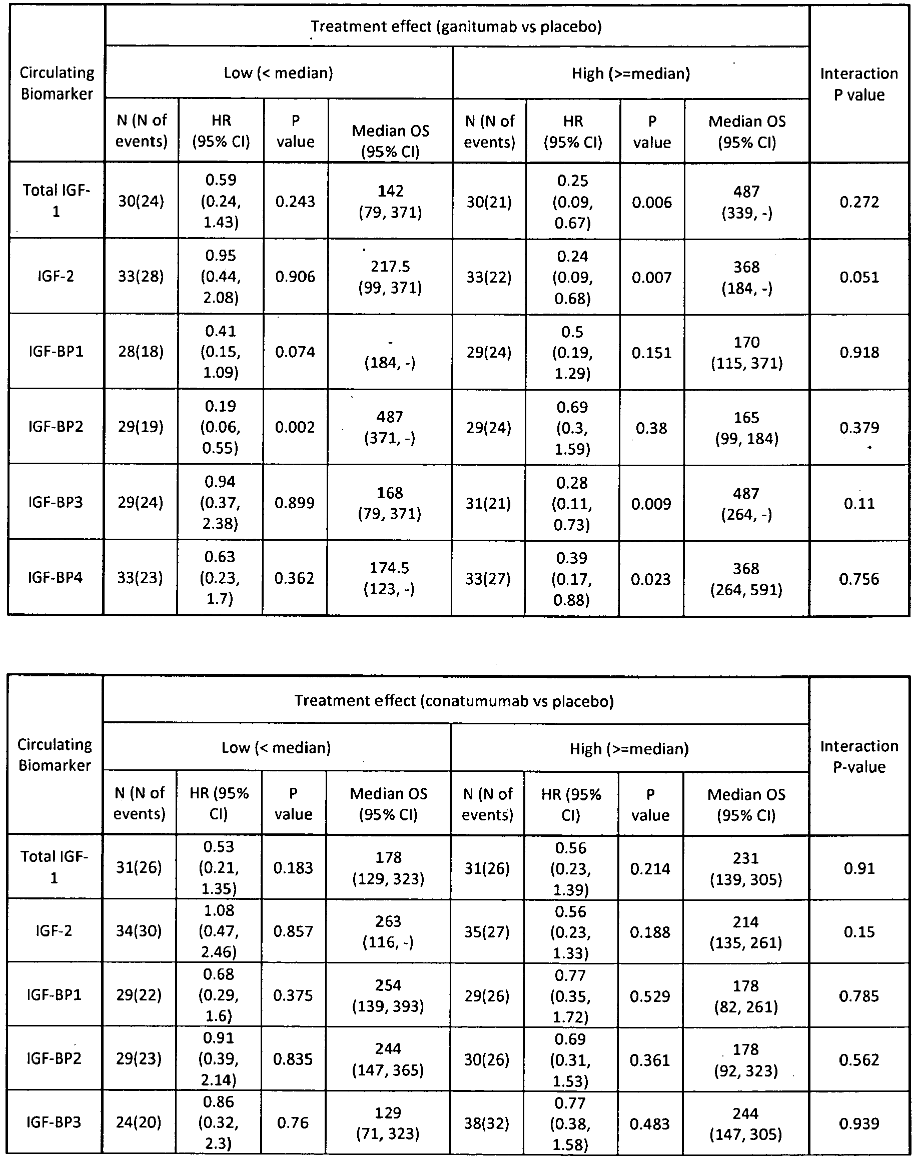

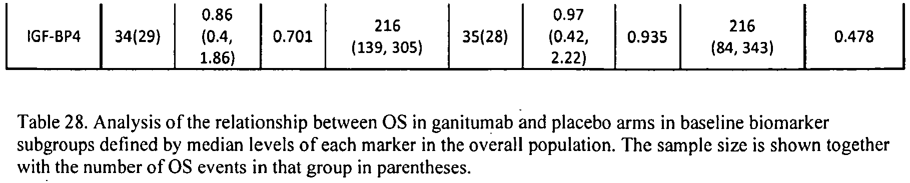

These relationships suggest that these markers can be used to predict OS in pancreatic cancer patients that are treated with an inhibitor of IGF-1 R. These data also suggest that markers that predict response to drugs that inhibit IGF-1 R will also be predictive of OS for drugs that act anywhere on the pathway upstream (i.e., agents that regulate the ligands or binding proteins) or downstream (i.e., agents that inhibit intracellular targets that are downstream of IGF-1R which would include, but are not limited to, constituent signaling proteins of the Ras/Raf and PI3K signaling pathways).

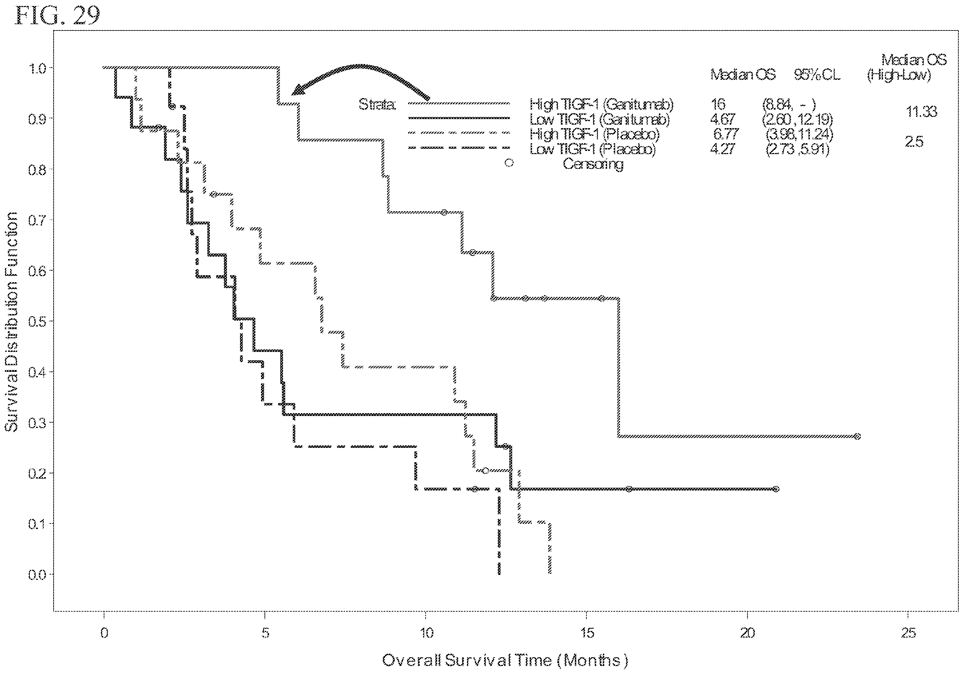

Having shown that predictive relationships exist between these circulating proteins and OS for an IGF-1R inhibitor, it was next demonstrated that these predictive relationships are clinically meaningful, i.e., that the differences in OS between high and low biomarker defined subgroups are substantial. To represent these relationships Kaplan-Maier plots were generated that show the OS for the high and low subgroups by plotting the fraction of patients in each sub-group that survive against time. These plots are shown in Figures 29 through 33.

In the case of total IGF-1 (Figure 29; Table 29), there is a clear separation between the curves for high and low sub-groups in the ganitumab arm, while the curves for high and low sub-groups are indistinguishable from each other in the placebo arm.

**Table 29**

| | | **Median KM OS Time, Months (95% CI)** | |
|---|---|---|---|
| **Baseline Biomarker** | **Biomarker Subgroup** | **Ganitumab + Gemcitabine** | **Placebo + Gemcitabine** |
| Total IGF-1 | Low | 4.67(2.60-12.19) | 4.27 (2.73-5.91) |
| | High | 16 (8.84-NE) | 6.77 (3.98-11.24) |
| Free IGF-1 | Low | 8.84 (3.25-16) | 6.57 (4.07, 11.24) |
| | High | 11.14 (5.42-NE) | 3.55 (1.15, 10.91) |
| IGF-2 | Low | 5.59 (2.60-12.19) | 4.86 (2.73-12.29) |
| | High | 16 (6.05-NE) | 5.91 (3.15-11.24) |
| IGFBP-1 | Low | NE (6.05-NE) | 7.43 (3.98-12.29) |
| | High | 5.59 (3.78-12.19) | 4.07 (2.73-4.93) |
| IGFBP-2 | Low | 12.65 (11.14-NE) | 6.57 (2.73-12.29) |
| | High | 5.52 (3.25-8.84) | 4.86 (2.89-6.77) |
| IGFBP-3 | Low | 5.52 (2.60-12.19) | 4.27 (2.60-7.43) |
| | High | 16 (8.67-NE) | 6.77 (3.98-11.24) |
| IGFBP-4 | Low | 8.59 (4.04-NE) | 6.77 (4.07-11.24) |
| | High | 12.09 (5.59-16.00) | 3.71 (2.30-11.50) |

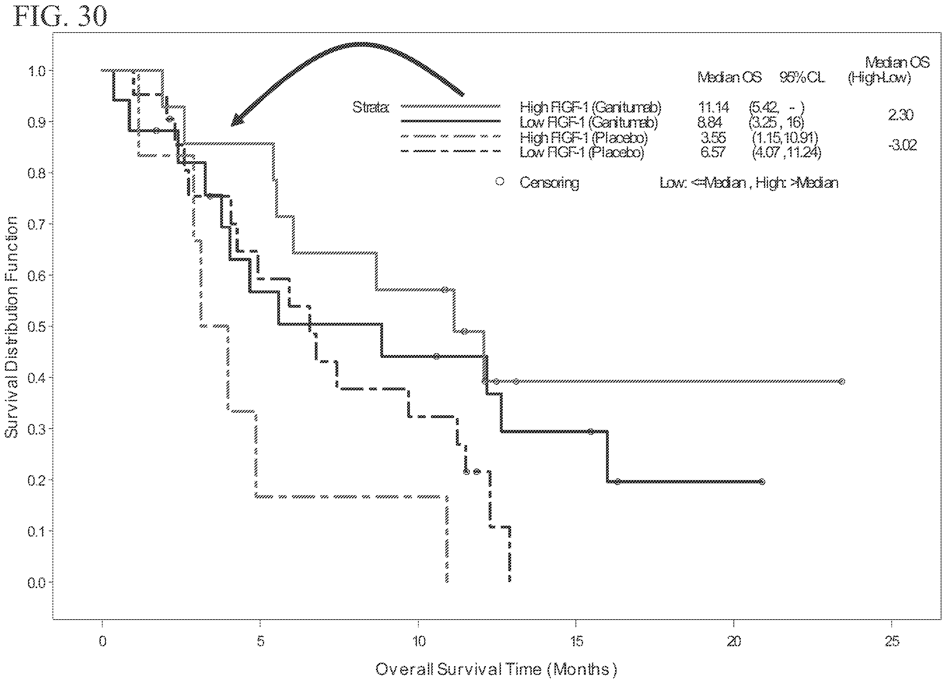

For free IGF-1 (Figure 30; Table 29), in the high subgroup the median OS in the ganitumab treatment arm was 11.14 months compared to 3.55 months in the placebo arm, a difference of 7.59 months.

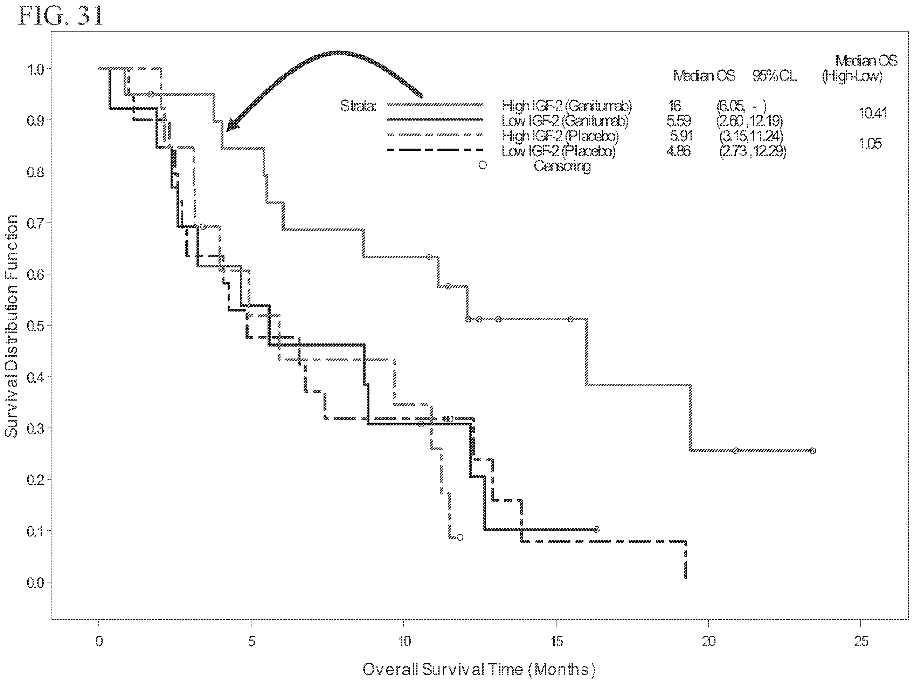

For IGF-2 (Figure 31; Table 29), again the difference between high and low subgroup OS profiles is apparent in ganitumab arm, with the high subgroup showing improved OS in comparison to the low subgroup and both subgroups in the placebo arms.

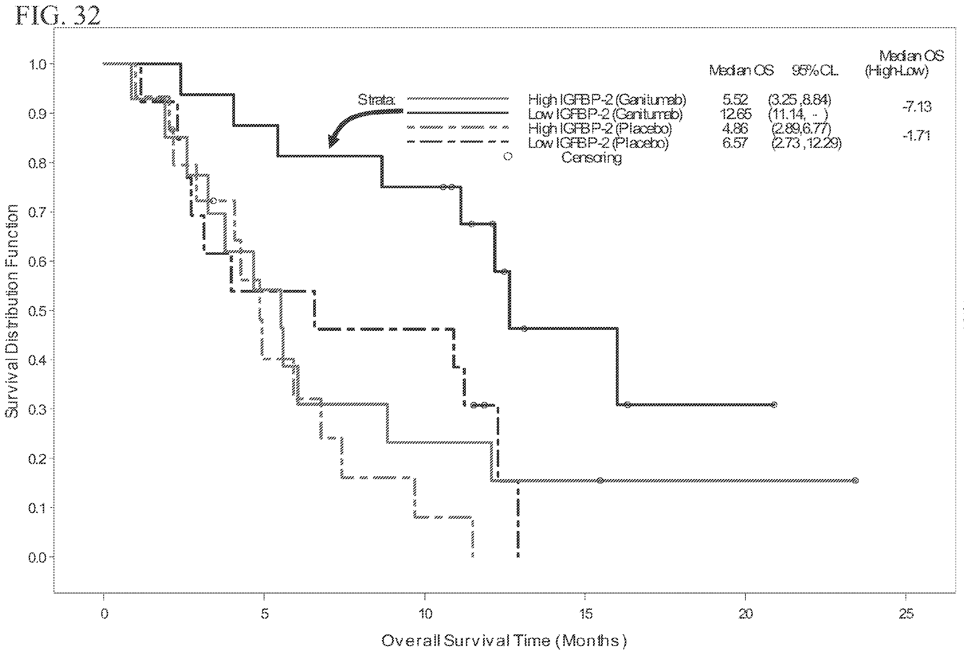

For IGFBP-2 (Figure 32; Table 29), again a difference between high and low subgroup OS profiles in the ganitumab arm is apparent, however in this case the low subgroup in the ganitumab arm shows improved OS in comparison to the high subgroup in the ganitumab arm and to both subgroups in the placebo arm.

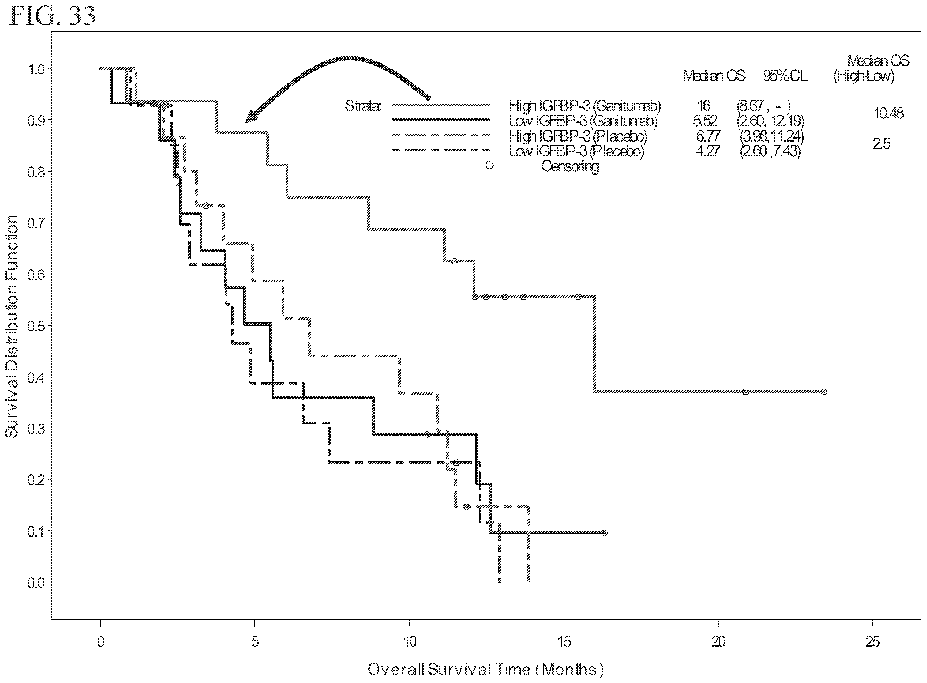

For IGFBP-3 (Figure 33; Table 29), again the difference between high and low subgroup OS profiles is apparent, with the high subgroup showing improved OS in comparison to the low subgroup and both subgroups in the placebo arms.

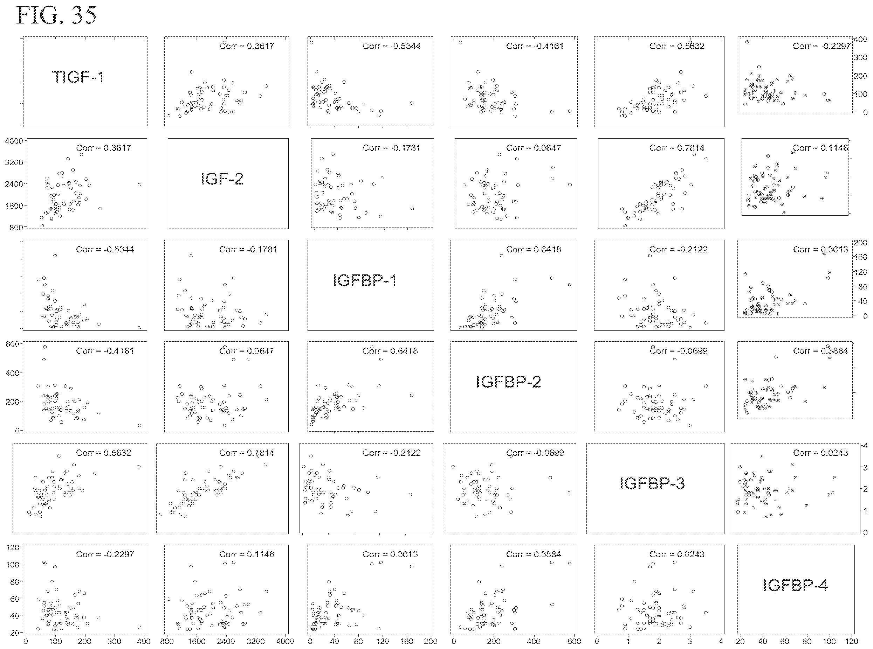

Analysis of the correlations between these markers (non-parametric Spearman correlation) reveals that IGF-1 and IGF-2 levels both significantly correlate with IGFBP-3 levels. No relationship was observed between IGFBP-2 and IGFBP-3 or IGFBP-2 and IGF-2 suggesting that these markers predict different patient populations and that the combination of these markers as a composite panel would be more predictive than any of these alone, as shown in Figure 35.

As shown in Figure 35, the correlations between many of the circulating biomarkers were not strong suggesting that they may operate as independent predictors of efficacy. This suggests that ratios of these markers are predictive. In order to compare the predictive potential for biomarker ratios with the individual biomarkers alone, sensitivity and specificity for prediction of nine month survival status were determined through analysis of receiver operator characteristic (ROC) with predictive values presented as the area under the ROC curve (AUC_{ROC}). The strongest effects were observed for the ratios IGF-2/IGFBP-2 and IGFBP-2/IGFBP-3. AUC_{ROC} was higher with the ratio IGF-2/IGFBP-2 (AUC_{ROC} =0.92) than with the individual factors (IGF-2: AUC_{ROC}= 0.727; IGFBP-2: AUC_{ROC}=0.772). Likewise, the ratio IGFBP-2/IGFBP-3 had a higher AUC_{ROC} (0.906) than either factor alone (IGFBP-2: AUC_{ROC} =0.772; IGFBP-3 AUC_{ROC} =0.715).

The effect of treatment (ganitumab versus placebo) on OS was analyzed in patients who had higher- or lower-than-median values of these ratios. The median ratio of IGF-2/IGFBP-2 was 11.1509. The median ratio of IGFBP-2/IGFBP-3 was 0.0834. Patients with either a higher-than-median IGF-2/IGFBP-2 ratio or a lower-than-median IGFBP-2/IGFBP-3 ratio were more likely to show a ganitumab-dependent increase in OS. Patients with a lower-than-median IGF-2/IGFBP-2 ratio and a higher-than-median IGFBP-2/IGFBP-3 ratio were less likely to show a ganitumab-dependent increase in OS. These observations are consistent with the subgroup analysis based on single biomarkers (i.e., patients with lower than median IGFBP-2, or higher than median IGF-2, or higher than median IGFBP-3, were more likely to show ganitumab-dependent increases in OS).

## Claims

1. A method for assessing the prognosis for a patient comprising determining the amount of a circulating biomarker, wherein said patient has pancreatic cancer and is being treated with ganitumab, wherein a higher concentration of said circulating biomarker indicates that said patient has a better prognosis of a ganitumab-dependent increase in overall survival, and wherein said circulating biomarker is total IGF-2 and wherein said patient's total IGF-2 concentration is higher if it is greater than 1734 ng/mL.

2. The method of claim 1, wherein said patient has a pancreatic cancer tumor.

3. The method of claim 2, wherein said pancreatic cancer tumor is a metastatic pancreatic cancer tumor or a locally advanced pancreatic cancer tumor.

4. The method of claim 1, further comprising determining the concentration of a second circulating biomarker selected from total IGF-1, IGFBP-2, IGFBP-3, the ratio of IGF-2/IGFBP-2, and the ratio of IGFBP-2/IGFBP-3.

5. The method of claim 4, further comprising determining the concentration of a third circulating biomarker selected from total IGF-1, IGFBP-2, IGFBP-3, the ratio of IGF-2/IGFBP-2, and the ratio of IGFBP-2/IGFBP-3.

6. The method of claim 5, further comprising determining the concentration of a fourth circulating biomarker selected from total IGF-1, IGFBP-2, IGFBP-3, the ratio of IGF-2/IGFBP-2, and the ratio of IGFBP-2/IGFBP-3.

7. The method of claim 4, wherein said second biomarker is the ratio of IGF-2/IGFBP-2.

8. The method of claim 4, wherein said second biomarker is the ratio of IGFBP-2/IGFBP-3.

## Patentansprüche

1. Verfahren zur Beurteilung der Prognose für einen Patienten, umfassend das Bestimmen der Menge eines zirkulierenden Biomarkers, wobei der Patient Bauchspeicheldrüsenkrebs hat und mit Ganitumab behandelt wird, wobei eine höhere Konzentration des zirkulierenden Biomarkers anzeigt, dass der Patient eine besser Prognose für einen Ganitumab-abhängigen Anstieg des Gesamtüberlebens hat, und wobei der zirkulierende Biomarker Gesamt-IGF-2 ist und wobei die Gesamt-IGF-2 Konzentration des Patienten höher ist, wenn sie mehr als 1.734 ng/mL beträgt.

2. Verfahren nach Anspruch 1, wobei der Patient einen Bauchspeicheldrüsenkrebs-Tumor hat.

3. Verfahren nach Anspruch 2, wobei der Bauchspeicheldrüsenkrebs-Tumor ein metastasierender Bauchspeicheldrüsenkrebs-Tumor oder ein lokal fortgeschrittener Bauchspeicheldrüsenkrebs-Tumor ist.

4. Verfahren nach Anspruch 1, ferner umfassend das Bestimmen der Konzentration eines zweiten zirkulierenden Biomarkers, der ausgewählt ist aus Gesamt-IGF-1, IGFBP-2, IGFBP-3, dem Verhältnis IGF-2/IGFBP-2 und dem Verhältnis IGFBP-2/IGFBP-3.

5. Verfahren nach Anspruch 4, ferner umfassend das Bestimmen der Konzentration eines dritten zirkulierenden Biomarkers, der ausgewählt ist aus Gesamt-IGF-1, IGFBP-2, IGFBP-3, dem Verhältnis IGF-2/IGFBP-2 und dem Verhältnis IGFBP-2/IGFBP-3.

6. Verfahren nach Anspruch 5, ferner umfassend das Bestimmen der Konzentration eines vierten zirkulierenden Biomarkers, der ausgewählt ist aus Gesamt-IGF-1, IGFBP-2, IGFBP-3, dem Verhältnis IGF-2/IGFBP-2 und dem Verhältnis IGFBP-2/IGFBP-3.

7. Verfahren nach Anspruch 4, wobei der zweite Biomarker das Verhältnis IGF-2/IGFBP-2 ist.

8. Verfahren nach Anspruch 4, der zweite Biomarker das Verhältnis IGFBP-2/IGFBP-3 ist.

## Revendications

1. Procédé d'évaluation du pronostic d'un patient comprenant la détermination de la quantité d'un biomarqueur circulant, dans lequel ledit patient souffre d'un cancer pancréatique et est traité avec du ganitumab, dans lequel une concentration plus élevée dudit biomarqueur circulant indique que ledit patient a un meilleur pronostic d'augmentation de la survie globale dépendant du ganitumab, et dans lequel ledit biomarqueur circulant est l'IGF-2 total et dans lequel ladite concentration en IGF-2 total du patient est plus élevée si elle est supérieure à 1.734 ng/ml.

2. Procédé selon la revendication 1, dans lequel ledit patient a une tumeur cancéreuse du pancréas.

3. Procédé selon la revendication 2, dans lequel ladite tumeur cancéreuse du pancréas est une tumeur cancéreuse du pancréas métastatique ou une tumeur cancéreuse du pancréas localement avancée.

4. Procédé selon la revendication 1, comprenant en outre la détermination de la concentration en un deuxième biomarqueur circulant sélectionné parmi l'IGF-1 total, l'IGFBP-2, l'IGFBP-3, le rapport IGF-2/IGFBP-2 et le rapport IGFBP-2/IGFBP-3.

5. Procédé selon la revendication 4, comprenant en outre la détermination de la concentration en un troisième biomarqueur circulant sélectionné parmi l'IGF-1 total, l'IGFBP-2, l'IGFBP-3, le rapport IGF-2/IGFBP-2 et le rapport IGFBP-2/IGFBP-3.

6. Procédé selon la revendication 5, comprenant en outre la détermination de la concentration en un quatrième biomarqueur circulant sélectionné parmi l'IGF-1 total, l'IGFBP-2, l'IGFBP-3, le rapport IGF-2/IGFBP-2 et le rapport IGFBP-2/IGFBP-3.

7. Procédé selon la revendication 4, dans lequel ledit deuxième biomarqueur est le rapport IGF-2/IGFBP-2.

8. Procédé selon la revendication 4, dans lequel ledit deuxième biomarqueur est le rapport IGFBP-2/IGFBP-3.
